# EUROPEAN PATENT APPLICATION

(11) **EP 2 749 570 A2**
(43) Date of publication of application: **02.07.2014**
(21) Application number: 13189780.3
(22) Date of filing: 02.09.2011
(51) Int. Cl.: C07K 16/12

(54) **Staphylococcus aureus specific human recombinant polyclonal antibodies and uses thereof**

(30) Priority: 02.09.2010 US 379580 P; 08.09.2010 US 380934 P
(62) Divisional of application: 11785505.6
(71) Applicant: Excelimmune, Inc., Woburn, MA 01801 (US)
(72) Inventor: Coljee, Vincent, Watertown, MA 02472 (US)
(74) Representative: EIP

(57) **Abstract**

Human antibodies that bind to one or more strains *Staphylococcus aureus* strains, including methicillin resistant strains, are disclosed. Also disclosed are human recombinant polyclonal antibody compositions, and therapeutic methods for using the antibodies.

SEQUENCE LISTING

<110> EXCELIMMUNE, INC.

<120> STAPHYLOCOCCUS AUREUS SPECIFIC HUMAN RECOMBINANT POLYCLONAL
      ANTIBODIES AND USES THEREOF

<130> EXL-001PC

<140> PCT/US2011/050422
<141> 2011-09-02

<150> 61/380,934
<151> 2010-09-08

<150> 61/379,580
<151> 2010-09-02

<160> 381

<170> PatentIn version 3.5

<210> 1
<211> 343
<212> DNA
<213> Homo sapiens

<400> 1
gaggtgcagc tggtggagtc tgggggaggc ttggtaaagc ctggggggtc ccttagactc      60

tcctgtgcag cctctggatt cactttcagt aacgcctgga tgacctgggt ccgccaggct     120

ccagggaagg ggctggagtg ggttggccgt attaagagca aaactgatgg tgggacaaca     180

gactacgctg cacccgtgaa aggcagattc accatctcaa gagatgattc aataaacaca     240

ctgtatctgc aaatgaacag cctgaaaacc gaggacacag ccgtgtatta ttgtaggacg     300

gggggctact ggggccaggg aaccctggtc accgtctcga gtg                       343


<210> 2
<211> 114
<212> PRT
<213> Homo sapiens

<400> 2
Glu Val Gln Leu Val Glu Ser Gly Gly Gly Leu Val Lys Pro Gly Gly
1               5                   10                  15


Ser Leu Arg Leu Ser Cys Ala Ala Ser Gly Phe Thr Phe Ser Asn Ala
            20                  25                  30


Trp Met Thr Trp Val Arg Gln Ala Pro Gly Lys Gly Leu Glu Trp Val
        35                  40                  45


Gly Arg Ile Lys Ser Lys Thr Asp Gly Gly Thr Thr Asp Tyr Ala Ala
    50                  55                  60

Pro Val Lys Gly Arg Phe Thr Ile Ser Arg Asp Asp Ser Ile Asn Thr
65                  70                  75                  80

Leu Tyr Leu Gln Met Asn Ser Leu Lys Thr Glu Asp Thr Ala Val Tyr
                85                  90                  95

Tyr Cys Arg Thr Gly Gly Tyr Trp Gly Gln Gly Thr Leu Val Thr Val
            100                 105                 110

Ser Ser

<210> 3
<211> 328
<212> DNA
<213> Homo sapiens

<400> 3
caggctgtgg tgactcagga gccctcactg actgtgtccc caggagggac agtcactctc      60

acctgtggct ccagcactgg agctgtcacc agtggtcatt atccctactg gttccagcag     120

aagcctggcc aagcccccag gacactgatt tatgatacaa gcaagaaaca ttcctggaca     180

cctgcccggt tctcaggctc cctccttggg ggcaaagctg ccctgaccct ttcgggtgcg     240

cagcctgagg atgaggctga gtattactgc ttggtctccg atagtggtgc tcggatcttc     300

ggacctggga ccaaggtcac cgtcctag                                        328

<210> 4
<211> 109
<212> PRT
<213> Homo sapiens

<400> 4
Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

His Tyr Pro Tyr Trp Phe Gln Gln Lys Pro Gly Gln Ala Pro Arg Thr
        35                  40                  45

Leu Ile Tyr Asp Thr Ser Lys Lys His Ser Trp Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Leu Val Ser Asp Ser Gly
                85                  90                  95

Ala Arg Ile Phe Gly Pro Gly Thr Lys Val Thr Val Leu
            100                 105

<210> 5
<211> 8
<212> PRT
<213> Homo sapiens

<400> 5
Gly Phe Thr Phe Ser Asn Ala Trp
1               5

<210> 6
<211> 10
<212> PRT
<213> Homo sapiens

<400> 6
Ile Lys Ser Lys Thr Asp Gly Gly Thr Thr
1               5                   10

<210> 7
<211> 7
<212> PRT
<213> Homo sapiens

<400> 7
Cys Arg Thr Gly Gly Tyr Trp
1               5

<210> 8
<211> 9
<212> PRT
<213> Homo sapiens

<400> 8
Thr Gly Ala Val Thr Ser Gly His Tyr
1               5

<210> 9
<211> 11
<212> PRT
<213> Homo sapiens

<400> 9
Cys Leu Val Ser Asp Ser Gly Ala Arg Ile Phe
1               5                   10

<210> 10
<211> 364
<212> DNA
<213> Homo sapiens

<400> 10
caggttcagc tggtgcagtc tggacctgaa atgaagaagc ctggggcctc agtgaaggtc        60

tcctgtcagg cctctggtta cacctttacc attaatggaa ttagttgggt gcgacaggcc 120

cctggacaac ggcctgagtg gatggggtgg atcagcacta gtaatggtca gacaaacttt 180

gcacagaatt tccagggcag actcaccttg accacagaca catccacgac cacggccttc 240

atggagttga tgagcctcac atctgccgac acggccgtat actactgtgc gagaaatcca 300

tacggcaatt cgcgcagatt ctttgactac tggggccagg gcaccctggt caccgtctcg 360

agtg 364

<210> 11
<211> 121
<212> PRT
<213> Homo sapiens

<400> 11
Gln Val Gln Leu Val Gln Ser Gly Pro Glu Met Lys Lys Pro Gly Ala
1               5                   10                  15

Ser Val Lys Val Ser Cys Gln Ala Ser Gly Tyr Thr Phe Thr Ile Asn
            20                  25                  30

Gly Ile Ser Trp Val Arg Gln Ala Pro Gly Gln Arg Pro Glu Trp Met
        35                  40                  45

Gly Trp Ile Ser Thr Ser Asn Gly Gln Thr Asn Phe Ala Gln Asn Phe
    50                  55                  60

Gln Gly Arg Leu Thr Leu Thr Thr Asp Thr Ser Thr Thr Thr Ala Phe
65                  70                  75                  80

Met Glu Leu Met Ser Leu Thr Ser Ala Asp Thr Ala Val Tyr Tyr Cys
                85                  90                  95

Ala Arg Asn Pro Tyr Gly Asn Ser Arg Arg Phe Phe Asp Tyr Trp Gly
            100                 105                 110

Gln Gly Thr Leu Val Thr Val Ser Ser
        115                 120

<210> 12
<211> 322
<212> DNA
<213> Homo sapiens

<400> 12
gacatccaga tgacccagtc tccatcctcc ctgtctgcct ctgttggcga cagagtcatc 60

atcacttgcc ggacaagtca gaccatcacc aattacataa attggtatca gcaaaaacca 120

gggaaagccc cgcagctcct gatctatggt gtatccagtt tgcaaagtgg ggtcccatca 180

aggttcagtg gcggtggatc tgggacagaa ttcactctca ccatcagcag tctgcaacct     240

gaagactttg caacttatta ctgtcaacag agttataggg cccctcaaac attcggccaa     300

gggaccaagc tggaaatcaa ac                                              322

<210> 13
<211> 107
<212> PRT
<213> Homo sapiens

<400> 13
Asp Ile Gln Met Thr Gln Ser Pro Ser Ser Leu Ser Ala Ser Val Gly
1               5                   10                  15

Asp Arg Val Ile Ile Thr Cys Arg Thr Ser Gln Thr Ile Thr Asn Tyr
            20                  25                  30

Ile Asn Trp Tyr Gln Gln Lys Pro Gly Lys Ala Pro Gln Leu Leu Ile
        35                  40                  45

Tyr Gly Val Ser Ser Leu Gln Ser Gly Val Pro Ser Arg Phe Ser Gly
    50                  55                  60

Gly Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Ser Tyr Arg Ala Pro Gln
                85                  90                  95

Thr Phe Gly Gln Gly Thr Lys Leu Glu Ile Lys
            100                 105

<210> 14
<211> 8
<212> PRT
<213> Homo sapiens

<400> 14
Gly Tyr Thr Phe Thr Ile Asn Gly
1               5

<210> 15
<211> 8
<212> PRT
<213> Homo sapiens

<400> 15
Ile Ser Thr Ser Asn Gly Gln Thr
1               5

<210> 16

<211> 16
<212> PRT
<213> Homo sapiens

<400> 16
Cys Ala Arg Asn Pro Tyr Gly Asn Ser Arg Arg Phe Phe Asp Tyr Trp
1               5                   10                  15


<210> 17
<211> 6
<212> PRT
<213> Homo sapiens

<400> 17
Gln Thr Ile Thr Asn Tyr
1               5


<210> 18
<211> 11
<212> PRT
<213> Homo sapiens

<400> 18
Cys Gln Gln Ser Tyr Arg Ala Pro Gln Thr Phe
1               5                   10


<210> 19
<211> 352
<212> DNA
<213> Homo sapiens

<400> 19
gaggtgcagc tggtggagtc tgggggaggc ttggtgcagc cgggggggtc tctgagactc      60

tcttgtgcag cctctggttt cgccttcagc agccacgaga tgcactgggt tcgtcaaggg     120

ataggacaca gtttggagtg ggtctcagct attggccttg ctggtgacac atattattca     180

gactccgtga agggccgctt caccatctcc agagaaaacg ccaagaacac tttgtatctt     240

gaaatgaaca atttgagagt cggcgacacg gctgtctatt actgtgtcaa agaaggggct     300

cattgggaat ttgactcctg gggccaggga accctggtca ccgtctcgag tg             352


<210> 20
<211> 117
<212> PRT
<213> Homo sapiens

<400> 20
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ala Phe Ser Ser His
            20                  25                  30


Glu Met His Trp Val Arg Gln Gly Ile Gly His Ser Leu Glu Trp Val

        35                  40                  45

Ser Ala Ile Gly Leu Ala Gly Asp Thr Tyr Tyr Ser Asp Ser Val Lys
    50                  55                  60

Gly Arg Phe Thr Ile Ser Arg Glu Asn Ala Lys Asn Thr Leu Tyr Leu
65                  70                  75                  80

Glu Met Asn Asn Leu Arg Val Gly Asp Thr Ala Val Tyr Tyr Cys Val
                85                  90                  95

Lys Glu Gly Ala His Trp Glu Phe Asp Ser Trp Gly Gln Gly Thr Leu
            100                 105                 110

Val Thr Val Ser Ser
        115

<210> 21
<211> 328
<212> DNA
<213> Homo sapiens

<400> 21
cagtctgtgc tgacgcagcc gccctcagtg tctggggccc ctggacagag ggtcatcatc 60

tcctgcactg ggagcagctc caacctcggg gcaagtcttg atgtacactg gtaccagcgc 120

cttccaggga caggcccccg actcctcatc tatgctaaca acaatcggcc ctcaggggtc 180

cctgaccgat tctctggctc caagtctggc acctcagcct ccctggccat caccgggctc 240

caggctgaag atgagggtga ttattactgc cagtcctatg acggcagcct gagggtgttc 300

ggcggaggga ccaggctgac cgtcctag 328

<210> 22
<211> 109
<212> PRT
<213> Homo sapiens

<400> 22
Gln Ser Val Leu Thr Gln Pro Pro Ser Val Ser Gly Ala Pro Gly Gln
1               5                   10                  15

Arg Val Ile Ile Ser Cys Thr Gly Ser Ser Ser Asn Leu Gly Ala Ser
            20                  25                  30

Leu Asp Val His Trp Tyr Gln Arg Leu Pro Gly Thr Gly Pro Arg Leu
        35                  40                  45

Leu Ile Tyr Ala Asn Asn Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Gly Asp Tyr Tyr Cys Gln Ser Tyr Asp Gly Ser
                85                  90                  95

Leu Arg Val Phe Gly Gly Gly Thr Arg Leu Thr Val Leu
            100                 105

<210> 23
<211> 8
<212> PRT
<213> Homo sapiens

<400> 23
Gly Phe Ala Phe Ser Ser His Glu
1               5

<210> 24
<211> 7
<212> PRT
<213> Homo sapiens

<400> 24
Ile Gly Leu Ala Gly Asp Thr
1               5

<210> 25
<211> 13
<212> PRT
<213> Homo sapiens

<400> 25
Cys Val Lys Glu Gly Ala His Trp Glu Phe Asp Ser Trp
1               5                   10

<210> 26
<211> 9
<212> PRT
<213> Homo sapiens

<400> 26
Ser Ser Asn Leu Gly Ala Ser Leu Asp
1               5

<210> 27
<211> 11
<212> PRT
<213> Homo sapiens

<400> 27

<210> 28
<211> 379
<212> DNA
<213> Homo sapiens

<400> 28
caggtgcagc tggtggagtc tgggggaggc ttgttcaagc ctggagggtc cctgagactg 60

tcctgtgcag cctctggatt caccttcagt gactactaca tgagctggat ccggcaggct 120

ccagggaagg ggctggagtg gatttcatac attagtagta gtggtaggac cataaagtac 180

gcagactctg tgaagggccg attcaccatc tccagggaca acgccaagaa ctcactttat 240

ctgcaaatga acagcctgag agccgaggac acggccgtgt attattgtgc gagagatgga 300

ggatattgtg gaagtgcaaa ctgccttcat gacgcttttg atatctgggg ccaagggaca 360

atggtcaccg tctcgagtg 379


<210> 29
<211> 126
<212> PRT
<213> Homo sapiens

<400> 29
Gln Val Gln Leu Val Glu Ser Gly Gly Gly Leu Phe Lys Pro Gly Gly
1               5                   10                  15


Ser Leu Arg Leu Ser Cys Ala Ala Ser Gly Phe Thr Phe Ser Asp Tyr
            20                  25                  30


Tyr Met Ser Trp Ile Arg Gln Ala Pro Gly Lys Gly Leu Glu Trp Ile
        35                  40                  45


Ser Tyr Ile Ser Ser Ser Gly Arg Thr Ile Lys Tyr Ala Asp Ser Val
    50                  55                  60


Lys Gly Arg Phe Thr Ile Ser Arg Asp Asn Ala Lys Asn Ser Leu Tyr
65                  70                  75                  80


Leu Gln Met Asn Ser Leu Arg Ala Glu Asp Thr Ala Val Tyr Tyr Cys
                85                  90                  95


Ala Arg Asp Gly Gly Tyr Cys Gly Ser Ala Asn Cys Leu His Asp Ala
            100                 105                 110


Phe Asp Ile Trp Gly Gln Gly Thr Met Val Thr Val Ser Ser
        115                 120                 125


<210> 30
<211> 319
<212> DNA
<213> Homo sapiens

<400> 30
gacatccaga tgacccagtc tccttccacc ctgtctgcat ctgtaggaga cagagtcacc 60

atcacttgcc gggccagtca gagtattagt agctggttgg cctggtatca gcagaaacca 120

gggaaagccc ctaagctcct gatctataag gcgtctagtt tagaaagtgg ggtcccatca 180

aggttcagcg gcagtggatc tgggacagaa ttcactctca ccatcagcag cctgcagcct 240

gatgattttg caacttatta ctgccaacag tataatagtt attcgacgtt cggccaaggg 300

accaaggtgg aaatcaaac 319

<210> 31
<211> 106
<212> PRT
<213> Homo sapiens

<400> 31
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Ser Thr
                85                  90                  95

Phe Gly Gln Gly Thr Lys Val Glu Ile Lys
            100                 105

<210> 32
<211> 8
<212> PRT
<213> Homo sapiens

<400> 32
Gly Phe Thr Phe Ser Asp Tyr Tyr
1               5

<210> 33
<211> 11
<212> PRT
<213> Homo sapiens

<400> 33
Ile Ser Tyr Ile Ser Ser Ser Gly Arg Thr Ile
1               5                   10

<210> 34
<211> 21
<212> PRT
<213> Homo sapiens

<400> 34
Cys Ala Arg Asp Gly Gly Tyr Cys Gly Ser Ala Asn Cys Leu His Asp
1               5                   10                  15

Ala Phe Asp Ile Trp
            20

<210> 35
<211> 6
<212> PRT
<213> Homo sapiens

<400> 35
Gln Ser Ile Ser Ser Trp
1               5

<210> 36
<211> 10
<212> PRT
<213> Homo sapiens

<400> 36

<210> 37
<211> 385
<212> DNA
<213> Homo sapiens

<400> 37
caggtcacct tgaaggagtc tggtcccacg ctggtgaaac ccacacagac cctcacgctg      60

acgtgcacct tctctgggtt ttcgctcagc actagtggag agggtgtggc ctggatccgt     120

caggccccag gaaaggccct ggagtggctt gcactcattt attgggatga tgataagcgc     180

tacaacccat ctctgaagag caggctcacc atcaccaagg acaactccag aaaccaagtg     240

gtccttacaa tgaccaacat ggaccctgtg gacacagcca catattactg tgtacacagg     300

gggatgaata gggtttttgg ggtagtttat aattcgggct ggttcgaccc ctggggccag     360

ggaaccctgg tcaccgtctc gagtg                                           385

<210> 38
<211> 128

<212> PRT
<213> Homo sapiens

<400> 38
Gln Val Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Glu Gly Val Ala Trp Ile Arg Gln Ala Pro Gly Lys Ala Leu Glu
        35                  40                  45

Trp Leu Ala Leu Ile Tyr Trp Asp Asp Asp Lys Arg Tyr Asn Pro Ser
    50                  55                  60

Leu Lys Ser Arg Leu Thr Ile Thr Lys Asp Asn Ser Arg Asn Gln Val
65                  70                  75                  80

Val Leu Thr Met Thr Asn Met Asp Pro Val Asp Thr Ala Thr Tyr Tyr
                85                  90                  95

Cys Val His Arg Gly Met Asn Arg Val Phe Gly Val Val Tyr Asn Ser
            100                 105                 110

Gly Trp Phe Asp Pro Trp Gly Gln Gly Thr Leu Val Thr Val Ser Ser
        115                 120                 125

<210> 39
<211> 325
<212> DNA
<213> Homo sapiens

<400> 39
gaaattgtgt tgacgcagtc tccaggcacc ctgtctttgt ctccagggga aagagccacc      60

ctctcctgta gggccagtca gagcgttaga agcagccagc tagcctggta ccagcagaag     120

cctggccagg ctcccagcct cctcatgtat gacgcatcta agagggccac tggcatccca     180

gacaggttca gtggcagtgg gtctgggaca gacttcactc tcaccatcag cagactggag     240

cctgaagatt ttgtagtgta tttctgtcag cagtatgata catcaccgtg gacgttcggc     300

caagggacca aggtggaaat caaac                                           325

<210> 40
<211> 108
<212> PRT
<213> Homo sapiens

<400> 40
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Arg Ser Ser
            20                  25                  30

Gln Leu Ala Trp Tyr Gln Gln Lys Pro Gly Gln Ala Pro Ser Leu Leu
        35                  40                  45

Met Tyr Asp Ala Ser Lys Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Val Val Tyr Phe Cys Gln Gln Tyr Asp Thr Ser Pro
                85                  90                  95

Trp Thr Phe Gly Gln Gly Thr Lys Val Glu Ile Lys
            100                 105

<210> 41
<211> 10
<212> PRT
<213> Homo sapiens

<400> 41
Gly Phe Ser Leu Ser Thr Ser Gly Glu Gly
1               5                   10

<210> 42
<211> 7
<212> PRT
<213> Homo sapiens

<400> 42
Ile Tyr Trp Asp Asp Asp Lys
1               5

<210> 43
<211> 22
<212> PRT
<213> Homo sapiens

<400> 43
Cys Val His Arg Gly Met Asn Arg Val Phe Gly Val Val Tyr Asn Ser
1               5                   10                  15

Gly Trp Phe Asp Pro Trp
            20

<210> 44
<211> 7
<212> PRT

<213> Homo sapiens

<400> 44
Gln Ser Val Arg Ser Ser Gln
1               5

<210> 45
<211> 11
<212> PRT
<213> Homo sapiens

<400> 45

<210> 46
<211> 358
<212> DNA
<213> Homo sapiens

<400> 46
caggtgcagc tacagcagtg gggcgcagga ctgttgaagc cttcggagac cctgtccctc      60

agttgcgctg tctccggtac gtccttcagt gattactact gggcctggat ccgtcagccc     120

ccagggaagg ggctggagtg gattggggaa atcaatcaga gtggacacac taacgacaac     180

ccgtccttca agagtcgagt caccatgtta gtagacaagt ccaagaacca gttctccctg     240

aggatgactt ctgtgaccgc cgcggacacg gctgtatatt actgtgcgag atttccttgg     300

gtgggacccg agggttttga ccactggggc cagggaaccc tggtcaccgt ctcgagtg        358

<210> 47
<211> 119
<212> PRT
<213> Homo sapiens

<400> 47
Gln Val Gln Leu Gln Gln Trp Gly Ala Gly Leu Leu Lys Pro Ser Glu
1               5                   10                  15

Thr Leu Ser Leu Ser Cys Ala Val Ser Gly Thr Ser Phe Ser Asp Tyr
            20                  25                  30

Tyr Trp Ala Trp Ile Arg Gln Pro Pro Gly Lys Gly Leu Glu Trp Ile
        35                  40                  45

Gly Glu Ile Asn Gln Ser Gly His Thr Asn Asp Asn Pro Ser Phe Lys
    50                  55                  60

Ser Arg Val Thr Met Leu Val Asp Lys Ser Lys Asn Gln Phe Ser Leu
65                  70                  75                  80

Arg Met Thr Ser Val Thr Ala Ala Asp Thr Ala Val Tyr Tyr Cys Ala

85                  90                  95

Arg Phe Pro Trp Val Gly Pro Glu Gly Phe Asp His Trp Gly Gln Gly
            100                 105                 110

Thr Leu Val Thr Val Ser Ser
        115

<210> 48
<211> 382
<212> DNA
<213> Homo sapiens

<400> 48
gaaattgtgc tgactcagtc tccagacttt cagtctggga ctccaaagga gatggtcacc      60

atcacctgcc gggccagtca gaccattggt aacagcttac actggtacca acagaaacca     120

ggtcagtctc caaagctcct catcaaatat ggttcccagt ccatgtcagg ggtcccctcg     180

cggttcagtg gcagtggatt tgggacagat ttcaccctga ccatcaacag cctggaagct     240

gaagatgctg cagtgtatta ctgtcttcag agtggtggtt ttccgtggac gttcggccaa     300

gggaccaagg tggagatcag acgaactgtg gctgcaccat ctgtcttcat cttcccgcca     360

tctgatgagc agttgaaatc tg                                              382

<210> 49
<211> 107
<212> PRT
<213> Homo sapiens

<400> 49
Glu Ile Val Leu Thr Gln Ser Pro Asp Phe Gln Ser Gly Thr Pro Lys
1               5                   10                  15

Glu Met Val Thr Ile Thr Cys Arg Ala Ser Gln Thr Ile Gly Asn Ser
            20                  25                  30

Leu His Trp Tyr Gln Gln Lys Pro Gly Gln Ser Pro Lys Leu Leu Ile
        35                  40                  45

Lys Tyr Gly Ser Gln Ser Met Ser Gly Val Pro Ser Arg Phe Ser Gly
    50                  55                  60

Ser Gly Phe Gly Thr Asp Phe Thr Leu Thr Ile Asn Ser Leu Glu Ala
65                  70                  75                  80

Glu Asp Ala Ala Val Tyr Tyr Cys Leu Gln Ser Gly Gly Phe Pro Trp
                85                  90                  95

Thr Phe Gly Gln Gly Thr Lys Val Glu Ile Arg

100                 105

<210> 50
<211> 8
<212> PRT
<213> Homo sapiens

<400> 50
Gly Thr Ser Phe Ser Asp Tyr Tyr
1               5

<210> 51
<211> 7
<212> PRT
<213> Homo sapiens

<400> 51
Ile Asn Gln Ser Gly His Thr
1               5

<210> 52
<211> 15
<212> PRT
<213> Homo sapiens

<400> 52
Cys Ala Arg Phe Pro Trp Val Gly Pro Glu Gly Phe Asp His Trp
1               5                   10                  15

<210> 53
<211> 6
<212> PRT
<213> Homo sapiens

<400> 53
Gln Thr Ile Gly Asn Ser
1               5

<210> 54
<211> 11
<212> PRT
<213> Homo sapiens

<400> 54
Cys Leu Gln Ser Gly Gly Phe Pro Trp Thr Phe
1               5                   10

<210> 55
<211> 367
<212> DNA
<213> Homo sapiens

<400> 55
caggtccagc tggtacagtc tggggctgag atgaagaagc ctggggcctc agtgaaggtc      60

tcctgcaagg gttccggatc cagtctccct tatctttcga tacaatgggt gcgacaggcc     120

cctggaaaag gccttgagtg gatgggaggc tttgatcctg aagacggtga aacaatctac 180

aacgagaaat tcgagggcag aatcaccatg gccgaggaca catcgacaga cacagcctac 240

atggaactca gcagcctcac atctgacgac acggccgtct attattgcgc aacaagcctc 300

atccgtaacg gtctttatga agcgttcgaa cactggggcc agggaaccct ggtcaccgtc 360

tcgagtg 367

<210> 56
<211> 122
<212> PRT
<213> Homo sapiens

<400> 56
Gln Val Gln Leu Val Gln Ser Gly Ala Glu Met Lys Lys Pro Gly Ala
1               5                   10                  15

Ser Val Lys Val Ser Cys Lys Gly Ser Gly Ser Ser Leu Pro Tyr Leu
            20                  25                  30

Ser Ile Gln Trp Val Arg Gln Ala Pro Gly Lys Gly Leu Glu Trp Met
        35                  40                  45

Gly Gly Phe Asp Pro Glu Asp Gly Glu Thr Ile Tyr Asn Glu Lys Phe
    50                  55                  60

Glu Gly Arg Ile Thr Met Ala Glu Asp Thr Ser Thr Asp Thr Ala Tyr
65                  70                  75                  80

Met Glu Leu Ser Ser Leu Thr Ser Asp Asp Thr Ala Val Tyr Tyr Cys
                85                  90                  95

Ala Thr Ser Leu Ile Arg Asn Gly Leu Tyr Glu Ala Phe Glu His Trp
            100                 105                 110

Gly Gln Gly Thr Leu Val Thr Val Ser Ser
        115                 120

<210> 57
<211> 322
<212> DNA
<213> Homo sapiens

<400> 57
gacatccaga tgacccagtc tccatcctcc ctgtctgcat ctgtaggaga cagagtcacc 60

atcacttgcc aggcgagtga tgacattaaa aagaatttaa attggtatca acaaaaaaca 120

gggacagccc ctaagctcct ggtctacgac gcatccaatt tggagacagg ggtcccatcg 180

aggttccgtg gacgtgcatc tgggacacaa tatactttca ccatcacgaa tctgcagcct 240

caagattctg gaatatatta ctgtcaacaa tatgacaatg tcccgctcag ttttggcgga 300

gggaccaagg tggaggtcag ac 322

<210> 58
<211> 107
<212> PRT
<213> Homo sapiens

<400> 58
Asp Ile Gln Met Thr Gln Ser Pro Ser Ser Leu Ser Ala Ser Val Gly
1               5                   10                  15

Asp Arg Val Thr Ile Thr Cys Gln Ala Ser Asp Asp Ile Lys Lys Asn
            20                  25                  30

Leu Asn Trp Tyr Gln Gln Lys Thr Gly Thr Ala Pro Lys Leu Leu Val
        35                  40                  45

Tyr Asp Ala Ser Asn Leu Glu Thr Gly Val Pro Ser Arg Phe Arg Gly
    50                  55                  60

Arg Ala Ser Gly Thr Gln Tyr Thr Phe Thr Ile Thr Asn Leu Gln Pro
65                  70                  75                  80

Gln Asp Ser Gly Ile Tyr Tyr Cys Gln Gln Tyr Asp Asn Val Pro Leu
                85                  90                  95

Ser Phe Gly Gly Gly Thr Lys Val Glu Val Arg
            100                 105

<210> 59
<211> 8
<212> PRT
<213> Homo sapiens

<400> 59
Gly Ser Ser Leu Pro Tyr Leu Ser
1               5

<210> 60
<211> 8
<212> PRT
<213> Homo sapiens

<400> 60
Phe Asp Pro Glu Asp Gly Glu Thr
1               5

<210> 61
<211> 17
<212> PRT
<213> Homo sapiens

<400> 61
Cys Ala Thr Ser Leu Ile Arg Asn Gly Leu Tyr Glu Ala Phe Glu His
1               5                   10                  15

Trp

<210> 62
<211> 6
<212> PRT
<213> Homo sapiens

<400> 62
Asp Asp Ile Lys Lys Asn
1               5

<210> 63
<211> 11
<212> PRT
<213> Homo sapiens

<400> 63

<210> 64
<211> 382
<212> DNA
<213> Homo sapiens

<400> 64
caggtgcagc tggtgcagtc tggggctgag gtgaagatgc ctgagtcctc ggtgagggtc      60

tcctgcagga tttctggagg caacttcaaa aattatattt ttaactgggt gcgacaggcc     120

cccggacaag ggcttgagtg ggttggaagg atcaacccaa aagttggaaa tgaaaatcac     180

atacagagat atgggaacag actcaccctc accgcggacg attccaggag aacagtttac     240

atggagttga gcagcctgac aacagacgac acggccgtat attattgtgc gacattacgt     300

gttattggta accggagact acgaccggtg ggcggaattt ttgatatctg gggccaaggg     360

acaatggtca ccgtctcgag tg                                              382

<210> 65
<211> 127
<212> PRT
<213> Homo sapiens

<400> 65
Gln Val Gln Leu Val Gln Ser Gly Ala Glu Val Lys Met Pro Glu Ser
1               5                   10                  15

Ser Val Arg Val Ser Cys Arg Ile Ser Gly Gly Asn Phe Lys Asn Tyr
            20                  25                  30

Ile Phe Asn Trp Val Arg Gln Ala Pro Gly Gln Gly Leu Glu Trp Val
        35                  40                  45

Gly Arg Ile Asn Pro Lys Val Gly Asn Glu Asn His Ile Gln Arg Tyr
    50                  55                  60

Gly Asn Arg Leu Thr Leu Thr Ala Asp Asp Ser Arg Arg Thr Val Tyr
65                  70                  75                  80

Met Glu Leu Ser Ser Leu Thr Thr Asp Asp Thr Ala Val Tyr Tyr Cys
                85                  90                  95

Ala Thr Leu Arg Val Ile Gly Asn Arg Arg Leu Arg Pro Val Gly Gly
            100                 105                 110

Ile Phe Asp Ile Trp Gly Gln Gly Thr Met Val Thr Val Ser Ser
        115                 120                 125

<210> 66
<211> 322
<212> DNA
<213> Homo sapiens

<400> 66
gacatccaga tgacccagtc tccatcctcc ctgtctgcgt ctgtgggaga cagagtcacc      60

atcacttgcc aggcgagtca ggacgtgagt cactatgtaa attggtatca acaaaaacca     120

gggaaagccc caaaagccct ggtctacgag gcatccaatt tggaaacagg ggtcccatca     180

agattcagtg gaagtggatc tgggacacat ttcaccttca ccatcaacag cctgcagcct     240

gaagatatcg cgacttattt ctgtcaacat gatgcagagc tgcctccctc tttcggagga     300

gggaccaagg tggagatcaa ac                                              322

<210> 67
<211> 107
<212> PRT
<213> Homo sapiens

<400> 67
Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Val Ser His Tyr
            20                  25                  30

Val Asn Trp Tyr Gln Gln Lys Pro Gly Lys Ala Pro Lys Ala Leu Val
        35                  40                  45

Tyr Glu Ala Ser Asn Leu Glu Thr Gly Val Pro Ser Arg Phe Ser Gly
    50                  55                  60

Ser Gly Ser Gly Thr His Phe Thr Phe Thr Ile Asn Ser Leu Gln Pro
65                  70                  75                  80

Glu Asp Ile Ala Thr Tyr Phe Cys Gln His Asp Ala Glu Leu Pro Pro
                85                  90                  95

Ser Phe Gly Gly Gly Thr Lys Val Glu Ile Lys
            100                 105

<210> 68
<211> 8
<212> PRT
<213> Homo sapiens

<400> 68
Gly Gly Asn Phe Lys Asn Tyr Ile
1               5

<210> 69
<211> 8
<212> PRT
<213> Homo sapiens

<400> 69
Ile Asn Pro Lys Val Gly Asn Glu
1               5

<210> 70
<211> 22
<212> PRT
<213> Homo sapiens

<400> 70
Cys Ala Thr Leu Arg Val Ile Gly Asn Arg Arg Leu Arg Pro Val Gly
1               5                   10                  15

Gly Ile Phe Asp Ile Trp
            20

<210> 71
<211> 6
<212> PRT
<213> Homo sapiens

<400> 71
Gln Asp Val Ser His Tyr
1               5

<210> 72
<211> 11
<212> PRT

<213> Homo sapiens

<400> 72
Cys Gln His Asp Ala Glu Leu Pro Pro Ser Phe
1               5                   10

<210> 73
<211> 364
<212> DNA
<213> Homo sapiens

<400> 73
caggttcagc tggtgcagtc tggagctgaa gtgaagaagc ctggggcctc agtgaaggtc      60

tcctgcaagt cttctggtca ctcctttacc gcctatggca tcagctgggt gcgacaggcc     120

cctggacaag ggcttgagtg gatggggtgg atcagcacta acaatggcaa cacaaactat     180

agagagaagt tccaggacag actctccttg accacggaca catccacgag cacagtctac     240

atggaactga ggagcctgac atctgacgac acggccgtgt attactgtgc gagaaatcct     300

tatggcaatt cgcggagatt cttagactac tggggccagg gaaccctggt caccgtctcg     360

agtg                                                                  364

<210> 74
<211> 121
<212> PRT
<213> Homo sapiens

<400> 74
Gln Val Gln Leu Val Gln Ser Gly Ala Glu Val Lys Lys Pro Gly Ala
1               5                   10                  15

Ser Val Lys Val Ser Cys Lys Ser Ser Gly His Ser Phe Thr Ala Tyr
            20                  25                  30

Gly Ile Ser Trp Val Arg Gln Ala Pro Gly Gln Gly Leu Glu Trp Met
        35                  40                  45

Gly Trp Ile Ser Thr Asn Asn Gly Asn Thr Asn Tyr Arg Glu Lys Phe
    50                  55                  60

Gln Asp Arg Leu Ser Leu Thr Thr Asp Thr Ser Thr Ser Thr Val Tyr
65                  70                  75                  80

Met Glu Leu Arg Ser Leu Thr Ser Asp Asp Thr Ala Val Tyr Tyr Cys
                85                  90                  95

Ala Arg Asn Pro Tyr Gly Asn Ser Arg Arg Phe Leu Asp Tyr Trp Gly
            100                 105                 110

Gln Gly Thr Leu Val Thr Val Ser Ser

115                 120

<210> 75
<211> 322
<212> DNA
<213> Homo sapiens

<400> 75
gacatccaga tgacccagtc tccatcctcc ctgtctgcat ctgtcggaga cagagtcatc      60

atcacttgcc gggcaagtca gaacattagc ccctatttaa attggtatca gcagaagcca     120

gggagagccc ctgagctcct gatctatggt acgaccactg tacaaactgg ggtcccgtca     180

cggttcagtg gcattggatc tgggacagat ttcactctca ccatcagcag tctgcaacct     240

gaagattttg caacttatta ctgtcaacag agctacaggg cccctcaaac gttcggccaa     300

gggaccaagg tggaagtcaa gc                                              322

<210> 76
<211> 107
<212> PRT
<213> Homo sapiens

<400> 76
Asp Ile Gln Met Thr Gln Ser Pro Ser Ser Leu Ser Ala Ser Val Gly
1               5                   10                  15

Asp Arg Val Ile Ile Thr Cys Arg Ala Ser Gln Asn Ile Ser Pro Tyr
            20                  25                  30

Leu Asn Trp Tyr Gln Gln Lys Pro Gly Arg Ala Pro Glu Leu Leu Ile
        35                  40                  45

Tyr Gly Thr Thr Thr Val Gln Thr Gly Val Pro Ser Arg Phe Ser Gly
    50                  55                  60

Ile Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Arg Ala Pro Gln
                85                  90                  95

Thr Phe Gly Gln Gly Thr Lys Val Glu Val Lys
            100                 105

<210> 77
<211> 8
<212> PRT
<213> Homo sapiens

<400> 77
Gly His Ser Phe Thr Ala Tyr Gly

1               5


<210> 78
<211> 8
<212> PRT
<213> Homo sapiens

<400> 78
Ile Ser Thr Asn Asn Gly Asn Thr
1               5


<210> 79
<211> 16
<212> PRT
<213> Homo sapiens

<400> 79
Cys Ala Arg Asn Pro Tyr Gly Asn Ser Arg Arg Phe Leu Asp Tyr Trp
1               5                   10                  15


<210> 80
<211> 6
<212> PRT
<213> Homo sapiens

<400> 80
Gln Asn Ile Ser Pro Tyr
1               5


<210> 81
<211> 11
<212> PRT
<213> Homo sapiens

<400> 81
Cys Gln Gln Ser Tyr Arg Ala Pro Gln Thr Phe
1               5                   10


<210> 82
<211> 376
<212> DNA
<213> Homo sapiens

<400> 82
caggtcacct tgaaggagtc tggtcctccg ctggtgaatc ccacagagac cctcacggtg      60

acctgcaatt tccctggctt ctcattcacc tctagtggag agggtgtggg ctggatccgt     120

cagcccccag gaaaggccct ggaatggctt gctcacattt attgggatgg cgataagcgg     180

ttcaacacat ctctgaaaaa cagactcaac atcaccaagg acacctctaa gaatcaggtg     240

gtcctcactt tgaccaacgt ggcccctgtg gacacagcca cgtatttctg tgtccacctg     300

acacgcccat ccttttggag tccctattac ctttttgacc tctggggcca gggaaccctg     360

gtcaccgtct cgagtg                                                     376

<210> 83
<211> 125
<212> PRT
<213> Homo sapiens

<400> 83
Gln Val Thr Leu Lys Glu Ser Gly Pro Pro Leu Val Asn Pro Thr Glu
1               5                   10                  15

Thr Leu Thr Val Thr Cys Asn Phe Pro Gly Phe Ser Phe Thr Ser Ser
            20                  25                  30

Gly Glu Gly Val Gly Trp Ile Arg Gln Pro Pro Gly Lys Ala Leu Glu
        35                  40                  45

Trp Leu Ala His Ile Tyr Trp Asp Gly Asp Lys Arg Phe Asn Thr Ser
    50                  55                  60

Leu Lys Asn Arg Leu Asn Ile Thr Lys Asp Thr Ser Lys Asn Gln Val
65                  70                  75                  80

Val Leu Thr Leu Thr Asn Val Ala Pro Val Asp Thr Ala Thr Tyr Phe
                85                  90                  95

Cys Val His Leu Thr Arg Pro Ser Phe Trp Ser Pro Tyr Tyr Leu Phe
            100                 105                 110

Asp Leu Trp Gly Gln Gly Thr Leu Val Thr Val Ser Ser
        115                 120                 125

<210> 84
<211> 319
<212> DNA
<213> Homo sapiens

<400> 84
gacatccagt tgacccagtc tccgccctcc ctgtctgcat ctgtcggaga cagtgtcacc      60

ataacttgcc gggcaagtca atatattagt acgtatttaa attggtatca gcagaaaccg     120

gggaaagccc ccagactctt gatctatggt gcatccaatt tgcacagtgg ggtctcatca     180

aggttcagtg gcagtagatc tgggacagat ttcactctca ccataaccag tctacagcct     240

gaagattttg cgacttacta ctgccaacag agttccactt tgtggtcgtt cggccaaggg     300

accaaggtgg agatgcacc                                                  319

<210> 85
<211> 106
<212> PRT
<213> Homo sapiens

<400> 85
Asp Ile Gln Leu Thr Gln Ser Pro Pro Ser Leu Ser Ala Ser Val Gly
1               5                   10                  15

Asp Ser Val Thr Ile Thr Cys Arg Ala Ser Gln Tyr Ile Ser Thr Tyr
            20                  25                  30

Leu Asn Trp Tyr Gln Gln Lys Pro Gly Lys Ala Pro Arg Leu Leu Ile
        35                  40                  45

Tyr Gly Ala Ser Asn Leu His Ser Gly Val Ser Ser Arg Phe Ser Gly
    50                  55                  60

Ser Arg Ser Gly Thr Asp Phe Thr Leu Thr Ile Thr Ser Leu Gln Pro
65                  70                  75                  80

Glu Asp Phe Ala Thr Tyr Tyr Cys Gln Gln Ser Ser Thr Leu Trp Ser
                85                  90                  95

Phe Gly Gln Gly Thr Lys Val Glu Met His
            100                 105


<210> 86
<211> 10
<212> PRT
<213> Homo sapiens

<400> 86
Gly Phe Ser Phe Thr Ser Ser Gly Glu Gly
1               5                   10


<210> 87
<211> 7
<212> PRT
<213> Homo sapiens

<400> 87
Ile Tyr Trp Asp Gly Asp Lys
1               5


<210> 88
<211> 19
<212> PRT
<213> Homo sapiens

<400> 88
Cys Val His Leu Thr Arg Pro Ser Phe Trp Ser Pro Tyr Tyr Leu Phe
1               5                   10                  15

Asp Leu Trp

<210> 89
<211> 7
<212> PRT
<213> Homo sapiens

<400> 89
Gln Tyr Ile Ser Thr Tyr Leu
1               5


<210> 90
<211> 10
<212> PRT
<213> Homo sapiens

<400> 90
Cys Gln Gln Ser Ser Thr Leu Trp Ser Phe
1               5                   10


<210> 91
<211> 358
<212> DNA
<213> Homo sapiens

<400> 91
caggttcagc tggtgcagtc tggaggtgaa atgaggaaac ctggggcctc attgaaggtc      60

tcctgcaagg cctctaatta caccttcacc agttttggtc tgacctggct gcgacaggcc     120

cctgggcaag gccctgagtg gctgggctgg atcagtcctt ataatggaaa caccaactat     180

gcacagaact tccgcgacag actcaccatg accacagaca cgtccacgag cacagcctac     240

atggagctga agagcctgac atctgacgac acggccgtct attactgtgc cagaactaac     300

tgggggaatt ggtacctcga tctctggggc cctggcaccc tggtcactgt ctcgagtg       358


<210> 92
<211> 119
<212> PRT
<213> Homo sapiens

<400> 92
Gln Val Gln Leu Val Gln Ser Gly Gly Glu Met Arg Lys Pro Gly Ala
1               5                   10                  15


Ser Leu Lys Val Ser Cys Lys Ala Ser Asn Tyr Thr Phe Thr Ser Phe
            20                  25                  30


Gly Leu Thr Trp Leu Arg Gln Ala Pro Gly Gln Gly Pro Glu Trp Leu
        35                  40                  45


Gly Trp Ile Ser Pro Tyr Asn Gly Asn Thr Asn Tyr Ala Gln Asn Phe
    50                  55                  60


Arg Asp Arg Leu Thr Met Thr Thr Asp Thr Ser Thr Ser Thr Ala Tyr
65                  70                  75                  80

Met Glu Leu Lys Ser Leu Thr Ser Asp Asp Thr Ala Val Tyr Tyr Cys
                85                  90                  95

Ala Arg Thr Asn Trp Gly Asn Trp Tyr Leu Asp Leu Trp Gly Pro Gly
            100                 105                 110

Thr Leu Val Thr Val Ser Ser
        115

<210> 93
<211> 340
<212> DNA
<213> Homo sapiens

<400> 93
gacatcgtga tgacccagtc tccagactcc ctggctgtgg ctctgggcga gcgggccacc      60

atcagctgca agtccagcca gactatcttg ttcggctcca acaacaagaa ctacttacag     120

tggtttcagc agaaacccgg acagcctcct aagttactca tttactgggc atctacccgg     180

gaatccgggg tccctgaccg attcagtggc gacgggtctg ggacagactt cactctctcc     240

atcgacaacc tacaggctga agatgtggca ctttattatt gccagcaata ttttagtaat     300

ccgtatactt tcggccaggg gaccaaattg gagatcaaac                           340

<210> 94
<211> 113
<212> PRT
<213> Homo sapiens

<400> 94
Asp Ile Val Met Thr Gln Ser Pro Asp Ser Leu Ala Val Ala Leu Gly
1               5                   10                  15

Glu Arg Ala Thr Ile Ser Cys Lys Ser Ser Gln Thr Ile Leu Phe Gly
            20                  25                  30

Ser Asn Asn Lys Asn Tyr Leu Gln Trp Phe Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Asp Gly Ser Gly Thr Asp Phe Thr Leu Ser
65                  70                  75                  80

Ile Asp Asn Leu Gln Ala Glu Asp Val Ala Leu Tyr Tyr Cys Gln Gln
                85                  90                  95

Tyr Phe Ser Asn Pro Tyr Thr Phe Gly Gln Gly Thr Lys Leu Glu Ile
            100                 105                 110

Lys

<210> 95
<211> 8
<212> PRT
<213> Homo sapiens

<400> 95
Asn Tyr Thr Phe Thr Ser Phe Gly
1               5

<210> 96
<211> 8
<212> PRT
<213> Homo sapiens

<400> 96
Ile Ser Pro Tyr Asn Gly Asn Thr
1               5

<210> 97
<211> 14
<212> PRT
<213> Homo sapiens

<400> 97
Cys Ala Arg Thr Asn Trp Gly Asn Trp Tyr Leu Asp Leu Trp
1               5                   10

<210> 98
<211> 12
<212> PRT
<213> Homo sapiens

<400> 98
Gln Thr Ile Leu Phe Gly Ser Asn Asn Lys Asn Tyr
1               5                   10

<210> 99
<211> 11
<212> PRT
<213> Homo sapiens

<400> 99
Cys Gln Gln Tyr Phe Ser Asn Pro Tyr Thr Phe
1               5                   10

<210> 100
<211> 361
<212> DNA
<213> Homo sapiens

<400> 100
caggtccagc ttgtgcagtc tggggctgag gtgaagaagc ctggggcctc agtgaaaatt      60

gcctgcaaga cctctggata catcttcgtt gactatgctg tacaatgggt gcgccaggcc     120

cccggacaga ggcttgagtg gatggggtgg ctccagtcag ccagtggcaa gacgagctcg     180

tcacatagtc tccaagacag agtctccatt acccgggaca catccgcgaa cacagcctac     240

ttggaactga ggagtctgac ttctgaagat acggctgtct actactgtgc gagagggagt     300

ataagtcatt acgccccctt tgactactgg ggccagggaa ccctggtcac cgtctcgagt     360

g                                                                     361

<210> 101
<211> 120
<212> PRT
<213> Homo sapiens

<400> 101
Gln Val Gln Leu Val Gln Ser Gly Ala Glu Val Lys Lys Pro Gly Ala
1               5                   10                  15

Ser Val Lys Ile Ala Cys Lys Thr Ser Gly Tyr Ile Phe Val Asp Tyr
            20                  25                  30

Ala Val Gln Trp Val Arg Gln Ala Pro Gly Gln Arg Leu Glu Trp Met
        35                  40                  45

Gly Trp Leu Gln Ser Ala Ser Gly Lys Thr Ser Ser Ser His Ser Leu
    50                  55                  60

Gln Asp Arg Val Ser Ile Thr Arg Asp Thr Ser Ala Asn Thr Ala Tyr
65                  70                  75                  80

Leu Glu Leu Arg Ser Leu Thr Ser Glu Asp Thr Ala Val Tyr Tyr Cys
                85                  90                  95

Ala Arg Gly Ser Ile Ser His Tyr Ala Pro Phe Asp Tyr Trp Gly Gln
            100                 105                 110

Gly Thr Leu Val Thr Val Ser Ser
        115                 120

<210> 102
<211> 340
<212> DNA
<213> Homo sapiens

<400> 102
gacatcgtga tgacccagtc tccagactcc ctggctgtgt ctctgggcga gagggccacc      60

atcgactgca agtccagtca gaatatttta tatacttcca acaataagag tttcttagct     120

tggtaccaac agaaaccagg acagcctcct aaattgctca tttactgggc gtctacccga     180

gagttcgggg tccctgcgcg attcagtggc agcgggtctg ggacagattt cactctcacc     240

atcaacaacc tgcagggtga agatgtggcc atttattact gtcaacagta tttttctact     300

ccatacactt ttggccaggg gaccaagctg gagatcaaac                          340

<210> 103
<211> 113
<212> PRT
<213> Homo sapiens

<400> 103
Asp Ile Val Met Thr Gln Ser Pro Asp Ser Leu Ala Val Ser Leu Gly
1               5                   10                  15

Glu Arg Ala Thr Ile Asp Cys Lys Ser Ser Gln Asn Ile Leu Tyr Thr
            20                  25                  30

Ser Asn Asn Lys Ser Phe Leu Ala Trp Tyr Gln Gln Lys Pro Gly Gln
        35                  40                  45

Pro Pro Lys Leu Leu Ile Tyr Trp Ala Ser Thr Arg Glu Phe Gly Val
    50                  55                  60

Pro Ala Arg Phe Ser Gly Ser Gly Ser Gly Thr Asp Phe Thr Leu Thr
65                  70                  75                  80

Ile Asn Asn Leu Gln Gly Glu Asp Val Ala Ile Tyr Tyr Cys Gln Gln
                85                  90                  95

Tyr Phe Ser Thr Pro Tyr Thr Phe Gly Gln Gly Thr Lys Leu Glu Ile
            100                 105                 110

Lys

<210> 104
<211> 8
<212> PRT
<213> Homo sapiens

<400> 104
Gly Tyr Ile Phe Val Asp Tyr Ala
1               5

<210> 105
<211> 8
<212> PRT
<213> Homo sapiens

<400> 105
Leu Gln Ser Ala Ser Gly Lys Thr
1               5

<210> 106
<211> 15
<212> PRT
<213> Homo sapiens

<400> 106
Cys Ala Arg Gly Ser Ile Ser His Tyr Ala Pro Phe Asp Tyr Trp
1               5                   10                  15

<210> 107
<211> 12
<212> PRT
<213> Homo sapiens

<400> 107
Gln Asn Ile Leu Tyr Thr Ser Asn Asn Lys Ser Phe
1               5                   10

<210> 108
<211> 11
<212> PRT
<213> Homo sapiens

<400> 108

<210> 109
<211> 361
<212> DNA
<213> Homo sapiens

<400> 109
caggtgcagc tgcaggagtc gggcccagga ctggtgaagc cctcggagac cctgtccctc      60

acgtgcactg tctctggtgc ctacatcact aatgactact ggagctggat ccggcagccc     120

gccgggaagg gactggcgtg gattgggcgt ctctatacca gtgggagcac caactacaac     180

ccctccctca agagtcgagt caccatgtca gtagacacgt ccaaaaacca gttctccctg     240

aggctcacct ctgtgaccgc cgcggacacg gccgtttatt attgtgcgag agatgagtgt     300

agtggtggtg ggtgcttcca tgagaactgg ggccagggaa ccctggtcac cgtctcgagt     360

g                                                                     361

<210> 110
<211> 120
<212> PRT
<213> Homo sapiens

<400> 110
Gln Val Gln Leu Gln Glu Ser Gly Pro Gly Leu Val Lys Pro Ser Glu

1               5                   10                  15

Thr Leu Ser Leu Thr Cys Thr Val Ser Gly Ala Tyr Ile Thr Asn Asp
            20                  25                  30

Tyr Trp Ser Trp Ile Arg Gln Pro Ala Gly Lys Gly Leu Ala Trp Ile
        35                  40                  45

Gly Arg Leu Tyr Thr Ser Gly Ser Thr Asn Tyr Asn Pro Ser Leu Lys
    50                  55                  60

Ser Arg Val Thr Met Ser Val Asp Thr Ser Lys Asn Gln Phe Ser Leu
65                  70                  75                  80

Arg Leu Thr Ser Val Thr Ala Ala Asp Thr Ala Val Tyr Tyr Cys Ala
                85                  90                  95

Arg Asp Glu Cys Ser Gly Gly Gly Cys Phe His Glu Asn Trp Gly Gln
            100                 105                 110

Gly Thr Leu Val Thr Val Ser Ser
        115                 120

<210> 111
<211> 325
<212> DNA
<213> Homo sapiens

<400> 111
gaaattgtgt tgacgcagtc tccaggcacc ctgtctttgt ctccagggga aagagccacc      60

ctctcctgca gggccagcca gagtattagc agcagctact tggcctggta ccagcagaag     120

cgtggccagg ctcccaggct cctcatctat ggtgcatcca gtagggccac tggcatccca     180

gacaggttca gtggcagtgg gactgggaca gacttcactc tcaccatcag cagactggag     240

cctgacgatt ttgcacttta ttactgtcag cagtatgata ggtcaccgct cactttcggc     300

ggagggacca aggtggagat caaac                                           325

<210> 112
<211> 108
<212> PRT
<213> Homo sapiens

<400> 112
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Ile Ser Ser Ser
            20                  25                  30

Tyr Leu Ala Trp Tyr Gln Gln Lys Arg Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Thr Gly Thr Asp Phe Thr Leu Thr Ile Ser Arg Leu Glu
65                  70                  75                  80

Pro Asp Asp Phe Ala Leu Tyr Tyr Cys Gln Gln Tyr Asp Arg Ser Pro
                85                  90                  95

Leu Thr Phe Gly Gly Gly Thr Lys Val Glu Ile Lys
            100                 105

<210> 113
<211> 8
<212> PRT
<213> Homo sapiens

<400> 113
Gly Ala Tyr Ile Thr Asn Asp Tyr
1               5

<210> 114
<211> 7
<212> PRT
<213> Homo sapiens

<400> 114
Leu Tyr Thr Ser Gly Ser Thr
1               5

<210> 115
<211> 16
<212> PRT
<213> Homo sapiens

<400> 115
Cys Ala Arg Asp Glu Cys Ser Gly Gly Gly Cys Phe His Glu Asn Trp
1               5                   10                  15

<210> 116
<211> 7
<212> PRT
<213> Homo sapiens

<400> 116
Gln Ser Ile Ser Ser Ser Tyr
1               5

<210> 117
<211> 11

<212> PRT
<213> Homo sapiens

<400> 117


<210> 118
<211> 1591
<212> DNA
<213> Homo sapiens

<400> 118
cctccaccaa gggcccatcg gtcttccccc tggcaccctc ctccaagagc acctctgggg      60

gcacagcggc cctgggctgc ctggtcaagg actacttccc cgaaccggtg acggtgtcgt     120

ggaactcagg cgccctgacc agcggcgtgc acaccttccc ggctgtccta cagtcctcag     180

gactctactc cctcagcagc gtggtgaccg tgccctccag cagcttgggc acccagacct     240

acatctgcaa cgtgaatcac aagcccagca acaccaaggt ggacaagaaa gttggtgaga     300

ggccagcaca gggagggagg gtgtctgctg gaagcaggct cagcgctcct gcctggacgc     360

atcccggcta tgcagcccca gtccagggca gcaaggcagg ccccgtctgc ctcttcaccc     420

ggagcctctg cccgccccac tcatgctcag ggagagggtc ttctggcttt ttcccaggct     480

ctgggcaggc acaggctagg tgcccctaac ccaggccctg cacacaaagg ggcaggtgct     540

gggctcagac ctgccaagag ccatatccgg gaggaccctg cccctgacct aagcccaccc     600

caaaggccaa actctccact ccctcagctc ggacaccttc tctcctccca gattccagta     660

actcccaatc ttctctctgc agagcccaaa tcttgtgaca aaactcacac atgcccaccg     720

tgcccaggta agccagccca ggcctcgccc tccagctcaa ggcgggacag gtgccctaga     780

gtagcctgca tccagggaca ggccccagcc gggtgctgac acgtccacct ccatctcttc     840

ctcagcacct gaactcctgg ggggaccgtc agtcttcctc ttccccccaa aacccaagga     900

caccctcatg atctcccgga cccctgaggt cacatgcgtg gtggtggacg tgagccacga     960

agaccctgag gtcaagttca actggtacgt ggacggcgtg gaggtgcata atgccaagac    1020

aaagccgcgg gaggagcagt acaacagcac gtaccgggtg gtcagcgtcc tcaccgtcct    1080

gcaccaggac tggctgaatg gcaaggagta caagtgcaag gtctccaaca aagccctccc    1140

agcccccatc gagaaaacca tctccaaagc caaaggtggg acccgtgggg tgcgagggcc    1200

acatggacag aggccggctc ggcccaccct ctgccctgag agtgaccgct gtaccaacct    1260

ctgtcctaca gggcagcccc gagaaccaca ggtgtacacc ctgcccccat cccgggatga    1320

gctgaccaag aaccaggtca gcctgacctg cctggtcaaa ggcttctatc ccagcgacat    1380

cgccgtggag tgggagagca atgggcagcc ggagaacaac tacaagacca cgcctcccgt    1440

gctggactcc gacggctcct tcttcctcta cagcaagctc accgtggaca agagcaggtg    1500

gcagcagggg aacgtcttct catgctccgt gatgcatgag gctctgcaca accactacac 1560

gcagaagagc ctctccctgt ctccgggtaa a 1591

<210> 119
<211> 989
<212> DNA
<213> Homo sapiens

<400> 119
cctccaccaa gggcccatcg gtcttccccc tggcaccctc ctccaagagc acctctgggg 60

gcacagcggc cctgggctgc ctggtcaagg actacttccc cgaaccggtg acggtgtcgt 120

ggaactcagg cgccctgacc agcggcgtgc acaccttccc ggctgtccta cagtcctcag 180

gactctactc cctcagcagc gtggtgaccg tgccctccag cagcttgggc acccagacct 240

acatctgcaa cgtgaatcac aagcccagca acaccaaggt ggacaagaaa gttgagccca 300

aatcttgtga caaaactcac acatgcccac cgtgcccagc acctgaactc ctggggggac 360

cgtcagtctt cctcttcccc ccaaaaccca aggacaccct catgatctcc cggacccctg 420

aggtcacatg cgtggtggtg gacgtgagcc acgaagaccc tgaggtcaag ttcaactggt 480

acgtggacgg cgtggaggtg cataatgcca agacaaagcc gcgggaggag cagtacaaca 540

gcacgtaccg tgtggtcagc gtcctcaccg tcctgcacca ggactggctg aatggcaagg 600

agtacaagtg caaggtctcc aacaaagccc tcccagcccc catcgagaaa accatctcca 660

aagccaaagg gcagccccga gaaccacagg tgtacaccct gcccccatcc cgggatgagc 720

tgaccaagaa ccaggtcagc ctgacctgcc tggtcaaagg cttctatccc agcgacatcg 780

ccgtggagtg ggagagcaat gggcagccgg agaacaacta caagaccacg cctcccgtgc 840

tggactccga cggctccttc ttcctctaca gcaagctcac cgtggacaag agcaggtggc 900

agcaggggaa cgtcttctca tgctccgtga tgcatgaggc tctgcacaac cactacacgc 960

agaagagcct ctccctgtct ccgggtaaa 989

<210> 120
<211> 329
<212> PRT
<213> Homo sapiens

<400> 120
Ser Thr Lys Gly Pro Ser Val Phe Pro Leu Ala Pro Ser Ser Lys Ser
1               5                   10                  15

Thr Ser Gly Gly Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Ser Leu Gly Thr Gln Thr Tyr
65                  70                  75                  80

Ile Cys Asn Val Asn His Lys Pro Ser Asn Thr Lys Val Asp Lys Lys
                85                  90                  95

Val Glu Pro Lys Ser Cys Asp Lys Thr His Thr Cys Pro Pro Cys Pro
            100                 105                 110

Ala Pro Glu Leu Leu Gly Gly Pro Ser Val Phe Leu Phe Pro Pro Lys
        115                 120                 125

Pro Lys Asp Thr Leu Met Ile Ser Arg Thr Pro Glu Val Thr Cys Val
    130                 135                 140

Val Val Asp Val Ser His Glu Asp Pro Glu Val Lys Phe Asn Trp Tyr
145                 150                 155                 160

Val Asp Gly Val Glu Val His Asn Ala Lys Thr Lys Pro Arg Glu Glu
                165                 170                 175

Gln Tyr Asn Ser Thr Tyr Arg Val Val Ser Val Leu Thr Val Leu His
            180                 185                 190

Gln Asp Trp Leu Asn Gly Lys Glu Tyr Lys Cys Lys Val Ser Asn Lys
        195                 200                 205

Ala Leu Pro Ala Pro Ile Glu Lys Thr Ile Ser Lys Ala Lys Gly Gln
    210                 215                 220

Pro Arg Glu Pro Gln Val Tyr Thr Leu Pro Pro Ser Arg Asp Glu Leu
225                 230                 235                 240

Thr Lys Asn Gln Val Ser Leu Thr Cys Leu Val Lys Gly Phe Tyr Pro
                245                 250                 255

Ser Asp Ile Ala Val Glu Trp Glu Ser Asn Gly Gln Pro Glu Asn Asn
            260                 265                 270

Tyr Lys Thr Thr Pro Pro Val Leu Asp Ser Asp Gly Ser Phe Phe Leu
        275                 280                 285

Tyr Ser Lys Leu Thr Val Asp Lys Ser Arg Trp Gln Gln Gly Asn Val

290                 295                 300

Phe Ser Cys Ser Val Met His Glu Ala Leu His Asn His Tyr Thr Gln
305                 310                 315                 320

Lys Ser Leu Ser Leu Ser Pro Gly Lys
                325

<210> 121
<211> 1584
<212> DNA
<213> Homo sapiens

<400> 121
cctccaccaa gggcccatcg gtcttccccc tggcgccctg ctccaggagc acctccgaga      60

gcacagccgc cctgggctgc ctggtcaagg actacttccc cgaaccggtg acggtgtcgt     120

ggaactcagg cgctctgacc agcggcgtgc acaccttccc agctgtccta cagtcctcag     180

gactctactc cctcagcagc gtggtgaccg tgccctccag caacttcggc acccagacct     240

acacctgcaa cgtagatcac aagcccagca acaccaaggt ggacaagaca gttggtgaga     300

ggccagctca gggagggagg gtgtctgctg gaagccaggc tcagccctcc tgcctggacg     360

caccccggct gtgcagcccc agcccagggc agcaaggcag gccccatctg tctcctcacc     420

cggaggcctc tgcccgcccc actcatgctc agggagaggg tcttctggct ttttccacca     480

ggctccaggc aggcacaggc tgggtgcccc taccccaggc ccttcacaca caggggcagg     540

tgcttggctc agacctgcca aaagccatat ccgggaggac cctgcccctg acctaagccg     600

accccaaagg ccaaactgtc cactccctca gctcggacac cttctctcct cccagatccg     660

agtaactccc aatcttctct ctgcagagcg caaatgttgt gtcgagtgcc caccgtgccc     720

aggtaagcca gcccaggcct cgccctccag ctcaaggcgg gacaggtgcc ctagagtagc     780

ctgcatccag ggacaggccc cagctgggtg ctgacacgtc cacctccatc tcttcctcag     840

caccacctgt ggcaggaccg tcagtcttcc tcttcccccc aaaacccaag gacaccctca     900

tgatctcccg gacccctgag gtcacgtgcg tggtggtgga cgtgagccac gaagaccccg     960

aggtccagtt caactggtac gtggacggcg tggaggtgca taatgccaag acaaagccac    1020

gggaggagca gttcaacagc acgttccgtg tggtcagcgt cctcaccgtt gtgcaccagg    1080

actggctgaa cggcaaggag tacaagtgca aggtctccaa caaaggcctc ccagccccca    1140

tcgagaaaac catctccaaa accaaaggtg ggacccgcgg ggtatgaggg ccacatggac    1200

agaggccggc tcggcccacc ctctgccctg ggagtgaccg ctgtgccaac ctctgtccct    1260

acagggcagc cccgagaacc acaggtgtac accctgcccc catcccggga ggagatgacc    1320

aagaaccagg tcagcctgac ctgcctggtc aaaggcttct accccagcga catcgccgtg    1380

gagtgggaga gcaatgggca gccggagaac aactacaaga ccacacctcc catgctggac 1440

tccgacggct ccttcttcct ctacagcaag ctcaccgtgg acaagagcag gtggcagcag 1500

gggaacgtct tctcatgctc cgtgatgcat gaggctctgc acaaccacta cacgcagaag 1560

agcctctccc tgtctccggg taaa 1584

<210> 122
<211> 977
<212> DNA
<213> Homo sapiens

<400> 122
cctccaccaa gggcccatcg gtcttccccc tggcgccctg ctccaggagc acctccgaga 60

gcacagccgc cctgggctgc ctggtcaagg actacttccc cgaaccggtg acggtgtcgt 120

ggaactcagg cgctctgacc agcggcgtgc acaccttccc agctgtccta cagtcctcag 180

gactctactc cctcagcagc gtggtgaccg tgccctccag caacttcggc acccagacct 240

acacctgcaa cgtagatcac aagcccagca acaccaaggt ggacaagaca gttgagcgca 300

aatgttgtgt cgagtgccca ccgtgcccag caccacctgt ggcaggaccg tcagtcttcc 360

tcttcccccc aaaacccaag gacaccctca tgatctcccg gacccctgag gtcacgtgcg 420

tggtggtgga cgtgagccac gaagaccccg aggtccagtt caactggtac gtggacggcg 480

tggaggtgca taatgccaag acaaagccac gggaggagca gttcaacagc acgttccgtg 540

tggtcagcgt cctcaccgtt gtgcaccagg actggctgaa cggcaaggag tacaagtgca 600

aggtctccaa caaaggcctc ccagccccca tcgagaaaac catctccaaa accaaagggc 660

agccccgaga accacaggtg tacaccctgc ccccatcccg ggaggagatg accaagaacc 720

aggtcagcct gacctgcctg gtcaaaggct tctaccccag cgacatcgcc gtggagtggg 780

agagcaatgg gcagccggag aacaactaca agaccacacc tcccatgctg gactccgacg 840

gctccttctt cctctacagc aagctcaccg tggacaagag caggtggcag caggggaacg 900

tcttctcatg ctccgtgatg catgaggctc tgcacaacca ctacacgcag aagagcctct 960

ccctgtctcc gggtaaa 977

<210> 123
<211> 325
<212> PRT
<213> Homo sapiens

<400> 123
Ser Thr Lys Gly Pro Ser Val Phe Pro Leu Ala Pro Cys Ser Arg Ser
1               5                   10                  15

Thr Ser Glu Ser Thr Ala Ala Leu Gly Cys Leu Val Lys Asp Tyr Phe
            20                  25                  30

Pro Glu Pro Val Thr Val Ser Trp Asn Ser Gly Ala Leu Thr Ser Gly
        35                  40                  45

Val His Thr Phe Pro Ala Val Leu Gln Ser Ser Gly Leu Tyr Ser Leu
    50                  55                  60

Ser Ser Val Val Thr Val Pro Ser Ser Asn Phe Gly Thr Gln Thr Tyr
65                  70                  75                  80

Thr Cys Asn Val Asp His Lys Pro Ser Asn Thr Lys Val Asp Lys Thr
                85                  90                  95

Val Glu Arg Lys Cys Cys Val Glu Cys Pro Pro Cys Pro Ala Pro Pro
            100                 105                 110

Val Ala Gly Pro Ser Val Phe Leu Phe Pro Pro Lys Pro Lys Asp Thr
        115                 120                 125

Leu Met Ile Ser Arg Thr Pro Glu Val Thr Cys Val Val Val Asp Val
    130                 135                 140

Ser His Glu Asp Pro Glu Val Gln Phe Asn Trp Tyr Val Asp Gly Val
145                 150                 155                 160

Glu Val His Asn Ala Lys Thr Lys Pro Arg Glu Glu Gln Phe Asn Ser
                165                 170                 175

Thr Phe Arg Val Val Ser Val Leu Thr Val Val His Gln Asp Trp Leu
            180                 185                 190

Asn Gly Lys Glu Tyr Lys Cys Lys Val Ser Asn Lys Gly Leu Pro Ala
        195                 200                 205

Pro Ile Glu Lys Thr Ile Ser Lys Thr Lys Gly Gln Pro Arg Glu Pro
    210                 215                 220

Gln Val Tyr Thr Leu Pro Pro Ser Arg Glu Glu Met Thr Lys Asn Gln
225                 230                 235                 240

Val Ser Leu Thr Cys Leu Val Lys Gly Phe Tyr Pro Ser Asp Ile Ala
                245                 250                 255

Val Glu Trp Glu Ser Asn Gly Gln Pro Glu Asn Asn Tyr Lys Thr Thr
            260                 265                 270

Pro Pro Met Leu Asp Ser Asp Gly Ser Phe Phe Leu Tyr Ser Lys Leu
        275                 280                 285

Thr Val Asp Lys Ser Arg Trp Gln Gln Gly Asn Val Phe Ser Cys Ser
    290                 295                 300

Val Met His Glu Ala Leu His Asn His Tyr Thr Gln Lys Ser Leu Ser
305                 310                 315                 320

Leu Ser Pro Gly Lys
                325

<210> 124
<211> 320
<212> DNA
<213> Homo sapiens

<400> 124
gaactgtggc tgcaccatct gtcttcatct tcccgccatc tgatgagcag ttgaaatctg      60

gaactgcctc tgttgtgtgc ctgctgaata acttctatcc cagagaggcc aaagtacagt     120

ggaaggtgga taacgccctc caatcgggta actcccagga gagtgtcaca gagcaggaca     180

gcaaggacag cacctacagc ctcagcagca ccctgacgct gagcaaagca gactacgaga     240

aacacaaagt ctacgcctgc gaagtcaccc atcagggcct gagctcgccc gtcacaaaga     300

gcttcaacag gggagagtgt                                                 320

<210> 125
<211> 107
<212> PRT
<213> Homo sapiens

<400> 125

<210> 126
<211> 317
<212> DNA
<213> Homo sapiens

<400> 126
gtcagcccaa ggccaacccc actgtcactc tgttcccgcc ctcctctgag gagctccaag      60

ccaacaaggc cacactagtg tgtctgatca gtgacttcta cccgggagct gtgacagtgg     120

cctggaaggc agatggcagc cccgtcaagg cgggagtgga gaccaccaaa ccctccaaac     180

agagcaacaa caagtacgcg gccagcagct acctgagcct gacgcccgag cagtggaagt     240

cccacagaag ctacagctgc caggtcacgc atgaagggag caccgtggag aagacagtgg     300

cccctacaga atgttca                                                    317

<210> 127
<211> 106
<212> PRT
<213> Homo sapiens

<400> 127
Gly Gln Pro Lys Ala Asn Pro Thr Val Thr Leu Phe Pro Pro Ser Ser
1               5                   10                  15

Glu Glu Leu Gln Ala Asn Lys Ala Thr Leu Val Cys Leu Ile Ser Asp
            20                  25                  30

Phe Tyr Pro Gly Ala Val Thr Val Ala Trp Lys Ala Asp Gly Ser Pro
        35                  40                  45

Val Lys Ala Gly Val Glu Thr Thr Lys Pro Ser Lys Gln Ser Asn Asn
    50                  55                  60

Lys Tyr Ala Ala Ser Ser Tyr Leu Ser Leu Thr Pro Glu Gln Trp Lys
65                  70                  75                  80

Ser His Arg Ser Tyr Ser Cys Gln Val Thr His Glu Gly Ser Thr Val
                85                  90                  95

Glu Lys Thr Val Ala Pro Thr Glu Cys Ser
            100                 105

<210> 128
<211> 1323
<212> DNA
<213> Homo sapiens

<400> 128

gaggtgcagc tggtggagtc tgggggaggc ttggtaaagc ctggggggtc ccttagactc 60

tcctgtgcag cctctggatt cactttcagt aacgcctgga tgacctgggt ccgccaggct 120

ccagggaagg ggctggagtg ggttggccgt attaagagca aaactgatgg tgggacaaca 180

gactacgctg cacccgtgaa aggcagattc accatctcaa gagatgattc aataaacaca 240

ctgtatctgc aaatgaacag cctgaaaacc gaggacacag ccgtgtatta ttgtaggacg 300

gggggctact ggggccaggg aaccctggtc accgtctcga gtgcctccac caagggccca 360

tcggtcttcc ccctggcgcc ctgctccagg agcacctccg agagcacagc cgccctgggc 420

tgcctggtca aggactactt ccccgaaccg gtgacggtgt cgtggaactc aggcgctctg 480

accagcggcg tgcacacctt cccagctgtc ctacagtcct caggactcta ctccctcagc 540

agcgtggtga ccgtgccctc cagcaacttc ggcacccaga cctacacctg caacgtagat 600

cacaagccca gcaacaccaa ggtggacaag acagttgagc gcaaatgttg tgtcgagtgc 660

ccaccgtgcc cagcaccacc tgtggcagga ccgtcagtct tcctcttccc cccaaaaccc 720

aaggacaccc tcatgatctc ccggacccct gaggtcacgt gcgtggtggt ggacgtgagc 780

cacgaagacc ccgaggtcca gttcaactgg tacgtggacg gcgtggaggt gcataatgcc 840

aagacaaagc cacgggagga gcagttcaac agcacgttcc gtgtggtcag cgtcctcacc 900

gttgtgcacc aggactggct gaacggcaag gagtacaagt gcaaggtctc caacaaaggc 960

ctcccagccc ccatcgagaa aaccatctcc aaaaccaaag ggcagccccg agaaccacag 1020

gtgtacaccc tgcccccatc ccgggaggag atgaccaaga accaggtcag cctgacctgc 1080

ctggtcaaag gcttctaccc cagcgacatc gccgtggagt gggagagcaa tgggcagccg 1140

gagaacaact acaagaccac acctcccatg ctggactccg acggctcctt cttcctctac 1200

agcaagctca ccgtggacaa gagcaggtgg cagcagggga acgtcttctc atgctccgtg 1260

atgcatgagg ctctgcacaa ccactacacg cagaagagcc tctccctgtc tccgggtaaa 1320

tga 1323

<210> 129
<211> 440
<212> PRT
<213> Homo sapiens

<400> 129
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asn Ala
            20                  25                  30

Trp Met Thr Trp Val Arg Gln Ala Pro Gly Lys Gly Leu Glu Trp Val
        35                  40                  45

Gly Arg Ile Lys Ser Lys Thr Asp Gly Gly Thr Thr Asp Tyr Ala Ala
    50                  55                  60

Pro Val Lys Gly Arg Phe Thr Ile Ser Arg Asp Asp Ser Ile Asn Thr
65                  70                  75                  80

Leu Tyr Leu Gln Met Asn Ser Leu Lys Thr Glu Asp Thr Ala Val Tyr
                85                  90                  95

Tyr Cys Arg Thr Gly Gly Tyr Trp Gly Gln Gly Thr Leu Val Thr Val
            100                 105                 110

Ser Ser Ala Ser Thr Lys Gly Pro Ser Val Phe Pro Leu Ala Pro Cys
        115                 120                 125

Ser Arg Ser Thr Ser Glu Ser Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Asn Phe Gly Thr
            180                 185                 190

Gln Thr Tyr Thr Cys Asn Val Asp His Lys Pro Ser Asn Thr Lys Val
        195                 200                 205

Asp Lys Thr Val Glu Arg Lys Cys Cys Val Glu Cys Pro Pro Cys Pro
    210                 215                 220

Ala Pro Pro Val Ala Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser His Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Phe Arg Val Val Ser Val Leu Thr Val Val His Gln

290                 295                 300

Asp Trp Leu Asn Gly Lys Glu Tyr Lys Cys Lys Val Ser Asn Lys Gly
305                 310                 315                 320

Leu Pro Ala Pro Ile Glu Lys Thr Ile Ser Lys Thr Lys Gly Gln Pro
                325                 330                 335

Arg Glu Pro Gln Val Tyr Thr Leu Pro Pro Ser Arg Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Met Leu Asp Ser Asp Gly Ser Phe Phe Leu Tyr
385                 390                 395                 400

Ser Lys Leu Thr Val Asp Lys Ser Arg Trp Gln Gln Gly Asn Val Phe
                405                 410                 415

Ser Cys Ser Val Met His Glu Ala Leu His Asn His Tyr Thr Gln Lys
            420                 425                 430

Ser Leu Ser Leu Ser Pro Gly Lys
        435                 440

<210> 130
<211> 645
<212> DNA
<213> Homo sapiens

<400> 130
caggctgtgg tgactcagga gccctcactg actgtgtccc caggagggac agtcactctc      60

acctgtggct ccagcactgg agctgtcacc agtggtcatt atccctactg gttccagcag     120

aagcctggcc aagcccccag gacactgatt tatgatacaa gcaagaaaca ttcctggaca     180

cctgcccggt tctcaggctc cctccttggg ggcaaagctg ccctgaccct ttcgggtgcg     240

cagcctgagg atgaggctga gtattactgc ttggtctccg atagtggtgc tcggatcttc     300

ggacctggga ccaaggtcac cgtcctaggt cagcccaagg ccaaccccac tgtcactctg     360

ttcccgccct cctctgagga gctccaagcc aacaaggcca cactagtgtg tctgatcagt     420

gacttctacc cgggagctgt gacagtggcc tggaaggcag atggcagccc cgtcaaggcg     480

ggagtggaga ccaccaaacc ctccaaacag agcaacaaca agtacgcggc cagcagctac     540

ctgagcctga cgcccgagca gtggaagtcc cacagaagct acagctgcca ggtcacgcat     600

gaagggagca ccgtggagaa gacagtggcc cctacagaat gttca                     645

<210> 131
<211> 215
<212> PRT
<213> Homo sapiens

<400> 131
Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

His Tyr Pro Tyr Trp Phe Gln Gln Lys Pro Gly Gln Ala Pro Arg Thr
        35                  40                  45

Leu Ile Tyr Asp Thr Ser Lys Lys His Ser Trp Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Leu Val Ser Asp Ser Gly
                85                  90                  95

Ala Arg Ile Phe Gly Pro Gly Thr Lys Val Thr Val Leu Gly Gln Pro
            100                 105                 110

Lys Ala Asn Pro Thr Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Gly Ser Pro Val Lys Ala
145                 150                 155                 160

Gly Val Glu Thr Thr Lys Pro Ser Lys Gln Ser Asn Asn Lys Tyr Ala
                165                 170                 175

Ala Ser Ser Tyr Leu Ser Leu Thr Pro Glu Gln Trp Lys Ser His Arg
            180                 185                 190

Ser Tyr Ser Cys Gln Val Thr His Glu Gly Ser Thr Val Glu Lys Thr
        195                 200                 205

Val Ala Pro Thr Glu Cys Ser
    210                 215

<210> 132
<211> 1344
<212> DNA
<213> Homo sapiens

<400> 132
caggttcagc tggtgcagtc tggacctgaa atgaagaagc ctggggcctc agtgaaggtc      60

tcctgtcagg cctctggtta cacctttacc attaatggaa ttagttgggt gcgacaggcc     120

cctggacaac ggcctgagtg gatggggtgg atcagcacta gtaatggtca gacaaacttt     180

gcacagaatt tccagggcag actcaccttg accacagaca catccacgac cacggccttc     240

atggagttga tgagcctcac atctgccgac acggccgtat actactgtgc gagaaatcca     300

tacggcaatt cgcgcagatt ctttgactac tggggccagg gaaccctggt caccgtctcg     360

agtgcctcca ccaagggccc atcggtcttc cccctggcgc cctgctccag gagcacctcc     420

gagagcacag ccgccctggg ctgcctggtc aaggactact tccccgaacc ggtgacggtg     480

tcgtggaact caggcgctct gaccagcggc gtgcacacct tcccagctgt cctacagtcc     540

tcaggactct actccctcag cagcgtggtg accgtgccct ccagcaactt cggcacccag     600

acctacacct gcaacgtaga tcacaagccc agcaacacca aggtggacaa gacagttgag     660

cgcaaatgtt gtgtcgagtg cccaccgtgc ccagcaccac ctgtggcagg accgtcagtc     720

ttcctcttcc ccccaaaacc caaggacacc ctcatgatct cccggacccc tgaggtcacg     780

tgcgtggtgg tggacgtgag ccacgaagac cccgaggtcc agttcaactg gtacgtggac     840

ggcgtggagg tgcataatgc caagacaaag ccacgggagg agcagttcaa cagcacgttc     900

cgtgtggtca gcgtcctcac cgttgtgcac caggactggc tgaacggcaa ggagtacaag     960

tgcaaggtct ccaacaaagg cctcccagcc cccatcgaga aaaccatctc caaaaccaaa    1020

gggcagcccc gagaaccaca ggtgtacacc ctgcccccat cccgggagga gatgaccaag    1080

aaccaggtca gcctgacctg cctggtcaaa ggcttctacc ccagcgacat cgccgtggag    1140

tgggagagca atgggcagcc ggagaacaac tacaagacca cacctcccat gctggactcc    1200

gacggctcct tcttcctcta cagcaagctc accgtggaca agagcaggtg gcagcagggg    1260

aacgtcttct catgctccgt gatgcatgag gctctgcaca accactacac gcagaagagc    1320

ctctccctgt ctccgggtaa atga                                           1344

<210> 133
<211> 447
<212> PRT
<213> Homo sapiens

<400> 133

<210> 134
<211> 642
<212> DNA
<213> Homo sapiens

<400> 134
gacatccaga tgacccagtc tccatcctcc ctgtctgcct ctgttggcga cagagtcatc      60

atcacttgcc ggacaagtca gaccatcacc aattacataa attggtatca gcaaaaacca     120

gggaaagccc cgcagctcct gatctatggt gtatccagtt tgcaaagtgg ggtcccatca     180

aggttcagtg gcggtggatc tgggacagaa ttcactctca ccatcagcag tctgcaacct     240

gaagactttg caacttatta ctgtcaacag agttataggg cccctcaaac attcggccaa 300

gggaccaagc tggaaatcaa acgaactgtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gt 642

<210> 135
<211> 214
<212> PRT
<213> Homo sapiens

<400> 135
Asp Ile Gln Met Thr Gln Ser Pro Ser Ser Leu Ser Ala Ser Val Gly
1               5                   10                  15

Asp Arg Val Ile Ile Thr Cys Arg Thr Ser Gln Thr Ile Thr Asn Tyr
            20                  25                  30

Ile Asn Trp Tyr Gln Gln Lys Pro Gly Lys Ala Pro Gln Leu Leu Ile
        35                  40                  45

Tyr Gly Val Ser Ser Leu Gln Ser Gly Val Pro Ser Arg Phe Ser Gly
    50                  55                  60

Gly Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Ser Tyr Arg Ala Pro Gln
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 136
<211> 1332
<212> DNA
<213> Homo sapiens

<400> 136
gaggtgcagc tggtggagtc tgggggaggc ttggtgcagc cgggggggtc tctgagactc      60

tcttgtgcag cctctggttt cgccttcagc agccacgaga tgcactgggt tcgtcaaggg     120

ataggacaca gtttggagtg ggtctcagct attggccttg ctggtgacac atattattca     180

gactccgtga agggccgctt caccatctcc agagaaaacg ccaagaacac tttgtatctt     240

gaaatgaaca atttgagagt cggcgacacg gctgtctatt actgtgtcaa agaaggggct     300

cattgggaat ttgactcctg gggccaggga accctggtca ccgtctcgag tgcctccacc     360

aagggcccat cggtcttccc cctggcgccc tgctccagga gcacctccga gagcacagcc     420

gccctgggct gcctggtcaa ggactacttc cccgaaccgg tgacggtgtc gtggaactca     480

ggcgctctga ccagcggcgt gcacaccttc ccagctgtcc tacagtcctc aggactctac     540

tccctcagca gcgtggtgac cgtgccctcc agcaacttcg gcacccagac ctacacctgc     600

aacgtagatc acaagcccag caacaccaag gtggacaaga cagttgagcg caaatgttgt     660

gtcgagtgcc caccgtgccc agcaccacct gtggcaggac cgtcagtctt cctcttcccc     720

ccaaaaccca aggacaccct catgatctcc cggacccctg aggtcacgtg cgtggtggtg     780

gacgtgagcc acgaagaccc cgaggtccag ttcaactggt acgtggacgg cgtggaggtg     840

cataatgcca agacaaagcc acgggaggag cagttcaaca gcacgttccg tgtggtcagc     900

gtcctcaccg ttgtgcacca ggactggctg aacggcaagg agtacaagtg caaggtctcc     960

aacaaaggcc tcccagcccc catcgagaaa accatctcca aaaccaaagg gcagccccga    1020

gaaccacagg tgtacaccct gcccccatcc cgggaggaga tgaccaagaa ccaggtcagc    1080

ctgacctgcc tggtcaaagg cttctacccc agcgacatcg ccgtggagtg ggagagcaat    1140

gggcagccgg agaacaacta caagaccaca cctcccatgc tggactccga cggctccttc    1200

ttcctctaca gcaagctcac cgtggacaag agcaggtggc agcaggggaa cgtcttctca    1260

tgctccgtga tgcatgaggc tctgcacaac cactacacgc agaagagcct ctccctgtct    1320

ccgggtaaat ga                                                          1332

<210> 137
<211> 443
<212> PRT
<213> Homo sapiens

<400> 137
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Ala Phe Ser Ser His
            20                  25                  30

Glu Met His Trp Val Arg Gln Gly Ile Gly His Ser Leu Glu Trp Val
        35                  40                  45

Ser Ala Ile Gly Leu Ala Gly Asp Thr Tyr Tyr Ser Asp Ser Val Lys
    50                  55                  60

Gly Arg Phe Thr Ile Ser Arg Glu Asn Ala Lys Asn Thr Leu Tyr Leu
65                  70                  75                  80

Glu Met Asn Asn Leu Arg Val Gly Asp Thr Ala Val Tyr Tyr Cys Val
                85                  90                  95

Lys Glu Gly Ala His Trp Glu Phe Asp Ser Trp Gly Gln Gly Thr Leu
            100                 105                 110

Val Thr Val Ser Ser Ala Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Asn
            180                 185                 190

Phe Gly Thr Gln Thr Tyr Thr Cys Asn Val Asp His Lys Pro Ser Asn
        195                 200                 205

Thr Lys Val Asp Lys Thr Val Glu Arg Lys Cys Cys Val Glu Cys Pro

210                 215                 220

Pro Cys Pro Ala Pro Pro Val Ala Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Phe Arg Val Val Ser Val Leu Thr Val
    290                 295                 300

Val His Gln Asp Trp Leu Asn Gly Lys Glu Tyr Lys Cys Lys Val Ser
305                 310                 315                 320

Asn Lys Gly Leu Pro Ala Pro Ile Glu Lys Thr Ile Ser Lys Thr Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Met Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys
        435                 440

<210> 138
<211> 645
<212> DNA
<213> Homo sapiens

<400> 138
cagtctgtgc tgacgcagcc gccctcagtg tctggggccc ctggacagag ggtcatcatc 60

tcctgcactg ggagcagctc caacctcggg gcaagtcttg atgtacactg gtaccagcgc 120

cttccaggga caggcccccg actcctcatc tatgctaaca acaatcggcc ctcaggggtc 180

cctgaccgat tctctggctc caagtctggc acctcagcct ccctggccat caccgggctc 240

caggctgaag atgagggtga ttattactgc cagtcctatg acggcagcct gagggtgttc 300

ggcggaggga ccaggctgac cgtcctaggt cagcccaagg ctgccccctc ggtcactctg 360

ttcccgccct cctctgagga gcttcaagcc aacaaggcca cactggtgtg tctcataagt 420

gacttctacc cgggagccgt gacagtggcc tggaaggcag atagcagccc cgtcaaggcg 480

ggagtggaga ccaccacacc ctccaaacaa agcaacaaca agtacgcggc cagcagctac 540

ctgagcctga cgcctgagca gtggaagtcc cacaaaagct acagctgcca ggtcacgcat 600

gaagggagca ccgtggagaa gacagtggcc cctacagaat gttca 645

<210> 139
<211> 215
<212> PRT
<213> Homo sapiens

<400> 139
Gln Ser Val Leu Thr Gln Pro Pro Ser Val Ser Gly Ala Pro Gly Gln
1               5                   10                  15

Arg Val Ile Ile Ser Cys Thr Gly Ser Ser Ser Asn Leu Gly Ala Ser
            20                  25                  30

Leu Asp Val His Trp Tyr Gln Arg Leu Pro Gly Thr Gly Pro Arg Leu
        35                  40                  45

Leu Ile Tyr Ala Asn Asn Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Ala Glu Asp Glu Gly Asp Tyr Tyr Cys Gln Ser Tyr Asp Gly Ser
                85                  90                  95

Leu Arg Val Phe Gly Gly Gly Thr Arg Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
    130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala
                165                 170                 175

Ala Ser Ser Tyr Leu Ser Leu Thr Pro Glu Gln Trp Lys Ser His Lys
            180                 185                 190

Ser Tyr Ser Cys Gln Val Thr His Glu Gly Ser Thr Val Glu Lys Thr
        195                 200                 205

Val Ala Pro Thr Glu Cys Ser
    210                 215

<210> 140
<211> 1359
<212> DNA
<213> Homo sapiens

<400> 140
caggtgcagc tggtggagtc tgggggaggc ttggtcaagc ctggagggtc cctgagactg      60

tcctgtgcag cctctggatt caccttcagt gactactaca tgagctggat ccggcaggct     120

ccagggaagg ggctggagtg gatttcatac attagtagta gtggtaggac cataaagtac     180

gcagactctg tgaagggccg attcaccatc tccagggaca acgccaagaa ctcactttat     240

ctgcaaatga acagcctgag agccgaggac acggccgtgt attattgtgc gagagatgga     300

ggatattgtg gaagtgcaaa ctgccttcat gacgcttttg atatctgggg ccaagggaca     360

atggtcaccg tctcgagtgc ctccaccaag ggcccatcgg tcttccccct ggcgccctgc     420

tccaggagca cctccgagag cacagccgcc ctgggctgcc tggtcaagga ctacttcccc     480

gaaccggtga cggtgtcgtg gaactcaggc gctctgacca gcggcgtgca caccttccca     540

gctgtcctac agtcctcagg actctactcc ctcagcagcg tggtgaccgt gccctccagc     600

aacttcggca cccagaccta cacctgcaac gtagatcaca agcccagcaa caccaaggtg     660

gacaagacag ttgagcgcaa atgttgtgtc gagtgcccac cgtgcccagc accacctgtg     720

gcaggaccgt cagtcttcct cttcccccca aaacccaagg acaccctcat gatctcccgg     780

acccctgagg tcacgtgcgt ggtggtggac gtgagccacg aagaccccga ggtccagttc     840

aactggtacg tggacggcgt ggaggtgcat aatgccaaga caaagccacg ggaggagcag     900

ttcaacagca cgttccgtgt ggtcagcgtc ctcaccgttg tgcaccagga ctggctgaac     960

ggcaaggagt acaagtgcaa ggtctccaac aaaggcctcc cagcccccat cgagaaaacc    1020

atctccaaaa ccaaagggca gccccgagaa ccacaggtgt acaccctgcc cccatcccgg 1080

gaggagatga ccaagaacca ggtcagcctg acctgcctgg tcaaaggctt ctaccccagc 1140

gacatcgccg tggagtggga gagcaatggg cagccggaga acaactacaa gaccacacct 1200

cccatgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaatga 1359

<210> 141
<211> 452
<212> PRT
<213> Homo sapiens

<400> 141
Gln Val Gln Leu Val Glu Ser Gly Gly Gly Leu Phe Lys Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Ile Arg Gln Ala Pro Gly Lys Gly Leu Glu Trp Ile
        35                  40                  45

Ser Tyr Ile Ser Ser Ser Gly Arg Thr Ile Lys Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Asp Gly Gly Tyr Cys Gly Ser Ala Asn Cys Leu His Asp Ala
            100                 105                 110

Phe Asp Ile Trp Gly Gln Gly Thr Met Val Thr Val Ser Ser Ala Ser
        115                 120                 125

Thr Lys Gly Pro Ser Val Phe Pro Leu Ala Pro Cys Ser Arg Ser Thr
    130                 135                 140

Ser Glu Ser Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Asn Phe Gly Thr Gln Thr Tyr Thr
        195                 200                 205

Cys Asn Val Asp His Lys Pro Ser Asn Thr Lys Val Asp Lys Thr Val
    210                 215                 220

Glu Arg Lys Cys Cys Val Glu Cys Pro Pro Cys Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Gln Phe Asn Trp Tyr Val Asp Gly Val Glu
        275                 280                 285

Val His Asn Ala Lys Thr Lys Pro Arg Glu Glu Gln Phe Asn Ser Thr
    290                 295                 300

Phe Arg Val Val Ser Val Leu Thr Val Val His Gln Asp Trp Leu Asn
305                 310                 315                 320

Gly Lys Glu Tyr Lys Cys Lys Val Ser Asn Lys Gly Leu Pro Ala Pro
                325                 330                 335

Ile Glu Lys Thr Ile Ser Lys Thr Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Met Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210> 142
<211> 639
<212> DNA
<213> Homo sapiens

<400> 142
gacatccaga tgacccagtc tccttccacc ctgtctgcat ctgtaggaga cagagtcacc      60

atcacttgcc gggccagtca gagtattagt agctggttgg cctggtatca gcagaaacca     120

gggaaagccc ctaagctcct gatctataag gcgtctagtt tagaaagtgg ggtcccatca     180

aggttcagcg gcagtggatc tgggacagaa ttcactctca ccatcagcag cctgcagcct     240

gatgattttg caacttatta ctgccaacag tataatagtt attcgacgtt cggccaaggg     300

accaaggtgg aaatcaaacg aactgtggct gcaccatctg tcttcatctt cccgccatct     360

gatgagcagt tgaaatctgg aactgcctct gttgtgtgcc tgctgaataa cttctatccc     420

agagaggcca aagtacagtg gaaggtggat aacgccctcc aatcgggtaa ctcccaggag     480

agtgtcacag agcaggacag caaggacagc acctacagcc tcagcagcac cctgacgctg     540

agcaaagcag actacgagaa acacaaagtc tacgcctgcg aagtcaccca tcagggcctg     600

agctcgcccg tcacaaagag cttcaacagg ggagagtgt                            639

<210> 143
<211> 213
<212> PRT
<213> Homo sapiens

<400> 143
Asp Ile Gln Met Thr Gln Ser Pro Ser Thr Leu Ser Ala Ser Val Gly
1               5                   10                  15

Asp Arg Val Thr Ile Thr Cys Arg Ala Ser Gln Ser Ile Ser Ser Trp
            20                  25                  30

Leu Ala Trp Tyr Gln Gln Lys Pro Gly Lys Ala Pro Lys Leu Leu Ile
        35                  40                  45

Tyr Lys Ala Ser Ser Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ser Tyr Ser Thr
                85                  90                  95

Phe Gly Gln Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210> 144
<211> 1365
<212> DNA
<213> Homo sapiens

<400> 144
caggtcacct tgaaggagtc tggtcccacg ctggtgaaac ccacacagac cctcacgctg      60

acgtgcacct tctctgggtt ttcgctcagc actagtggag agggtgtggc ctggatccgt     120

caggccccag gaaaggccct ggagtggctt gcactcattt attgggatga tgataagcgc     180

tacaacccat ctctgaagag caggctcacc atcaccaagg acaactccag aaaccaagtg     240

gtccttacaa tgaccaacat ggaccctgtg gacacagcca catattactg tgtacacagg     300

gggatgaata gggtttttgg ggtagtttat aattcgggct ggttcgaccc ctggggccag     360

ggaaccctgg tcaccgtctc gagtgcctcc accaagggcc catcggtctt ccccctggcg     420

ccctgctcca ggagcacctc cgagagcaca gccgccctgg gctgcctggt caaggactac     480

ttccccgaac cggtgacggt gtcgtggaac tcaggcgctc tgaccagcgg cgtgcacacc     540

ttcccagctg tcctacagtc ctcaggactc tactccctca gcagcgtggt gaccgtgccc     600

tccagcaact tcggcaccca gacctacacc tgcaacgtag atcacaagcc cagcaacacc 660

aaggtggaca agacagttga gcgcaaatgt tgtgtcgagt gcccaccgtg cccagcacca 720

cctgtggcag gaccgtcagt cttcctcttc cccccaaaac ccaaggacac cctcatgatc 780

tcccggaccc ctgaggtcac gtgcgtggtg gtggacgtga gccacgaaga ccccgaggtc 840

cagttcaact ggtacgtgga cggcgtggag gtgcataatg ccaagacaaa gccacgggag 900

gagcagttca acagcacgtt ccgtgtggtc agcgtcctca ccgttgtgca ccaggactgg 960

ctgaacggca aggagtacaa gtgcaaggtc tccaacaaag gcctcccagc ccccatcgag 1020

aaaaccatct ccaaaaccaa agggcagccc cgagaaccac aggtgtacac cctgccccca 1080

tcccgggagg agatgaccaa gaaccaggtc agcctgacct gcctggtcaa aggcttctac 1140

cccagcgaca tcgccgtgga gtgggagagc aatgggcagc cggagaacaa ctacaagacc 1200

acacctccca tgctggactc cgacggctcc ttcttcctct acagcaagct caccgtggac 1260

aagagcaggt ggcagcaggg gaacgtcttc tcatgctccg tgatgcatga ggctctgcac 1320

aaccactaca cgcagaagag cctctccctg tctccgggta aatga 1365

<210> 145
<211> 454
<212> PRT
<213> Homo sapiens

<400> 145
Gln Val Thr Leu Lys Glu Ser Gly Pro Thr Leu Val Lys Pro Thr Gln
1               5                   10                  15

Thr Leu Thr Leu Thr Cys Thr Phe Ser Gly Phe Ser Leu Ser Thr Ser
            20                  25                  30

Gly Glu Gly Val Ala Trp Ile Arg Gln Ala Pro Gly Lys Ala Leu Glu
        35                  40                  45

Trp Leu Ala Leu Ile Tyr Trp Asp Asp Asp Lys Arg Tyr Asn Pro Ser
    50                  55                  60

Leu Lys Ser Arg Leu Thr Ile Thr Lys Asp Asn Ser Arg Asn Gln Val
65                  70                  75                  80

Val Leu Thr Met Thr Asn Met Asp Pro Val Asp Thr Ala Thr Tyr Tyr
                85                  90                  95

Cys Val His Arg Gly Met Asn Arg Val Phe Gly Val Val Tyr Asn Ser
            100                 105                 110

Gly Trp Phe Asp Pro Trp Gly Gln Gly Thr Leu Val Thr Val Ser Ser

115                 120                 125

Ala Ser Thr Lys Gly Pro Ser Val Phe Pro Leu Ala Pro Cys Ser Arg
    130                 135                 140

Ser Thr Ser Glu Ser Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

Leu Ser Ser Val Val Thr Val Pro Ser Ser Asn Phe Gly Thr Gln Thr
        195                 200                 205

Tyr Thr Cys Asn Val Asp His Lys Pro Ser Asn Thr Lys Val Asp Lys
    210                 215                 220

Thr Val Glu Arg Lys Cys Cys Val Glu Cys Pro Pro Cys Pro Ala Pro
225                 230                 235                 240

Pro Val Ala Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Gln Phe Asn Trp Tyr Val Asp Gly
        275                 280                 285

Val Glu Val His Asn Ala Lys Thr Lys Pro Arg Glu Glu Gln Phe Asn
    290                 295                 300

Ser Thr Phe Arg Val Val Ser Val Leu Thr Val Val His Gln Asp Trp
305                 310                 315                 320

Leu Asn Gly Lys Glu Tyr Lys Cys Lys Val Ser Asn Lys Gly Leu Pro
                325                 330                 335

Ala Pro Ile Glu Lys Thr Ile Ser Lys Thr Lys Gly Gln Pro Arg Glu
            340                 345                 350

Pro Gln Val Tyr Thr Leu Pro Pro Ser Arg Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Met Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Asn His Tyr Thr Gln Lys Ser Leu
        435                 440                 445

Ser Leu Ser Pro Gly Lys
    450

<210> 146
<211> 645
<212> DNA
<213> Homo sapiens

<400> 146
gaaattgtgt tgacgcagtc tccaggcacc ctgtctttgt ctccagggga aagagccacc      60

ctctcctgta gggccagtca gagcgttaga agcagccagc tagcctggta ccagcagaag     120

cctggccagg ctcccagcct cctcatgtat gacgcatcta agagggccac tggcatccca     180

gacaggttca gtggcagtgg gtctgggaca gacttcactc tcaccatcag cagactggag     240

cctgaagatt ttgtagtgta tttctgtcag cagtatgata catcaccgtg gacgttcggc     300

caagggacca aggtggaaat caaacgaact gtggctgcac catctgtctt catcttcccg     360

ccatctgatg agcagttgaa atctggaact gcctctgttg tgtgcctgct gaataacttc     420

tatcccagag aggccaaagt acagtggaag gtggataacg ccctccaatc gggtaactcc     480

caggagagtg tcacagagca ggacagcaag gacagcacct acagcctcag cagcaccctg     540

acgctgagca aagcagacta cgagaaacac aaagtctacg cctgcgaagt cacccatcag     600

ggcctgagct cgcccgtcac aaagagcttc aacaggggag agtgt                     645

<210> 147
<211> 215
<212> PRT
<213> Homo sapiens

<400> 147
Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Arg Ser Ser
            20                  25                  30

Gln Leu Ala Trp Tyr Gln Gln Lys Pro Gly Gln Ala Pro Ser Leu Leu
        35                  40                  45

Met Tyr Asp Ala Ser Lys Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Val Val Tyr Phe Cys Gln Gln Tyr Asp Thr Ser Pro
                85                  90                  95

Trp Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 148
<211> 376
<212> DNA
<213> Homo sapiens

<400> 148
cagatcacct tgaaggagtc tggtcctacg ctggtgaacc ccacacagac cctcacgctg        60

acctgcacct tttctgggtt ctcattcacc tctagtggag aggctgtggg ctggatccgt       120

cagcccccag gaaaggccct ggaatggctt gctcacattt attgggatga tgatccgcgt       180

tatagtacat ctctgaagag cagactcacc atcaccaagg acacctccaa aaatcaggtg 240

gtcctctcta tgaccgacat tgaccctgcg gacacaggca catattactg tgcacacctg 300

agacgcccat ccctttggag tccttattat ctttttgact gctggggcca gggaaccctg 360

gtcaccgtct cgagtg 376

<210> 149
<211> 125
<212> PRT
<213> Homo sapiens

<400> 149
Gln Ile Thr Leu Lys Glu Ser Gly Pro Thr Leu Val Asn Pro Thr Gln
1               5                   10                  15

Thr Leu Thr Leu Thr Cys Thr Phe Ser Gly Phe Ser Phe Thr Ser Ser
            20                  25                  30

Gly Glu Ala Val Gly Trp Ile Arg Gln Pro Pro Gly Lys Ala Leu Glu
        35                  40                  45

Trp Leu Ala His Ile Tyr Trp Asp Asp Asp Pro Arg Tyr Ser Thr Ser
    50                  55                  60

Leu Lys Ser Arg Leu Thr Ile Thr Lys Asp Thr Ser Lys Asn Gln Val
65                  70                  75                  80

Val Leu Ser Met Thr Asp Ile Asp Pro Ala Asp Thr Gly Thr Tyr Tyr
                85                  90                  95

Cys Ala His Leu Arg Arg Pro Ser Leu Trp Ser Pro Tyr Tyr Leu Phe
            100                 105                 110

Asp Cys Trp Gly Gln Gly Thr Leu Val Thr Val Ser Ser
        115                 120                 125

<210> 150
<211> 319
<212> DNA
<213> Homo sapiens

<400> 150
gacatccaga tgacccagtc tccatcctcc ctgtctgcat ctgttggaga cagagtcacc 60

atcacttgcc gggcaagtca gagtattagt agctatttaa attggtatca actgaaacca 120

ggacaagccc ctaagctcct gatctatggt gcatccagtt tggaaaatgg ggtcccttca 180

cggttcagtg gcagtggctc tgggccagat ttcactctca ccattagcag tctgcaacct 240

gaagattttg caacttacta ctgtcaacag agttacaaaa tgtggtcatt cggccaaggg 300

accaaggtgg aaatccaac                                                319

<210> 151
<211> 106
<212> PRT
<213> Homo sapiens

<400> 151
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Leu Lys Pro Gly Gln Ala Pro Lys Leu Leu Ile
        35                  40                  45

Tyr Gly Ala Ser Ser Leu Glu Asn Gly Val Pro Ser Arg Phe Ser Gly
    50                  55                  60

Ser Gly Ser Gly Pro Asp Phe Thr Leu Thr Ile Ser Ser Leu Gln Pro
65                  70                  75                  80

Glu Asp Phe Ala Thr Tyr Tyr Cys Gln Gln Ser Tyr Lys Met Trp Ser
                85                  90                  95

Phe Gly Gln Gly Thr Lys Val Glu Ile Gln
            100                 105

<210> 152
<211> 355
<212> DNA
<213> Homo sapiens

<400> 152
caggttcagc tggtgcagtc tggagttgag gtgaagaagc ctggggcctc agtgaaggtc      60

tcctgcaagg cttctggcta cacctttacc aactttggct tcagttgggt gcggcaggcc     120

ccaggacaag ggcttgagtg gctgggatgg atcaacactc ataacggtaa cacaaactat     180

gcaccgagat tccaagacag agtcaccctg accacagaca catccacgac cacagcctac     240

atggagctga ggagcctgag atctgaggac acggccgtgt attattgtgc gagagatctt     300

gggggaagta gttccgccta ctggggacag ggaaccctgg tcaccgtctc gagtg          355

<210> 153
<211> 118
<212> PRT
<213> Homo sapiens

<400> 153

Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Phe
            20                  25                  30

Gly Phe Ser Trp Val Arg Gln Ala Pro Gly Gln Gly Leu Glu Trp Leu
        35                  40                  45

Gly Trp Ile Asn Thr His Asn Gly Asn Thr Asn Tyr Ala Pro Arg Phe
    50                  55                  60

Gln Asp Arg Val Thr Leu Thr Thr Asp Thr Ser Thr Thr Thr Ala Tyr
65                  70                  75                  80

Met Glu Leu Arg Ser Leu Arg Ser Glu Asp Thr Ala Val Tyr Tyr Cys
                85                  90                  95

Ala Arg Asp Leu Gly Gly Ser Ser Ser Ala Tyr Trp Gly Gln Gly Thr
            100                 105                 110

Leu Val Thr Val Ser Ser
        115

<210> 154
<211> 325
<212> DNA
<213> Homo sapiens

<400> 154
gaaattgtgt tgacgcagtc tccaggcacc ctgtctttgt ctccagggga gagagccacc      60

ctctcttgca gggccagtca cagtggtacc aacaactaca tcgcctggta ccagcagaaa     120

cctggccagc ctcccaggct cctcatctat ggtgcatcca gcagggcccc tggcatctca     180

gagaggttca gcggcagtgg gtctgggaca gacttcactc tcatcatcag cagactggag     240

cctgaagact ttggagtata ttactgtgag cagtatggga actcacctct gacgttcggc     300

caagggacca aggtggaaat caaac                                           325

<210> 155
<211> 108
<212> PRT
<213> Homo sapiens

<400> 155
Glu Ile Val Leu Thr Gln Ser Pro Gly Thr Leu Ser Leu Ser Pro Gly
1               5                   10                  15

Glu Arg Ala Thr Leu Ser Cys Arg Ala Ser His Ser Gly Thr Asn Asn
            20                  25                  30

Tyr Ile Ala Trp Tyr Gln Gln Lys Pro Gly Gln Pro Pro Arg Leu Leu
        35                  40                  45

Ile Tyr Gly Ala Ser Ser Arg Ala Pro Gly Ile Ser Glu Arg Phe Ser
    50                  55                  60

Gly Ser Gly Ser Gly Thr Asp Phe Thr Leu Ile Ile Ser Arg Leu Glu
65                  70                  75                  80

Pro Glu Asp Phe Gly Val Tyr Tyr Cys Glu Gln Tyr Gly Asn Ser Pro
                85                  90                  95

Leu Thr Phe Gly Gln Gly Thr Lys Val Glu Ile Lys
            100                 105

<210> 156
<211> 376
<212> DNA
<213> Homo sapiens

<400> 156
caggtgcagc tgcaggagtc gggcccagga ctggtggagt cttcggacgt cctgtccctc      60

acctgcactg tctctggtgg ctccatcagt aatgactatt gggcctggat ccggcagacc     120

ccagggaagg gactggagtg gattgggtct gtctattaca gtggcagaac cagtttcagc     180

ccctccctca agagtcgagt cactatgtca gtggacacga ccaagaacca attcttcctg     240

aacctgggct ctctgaccgt tgcggacacg gccgtgtatt actgtgcgag aactacaagg     300

ttgtcctttg actggttagt acactactat ggaatggacg tctggggcca ggggaccacg     360

gtcaccgtct cgagtg                                                     376

<210> 157
<211> 125
<212> PRT
<213> Homo sapiens

<400> 157
Gln Val Gln Leu Gln Glu Ser Gly Pro Gly Leu Val Glu Ser Ser Asp
1               5                   10                  15

Val Leu Ser Leu Thr Cys Thr Val Ser Gly Gly Ser Ile Ser Asn Asp
            20                  25                  30

Tyr Trp Ala Trp Ile Arg Gln Thr Pro Gly Lys Gly Leu Glu Trp Ile
        35                  40                  45

Gly Ser Val Tyr Tyr Ser Gly Arg Thr Ser Phe Ser Pro Ser Leu Lys
    50                  55                  60

Ser Arg Val Thr Met Ser Val Asp Thr Thr Lys Asn Gln Phe Phe Leu
65                  70                  75                  80

Asn Leu Gly Ser Leu Thr Val Ala Asp Thr Ala Val Tyr Tyr Cys Ala
                85                  90                  95

Arg Thr Thr Arg Leu Ser Phe Asp Trp Leu Val His Tyr Tyr Gly Met
            100                 105                 110

Asp Val Trp Gly Gln Gly Thr Thr Val Thr Val Ser Ser
        115                 120                 125

<210> 158
<211> 385
<212> DNA
<213> Homo sapiens

<400> 158
gacatccaga tgacccagtc tccatcgtcc ctgactgcat ctgtaggaga cagagtcacc      60

attatttgtc gggcaagtca gcccattggc agctatttga attggtatca gcagagtcca     120

gggaaagccc ctaaactcct catctatgct gcatccactt tgcagagtgg ggtcccatca     180

aggttcagtg gcagtggatc tgggacagat ttcactctca acatcagtgg tctgcaacct     240

gaagattttg caacttacta ctgtcaacag agttacaata accctttgtt caccttcggc     300

cctgggacca aagtggatat caaacgaact gtggctgcac catctgtctt catcttcccg     360

ccatctgatg agcagttgaa atctg                                           385

<210> 159
<211> 108
<212> PRT
<213> Homo sapiens

<400> 159
Asp Ile Gln Met Thr Gln Ser Pro Ser Ser Leu Thr Ala Ser Val Gly
1               5                   10                  15

Asp Arg Val Thr Ile Ile Cys Arg Ala Ser Gln Pro Ile Gly Ser Tyr
            20                  25                  30

Leu Asn Trp Tyr Gln Gln Ser Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Asn Ile Ser Gly Leu Gln Pro
65                  70                  75                  80

Glu Asp Phe Ala Thr Tyr Tyr Cys Gln Gln Ser Tyr Asn Asn Pro Leu
                85                  90                  95

Phe Thr Phe Gly Pro Gly Thr Lys Val Asp Ile Lys
            100                 105

<210> 160
<211> 373
<212> DNA
<213> Homo sapiens

<400> 160
gaggtgcagc tggtggagtc tgggggaagt ctggtacggc cgggggggtc cctgcgactc 60

tcctgtgcag cctctggatt catctttgat gattatggca tgagttgggt ccgccaagtt 120

ccaggggagg gcctggagtg ggtcgccggc attacttggg atggcgtgaa cacagattat 180

gcagactccg tgaagggccg cttcaccatc tccagagaca acgccaagaa ctccctgttt 240

ctcctaatga acagtctcag agccgaggac acggccctgt atcactgtgc gagagccaga 300

tactaccaag gcactggcct ccggtcctac ctcgacgagt ggggccaggg aaccctggtc 360

accgtctcga gtg 373

<210> 161
<211> 124
<212> PRT
<213> Homo sapiens

<400> 161
Glu Val Gln Leu Val Glu Ser Gly Gly Ser Leu Val Arg Pro Gly Gly
1               5                   10                  15

Ser Leu Arg Leu Ser Cys Ala Ala Ser Gly Phe Ile Phe Asp Asp Tyr
            20                  25                  30

Gly Met Ser Trp Val Arg Gln Val Pro Gly Glu Gly Leu Glu Trp Val
        35                  40                  45

Ala Gly Ile Thr Trp Asp Gly Val Asn Thr Asp Tyr Ala Asp Ser Val
    50                  55                  60

Lys Gly Arg Phe Thr Ile Ser Arg Asp Asn Ala Lys Asn Ser Leu Phe
65                  70                  75                  80

Leu Leu Met Asn Ser Leu Arg Ala Glu Asp Thr Ala Leu Tyr His Cys
                85                  90                  95

Ala Arg Ala Arg Tyr Tyr Gln Gly Thr Gly Leu Arg Ser Tyr Leu Asp
            100                 105                 110

Glu Trp Gly Gln Gly Thr Leu Val Thr Val Ser Ser
        115                 120

<210> 162
<211> 383
<212> DNA
<213> Homo sapiens

<400> 162
gacatccaga tgacccagtc tccatcctcc gtgtctgcat ctgtaggaga aagagtcact        60

atcacttgtc gggcgactca gggtattgac aactggttgg cctggtatca gcataaacca       120

gggaaagccc cgaagctcct gatttatgct gcatccactt tgcaaagtgg ggtcccgtcc       180

aggttcagcg gcagtggatc tgggacagat ttcactctca ccatcagcag cgtgcagcct       240

gaagatgttg caacttactt ttgtcaacag gctaagaatt tccctcgggg aggattcact       300

ttcggccctg ggaccaaagt cgctctcaaa cgaactgtgg ctgcaccatc tgtcttcatc       360

ttcccgccat ctgatgagca gtt                                               383

<210> 163
<211> 110
<212> PRT
<213> Homo sapiens

<400> 163
Asp Ile Gln Met Thr Gln Ser Pro Ser Ser Val Ser Ala Ser Val Gly
1               5                   10                  15

Glu Arg Val Thr Ile Thr Cys Arg Ala Thr Gln Gly Ile Asp Asn Trp
            20                  25                  30

Leu Ala Trp Tyr Gln His Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Val Gln Pro
65                  70                  75                  80

Glu Asp Val Ala Thr Tyr Phe Cys Gln Gln Ala Lys Asn Phe Pro Arg
                85                  90                  95

Gly Gly Phe Thr Phe Gly Pro Gly Thr Lys Val Ala Leu Lys
            100                 105                 110

<210> 164
<211> 355

<212> DNA
<213> Homo sapiens

<400> 164
caggtgcagc tggtggagtc tgggggaggc gtggtccagc ctgggaggtc cctgagactc      60

tcctgtgcag cctctggatt caccttcagt aaatttgcca tgcactgggt ccgccaggct     120

ccaggcaagg ggctggagtg ggtggcagct atagcgtctg atggatataa taaatactat     180

gcagactccg tgaagggcca attcaccgtc tccagagaca cttccaggaa cacgctgtat     240

ctccaaatgg acagcctgag acctgatgac acggctattt attactgtgc gtacggtcac     300

ttgtcttttt ggttcgaccc ctggggccag ggaaccctgg tcaccgtctc gagtg          355


<210> 165
<211> 118
<212> PRT
<213> Homo sapiens

<400> 165
Gln Val Gln Leu Val Glu Ser Gly Gly Gly Val Val Gln Pro Gly Arg
1               5                   10                  15


Ser Leu Arg Leu Ser Cys Ala Ala Ser Gly Phe Thr Phe Ser Lys Phe
            20                  25                  30


Ala Met His Trp Val Arg Gln Ala Pro Gly Lys Gly Leu Glu Trp Val
        35                  40                  45


Ala Ala Ile Ala Ser Asp Gly Tyr Asn Lys Tyr Tyr Ala Asp Ser Val
    50                  55                  60


Lys Gly Gln Phe Thr Val Ser Arg Asp Thr Ser Arg Asn Thr Leu Tyr
65                  70                  75                  80


Leu Gln Met Asp Ser Leu Arg Pro Asp Asp Thr Ala Ile Tyr Tyr Cys
                85                  90                  95


Ala Tyr Gly His Leu Ser Phe Trp Phe Asp Pro Trp Gly Gln Gly Thr
            100                 105                 110


Leu Val Thr Val Ser Ser
        115


<210> 166
<211> 382
<212> DNA
<213> Homo sapiens

<400> 166
gacatccaga tgacccagtc tccatcctcc ctgtccgcat ctgtaggaga cagagtcacc      60

atcacttgcc gggcaagtca ggacattgga aatggcttag gctggtatca acagatacca 120

gggaaagccc ctaagcgcct gatctatgct gcatcctctt tgcaaagtgg ggtcccatca 180

aggttcagcg gcagtggttc tgggacagag ttcactctca caatccacag cctgcagcct 240

gaagattttg caacttatta ctgtctacag cataattttt accctcgcac ttttggccag 300

gggaccaagc tggagatcaa gcgaactgtg gctgcaccat ctgtcttcat cttcccgcca 360

tctgatgagc agttgaaatc tg 382

<210> 167
<211> 107
<212> PRT
<213> Homo sapiens

<400> 167
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Asn Gly
            20                  25                  30

Leu Gly Trp Tyr Gln Gln Ile Pro Gly Lys Ala Pro Lys Arg Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Glu Phe Thr Leu Thr Ile His Ser Leu Gln Pro
65                  70                  75                  80

Glu Asp Phe Ala Thr Tyr Tyr Cys Leu Gln His Asn Phe Tyr Pro Arg
                85                  90                  95

Thr Phe Gly Gln Gly Thr Lys Leu Glu Ile Lys
            100                 105

<210> 168
<211> 367
<212> DNA
<213> Homo sapiens

<400> 168
caggtgcagc tggtgcagtc tggggctgag gtgaagaagc ctggggcctc agtgaaggtc 60

tcctgcaagg cttctggata caccttcacc gactactata tacactgggt gcgacaggcc 120

cctggacaag ggcttgagtg gatgggatgg atcaaccctt atagtggtgg cacaaactat 180

gcaccgaagt ttcagggcag gttcaccatg gccagggaca cgtccatcac cacaacctac 240

ttggacctgg gcaggctgac gtctagcgac acggccattt atttctgtgc gagagatttc 300

ttcagagcag cagctggcac ggccggagac tactggggcc agggaaccct ggtcaccgtc 360

tcgagtg 367

<210> 169
<211> 122
<212> PRT
<213> Homo sapiens

<400> 169
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Ile His Trp Val Arg Gln Ala Pro Gly Gln Gly Leu Glu Trp Met
        35                  40                  45

Gly Trp Ile Asn Pro Tyr Ser Gly Gly Thr Asn Tyr Ala Pro Lys Phe
    50                  55                  60

Gln Gly Arg Phe Thr Met Ala Arg Asp Thr Ser Ile Thr Thr Thr Tyr
65                  70                  75                  80

Leu Asp Leu Gly Arg Leu Thr Ser Ser Asp Thr Ala Ile Tyr Phe Cys
                85                  90                  95

Ala Arg Asp Phe Phe Arg Ala Ala Ala Gly Thr Ala Gly Asp Tyr Trp
            100                 105                 110

Gly Gln Gly Thr Leu Val Thr Val Ser Ser
        115                 120

<210> 170
<211> 397
<212> DNA
<213> Homo sapiens

<400> 170
gacatcgtga tgacccagtc tccagaagac tccctggctg tgtctctggg cgagagggcc 60

accatcaact gctccagcca gagtgcttta tacagcccca gcagtaagac ctacttagct 120

tggtaccagc agagaccggg acaccctcct aagcttctca tttactgggc atctacccgg 180

gtatccgggg tccctgaccg attcagtggc agcgggtctg ggacagattt cactctcacc 240

atcggcagcc tgcaggctga agatgtggca gtttattact gtcagtacta ttatggtact 300

gtcactttcg gcggagggac caaggtggag atcaaacgaa ctgtggctgc accatctgtc 360

ttcatcttcc cgccatctga tgagcagttg aaatctg 397

<210> 171
<211> 112
<212> PRT
<213> Homo sapiens

<400> 171
Ala Asp Ile Val Met Thr Gln Ser Pro Asp Ser Leu Ala Val Ser Leu
1               5                   10                  15

Gly Glu Arg Ala Thr Ile Asn Cys Ser Ser Gln Ser Ala Leu Tyr Ser
            20                  25                  30

Pro Ser Ser Lys Thr Tyr Leu Ala Trp Tyr Gln Gln Arg Pro Gly His
        35                  40                  45

Pro Pro Lys Leu Leu Ile Tyr Trp Ala Ser Thr Arg Val Ser Gly Val
    50                  55                  60

Pro Asp Arg Phe Ser Gly Ser Gly Ser Gly Thr Asp Phe Thr Leu Thr
65                  70                  75                  80

Ile Gly Ser Leu Gln Ala Glu Asp Val Ala Val Tyr Tyr Cys Gln Tyr
                85                  90                  95

Tyr Tyr Gly Thr Val Thr Phe Gly Gly Gly Thr Lys Val Glu Ile Lys
            100                 105                 110

<210> 172
<211> 376
<212> DNA
<213> Homo sapiens

<400> 172
caggtgcagc tgcaggagtc gggcccaaga ctggtgaagc cttcggagac cctgtccctc      60

acctgcactg tctctggtgg gtccatcaat agttattatt ggagttggat ccggcagccc     120

gtcgggaagg gactggagtg gattgggcgt atgtatcccg gtggtacctc cagttacaat     180

tcatccctca agagtcgagt catcatgtcg gtagacacgt cgaagaacca gttttctctg     240

aacctcactt ctgtgaccgc cgcagacacg gccgtatatt tctgtgcgag agatccaaca     300

tatcaccatg gcactagtgg ttatgtgggg acttttgatc tctggggcca agggacaatg     360

gtcaccgtct cgagtg                                                     376

<210> 173
<211> 125
<212> PRT
<213> Homo sapiens

<400> 173
Gln Val Gln Leu Gln Glu Ser Gly Pro Arg Leu Val Lys Pro Ser Glu

1               5                   10                  15

Thr Leu Ser Leu Thr Cys Thr Val Ser Gly Gly Ser Ile Asn Ser Tyr
            20                  25                  30

Tyr Trp Ser Trp Ile Arg Gln Pro Val Gly Lys Gly Leu Glu Trp Ile
        35                  40                  45

Gly Arg Met Tyr Pro Gly Gly Thr Ser Ser Tyr Asn Ser Ser Leu Lys
    50                  55                  60

Ser Arg Val Ile Met Ser Val Asp Thr Ser Lys Asn Gln Phe Ser Leu
65                  70                  75                  80

Asn Leu Thr Ser Val Thr Ala Ala Asp Thr Ala Val Tyr Phe Cys Ala
                85                  90                  95

Arg Asp Pro Thr Tyr His His Gly Thr Ser Gly Tyr Val Gly Thr Phe
            100                 105                 110

Asp Leu Trp Gly Gln Gly Thr Met Val Thr Val Ser Ser
        115                 120                 125

<210> 174
<211> 385
<212> DNA
<213> Homo sapiens

<400> 174
gaaatagtga tgacgcagtc tccagccacc ctgtctgcgt ctccagggga aagagtcacc      60

ctctcctgca gggccagtcc gagtgttcgc aacaatttag cctggtacca gcagaaacct     120

ggccagcctc ctaggctcct catctatggt gcatccacca aggtcatggg tactccaacc     180

aggttcagtg gcagtgggtc tgggacagag ttcactctca ccatcagcag cctgcagtct     240

gaagattttg cagtttatta ctgtcagcag tataatagct ggcctccgta cacttttagc     300

caggggacca aactggagat caaacgaact gtggctgcac catctgtctt catcttcccg     360

ccatctgatg agcagttgaa atctg                                           385

<210> 175
<211> 108
<212> PRT
<213> Homo sapiens

<400> 175
Asp Ile Val Met Thr Gln Ser Pro Ala Thr Leu Ser Ala Ser Pro Gly
1               5                   10                  15

Glu Arg Val Thr Leu Ser Cys Arg Ala Ser Pro Ser Val Arg Asn Asn

20                  25                  30

Leu Ala Trp Tyr Gln Gln Lys Pro Gly Gln Pro Pro Arg Leu Leu Ile
        35                  40                  45

Tyr Gly Ala Ser Thr Lys Val Met Gly Thr Pro Thr Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Ser Trp Pro Pro
                85                  90                  95

Tyr Thr Phe Ser Gln Gly Thr Lys Leu Glu Ile Lys
            100                 105

<210> 176
<211> 346
<212> DNA
<213> Homo sapiens

<400> 176
gaggtgcagc tggtggagtc ggggggaggc ctggtcaggc ctggggagtc cctgagactc      60

tcctgtgcag gctctggatt caccttcagc atgtttaccc tgaactgggt ccgccaggct     120

ccagggaagg gactggagtg ggtcgcatct attagtggta gtagcagttt agtgtttcat     180

gaagactcag tgaagggccg attcaccatc tccagagaca acgccaagaa ctcagtatat     240

ctgcaaatga acagcctgag agccgaagac acggctattt attactgtgt gagaggagat     300

agtcgcgact attggggcca gggaaccctg gtcaccgtct cgagtg                    346

<210> 177
<211> 115
<212> PRT
<213> Homo sapiens

<400> 177
Glu Val Gln Leu Val Glu Ser Gly Gly Gly Leu Val Arg Pro Gly Glu
1               5                   10                  15

Ser Leu Arg Leu Ser Cys Ala Gly Ser Gly Phe Thr Phe Ser Met Phe
            20                  25                  30

Thr Leu Asn Trp Val Arg Gln Ala Pro Gly Lys Gly Leu Glu Trp Val
        35                  40                  45

Ala Ser Ile Ser Gly Ser Ser Ser Leu Val Phe His Glu Asp Ser Val
    50                  55                  60

Lys Gly Arg Phe Thr Ile Ser Arg Asp Asn Ala Lys Asn Ser Val Tyr
65                  70                  75                  80

Leu Gln Met Asn Ser Leu Arg Ala Glu Asp Thr Ala Ile Tyr Tyr Cys
                85                  90                  95

Val Arg Gly Asp Ser Arg Asp Tyr Trp Gly Gln Gly Thr Leu Val Thr
            100                 105                 110

Val Ser Ser
        115

<210> 178
<211> 331
<212> DNA
<213> Homo sapiens

<400> 178
cagtctgtgt tgacgcagcc gccctcagtg tctgcggccc caggacagaa ggtcaccatc      60

tcctgctctg gaaggacctc caatattgga aataaccatg tatcctggta ccagcaattc     120

ccaggaacag cccccaaagt cctcatttat cgcaatgata ggcgaccctc agggattcct     180

gatcgattct ctggctccaa gtctgacacg tcagccaccc tgggcatcac cgggctccag     240

actggggacg aggccgatta ttactgcgga acatgggaca acagcctgag tggttgggtg     300

ttcggcggag ggaccaagct gaccgtccta g                                    331

<210> 179
<211> 110
<212> PRT
<213> Homo sapiens

<400> 179
Gln Ser Val Leu Thr Gln Pro Pro Ser Val Ser Ala Ala Pro Gly Gln
1               5                   10                  15

Lys Val Thr Ile Ser Cys Ser Gly Arg Thr Ser Asn Ile Gly Asn Asn
            20                  25                  30

His Val Ser Trp Tyr Gln Gln Phe Pro Gly Thr Ala Pro Lys Val Leu
        35                  40                  45

Ile Tyr Arg Asn Asp Arg Arg Pro Ser Gly Ile Pro Asp Arg Phe Ser
    50                  55                  60

Gly Ser Lys Ser Asp Thr Ser Ala Thr Leu Gly Ile Thr Gly Leu Gln
65                  70                  75                  80

Thr Gly Asp Glu Ala Asp Tyr Tyr Cys Gly Thr Trp Asp Asn Ser Leu

85                  90                  95

Ser Gly Trp Val Phe Gly Gly Gly Thr Lys Leu Thr Val Leu
            100                 105                 110

<210> 180
<211> 379
<212> DNA
<213> Homo sapiens

<400> 180
caggtgcagc tggtgcagtc tggggctgag gtgaagaggc ctggggcctc agtgaaggtc      60

tcctgtgaga cttctggata tagcttcacc actcatgatt tgaactgggt gcgacaggcc     120

gctggacagg ggcttgagtg gatgggatgg atgaacccta acactggtga cacatcctac     180

gcacagaggt tccagggcag agtcaccatg atcagggacg tctccataag tacagcctac     240

ttggaattga caggcctgac atttgatgac acggccgtct acttctgtgc gaggtcgaat     300

tctgcacctg tttcaactct tttacccgct gatgcttttg atgtctgggg ccaagggaca     360

atggtcaccg tctcgagtg                                                  379

<210> 181
<211> 125
<212> PRT
<213> Homo sapiens

<400> 181
Gln Val Gln Leu Val Gln Ser Gly Ala Glu Val Lys Arg Pro Gly Ala
1               5                   10                  15

Ser Val Lys Val Ser Cys Glu Thr Ser Gly Tyr Ser Phe Thr Thr Asp
            20                  25                  30

Leu Asn Trp Val Arg Gln Ala Ala Gly Gln Gly Leu Glu Trp Met Gly
        35                  40                  45

Trp Met Asn Pro Asn Thr Gly Asp Thr Ser Tyr Ala Gln Arg Phe Gln
    50                  55                  60

Gly Arg Val Thr Met Ile Arg Asp Val Ser Ile Ser Thr Ala Tyr Leu
65                  70                  75                  80

Glu Leu Thr Gly Leu Thr Phe Asp Asp Thr Ala Val Tyr Phe Cys Ala
                85                  90                  95

Arg Ser Asn Ser Ala Pro Val Ser Thr Leu Leu Pro Ala Asp Ala Phe
            100                 105                 110

Asp Val Trp Gly Gln Gly Thr Met Val Thr Val Ser Ser

115                 120                 125

<210> 182
<211> 325
<212> DNA
<213> Homo sapiens

<400> 182
gacatccaga tgacccagtc tccatcctcc ctgtctgcat ctataggaga cacagtcacc      60

atcacttgcc gggcaagtca gatcattaac agctatttaa attggtatca gcaaaaacca     120

ggggaagccc ctaaactcct gatctatgct gtatccaatt tgcaccgtgg ggtcccctca     180

aggttcagtg gcagtgggtc tgcggcagat ttcactctca ctatcagtgg tctgcaacct     240

gaagattttg caacttacta ctgtcaacag acttacacta ccgttgcgat caccttcggc     300

caagggacac gactggacat taaac                                           325

<210> 183
<211> 108
<212> PRT
<213> Homo sapiens

<400> 183
Asp Ile Gln Met Thr Gln Ser Pro Ser Ser Leu Ser Ala Ser Ile Gly
1               5                   10                  15

Asp Thr Val Thr Ile Thr Cys Arg Ala Ser Gln Ile Ile Asn Ser Tyr
            20                  25                  30

Leu Asn Trp Tyr Gln Gln Lys Pro Gly Glu Ala Pro Lys Leu Leu Ile
        35                  40                  45

Tyr Ala Val Ser Asn Leu His Arg Gly Val Pro Ser Arg Phe Ser Gly
    50                  55                  60

Ser Gly Ser Ala Ala Asp Phe Thr Leu Thr Ile Ser Gly Leu Gln Pro
65                  70                  75                  80

Glu Asp Phe Ala Thr Tyr Tyr Cys Gln Gln Thr Tyr Thr Thr Val Ala
                85                  90                  95

Ile Thr Phe Gly Gln Gly Thr Arg Leu Asp Ile Lys
            100                 105

<210> 184
<211> 373
<212> DNA
<213> Homo sapiens

<400> 184
caggtgcagc tggtgcagtc tggggcggag gtgaagaagc ctgggtcctc ggtgaacgtc      60

tcctgtaagg cctccgcagg gaccctcagc ggttatacca tcaactggtt gcgccaggcc     120

cctggacgag ggtttgagtg gctgggaagg atcatccctc accttggaat aggaaacaac     180

gcacataact gggaggccag agtcaccctt accgccgaca agtccacgga cactacctac     240

ctggagttgc gcgacctgag atctgacgac gtggccattt attactgcgc ggtggagttt     300

taccggctgg caacggtcac aaccccaacc cttgacttct ggggccaggg aaccctggtc     360

accgtctcga gtg                                                        373

<210> 185
<211> 122
<212> PRT
<213> Homo sapiens

<400> 185
Gln Val Gln Leu Val Gln Ser Gly Ala Glu Val Lys Lys Pro Gly Ser
1               5                   10                  15

Ser Val Asn Val Ser Cys Lys Ala Ser Ala Gly Thr Leu Ser Gly Tyr
            20                  25                  30

Thr Ile Asn Trp Leu Arg Gln Ala Pro Gly Arg Gly Phe Glu Trp Leu
        35                  40                  45

Gly Arg Ile Ile Pro Leu Gly Ile Gly Asn Asn Ala Asn Trp Glu Ala
    50                  55                  60

Arg Val Thr Leu Thr Ala Asp Lys Ser Thr Asp Thr Thr Tyr Leu Glu
65                  70                  75                  80

Leu Arg Asp Leu Arg Ser Asp Asp Val Ala Ile Tyr Tyr Cys Ala Val
                85                  90                  95

Glu Phe Tyr Arg Leu Ala Thr Val Thr Thr Pro Thr Leu Asp Phe Trp
            100                 105                 110

Gly Gln Gly Thr Leu Val Thr Val Ser Ser
        115                 120

<210> 186
<211> 325
<212> DNA
<213> Homo sapiens

<400> 186
tcctatgtgc tgactcagcc accctcggtg tccgtggccc caggaacgac ggccaggatt      60

acctgtgagg gggaccacat tggagctaaa agtgtgcact ggtaccagag gaagccaggc     120

caggcccctg tgttggtcgt ctatatgaat agtgaccggc cctcagggat ccctgagcga     180

ttgtctggct ccaactctgg gaacacggcc accctgagta tcagcagggt cgaggccgag 240

gatgaggccg actattattg tcaggtttgg gatagttttc gcgatcatca ggtattcggc 300

ggagggacca agttgaccgt cctga 325

<210> 187
<211> 108
<212> PRT
<213> Homo sapiens

<400> 187
Ser Tyr Val Leu Thr Gln Pro Pro Ser Val Ser Val Ala Pro Gly Thr
1               5                   10                  15

Thr Ala Arg Ile Thr Cys Glu Gly Asp His Ile Gly Ala Lys Ser Val
            20                  25                  30

His Trp Tyr Gln Arg Lys Pro Gly Gln Ala Pro Val Leu Val Val Tyr
        35                  40                  45

Met Asn Ser Asp Arg Pro Ser Gly Ile Pro Glu Arg Leu Ser Gly Ser
    50                  55                  60

Asn Ser Gly Asn Thr Ala Thr Leu Ser Ile Ser Arg Val Glu Ala Glu
65                  70                  75                  80

Asp Glu Ala Asp Tyr Tyr Cys Gln Val Trp Asp Ser Phe Arg Asp His
                85                  90                  95

Gln Val Phe Gly Gly Gly Thr Lys Leu Thr Val Leu
            100                 105

<210> 188
<211> 388
<212> DNA
<213> Homo sapiens

<400> 188
caggtcacct tgaaggagtc tggtcctgct atagtgaaac ccacacagac cctcacgctg 60

acctgcacct tctctgggtt ctcactccga actagtggag tgggtgtggg ctggatccgt 120

cagcccccag gaaaggccct tgagtggctt gcactcattt actgggatga tgataagcgc 180

tacaacccat ctctgaagac cagactcacc atcaccaagg actcctccag aaaccaggtg 240

ttcctcacaa tgaccaacat ggaccctgtg gacacagcca catacttctg tgcacaccga 300

caacatatta caaacagttt tggattggtt attgcgaatg atgcttttga catttggggc 360

caagggacaa tggtcaccgt ctcgagtg 388

<210> 189
<211> 127
<212> PRT
<213> Homo sapiens

<400> 189
Gln Val Thr Leu Lys Glu Ser Gly Pro Ala Ile Val Lys Pro Thr Gln
1               5                   10                  15

Thr Leu Thr Leu Thr Cys Thr Phe Ser Gly Phe Ser Leu Arg Thr Ser
            20                  25                  30

Gly Val Gly Val Gly Trp Ile Arg Gln Pro Pro Gly Lys Ala Leu Glu
        35                  40                  45

Trp Leu Ala Leu Ile Tyr Trp Asp Asp Asp Lys Arg Tyr Asn Pro Ser
    50                  55                  60

Leu Lys Thr Arg Leu Thr Ile Thr Lys Asp Ser Ser Arg Asn Gln Val
65                  70                  75                  80

Phe Leu Thr Met Thr Asn Met Asp Pro Val Asp Thr Ala Thr Tyr Phe
                85                  90                  95

Cys Ala Arg Gln Ile Thr Asn Ser Phe Gly Leu Val Ile Ala Asn Asp
            100                 105                 110

Ala Phe Asp Ile Trp Gly Gln Gly Thr Met Val Thr Val Ser Ser
        115                 120                 125

<210> 190
<211> 334
<212> DNA
<213> Homo sapiens

<400> 190
cagtctgccc tgactcagcc tccgtccgcg tccgggtctc ctggacagtc agtcaccatc      60

tcctgcactg gaaccagcag tgacgttggt cgttataatt atgtcgcctg gtatcaacaa     120

cacccaggca gagcccccag actcataatt tatggggtca ctaagcggcc ctcaggggtc     180

cctgatcgct tctctggctc caagtctggc aacacggcct ccctgaccgt ctctgggctc     240

caggctgaag atgaggctga ttattattgc agttcatatg caggcagcaa ctttttggag     300

gtgttcggcg gagggaccaa gctgaccgtc ctag                                 334

<210> 191
<211> 111
<212> PRT
<213> Homo sapiens

<400> 191

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Arg Tyr
            20                  25                  30

Asn Tyr Val Ala Trp Tyr Gln Gln His Pro Gly Arg Ala Pro Arg Leu
        35                  40                  45

Ile Ile Tyr Gly Val Thr Lys Arg Pro Ser Gly Val Pro Asp Arg Phe
    50                  55                  60

Ser Gly Ser Lys Ser Gly Asn Thr Ala Ser Leu Thr Val Ser Gly Leu
65                  70                  75                  80

Gln Ala Glu Asp Glu Ala Asp Tyr Tyr Cys Ser Ser Tyr Ala Gly Ser
                85                  90                  95

Asn Phe Leu Glu Val Phe Gly Gly Gly Thr Lys Leu Thr Val Leu
            100                 105                 110

<210> 192
<211> 379
<212> DNA
<213> Homo sapiens

<400> 192
caggtgcagc tggtgcagtc tggggctgag gtgaagaagc ctgggtcctc ggtgaaggtc      60

tcctgcaagg cttctggagg caccttcagc agctatgcaa tcaactgggt gcgacaggcc     120

cctggacaag gtcttgagtg gatgggaggg atcatcccta tgtttgatac agcaatctac     180

gcacagatgt tccagggcag agtcaccatc accgcggacg aatccacgag cacagcctac     240

atggagctga gcagcctgag atctgacgac acggccgtat attactgtgc gagagatcga     300

aacgattatt atgacagtag tggttattca ggcgcctttg accagtgggg ccagggaacc     360

ctggtcaccg tctcgagtg                                                  379

<210> 193
<211> 126
<212> PRT
<213> Homo sapiens

<400> 193
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Asn Trp Val Arg Gln Ala Pro Gly Gln Gly Leu Glu Trp Met
        35                  40                  45

Gly Gly Ile Ile Pro Met Phe Asp Thr Ala Ile Tyr Ala Gln Met Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Asp Asp Thr Ala Val Tyr Tyr Cys
                85                  90                  95

Ala Arg Asp Arg Asn Asp Tyr Tyr Asp Ser Ser Gly Tyr Ser Gly Ala
            100                 105                 110

Phe Asp Gln Trp Gly Gln Gly Thr Leu Val Thr Val Ser Ser
        115                 120                 125

<210> 194
<211> 325
<212> DNA
<213> Homo sapiens

<400> 194
gaaattgtgt tgacgcagtc tccaggcgcc ctgtctttgt ctccagggga aagagccacc      60

ctctcctgca gggccagtca gagtgttagc agcagctact tagcctggta ccagcagaaa     120

cctggccagg ctcccaggct cctcatctat ggtgcatcca gcagggccac tggcatccca     180

gacaggttca gtggcagtgg gtctgggaca gacttcactc tcacaatcag cagactggag     240

cctgaggatt ttgcagtgta ttactgtcag cagtatggta gctcacctcg cacttttggc     300

caggggacca agctggagat caaac                                           325

<210> 195
<211> 108
<212> PRT
<213> Homo sapiens

<400> 195
Glu Ile Val Leu Thr Gln Ser Pro Gly Ala Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Arg Thr Phe Gly Gln Gly Thr Lys Leu Glu Ile Lys
            100                 105

<210> 196
<211> 367
<212> DNA
<213> Homo sapiens

<400> 196
gaggtgcagc tggtggagtc cgggggaggc ttggtccagc ctggggggtc cctgaaactc      60

tcctgtgcag cctctgggtt cagcttcagt ggctctgctg tggaatgggt ccgccaggct     120

tccgggagag ggctggagtg gattggccat ataagaacca agaccaaaaa ttacgcgaca     180

gcgtatgctg cgtcggtgca aggcaggttc accatcttca gagatgattc aaggaacacg     240

gcgtatctgc agatgaacag tcttaaaacc gaggacacgg ccgtgtatta ttgttccaga     300

cacatcgaat ttagcagttc gtccgggccc cagatgggcc agggaaccct ggtcaccgtc     360

tcgagtg                                                               367

<210> 197
<211> 120
<212> PRT
<213> Homo sapiens

<400> 197
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Ser Phe Ser Gly Ser
            20                  25                  30

Ala Val Glu Trp Val Arg Gln Ala Ser Gly Arg Gly Leu Glu Trp Ile
        35                  40                  45

Gly Ile Arg Thr Lys Thr Lys Asn Tyr Ala Thr Ala Tyr Ala Ala Ser
    50                  55                  60

Val Gln Gly Arg Phe Thr Ile Phe Arg Asp Asp Ser Arg Asn Thr Ala
65                  70                  75                  80

Tyr Leu Gln Met Asn Ser Leu Lys Thr Glu Asp Thr Ala Val Tyr Tyr
                85                  90                  95

Cys Ser Arg Ile Glu Phe Ser Ser Ser Ser Gly Pro Gln Met Gly Gln
            100                 105                 110

Gly Thr Leu Val Thr Val Ser Ser
        115                 120

<210> 198
<211> 322
<212> DNA
<213> Homo sapiens

<400> 198
gacatccaga tgacccagtc tccttccacc ctgtctgcat ctgtaggcga cacagtcacc      60

atcacttgcc aggccagtca gaatattatt acttggttgg cctggtatca gcagaaacca     120

gggaaagccc ctaacatcct catctataag gcgtctggtt tagagagtgg ggtcccaccg     180

aggttcagcg gcagtgggtc ggggacagaa ttcactctca ccatcagcag cctgcagcct     240

gatgatgttg caacttatta ttgcctacaa tataaaactg agccgtggac gttcggccta     300

gggaccaagg tggagatcag ac                                              322

<210> 199
<211> 107
<212> PRT
<213> Homo sapiens

<400> 199
Asp Ile Gln Met Thr Gln Ser Pro Ser Thr Leu Ser Ala Ser Val Gly
1               5                   10                  15

Asp Thr Val Thr Ile Thr Cys Gln Ala Ser Gln Asn Ile Ile Thr Trp
            20                  25                  30

Leu Ala Trp Tyr Gln Gln Lys Pro Gly Lys Ala Pro Asn Ile Leu Ile
        35                  40                  45

Tyr Lys Ala Ser Gly Leu Glu Ser Gly Val Pro Pro Arg Phe Ser Gly
    50                  55                  60

Ser Gly Ser Gly Thr Glu Phe Thr Leu Thr Ile Ser Ser Leu Gln Pro
65                  70                  75                  80

Asp Asp Val Ala Thr Tyr Tyr Cys Leu Gln Tyr Lys Thr Glu Pro Trp
                85                  90                  95

Thr Phe Gly Leu Gly Thr Lys Val Glu Ile Arg
            100                 105

<210> 200
<211> 346
<212> DNA
<213> Homo sapiens

<400> 200
gaggtgcagc tggtggagtc tgggggaggc ttggcccagc cgggggagtc cctgagactc      60

tcctgtgcag cctccggatt cacctttagt gcctattgga tgagctgggt ccgccagact     120

ccagggaagg ggctggagtg ggtggccaac attaaccagg gtggagataa gacagactat     180

ttggactctg tgaagggccg attcaccgtc tccagagaca atgccaagaa cttagtgttt     240

ctgcaaatgg ccagcctgag agccgacgac acggccgtgt attactgtgg gagaggtaca     300

aaccaagact actggggcca gggaaccctg gtcaccgtct cgagtg                    346


<210> 201
<211> 115
<212> PRT
<213> Homo sapiens

<400> 201
Glu Val Gln Leu Val Glu Ser Gly Gly Gly Leu Ala Gln Pro Gly Glu
1               5                   10                  15

Ser Leu Arg Leu Ser Cys Ala Ala Ser Gly Phe Thr Phe Ser Ala Tyr
            20                  25                  30

Trp Met Ser Trp Val Arg Gln Thr Pro Gly Lys Gly Leu Glu Trp Val
        35                  40                  45

Ala Asn Ile Asn Gln Gly Gly Asp Lys Thr Asp Tyr Leu Asp Ser Val
    50                  55                  60

Lys Gly Arg Phe Thr Val Ser Arg Asp Asn Ala Lys Asn Leu Val Phe
65                  70                  75                  80

Leu Gln Met Ala Ser Leu Arg Ala Asp Asp Thr Ala Val Tyr Tyr Cys
                85                  90                  95

Gly Arg Gly Thr Asn Gln Asp Tyr Trp Gly Gln Gly Thr Leu Val Thr
            100                 105                 110

Val Ser Ser
        115


<210> 202
<211> 323
<212> DNA
<213> Homo sapiens

<400> 202

cagtctgtgc tgactcagcc accctcagcg tctgggaccc ccgggcagag ggtcaccatc 60

tcttgttctg gaagcagctc caatatcgga agtaataatg tatactggta ccaacaggtc 120

ccaggaacgg cccccaaatt tctcatctat ccgaatagtg agcggccctc aggggtccct 180

gaccgattct ctgcctccaa gtctggcacc tcagcctccc tggccatcag tggactccgg 240

tccgaggatg aggctactta ttactgtgca gcatgggatg acagcctaag aacttatgtc 300

ttcgcatctg ggaccaaggt cac 323

<210> 203
<211> 110
<212> PRT
<213> Homo sapiens

<400> 203
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Tyr Trp Tyr Gln Gln Val Pro Gly Thr Ala Pro Lys Phe Leu
        35                  40                  45

Ile Tyr Pro Asn Ser Glu Arg Pro Ser Gly Val Pro Asp Arg Phe Ser
    50                  55                  60

Ala Ser Lys Ser Gly Thr Ser Ala Ser Leu Ala Ile Ser Gly Leu Arg
65                  70                  75                  80

Ser Glu Asp Glu Ala Thr Tyr Tyr Cys Ala Ala Trp Asp Asp Ser Leu
                85                  90                  95

Arg Thr Tyr Val Phe Ala Ser Gly Thr Lys Val Thr Val Leu
            100                 105                 110

<210> 204
<211> 361
<212> DNA
<213> Homo sapiens

<400> 204
caggtgcagc tggtggagtc tgggggaggc gtggtccagc ctgggaggtc cctgagactc 60

tcctgtgcag cctccggatt caccctcagg agttatggca tgcactgggt ccgccaggct 120

ccaggcaagg gcctggagtg ggtggcagtt atatcatatg atggaagtaa gacatattat 180

agagactccg tgaagggccg attcaccatc tccagagaca attctaagaa cacactgtat 240

ttgcaaatga acagcctgcg agttgaggac acggctgtat attattgtgc gaaagaggat 300

aatttgggat ctgatgtttt tgatagatgg ggccaaggga caatggtcac cgtctcgagt     360

g                                                                   361

<210> 205
<211> 120
<212> PRT
<213> Homo sapiens

<400> 205
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Leu Arg Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Lys Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Val Glu Asp Thr Ala Val Tyr Tyr Cys
                85                  90                  95

Ala Lys Glu Asp Asn Leu Gly Ser Asp Val Phe Asp Arg Trp Gly Gln
            100                 105                 110

Gly Thr Met Val Thr Val Ser Ser
        115                 120

<210> 206
<211> 322
<212> DNA
<213> Homo sapiens

<400> 206
gacatcgtga tgacccagtc tccttccacc ctgtctgcat ctgtaggaga cagagttacc      60

atcacttgtc gggccagtca gagtgccagt cagtggttgg cctggtatca gcaaaaacca     120

gggaaagccc ctaatctcct aatctataag gcgtctaatt tacatagtgg ggtcccatca     180

aggttcagcg gcagtggatc tgggaccgaa ttcactctca ccatcagcag cctacagcct     240

gatgattttg caacttatta ctgccaacaa tacaacattt atccgtggac gttcggccaa     300

gggaccaagg tggaagtgag ac                                            322

<210> 207

<211> 107
<212> PRT
<213> Homo sapiens

<400> 207
Asp Ile Val Met Thr Gln Ser Pro Ser Thr Leu Ser Ala Ser Val Gly
1               5                   10                  15

Asp Arg Val Thr Ile Thr Cys Arg Ala Ser Gln Ser Ala Ser Gln Trp
            20                  25                  30

Leu Ala Trp Tyr Gln Gln Lys Pro Gly Lys Ala Pro Asn Leu Leu Ile
        35                  40                  45

Tyr Lys Ala Ser Asn Leu His Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ile Tyr Pro Trp
                85                  90                  95

Thr Phe Gly Gln Gly Thr Lys Val Glu Val Arg
            100                 105

<210> 208
<211> 361
<212> DNA
<213> Homo sapiens

<400> 208
caggtccagc tggtgcagtc tggggctgag gtgaaggagc cagggtcctc ggtgagggtc      60

tcctgcaagg cttctggacg catcttccgg aattatgctg tcagctgggt gcgacaggcc     120

cctggacaag ggcttgagtg ggtgggccgg ttcatccctt cggaaggaat aacaaactac     180

attccgacat tccgggacag actcaccttt actgcggaca agtccacgaa cacagccttc     240

attgacctga ctaacctgaa atatgaggac acggccatat attactgtgt gggtggcagg     300

ccaggcgctt tcgtcccctt tgactcctgg ggccagggaa ccctggtcac cgtctcgagt     360

g                                                                     361

<210> 209
<211> 120
<212> PRT
<213> Homo sapiens

<400> 209
Gln Val Gln Leu Val Gln Ser Gly Ala Glu Val Lys Glu Pro Gly Ser
1               5                   10                  15

Ser Val Arg Val Ser Cys Lys Ala Ser Gly Arg Ile Phe Arg Asn Tyr
            20                  25                  30

Ala Val Ser Trp Val Arg Gln Ala Pro Gly Gln Gly Leu Glu Trp Val
        35                  40                  45

Gly Arg Phe Ile Pro Ser Glu Gly Ile Thr Asn Tyr Ile Pro Thr Phe
    50                  55                  60

Arg Asp Arg Leu Thr Phe Thr Ala Asp Lys Ser Thr Asn Thr Ala Phe
65                  70                  75                  80

Ile Asp Leu Thr Asn Leu Lys Tyr Glu Asp Thr Ala Ile Tyr Tyr Cys
                85                  90                  95

Val Gly Gly Arg Pro Gly Ala Phe Val Pro Phe Asp Ser Trp Gly Gln
            100                 105                 110

Gly Thr Leu Val Thr Val Ser Ser
        115                 120

<210> 210
<211> 321
<212> DNA
<213> Homo sapiens

<400> 210
gaaatagtga tgacgcagtc tcctgccacc ctgtctgtgt ctccagggga aagagccacc      60

ctctcctgca gggccagtca gagtgtcagg ggcaacttag cctggtacca gcaaaaacct     120

ggccaggctc ccaggcttct catgtatgat gcttctacca gggccactag tttcccagcc     180

cggttcagtg gcagtgggtc tgggacagag ttcatctcac catcagcagt gtgcagtctg     240

aagattttgc actttattac tgtcagcagt ataatgactg gccccggact tttggccagg     300

ggaccaaggt ggagatcaaa c                                               321

<210> 211
<211> 107
<212> PRT
<213> Homo sapiens

<400> 211
Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Arg Gly Asn
            20                  25                  30

Leu Ala Trp Tyr Gln Gln Lys Pro Gly Gln Ala Pro Arg Leu Leu Met

        35                  40                  45

Tyr Asp Ala Ser Thr Arg Ala Thr Ser Phe Pro Ala Arg Phe Ser Gly
    50                  55                  60

Ser Gly Ser Gly Thr Glu Phe Thr Leu Thr Ile Ser Ser Val Gln Ser
65                  70                  75                  80

Glu Asp Phe Ala Leu Tyr Tyr Cys Gln Gln Tyr Asn Asp Trp Pro Arg
                85                  90                  95

Thr Phe Gly Gln Gly Thr Lys Val Glu Ile Lys
            100                 105

<210> 212
<211> 373
<212> DNA
<213> Homo sapiens

<400> 212
caggtgcagc tggtgcagtc tggggctgag gtgaagaagc ctgggtcctc ggtgagggtc 60

tcctgcaggg tttctggagg cagtttctcc agttatgacg tacactgggt gcgacaggcc 120

cctggacaag ggcttgagtg ggtgggaagg agcgtctcac gtgctgacag ctcaagtcac 180

acacgggagt tcggggacag agtcacgatt atcgcggaca aatccacgag tacagtttat 240

ttgacactga ccagtctgac ctctgacgac acggccatct attactgtgc ggcaggggat 300

tgtagtgatg gcgggtgctt ccgcacaccc ctgcacatct ggggccaggg caccctggtc 360

accgtctcga gtg 373

<210> 213
<211> 124
<212> PRT
<213> Homo sapiens

<400> 213
Gln Val Gln Leu Val Gln Ser Gly Ala Glu Val Lys Lys Pro Gly Ser
1               5                   10                  15

Ser Val Arg Val Ser Cys Arg Val Ser Gly Gly Ser Phe Ser Ser Tyr
            20                  25                  30

Asp Val His Trp Val Arg Gln Ala Pro Gly Gln Gly Leu Glu Trp Val
        35                  40                  45

Gly Arg Ser Val Ser Arg Ala Asp Ser Ser Ser His Thr Arg Glu Phe
    50                  55                  60

Gly Asp Arg Val Thr Ile Ile Ala Asp Lys Ser Thr Ser Thr Val Tyr

65                  70                  75                  80

Leu Thr Leu Thr Ser Leu Thr Ser Asp Asp Thr Ala Ile Tyr Tyr Cys
                85                  90                  95

Ala Ala Gly Asp Cys Ser Asp Gly Gly Cys Phe Arg Thr Pro Leu His
            100                 105                 110

Ile Trp Gly Gln Gly Thr Leu Val Thr Val Ser Ser
        115                 120

<210> 214
<211> 322
<212> DNA
<213> Homo sapiens

<400> 214
gaaatagtga tgacgcagtc tccagccact ctgtctctgt ctccagggga aagagccacc      60

ctctcctgca gggccagtca gagtctgaga accaacttag cctggtacca gcagcgacct     120

ggccaggctc ccaggctcct catctctggg gcatccacga gggccactgg tatcccagcc     180

aggttcagtg gcagtgggtc tgggacagaa ttcactctca ccattaggag cctgcagcct     240

gaagattttg cagtttatta ctgtcaacag tatcatgact ggcctcggac gttcggccaa     300

gggaccaagg tggatttcaa ac                                              322

<210> 215
<211> 107
<212> PRT
<213> Homo sapiens

<400> 215
Glu Ile Val Met Thr Gln Ser Pro Ala Thr Leu Ser Leu Ser Pro Gly
1               5                   10                  15

Glu Arg Ala Thr Leu Ser Cys Arg Ala Ser Gln Ser Leu Arg Thr Asn
            20                  25                  30

Leu Ala Trp Tyr Gln Gln Arg Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Glu Phe Thr Leu Thr Ile Arg Ser Leu Gln Pro
65                  70                  75                  80

Glu Asp Phe Ala Val Tyr Tyr Cys Gln Gln Tyr His Asp Trp Pro Arg
                85                  90                  95

Thr Phe Gly Gln Gly Thr Lys Val Asp Phe Lys
            100                 105

<210> 216
<211> 370
<212> DNA
<213> Homo sapiens

<400> 216
gaggtgcagc tggtgcagtc tggggcagaa gtgaaaaagg ccggggagtc tctgaggatc      60
tcctgtaagg gttctggata caactttaat agctattgga ttggctgggt gcgccagatg     120
cccgggaaag gcctggagtg gatggcaatc atctatcctg ctgactctga caggagatat     180
ggcccgtcct tccaaggcca ggtcaccatc tcagtcgaca agtctattaa caccgcgtac     240
ttgcagtgga gcagcctgaa ggcctcggac accgccatgt attattgtgc cagactcctc     300
agagttagta ctggttggga agatgctttt gatctgtggg gccaagggac aatggtcacc     360
gtctcgagtg                                                            370

<210> 217
<211> 123
<212> PRT
<213> Homo sapiens

<400> 217
Glu Val Gln Leu Val Gln Ser Gly Ala Glu Val Lys Lys Ala Gly Glu
1               5                   10                  15

Ser Leu Arg Ile Ser Cys Lys Gly Ser Gly Tyr Asn Phe Asn Ser Tyr
            20                  25                  30

Trp Ile Gly Trp Val Arg Gln Met Pro Gly Lys Gly Leu Glu Trp Met
        35                  40                  45

Ala Ile Ile Tyr Pro Ala Asp Ser Asp Arg Arg Tyr Gly Pro Ser Phe
    50                  55                  60

Gln Gly Gln Val Thr Ile Ser Val Asp Lys Ser Ile Asn Thr Ala Tyr
65                  70                  75                  80

Leu Gln Trp Ser Ser Leu Lys Ala Ser Asp Thr Ala Met Tyr Tyr Cys
                85                  90                  95

Ala Arg Leu Leu Arg Val Ser Thr Gly Trp Glu Asp Ala Phe Asp Leu
            100                 105                 110

Trp Gly Gln Gly Thr Met Val Thr Val Ser Ser
        115                 120

<210> 218
<211> 340
<212> DNA
<213> Homo sapiens

<400> 218
gacatcgtga tgacccagtc tccagactcc ctggttgtgt ctctgggcga gagggccacc 60

atcaagtgcc agtccagcca gagtgttttg tacagctcca ccaataagaa ctatttagcc 120

tggtaccagc agaaatcggg acagccacct aagctactca tttactgggc ctccacccgg 180

gaatccgggg tccctgaccg attcattggt agcggctcta ggacagattt cactctcacc 240

atcagcgacc tgcagcctga ggatgcggca gtttattact gtcagcagta ttatagtgat 300

tatctcactt tcggcggagg gaccaaggtg gagatcaaac 340


<210> 219
<211> 113
<212> PRT
<213> Homo sapiens

<400> 219
Asp Ile Val Met Thr Gln Ser Pro Asp Ser Leu Val Val Ser Leu Gly
1               5                   10                  15

Glu Arg Ala Thr Ile Lys Cys Gln Ser Ser Gln Ser Val Leu Tyr Ser
            20                  25                  30

Ser Thr Asn Lys Asn Tyr Leu Ala Trp Tyr Gln Gln Lys Ser Gly Gln
        35                  40                  45

Pro Pro Lys Leu Leu Ile Tyr Trp Ala Ser Thr Arg Glu Ser Gly Val
    50                  55                  60

Pro Asp Arg Phe Ile Gly Ser Gly Ser Arg Thr Asp Phe Thr Leu Thr
65                  70                  75                  80

Ile Ser Asp Leu Gln Pro Glu Asp Ala Ala Val Tyr Tyr Cys Gln Gln
                85                  90                  95

Tyr Tyr Ser Asp Tyr Leu Thr Phe Gly Gly Gly Thr Lys Val Glu Ile
            100                 105                 110

Lys


<210> 220
<211> 10
<212> PRT
<213> Homo sapiens

<400> 220
Gly Phe Ser Phe Thr Ser Ser Gly Glu Ala
1               5                   10


<210> 221
<211> 7
<212> PRT
<213> Homo sapiens

<400> 221
Ile Tyr Trp Asp Asp Asp Pro
1               5


<210> 222
<211> 19
<212> PRT
<213> Homo sapiens

<400> 222
Cys Ala His Leu Arg Arg Pro Ser Leu Trp Ser Pro Tyr Tyr Leu Phe
1               5                   10                  15


Asp Cys Trp




<210> 223
<211> 5
<212> PRT
<213> Homo sapiens

<400> 223
Gln Ser Ile Ser Ser
1               5


<210> 224
<211> 10
<212> PRT
<213> Homo sapiens

<400> 224
Cys Gln Gln Ser Tyr Lys Met Trp Ser Phe
1               5                   10


<210> 225
<211> 8
<212> PRT
<213> Homo sapiens

<400> 225
Gly Tyr Thr Phe Thr Asn Phe Gly
1               5


<210> 226
<211> 8
<212> PRT
<213> Homo sapiens

<400> 226
Ile Asn Thr His Asn Gly Asn Thr
1               5

<210> 227
<211> 17
<212> PRT
<213> Homo sapiens

<400> 227
Cys Ala Arg Asp Phe Phe Arg Ala Ala Ala Gly Thr Ala Gly Asp Tyr
1               5                   10                  15

Trp

<210> 228
<211> 7
<212> PRT
<213> Homo sapiens

<400> 228
His Ser Gly Thr Asn Asn Tyr
1               5

<210> 229
<211> 10
<212> PRT
<213> Homo sapiens

<400> 229
Cys Gln Gln Ser Tyr Lys Met Trp Ser Phe
1               5                   10

<210> 230
<211> 8
<212> PRT
<213> Homo sapiens

<400> 230
Gly Gly Ser Ile Ser Asn Asp Tyr
1               5

<210> 231
<211> 7
<212> PRT
<213> Homo sapiens

<400> 231
Val Tyr Tyr Ser Gly Arg Thr
1               5

<210> 232
<211> 21
<212> PRT

<213> Homo sapiens

<400> 232
Cys Ala Arg Thr Thr Arg Leu Ser Phe Asp Trp Leu Val His Tyr Tyr
1               5                   10                  15

Gly Met Asp Val Trp
            20

<210> 233
<211> 6
<212> PRT
<213> Homo sapiens

<400> 233
Gln Pro Ile Gly Ser Tyr
1               5

<210> 234
<211> 12
<212> PRT
<213> Homo sapiens

<400> 234
Cys Gln Gln Ser Tyr Asn Asn Pro Leu Phe Thr Phe
1               5                   10

<210> 235
<211> 8
<212> PRT
<213> Homo sapiens

<400> 235
Gly Phe Ile Phe Asp Asp Tyr Gly
1               5

<210> 236
<211> 8
<212> PRT
<213> Homo sapiens

<400> 236
Ile Thr Trp Asp Gly Val Asn Thr
1               5

<210> 237
<211> 19
<212> PRT
<213> Homo sapiens

<400> 237
Cys Ala Arg Ala Arg Tyr Tyr Gln Gly Thr Gly Leu Arg Ser Tyr Leu
1               5                   10                  15

Asp Glu Trp

<210> 238
<211> 6
<212> PRT
<213> Homo sapiens

<400> 238
Gln Gly Ile Asp Asn Trp
1               5


<210> 239
<211> 14
<212> PRT
<213> Homo sapiens

<400> 239
Cys Gln Gln Ala Lys Asn Phe Pro Arg Gly Gly Phe Thr Phe
1               5                   10


<210> 240
<211> 8
<212> PRT
<213> Homo sapiens

<400> 240
Gly Phe Thr Phe Ser Lys Phe Ala
1               5


<210> 241
<211> 8
<212> PRT
<213> Homo sapiens

<400> 241
Ile Ala Ser Asp Gly Tyr Asn Lys
1               5


<210> 242
<211> 13
<212> PRT
<213> Homo sapiens

<400> 242
Cys Ala Tyr Gly His Leu Ser Phe Trp Phe Asp Pro Trp
1               5                   10


<210> 243
<211> 6
<212> PRT
<213> Homo sapiens

<400> 243
Gln Asp Ile Gly Asn Gly
1               5


<210> 244

<211> 11
<212> PRT
<213> Homo sapiens

<400> 244
Cys Leu Gln His Asn Phe Tyr Pro Arg Thr Phe
1               5                   10


<210> 245
<211> 8
<212> PRT
<213> Homo sapiens

<400> 245
Gly Tyr Thr Phe Thr Asp Tyr Tyr
1               5


<210> 246
<211> 8
<212> PRT
<213> Homo sapiens

<400> 246
Ile Asn Pro Tyr Ser Gly Gly Thr
1               5


<210> 247
<211> 17
<212> PRT
<213> Homo sapiens

<400> 247
Cys Ala Arg Asp Phe Phe Arg Ala Ala Ala Gly Thr Ala Gly Asp Tyr
1               5                   10                  15


Trp




<210> 248
<211> 12
<212> PRT
<213> Homo sapiens

<400> 248
Gln Ser Ala Leu Tyr Ser Pro Ser Ser Lys Thr Tyr
1               5                   10


<210> 249
<211> 10
<212> PRT
<213> Homo sapiens

<400> 249
Cys Gln Tyr Tyr Tyr Gly Thr Val Thr Phe
1               5                   10

<210> 250
<211> 8
<212> PRT
<213> Homo sapiens

<400> 250
Gly Gly Ser Ile Asn Ser Tyr Tyr
1               5


<210> 251
<211> 7
<212> PRT
<213> Homo sapiens

<400> 251
Met Tyr Pro Gly Gly Thr Ser
1               5


<210> 252
<211> 21
<212> PRT
<213> Homo sapiens

<400> 252
Cys Ala Arg Asp Pro Thr Tyr His His Gly Thr Ser Gly Tyr Val Gly
1               5                   10                  15


Thr Phe Asp Leu Trp
            20


<210> 253
<211> 6
<212> PRT
<213> Homo sapiens

<400> 253
Pro Ser Val Arg Asn Asn
1               5


<210> 254
<211> 12
<212> PRT
<213> Homo sapiens

<400> 254


<210> 255
<211> 8
<212> PRT
<213> Homo sapiens

<400> 255
Gly Phe Thr Phe Ser Met Phe Thr
1               5

<210> 256
<211> 8
<212> PRT
<213> Homo sapiens

<400> 256
Ile Ser Gly Ser Ser Ser Leu Val
1               5


<210> 257
<211> 10
<212> PRT
<213> Homo sapiens

<400> 257


<210> 258
<211> 8
<212> PRT
<213> Homo sapiens

<400> 258
Thr Ser Asn Ile Gly Asn Asn His
1               5


<210> 259
<211> 13
<212> PRT
<213> Homo sapiens

<400> 259


<210> 260
<211> 7
<212> PRT
<213> Homo sapiens

<400> 260
Gly Tyr Ser Phe Thr Thr Asp
1               5


<210> 261
<211> 8
<212> PRT
<213> Homo sapiens

<400> 261
Met Asn Pro Asn Thr Gly Asp Thr
1               5


<210> 262
<211> 21

<212> PRT
<213> Homo sapiens

<400> 262
Cys Ala Arg Ser Asn Ser Ala Pro Val Ser Thr Leu Leu Pro Ala Asp
1               5                   10                  15

Ala Phe Asp Val Trp
            20

<210> 263
<211> 6
<212> PRT
<213> Homo sapiens

<400> 263
Gln Ile Ile Asn Ser Tyr
1               5

<210> 264
<211> 12
<212> PRT
<213> Homo sapiens

<400> 264
Cys Gln Gln Thr Tyr Thr Thr Val Ala Ile Thr Phe
1               5                   10

<210> 265
<211> 8
<212> PRT
<213> Homo sapiens

<400> 265
Ala Gly Thr Leu Ser Gly Tyr Thr
1               5

<210> 266
<211> 7
<212> PRT
<213> Homo sapiens

<400> 266
Ile Ile Pro Leu Gly Ile Gly
1               5

<210> 267
<211> 19
<212> PRT
<213> Homo sapiens

<400> 267
Cys Ala Val Glu Phe Tyr Arg Leu Ala Thr Val Thr Thr Pro Thr Leu
1               5                   10                  15

Asp Phe Trp

<210> 268
<211> 6
<212> PRT
<213> Homo sapiens

<400> 268
His Ile Gly Ala Lys Ser
1               5


<210> 269
<211> 13
<212> PRT
<213> Homo sapiens

<400> 269
Cys Gln Val Trp Asp Ser Phe Arg Asp His Gln Val Phe
1               5                   10


<210> 270
<211> 10
<212> PRT
<213> Homo sapiens

<400> 270
Gly Phe Ser Leu Arg Thr Ser Gly Val Gly
1               5                   10


<210> 271
<211> 7
<212> PRT
<213> Homo sapiens

<400> 271
Ile Tyr Trp Asp Asp Asp Lys
1               5


<210> 272
<211> 21
<212> PRT
<213> Homo sapiens

<400> 272
Cys Ala Arg Gln Ile Thr Asn Ser Phe Gly Leu Val Ile Ala Asn Asp
1               5                   10                  15


Ala Phe Asp Ile Trp
            20


<210> 273
<211> 9
<212> PRT
<213> Homo sapiens

<400> 273

Ser Ser Asp Val Gly Arg Tyr Asn Tyr
1               5

<210> 274
<211> 13
<212> PRT
<213> Homo sapiens

<400> 274
Cys Ser Ser Tyr Ala Gly Ser Asn Phe Leu Glu Val Phe
1               5                   10

<210> 275
<211> 8
<212> PRT
<213> Homo sapiens

<400> 275
Gly Gly Thr Phe Ser Ser Tyr Ala
1               5

<210> 276
<211> 8
<212> PRT
<213> Homo sapiens

<400> 276
Ile Ile Pro Met Phe Asp Thr Ala
1               5

<210> 277
<211> 21
<212> PRT
<213> Homo sapiens

<400> 277
Cys Ala Arg Asp Arg Asn Asp Tyr Tyr Asp Ser Ser Gly Tyr Ser Gly
1               5                   10                  15

Ala Phe Asp Gln Trp
            20

<210> 278
<211> 7
<212> PRT
<213> Homo sapiens

<400> 278
Gln Ser Val Ser Ser Ser Tyr
1               5

<210> 279
<211> 11
<212> PRT
<213> Homo sapiens

<400> 279

<210> 280
<211> 8
<212> PRT
<213> Homo sapiens

<400> 280
Gly Phe Ser Phe Ser Gly Ser Ala
1               5

<210> 281
<211> 10
<212> PRT
<213> Homo sapiens

<400> 281
Ile Arg Thr Lys Thr Lys Asn Tyr Ala Thr
1               5                   10

<210> 282
<211> 14
<212> PRT
<213> Homo sapiens

<400> 282
Cys Ser Arg Ile Glu Phe Ser Ser Ser Ser Gly Pro Gln Met
1               5                   10

<210> 283
<211> 6
<212> PRT
<213> Homo sapiens

<400> 283
Gln Asn Ile Ile Thr Trp
1               5

<210> 284
<211> 11
<212> PRT
<213> Homo sapiens

<400> 284
Cys Leu Gln Tyr Lys Thr Glu Pro Trp Thr Phe
1               5                   10

<210> 285
<211> 8
<212> PRT
<213> Homo sapiens

<400> 285
Gly Phe Thr Phe Ser Ala Tyr Trp
1               5

<210> 286
<211> 8
<212> PRT
<213> Homo sapiens

<400> 286
Ile Asn Gln Gly Gly Asp Lys Thr
1               5


<210> 287
<211> 10
<212> PRT
<213> Homo sapiens

<400> 287
Cys Gly Arg Gly Thr Asn Gln Asp Tyr Trp
1               5                   10


<210> 288
<211> 8
<212> PRT
<213> Homo sapiens

<400> 288
Ser Ser Asn Ile Gly Ser Asn Asn
1               5


<210> 289
<211> 13
<212> PRT
<213> Homo sapiens

<400> 289
Cys Ala Ala Trp Asp Asp Ser Leu Arg Thr Tyr Val Phe
1               5                   10


<210> 290
<211> 8
<212> PRT
<213> Homo sapiens

<400> 290
Gly Phe Thr Leu Arg Ser Tyr Gly
1               5


<210> 291
<211> 8
<212> PRT
<213> Homo sapiens

<400> 291
Ile Ser Tyr Asp Gly Ser Lys Thr
1               5


<210> 292

<211> 15
<212> PRT
<213> Homo sapiens

<400> 292
Cys Ala Lys Glu Asp Asn Leu Gly Ser Asp Val Phe Asp Arg Trp
1               5                   10                  15


<210> 293
<211> 6
<212> PRT
<213> Homo sapiens

<400> 293
Gln Ser Ala Ser Gln Trp
1               5


<210> 294
<211> 11
<212> PRT
<213> Homo sapiens

<400> 294


<210> 295
<211> 8
<212> PRT
<213> Homo sapiens

<400> 295
Gly Arg Ile Phe Arg Asn Tyr Ala
1               5


<210> 296
<211> 8
<212> PRT
<213> Homo sapiens

<400> 296
Phe Ile Pro Ser Glu Gly Ile Thr
1               5


<210> 297
<211> 15
<212> PRT
<213> Homo sapiens

<400> 297
Cys Val Gly Gly Arg Pro Gly Ala Phe Val Pro Phe Asp Ser Trp
1               5                   10                  15


<210> 298
<211> 6
<212> PRT
<213> Homo sapiens

<400> 298
Gln Ser Val Arg Gly Asn
1               5


<210> 299
<211> 11
<212> PRT
<213> Homo sapiens

<400> 299


<210> 300
<211> 8
<212> PRT
<213> Homo sapiens

<400> 300
Gly Gly Ser Phe Ser Ser Tyr Asp
1               5


<210> 301
<211> 8
<212> PRT
<213> Homo sapiens

<400> 301
Ser Val Ser Arg Ala Asp Ser Ser
1               5


<210> 302
<211> 19
<212> PRT
<213> Homo sapiens

<400> 302
Cys Ala Ala Gly Asp Cys Ser Asp Gly Gly Cys Phe Arg Thr Pro Leu
1               5                   10                  15


His Ile Trp



<210> 303
<211> 6
<212> PRT
<213> Homo sapiens

<400> 303
Gln Ser Leu Arg Thr Asn
1               5


<210> 304
<211> 11
<212> PRT

<213> Homo sapiens

<400> 304


<210> 305
<211> 8
<212> PRT
<213> Homo sapiens

<400> 305
Gly Tyr Asn Phe Asn Ser Tyr Trp
1               5


<210> 306
<211> 8
<212> PRT
<213> Homo sapiens

<400> 306
Ile Tyr Pro Ala Asp Ser Asp Arg
1               5


<210> 307
<211> 18
<212> PRT
<213> Homo sapiens

<400> 307
Cys Ala Arg Leu Leu Arg Val Ser Thr Gly Trp Glu Asp Ala Phe Asp
1               5                   10                  15


Leu Trp




<210> 308
<211> 12
<212> PRT
<213> Homo sapiens

<400> 308
Gln Ser Val Leu Tyr Ser Ser Thr Asn Lys Asn Tyr
1               5                   10


<210> 309
<211> 11
<212> PRT
<213> Homo sapiens

<400> 309
Cys Gln Gln Tyr Tyr Ser Asp Tyr Leu Thr Phe
1               5                   10


<210> 310
<211> 1353

<212> DNA
<213> Homo sapiens

<400> 310
cagatcacct tgaaggagtc tggtcctacg ctggtgaacc ccacacagac cctcacgctg 60

acctgcacct tttctgggtt ctcattcacc tctagtggag aggctgtggg ctggatccgt 120

cagcccccag gaaaggccct ggaatggctt gctcacattt attgggatga tgatccgcgt 180

tatagtacat ctctgaagag cagactcacc atcaccaagg acacctccaa aaatcaggtg 240

gtcctctcta tgaccgacat tgaccctgcg gacacaggca catattactg tgcacacctg 300

agacgcccat ccctttggag tccttattat ctttttgact gctggggcca gggaaccctg 360

gtcaccgtct cgagtgcctc caccaagggc ccatcggtct tccccctggc gccctgctcc 420

aggagcacct ccgagagcac agccgccctg ggctgcctgg tcaaggacta cttccccgaa 480

ccggtgacgg tgtcgtggaa ctcaggcgct ctgaccagcg gcgtgcacac cttcccagct 540

gtcctacagt cctcaggact ctactccctc agcagcgtgg tgaccgtgcc ctccagcaac 600

ttcggcaccc agacctacac ctgcaacgta gatcacaagc ccagcaacac caaggtggac 660

aagacagttg agcgcaaatg ttgtgtcgag tgcccaccgt gcccagcacc acctgtggca 720

ggaccgtcag tcttcctctt ccccccaaaa cccaaggaca ccctcatgat ctcccggacc 780

cctgaggtca cgtgcgtggt ggtggacgtg agccacgaag accccgaggt ccagttcaac 840

tggtacgtgg acggcgtgga ggtgcataat gccaagacaa agccacggga ggagcagttc 900

aacagcacgt tccgtgtggt cagcgtcctc accgttgtgc accaggactg gctgaacggc 960

aaggagtaca agtgcaaggt ctccaacaaa ggcctcccag cccccatcga gaaaaccatc 1020

tccaaaacca aagggcagcc ccgagaacca caggtgtaca ccctgccccc atcccgggag 1080

gagatgacca agaaccaggt cagcctgacc tgcctggtca aaggcttcta ccccagcgac 1140

atcgccgtgg agtgggagag caatgggcag ccggagaaca actacaagac cacacctccc 1200

atgctggact ccgacggctc cttcttcctc tacagcaagc tcaccgtgga caagagcagg 1260

tggcagcagg ggaacgtctt ctcatgctcc gtgatgcatg aggctctgca caaccactac 1320

acgcagaaga gcctctccct gtctccgggt aaa 1353

<210> 311
<211> 450
<212> PRT
<213> Homo sapiens

<400> 311
Gln Ile Thr Leu Lys Glu Ser Gly Pro Thr Leu Val Asn Pro Thr Gln
1               5                   10                  15

Thr Leu Thr Leu Thr Cys Thr Phe Ser Gly Phe Ser Phe Thr Ser Ser
            20                  25                  30

Gly Glu Ala Val Gly Trp Ile Arg Gln Pro Pro Gly Lys Ala Leu Glu
        35                  40                  45

Trp Leu Ala His Ile Tyr Trp Asp Asp Asp Pro Arg Tyr Ser Thr Ser
    50                  55                  60

Leu Lys Ser Arg Leu Thr Ile Thr Lys Asp Thr Ser Lys Asn Gln Val
65                  70                  75                  80

Val Leu Ser Met Thr Asp Ile Asp Pro Ala Asp Thr Gly Thr Tyr Tyr
                85                  90                  95

Cys Ala His Leu Arg Arg Pro Ser Leu Trp Ser Pro Tyr Tyr Leu Phe
            100                 105                 110

Asp Cys Trp Gly Gln Gly Thr Leu Val Thr Val Ser Ser Ser Thr Lys
        115                 120                 125

Gly Pro Ser Val Phe Pro Leu Ala Pro Cys Ser Arg Ser Thr Ser Glu
    130                 135                 140

Ser Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Asn Phe Gly Thr Gln Thr Tyr Thr Cys Asn
        195                 200                 205

Val Asp His Lys Pro Ser Asn Thr Lys Val Asp Lys Thr Val Glu Arg
    210                 215                 220

Lys Cys Cys Val Glu Cys Pro Pro Cys Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Gln Phe Asn Trp Tyr Val Asp Gly Val Glu Val His

275                 280                 285

Asn Ala Lys Thr Lys Pro Arg Glu Glu Gln Phe Asn Ser Thr Phe Arg
    290                 295                 300

Val Val Ser Val Leu Thr Val Val His Gln Asp Trp Leu Asn Gly Lys
305                 310                 315                 320

Glu Tyr Lys Cys Lys Val Ser Asn Lys Gly Leu Pro Ala Pro Ile Glu
                325                 330                 335

Lys Thr Ile Ser Lys Thr Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Met
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 312
<211> 639
<212> DNA
<213> Homo sapiens

<400> 312
gacatccaga tgacccagtc tccatcctcc ctgtctgcat ctgttggaga cagagtcacc      60

atcacttgcc gggcaagtca gagtattagt agctatttaa attggtatca actgaaacca     120

ggacaagccc ctaagctcct gatctatggt gcatccagtt tggaaaatgg ggtcccttca     180

cggttcagtg gcagtggctc tgggccagat ttcactctca ccattagcag tctgcaacct     240

gaagattttg caacttacta ctgtcaacag agttacaaaa tgtggtcatt cggccaaggg     300

accaaggtgg aaatccaacg aactgtggct gcaccatctg tcttcatctt cccgccatct 360

gatgagcagt tgaaatctgg aactgcctct gttgtgtgcc tgctgaataa cttctatccc 420

agagaggcca aagtacagtg gaaggtggat aacgccctcc aatcgggtaa ctcccaggag 480

agtgtcacag agcaggacag caaggacagc acctacagcc tcagcagcac cctgacgctg 540

agcaaagcag actacgagaa acacaaagtc tacgcctgcg aagtcaccca tcagggcctg 600

agctcgcccg tcacaaagag cttcaacagg ggagagtgt 639

<210> 313
<211> 213
<212> PRT
<213> Homo sapiens

<400> 313
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Leu Lys Pro Gly Gln Ala Pro Lys Leu Leu Ile
        35                  40                  45

Tyr Gly Ala Ser Ser Leu Glu Asn Gly Val Pro Ser Arg Phe Ser Gly
    50                  55                  60

Ser Gly Ser Gly Pro Asp Phe Thr Leu Thr Ile Ser Ser Leu Gln Pro
65                  70                  75                  80

Glu Asp Phe Ala Thr Tyr Tyr Cys Gln Gln Ser Tyr Lys Met Trp Ser
                85                  90                  95

Phe Gly Gln Gly Thr Lys Val Glu Ile Gln Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210> 314
<211> 1332
<212> DNA
<213> Homo sapiens

<400> 314
caggttcagc tggtgcagtc tggagttgag gtgaagaagc ctggggcctc agtgaaggtc      60

tcctgcaagg cttctggcta cacctttacc aactttggct tcagttgggt gcggcaggcc     120

ccaggacaag ggcttgagtg gctgggatgg atcaacactc ataacggtaa cacaaactat     180

gcaccgagat tccaagacag agtcaccctg accacagaca catccacgac cacagcctac     240

atggagctga ggagcctgag atctgaggac acggccgtgt attattgtgc gagagatctt     300

gggggaagta gttccgccta ctggggacag ggaaccctgg tcaccgtctc gagtgcctcc     360

accaagggcc catcggtctt ccccctggcg ccctgctcca ggagcacctc cgagagcaca     420

gccgccctgg gctgcctggt caaggactac ttccccgaac cggtgacggt gtcgtggaac     480

tcaggcgctc tgaccagcgg cgtgcacacc ttcccagctg tcctacagtc ctcaggactc     540

tactccctca gcagcgtggt gaccgtgccc tccagcaact tcggcaccca gacctacacc     600

tgcaacgtag atcacaagcc cagcaacacc aaggtggaca agacagttga gcgcaaatgt     660

tgtgtcgagt gcccaccgtg cccagcacca cctgtggcag gaccgtcagt cttcctcttc     720

cccccaaaac ccaaggacac cctcatgatc tcccggaccc ctgaggtcac gtgcgtggtg     780

gtggacgtga gccacgaaga ccccgaggtc cagttcaact ggtacgtgga cggcgtggag     840

gtgcataatg ccaagacaaa gccacgggag gagcagttca acagcacgtt ccgtgtggtc     900

agcgtcctca ccgttgtgca ccaggactgg ctgaacggca aggagtacaa gtgcaaggtc     960

tccaacaaag gcctcccagc ccccatcgag aaaaccatct ccaaaaccaa agggcagccc    1020

cgagaaccac aggtgtacac cctgccccca tcccgggagg agatgaccaa gaaccaggtc    1080

agcctgacct gcctggtcaa aggcttctac cccagcgaca tcgccgtgga gtgggagagc    1140

aatgggcagc cggagaacaa ctacaagacc acacctccca tgctggactc cgacggctcc    1200

ttcttcctct acagcaagct caccgtggac aagagcaggt ggcagcaggg gaacgtcttc    1260

tcatgctccg tgatgcatga ggctctgcac aaccactaca cgcagaagag cctctccctg    1320

tctccgggta aa                                                        1332

<210> 315
<211> 443
<212> PRT
<213> Homo sapiens

<400> 315
Gln Val Gln Leu Val Gln Ser Gly Val Glu Val Lys Lys Pro Gly Ala
1               5                   10                  15

Ser Val Lys Val Ser Cys Lys Ala Ser Gly Tyr Thr Phe Thr Asn Phe
            20                  25                  30

Gly Phe Ser Trp Val Arg Gln Ala Pro Gly Gln Gly Leu Glu Trp Leu
        35                  40                  45

Gly Trp Ile Asn Thr His Asn Gly Asn Thr Asn Tyr Ala Pro Arg Phe
    50                  55                  60

Gln Asp Arg Val Thr Leu Thr Thr Asp Thr Ser Thr Thr Thr Ala Tyr
65                  70                  75                  80

Met Glu Leu Arg Ser Leu Arg Ser Glu Asp Thr Ala Val Tyr Tyr Cys
                85                  90                  95

Ala Arg Asp Leu Gly Gly Ser Ser Ser Ala Tyr Trp Gly Gln Gly Thr
            100                 105                 110

Leu Val Thr Val Ser Ser Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Asn
            180                 185                 190

Phe Gly Thr Gln Thr Tyr Thr Cys Asn Val Asp His Lys Pro Ser Asn
        195                 200                 205

Thr Lys Val Asp Lys Thr Val Glu Arg Lys Cys Cys Val Glu Cys Pro
    210                 215                 220

Pro Cys Pro Ala Pro Pro Val Ala Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Phe Arg Val Val Ser Val Leu Thr Val
    290                 295                 300

Val His Gln Asp Trp Leu Asn Gly Lys Glu Tyr Lys Cys Lys Val Ser
305                 310                 315                 320

Asn Lys Gly Leu Pro Ala Pro Ile Glu Lys Thr Ile Ser Lys Thr Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Met Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys
        435                 440

<210> 316
<211> 645
<212> DNA
<213> Homo sapiens

<400> 316

gaaattgtgt tgacgcagtc tccaggcacc ctgtctttgt ctccagggga gagagccacc 60

ctctcttgca gggccagtca cagtggtacc aacaactaca tcgcctggta ccagcagaaa 120

cctggccagc ctcccaggct cctcatctat ggtgcatcca gcagggcccc tggcatctca 180

gagaggttca gcggcagtgg gtctgggaca gacttcactc tcatcatcag cagactggag 240

cctgaagact ttggagtata ttactgtgag cagtatggga actcacctct gacgttcggc 300

caagggacca aggtggaaat caaacgaact gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgt 645

<210> 317
<211> 215
<212> PRT
<213> Homo sapiens

<400> 317
Glu Ile Val Leu Thr Gln Ser Pro Gly Thr Leu Ser Leu Ser Pro Gly
1               5                   10                  15

Glu Arg Ala Thr Leu Ser Cys Arg Ala Ser His Ser Gly Thr Asn Asn
            20                  25                  30

Tyr Ile Ala Trp Tyr Gln Gln Lys Pro Gly Gln Pro Pro Arg Leu Leu
        35                  40                  45

Ile Tyr Gly Ala Ser Ser Arg Ala Pro Gly Ile Ser Glu Arg Phe Ser
    50                  55                  60

Gly Ser Gly Ser Gly Thr Asp Phe Thr Leu Ile Ile Ser Arg Leu Glu
65                  70                  75                  80

Pro Glu Asp Phe Gly Val Tyr Tyr Cys Glu Gln Tyr Gly Asn Ser Pro
                85                  90                  95

Leu Thr Phe Gly Gln Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 318
<211> 1353
<212> DNA
<213> Homo sapiens

<400> 318
caggtgcagc tgcaggagtc gggcccagga ctggtggagt cttcggacgt cctgtccctc      60

acctgcactg tctctggtgg ctccatcagt aatgactatt gggcctggat ccggcagacc     120

ccagggaagg gactggagtg gattgggtct gtctattaca gtggcagaac cagtttcagc     180

ccctccctca agagtcgagt cactatgtca gtggacacga ccaagaacca attcttcctg     240

aacctgggct ctctgaccgt tgcggacacg gccgtgtatt actgtgcgag aactacaagg     300

ttgtcctttg actggttagt acactactat ggaatggacg tctggggcca ggggaccacg     360

gtcaccgtct cgagtgcctc caccaagggc ccatcggtct tccccctggc gccctgctcc     420

aggagcacct ccgagagcac agccgccctg ggctgcctgg tcaaggacta cttccccgaa     480

ccggtgacgg tgtcgtggaa ctcaggcgct ctgaccagcg gcgtgcacac cttcccagct     540

gtcctacagt cctcaggact ctactccctc agcagcgtgg tgaccgtgcc ctccagcaac     600

ttcggcaccc agacctacac ctgcaacgta gatcacaagc ccagcaacac caaggtggac     660

aagacagttg agcgcaaatg ttgtgtcgag tgcccaccgt gcccagcacc acctgtggca     720

ggaccgtcag tcttcctctt ccccccaaaa cccaaggaca ccctcatgat ctcccggacc     780

cctgaggtca cgtgcgtggt ggtggacgtg agccacgaag accccgaggt ccagttcaac     840

tggtacgtgg acggcgtgga ggtgcataat gccaagacaa agccacggga ggagcagttc     900

aacagcacgt tccgtgtggt cagcgtcctc accgttgtgc accaggactg gctgaacggc     960

aaggagtaca agtgcaaggt ctccaacaaa ggcctcccag cccccatcga gaaaaccatc    1020

tccaaaacca aagggcagcc ccgagaacca caggtgtaca ccctgccccc atcccgggag    1080

gagatgacca agaaccaggt cagcctgacc tgcctggtca aaggcttcta ccccagcgac 1140

atcgccgtgg agtgggagag caatgggcag ccggagaaca actacaagac cacacctccc 1200

atgctggact ccgacggctc cttcttcctc tacagcaagc tcaccgtgga caagagcagg 1260

tggcagcagg ggaacgtctt ctcatgctcc gtgatgcatg aggctctgca caaccactac 1320

acgcagaaga gcctctccct gtctccgggt aaa 1353

<210> 319
<211> 450
<212> PRT
<213> Homo sapiens

<400> 319
Gln Val Gln Leu Gln Glu Ser Gly Pro Gly Leu Val Glu Ser Ser Asp
1               5                   10                  15

Val Leu Ser Leu Thr Cys Thr Val Ser Gly Gly Ser Ile Ser Asn Asp
            20                  25                  30

Tyr Trp Ala Trp Ile Arg Gln Thr Pro Gly Lys Gly Leu Glu Trp Ile
        35                  40                  45

Gly Ser Val Tyr Tyr Ser Gly Arg Thr Ser Phe Ser Pro Ser Leu Lys
    50                  55                  60

Ser Arg Val Thr Met Ser Val Asp Thr Thr Lys Asn Gln Phe Phe Leu
65                  70                  75                  80

Asn Leu Gly Ser Leu Thr Val Ala Asp Thr Ala Val Tyr Tyr Cys Ala
                85                  90                  95

Arg Thr Thr Arg Leu Ser Phe Asp Trp Leu Val His Tyr Tyr Gly Met
            100                 105                 110

Asp Val Trp Gly Gln Gly Thr Thr Val Thr Val Ser Ser Ser Thr Lys
        115                 120                 125

Gly Pro Ser Val Phe Pro Leu Ala Pro Cys Ser Arg Ser Thr Ser Glu
    130                 135                 140

Ser Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val

            180                 185                 190

Val Thr Val Pro Ser Ser Asn Phe Gly Thr Gln Thr Tyr Thr Cys Asn
        195                 200                 205

Val Asp His Lys Pro Ser Asn Thr Lys Val Asp Lys Thr Val Glu Arg
    210                 215                 220

Lys Cys Cys Val Glu Cys Pro Pro Cys Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Phe Asn Ser Thr Phe Arg
    290                 295                 300

Val Val Ser Val Leu Thr Val Val His Gln Asp Trp Leu Asn Gly Lys
305                 310                 315                 320

Glu Tyr Lys Cys Lys Val Ser Asn Lys Gly Leu Pro Ala Pro Ile Glu
                325                 330                 335

Lys Thr Ile Ser Lys Thr Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Met
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 320
<211> 645
<212> DNA
<213> Homo sapiens

<400> 320
gacatccaga tgacccagtc tccatcgtcc ctgactgcat ctgtaggaga cagagtcacc      60

attatttgtc gggcaagtca gcccattggc agctatttga attggtatca gcagagtcca     120

gggaaagccc ctaaactcct catctatgct gcatccactt tgcagagtgg ggtcccatca     180

aggttcagtg gcagtggatc tgggacagat ttcactctca acatcagtgg tctgcaacct     240

gaagattttg caacttacta ctgtcaacag agttacaata accctttgtt caccttcggc     300

cctgggacca aagtggatat caaacgaact gtggctgcac catctgtctt catcttcccg     360

ccatctgatg agcagttgaa atctggaact gcctctgttg tgtgcctgct gaataacttc     420

tatcccagag aggccaaagt acagtggaag gtggataacg ccctccaatc gggtaactcc     480

caggagagtg tcacagagca ggacagcaag gacagcacct acagcctcag cagcaccctg     540

acgctgagca aagcagacta cgagaaacac aaagtctacg cctgcgaagt cacccatcag     600

ggcctgagct cgcccgtcac aaagagcttc aacaggggag agtgt                     645

<210> 321
<211> 215
<212> PRT
<213> Homo sapiens

<400> 321
Asp Ile Gln Met Thr Gln Ser Pro Ser Ser Leu Thr Ala Ser Val Gly
1               5                   10                  15

Asp Arg Val Thr Ile Ile Cys Arg Ala Ser Gln Pro Ile Gly Ser Tyr
            20                  25                  30

Leu Asn Trp Tyr Gln Gln Ser Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Asn Ile Ser Gly Leu Gln Pro
65                  70                  75                  80

Glu Asp Phe Ala Thr Tyr Tyr Cys Gln Gln Ser Tyr Asn Asn Pro Leu
                85                  90                  95

Phe Thr Phe Gly Pro Gly Thr Lys Val Asp Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 322
<211> 1350
<212> DNA
<213> Homo sapiens

<400> 322
gaggtgcagc tggtggagtc tgggggaagt ctggtacggc cgggggggtc cctgcgactc      60

tcctgtgcag cctctggatt catctttgat gattatggca tgagttgggt ccgccaagtt     120

ccaggggagg gcctggagtg ggtcgccggc attacttggg atggcgtgaa cacagattat     180

gcagactccg tgaagggccg cttcaccatc tccagagaca acgccaagaa ctccctgttt     240

ctcctaatga acagtctcag agccgaggac acggccctgt atcactgtgc gagagccaga     300

tactaccaag gcactggcct ccggtcctac ctcgacgagt ggggccaggg aaccctggtc     360

accgtctcga gtgcctccac caagggccca tcggtcttcc ccctggcgcc ctgctccagg     420

agcacctccg agagcacagc cgccctgggc tgcctggtca aggactactt ccccgaaccg     480

gtgacggtgt cgtggaactc aggcgctctg accagcggcg tgcacacctt cccagctgtc     540

ctacagtcct caggactcta ctccctcagc agcgtggtga ccgtgccctc cagcaacttc     600

ggcacccaga cctacacctg caacgtagat cacaagccca gcaacaccaa ggtggacaag     660

acagttgagc gcaaatgttg tgtcgagtgc ccaccgtgcc cagcaccacc tgtggcagga 720

ccgtcagtct tcctcttccc cccaaaaccc aaggacaccc tcatgatctc ccggacccct 780

gaggtcacgt gcgtggtggt ggacgtgagc cacgaagacc ccgaggtcca gttcaactgg 840

tacgtggacg gcgtggaggt gcataatgcc aagacaaagc cacgggagga gcagttcaac 900

agcacgttcc gtgtggtcag cgtcctcacc gttgtgcacc aggactggct gaacggcaag 960

gagtacaagt gcaaggtctc caacaaaggc ctcccagccc ccatcgagaa aaccatctcc 1020

aaaaccaaag ggcagccccg agaaccacag gtgtacaccc tgcccccatc ccgggaggag 1080

atgaccaaga accaggtcag cctgacctgc ctggtcaaag gcttctaccc cagcgacatc 1140

gccgtggagt gggagagcaa tgggcagccg gagaacaact acaagaccac acctcccatg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 323
<211> 449
<212> PRT
<213> Homo sapiens

<400> 323
Glu Val Gln Leu Val Glu Ser Gly Gly Ser Leu Val Arg Pro Gly Gly
1               5                   10                  15

Ser Leu Arg Leu Ser Cys Ala Ala Ser Gly Phe Ile Phe Asp Asp Tyr
            20                  25                  30

Gly Met Ser Trp Val Arg Gln Val Pro Gly Glu Gly Leu Glu Trp Val
        35                  40                  45

Ala Gly Ile Thr Trp Asp Gly Val Asn Thr Asp Tyr Ala Asp Ser Val
    50                  55                  60

Lys Gly Arg Phe Thr Ile Ser Arg Asp Asn Ala Lys Asn Ser Leu Phe
65                  70                  75                  80

Leu Leu Met Asn Ser Leu Arg Ala Glu Asp Thr Ala Leu Tyr His Cys
                85                  90                  95

Ala Arg Ala Arg Tyr Tyr Gln Gly Thr Gly Leu Arg Ser Tyr Leu Asp
            100                 105                 110

Glu Trp Gly Gln Gly Thr Leu Val Thr Val Ser Ser Ser Thr Lys Gly
        115                 120                 125

Pro Ser Val Phe Pro Leu Ala Pro Cys Ser Arg Ser Thr Ser Glu Ser
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Asn Phe Gly Thr Gln Thr Tyr Thr Cys Asn Val
        195                 200                 205

Asp His Lys Pro Ser Asn Thr Lys Val Asp Lys Thr Val Glu Arg Lys
    210                 215                 220

Cys Cys Val Glu Cys Pro Pro Cys Pro Ala Pro Pro Val Ala Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Phe Arg Val
    290                 295                 300

Val Ser Val Leu Thr Val Val His Gln Asp Trp Leu Asn Gly Lys Glu
305                 310                 315                 320

Tyr Lys Cys Lys Val Ser Asn Lys Gly Leu Pro Ala Pro Ile Glu Lys
                325                 330                 335

Thr Ile Ser Lys Thr Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Met Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210> 324
<211> 651
<212> DNA
<213> Homo sapiens

<400> 324
gacatccaga tgacccagtc tccatcctcc gtgtctgcat ctgtaggaga aagagtcact      60

atcacttgtc gggcgactca gggtattgac aactggttgg cctggtatca gcataaacca     120

gggaaagccc cgaagctcct gatttatgct gcatccactt tgcaaagtgg ggtcccgtcc     180

aggttcagcg gcagtggatc tgggacagat ttcactctca ccatcagcag cgtgcagcct     240

gaagatgttg caacttactt ttgtcaacag gctaagaatt tccctcgggg aggattcact     300

ttcggccctg ggaccaaagt cgctctcaaa cgaactgtgg ctgcaccatc tgtcttcatc     360

ttcccgccat ctgatgagca gttgaaatct ggaactgcct ctgttgtgtg cctgctgaat     420

aacttctatc ccagagaggc caaagtacag tggaaggtgg ataacgccct ccaatcgggt     480

aactcccagg agagtgtcac agagcaggac agcaaggaca gcacctacag cctcagcagc     540

accctgacgc tgagcaaagc agactacgag aaacacaaag tctacgcctg cgaagtcacc     600

catcagggcc tgagctcgcc cgtcacaaag agcttcaaca ggggagagtg t              651

<210> 325
<211> 217
<212> PRT
<213> Homo sapiens

<400> 325
Asp Ile Gln Met Thr Gln Ser Pro Ser Ser Val Ser Ala Ser Val Gly
1               5                   10                  15

Glu Arg Val Thr Ile Thr Cys Arg Ala Thr Gln Gly Ile Asp Asn Trp
            20                  25                  30

Leu Ala Trp Tyr Gln His Lys Pro Gly Lys Ala Pro Lys Leu Leu Ile
        35                  40                  45

Tyr Ala Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Val Gln Pro
65                  70                  75                  80

Glu Asp Val Ala Thr Tyr Phe Cys Gln Gln Ala Lys Asn Phe Pro Arg
                85                  90                  95

Gly Gly Phe Thr Phe Gly Pro Gly Thr Lys Val Ala Leu Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215

<210> 326
<211> 1332
<212> DNA
<213> Homo sapiens

<400> 326
caggtgcagc tggtggagtc tgggggaggc gtggtccagc ctgggaggtc cctgagactc      60

tcctgtgcag cctctggatt caccttcagt aaatttgcca tgcactgggt ccgccaggct     120

ccaggcaagg ggctggagtg ggtggcagct atagcgtctg atggatataa taaatactat     180

gcagactccg tgaagggcca attcaccgtc tccagagaca cttccaggaa cacgctgtat     240

ctccaaatgg acagcctgag acctgatgac acggctattt attactgtgc gtacggtcac 300

ttgtcttttt ggttcgaccc ctggggccag ggaaccctgg tcaccgtctc gagtgcctcc 360

accaagggcc catcggtctt ccccctggcg ccctgctcca ggagcacctc cgagagcaca 420

gccgccctgg gctgcctggt caaggactac ttccccgaac cggtgacggt gtcgtggaac 480

tcaggcgctc tgaccagcgg cgtgcacacc ttcccagctg tcctacagtc ctcaggactc 540

tactccctca gcagcgtggt gaccgtgccc tccagcaact tcggcaccca gacctacacc 600

tgcaacgtag atcacaagcc cagcaacacc aaggtggaca agacagttga gcgcaaatgt 660

tgtgtcgagt gcccaccgtg cccagcacca cctgtggcag gaccgtcagt cttcctcttc 720

cccccaaaac ccaaggacac cctcatgatc tcccggaccc ctgaggtcac gtgcgtggtg 780

gtggacgtga gccacgaaga ccccgaggtc cagttcaact ggtacgtgga cggcgtggag 840

gtgcataatg ccaagacaaa gccacgggag gagcagttca acagcacgtt ccgtgtggtc 900

agcgtcctca ccgttgtgca ccaggactgg ctgaacggca aggagtacaa gtgcaaggtc 960

tccaacaaag gcctcccagc ccccatcgag aaaaccatct ccaaaaccaa agggcagccc 1020

cgagaaccac aggtgtacac cctgccccca tcccgggagg agatgaccaa gaaccaggtc 1080

agcctgacct gcctggtcaa aggcttctac cccagcgaca tcgccgtgga gtgggagagc 1140

aatgggcagc cggagaacaa ctacaagacc acacctccca tgctggactc cgacggctcc 1200

ttcttcctct acagcaagct caccgtggac aagagcaggt ggcagcaggg gaacgtcttc 1260

tcatgctccg tgatgcatga ggctctgcac aaccactaca cgcagaagag cctctccctg 1320

tctccgggta aa 1332

<210> 327
<211> 443
<212> PRT
<213> Homo sapiens

<400> 327
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Lys Phe
            20                  25                  30

Ala Met His Trp Val Arg Gln Ala Pro Gly Lys Gly Leu Glu Trp Val
        35                  40                  45

Ala Ala Ile Ala Ser Asp Gly Tyr Asn Lys Tyr Tyr Ala Asp Ser Val
    50                  55                  60

Lys Gly Gln Phe Thr Val Ser Arg Asp Thr Ser Arg Asn Thr Leu Tyr

65                  70                  75                  80

Leu Gln Met Asp Ser Leu Arg Pro Asp Asp Thr Ala Ile Tyr Tyr Cys
                85                  90                  95

Ala Tyr Gly His Leu Ser Phe Trp Phe Asp Pro Trp Gly Gln Gly Thr
            100                 105                 110

Leu Val Thr Val Ser Ser Ser Thr Lys Gly Pro Ser Val Phe Pro Leu
        115                 120                 125

Ala Pro Cys Ser Arg Ser Thr Ser Glu Ser Thr Ala Ala Leu Gly Cys
    130                 135                 140

Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Asn
            180                 185                 190

Phe Gly Thr Gln Thr Tyr Thr Cys Asn Val Asp His Lys Pro Ser Asn
        195                 200                 205

Thr Lys Val Asp Lys Thr Val Glu Arg Lys Cys Cys Val Glu Cys Pro
    210                 215                 220

Pro Cys Pro Ala Pro Pro Val Ala Gly Pro Ser Val Phe Leu Phe Pro
225                 230                 235                 240

Pro Lys Pro Lys Asp Thr Leu Met Ile Ser Arg Thr Pro Glu Val Thr
                245                 250                 255

Cys Val Val Val Asp Val Ser His Glu Asp Pro Glu Val Gln Phe Asn
            260                 265                 270

Trp Tyr Val Asp Gly Val Glu Val His Asn Ala Lys Thr Lys Pro Arg
        275                 280                 285

Glu Glu Gln Phe Asn Ser Thr Phe Arg Val Val Ser Val Leu Thr Val
    290                 295                 300

Val His Gln Asp Trp Leu Asn Gly Lys Glu Tyr Lys Cys Lys Val Ser
305                 310                 315                 320

Asn Lys Gly Leu Pro Ala Pro Ile Glu Lys Thr Ile Ser Lys Thr Lys
                325                 330                 335

Gly Gln Pro Arg Glu Pro Gln Val Tyr Thr Leu Pro Pro Ser Arg Glu
            340                 345                 350

Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380

Asn Asn Tyr Lys Thr Thr Pro Pro Met Leu Asp Ser Asp Gly Ser Phe
385                 390                 395                 400

Phe Leu Tyr Ser Lys Leu Thr Val Asp Lys Ser Arg Trp Gln Gln Gly
                405                 410                 415

Asn Val Phe Ser Cys Ser Val Met His Glu Ala Leu His Asn His Tyr
            420                 425                 430

Thr Gln Lys Ser Leu Ser Leu Ser Pro Gly Lys
        435                 440

<210> 328
<211> 642
<212> DNA
<213> Homo sapiens

<400> 328
gacatccaga tgacccagtc tccatcctcc ctgtccgcat ctgtaggaga cagagtcacc      60

atcacttgcc gggcaagtca ggacattgga aatggcttag gctggtatca acagatacca     120

gggaaagccc ctaagcgcct gatctatgct gcatcctctt tgcaaagtgg ggtcccatca     180

aggttcagcg gcagtggttc tgggacagag ttcactctca caatccacag cctgcagcct     240

gaagattttg caacttatta ctgtctacag cataattttt accctcgcac ttttggccag     300

gggaccaagc tggagatcaa gcgaactgtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gt                        642

<210> 329
<211> 214

<212> PRT
<213> Homo sapiens

<400> 329
Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Asn Gly
            20                  25                  30

Leu Gly Trp Tyr Gln Gln Ile Pro Gly Lys Ala Pro Lys Arg Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Glu Phe Thr Leu Thr Ile His Ser Leu Gln Pro
65                  70                  75                  80

Glu Asp Phe Ala Thr Tyr Tyr Cys Leu Gln His Asn Phe Tyr Pro Arg
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 330
<211> 1344
<212> DNA

<213> Homo sapiens

<400> 330
caggtgcagc tggtgcagtc tggggctgag gtgaagaagc ctggggcctc agtgaaggtc 60

tcctgcaagg cttctggata caccttcacc gactactata tacactgggt gcgacaggcc 120

cctggacaag ggcttgagtg gatgggatgg atcaaccctt atagtggtgg cacaaactat 180

gcaccgaagt ttcagggcag gttcaccatg gccagggaca cgtccatcac cacaacctac 240

ttggacctgg gcaggctgac gtctagcgac acggccattt atttctgtgc gagagatttc 300

ttcagagcag cagctggcac ggccggagac tactggggcc agggaaccct ggtcaccgtc 360

tcgagtgcct ccaccaaggg cccatcggtc ttccccctgg cgccctgctc caggagcacc 420

tccgagagca cagccgccct gggctgcctg gtcaaggact acttccccga accggtgacg 480

gtgtcgtgga actcaggcgc tctgaccagc ggcgtgcaca ccttcccagc tgtcctacag 540

tcctcaggac tctactccct cagcagcgtg gtgaccgtgc cctccagcaa cttcggcacc 600

cagacctaca cctgcaacgt agatcacaag cccagcaaca ccaaggtgga caagacagtt 660

gagcgcaaat gttgtgtcga gtgcccaccg tgcccagcac cacctgtggc aggaccgtca 720

gtcttcctct tccccccaaa acccaaggac accctcatga tctcccggac ccctgaggtc 780

acgtgcgtgg tggtggacgt gagccacgaa gaccccgagg tccagttcaa ctggtacgtg 840

gacggcgtgg aggtgcataa tgccaagaca aagccacggg aggagcagtt caacagcacg 900

ttccgtgtgg tcagcgtcct caccgttgtg caccaggact ggctgaacgg caaggagtac 960

aagtgcaagg tctccaacaa aggcctccca gcccccatcg agaaaaccat ctccaaaacc 1020

aaagggcagc cccgagaacc acaggtgtac accctgcccc catcccggga ggagatgacc 1080

aagaaccagg tcagcctgac ctgcctggtc aaaggcttct accccagcga catcgccgtg 1140

gagtgggaga gcaatgggca gccggagaac aactacaaga ccacacctcc catgctggac 1200

tccgacggct ccttcttcct ctacagcaag ctcaccgtgg acaagagcag gtggcagcag 1260

gggaacgtct tctcatgctc cgtgatgcat gaggctctgc acaaccacta cacgcagaag 1320

agcctctccc tgtctccggg taaa 1344

<210> 331
<211> 447
<212> PRT
<213> Homo sapiens

<400> 331
Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Tyr Ile His Trp Val Arg Gln Ala Pro Gly Gln Gly Leu Glu Trp Met
        35                  40                  45

Gly Trp Ile Asn Pro Tyr Ser Gly Gly Thr Asn Tyr Ala Pro Lys Phe
    50                  55                  60

Gln Gly Arg Phe Thr Met Ala Arg Asp Thr Ser Ile Thr Thr Thr Tyr
65                  70                  75                  80

Leu Asp Leu Gly Arg Leu Thr Ser Ser Asp Thr Ala Ile Tyr Phe Cys
                85                  90                  95

Ala Arg Asp Phe Phe Arg Ala Ala Ala Gly Thr Ala Gly Asp Tyr Trp
            100                 105                 110

Gly Gln Gly Thr Leu Val Thr Val Ser Ser Ser Thr Lys Gly Pro Ser
        115                 120                 125

Val Phe Pro Leu Ala Pro Cys Ser Arg Ser Thr Ser Glu Ser Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Asn Phe Gly Thr Gln Thr Tyr Thr Cys Asn Val Asp His
        195                 200                 205

Lys Pro Ser Asn Thr Lys Val Asp Lys Thr Val Glu Arg Lys Cys Cys
    210                 215                 220

Val Glu Cys Pro Pro Cys Pro Ala Pro Pro Val Ala Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Phe Arg Val Val Ser
    290                 295                 300

Val Leu Thr Val Val His Gln Asp Trp Leu Asn Gly Lys Glu Tyr Lys
305                 310                 315                 320

Cys Lys Val Ser Asn Lys Gly Leu Pro Ala Pro Ile Glu Lys Thr Ile
                325                 330                 335

Ser Lys Thr Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Met Leu Asp Ser
385                 390                 395                 400

Asp Gly Ser Phe Phe Leu Tyr Ser Lys Leu Thr Val Asp Lys Ser Arg
                405                 410                 415

Trp Gln Gln Gly Asn Val Phe Ser Cys Ser Val Met His Glu Ala Leu
            420                 425                 430

His Asn His Tyr Thr Gln Lys Ser Leu Ser Leu Ser Pro Gly Lys
        435                 440                 445

<210> 332
<211> 657
<212> DNA
<213> Homo sapiens

<400> 332
gacatcgtga tgacccagtc tccagaagac tccctggctg tgtctctggg cgagagggcc      60

accatcaact gctccagcca gagtgcttta tacagcccca gcagtaagac ctacttagct     120

tggtaccagc agagaccggg acaccctcct aagcttctca tttactgggc atctacccgg     180

gtatccgggg tccctgaccg attcagtggc agcgggtctg ggacagattt cactctcacc     240

atcggcagcc tgcaggctga agatgtggca gtttattact gtcagtacta ttatggtact     300

gtcactttcg gcggagggac caaggtggag atcaaacgaa ctgtggctgc accatctgtc     360

ttcatcttcc cgccatctga tgagcagttg aaatctggaa ctgcctctgt tgtgtgcctg     420

ctgaataact tctatcccag agaggccaaa gtacagtgga aggtggataa cgccctccaa     480

tcgggtaact cccaggagag tgtcacagag caggacagca aggacagcac ctacagcctc 540

agcagcaccc tgacgctgag caaagcagac tacgagaaac acaaagtcta cgcctgcgaa 600

gtcacccatc agggcctgag ctcgcccgtc acaaagagct tcaacagggg agagtgt 657

<210> 333
<211> 219
<212> PRT
<213> Homo sapiens

<400> 333
Ala Asp Ile Val Met Thr Gln Ser Pro Asp Ser Leu Ala Val Ser Leu
1               5                   10                  15

Gly Glu Arg Ala Thr Ile Asn Cys Ser Ser Gln Ser Ala Leu Tyr Ser
            20                  25                  30

Pro Ser Ser Lys Thr Tyr Leu Ala Trp Tyr Gln Gln Arg Pro Gly His
        35                  40                  45

Pro Pro Lys Leu Leu Ile Tyr Trp Ala Ser Thr Arg Val Ser Gly Val
    50                  55                  60

Pro Asp Arg Phe Ser Gly Ser Gly Ser Gly Thr Asp Phe Thr Leu Thr
65                  70                  75                  80

Ile Gly Ser Leu Gln Ala Glu Asp Val Ala Val Tyr Tyr Cys Gln Tyr
                85                  90                  95

Tyr Tyr Gly Thr Val Thr Phe Gly Gly Gly Thr Lys Val Glu Ile Lys
            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser

195                 200                 205

Pro Val Thr Lys Ser Phe Asn Arg Gly Glu Cys
    210                 215

<210> 334
<211> 1353
<212> DNA
<213> Homo sapiens

<400> 334
caggtgcagc tgcaggagtc gggcccaaga ctggtgaagc cttcggagac cctgtccctc      60

acctgcactg tctctggtgg gtccatcaat agttattatt ggagttggat ccggcagccc     120

gtcgggaagg gactggagtg gattgggcgt atgtatcccg gtggtacctc cagttacaat     180

tcatccctca agagtcgagt catcatgtcg gtagacacgt cgaagaacca gttttctctg     240

aacctcactt ctgtgaccgc cgcagacacg gccgtatatt tctgtgcgag agatccaaca     300

tatcaccatg gcactagtgg ttatgtgggg acttttgatc tctggggcca agggacaatg     360

gtcaccgtct cgagtgcctc caccaagggc ccatcggtct tccccctggc gccctgctcc     420

aggagcacct ccgagagcac agccgccctg ggctgcctgg tcaaggacta cttccccgaa     480

ccggtgacgg tgtcgtggaa ctcaggcgct ctgaccagcg gcgtgcacac cttcccagct     540

gtcctacagt cctcaggact ctactccctc agcagcgtgg tgaccgtgcc ctccagcaac     600

ttcggcaccc agacctacac ctgcaacgta gatcacaagc ccagcaacac caaggtggac     660

aagacagttg agcgcaaatg ttgtgtcgag tgcccaccgt gcccagcacc acctgtggca     720

ggaccgtcag tcttcctctt ccccccaaaa cccaaggaca ccctcatgat ctcccggacc     780

cctgaggtca cgtgcgtggt ggtggacgtg agccacgaag accccgaggt ccagttcaac     840

tggtacgtgg acggcgtgga ggtgcataat gccaagacaa agccacggga ggagcagttc     900

aacagcacgt tccgtgtggt cagcgtcctc accgttgtgc accaggactg gctgaacggc     960

aaggagtaca agtgcaaggt ctccaacaaa ggcctcccag cccccatcga gaaaaccatc    1020

tccaaaacca aagggcagcc ccgagaacca caggtgtaca ccctgccccc atcccgggag    1080

gagatgacca agaaccaggt cagcctgacc tgcctggtca aaggcttcta ccccagcgac    1140

atcgccgtgg agtgggagag caatgggcag ccggagaaca actacaagac cacacctccc    1200

atgctggact ccgacggctc cttcttcctc tacagcaagc tcaccgtgga caagagcagg    1260

tggcagcagg ggaacgtctt ctcatgctcc gtgatgcatg aggctctgca caaccactac    1320

acgcagaaga gcctctccct gtctccgggt aaa                                 1353

<210> 335
<211> 450
<212> PRT

<213> Homo sapiens

<400> 335
Gln Val Gln Leu Gln Glu Ser Gly Pro Arg Leu Val Lys Pro Ser Glu
1               5                   10                  15

Thr Leu Ser Leu Thr Cys Thr Val Ser Gly Gly Ser Ile Asn Ser Tyr
            20                  25                  30

Tyr Trp Ser Trp Ile Arg Gln Pro Val Gly Lys Gly Leu Glu Trp Ile
        35                  40                  45

Gly Arg Met Tyr Pro Gly Gly Thr Ser Ser Tyr Asn Ser Ser Leu Lys
    50                  55                  60

Ser Arg Val Ile Met Ser Val Asp Thr Ser Lys Asn Gln Phe Ser Leu
65                  70                  75                  80

Asn Leu Thr Ser Val Thr Ala Ala Asp Thr Ala Val Tyr Phe Cys Ala
                85                  90                  95

Arg Asp Pro Thr Tyr His His Gly Thr Ser Gly Tyr Val Gly Thr Phe
            100                 105                 110

Asp Leu Trp Gly Gln Gly Thr Met Val Thr Val Ser Ser Ser Thr Lys
        115                 120                 125

Gly Pro Ser Val Phe Pro Leu Ala Pro Cys Ser Arg Ser Thr Ser Glu
    130                 135                 140

Ser Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Asn Phe Gly Thr Gln Thr Tyr Thr Cys Asn
        195                 200                 205

Val Asp His Lys Pro Ser Asn Thr Lys Val Asp Lys Thr Val Glu Arg
    210                 215                 220

Lys Cys Cys Val Glu Cys Pro Pro Cys Pro Ala Pro Pro Val Ala Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Gln Phe Asn Trp Tyr Val Asp Gly Val Glu Val His
        275                 280                 285

Asn Ala Lys Thr Lys Pro Arg Glu Glu Gln Phe Asn Ser Thr Phe Arg
    290                 295                 300

Val Val Ser Val Leu Thr Val Val His Gln Asp Trp Leu Asn Gly Lys
305                 310                 315                 320

Glu Tyr Lys Cys Lys Val Ser Asn Lys Gly Leu Pro Ala Pro Ile Glu
                325                 330                 335

Lys Thr Ile Ser Lys Thr Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Met
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 336
<211> 645
<212> DNA
<213> Homo sapiens

<400> 336
gaaatagtga tgacgcagtc tccagccacc ctgtctgcgt ctccagggga aagagtcacc        60

ctctcctgca gggccagtcc gagtgttcgc aacaatttag cctggtacca gcagaaacct 120

ggccagcctc ctaggctcct catctatggt gcatccacca aggtcatggg tactccaacc 180

aggttcagtg gcagtgggtc tgggacagag ttcactctca ccatcagcag cctgcagtct 240

gaagattttg cagtttatta ctgtcagcag tataatagct ggcctccgta cacttttagc 300

caggggacca aactggagat caaacgaact gtggctgcac catctgtctt catcttcccg 360

ccatctgatg agcagttgaa atctggaact gcctctgttg tgtgcctgct gaataacttc 420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgt 645

<210> 337
<211> 215
<212> PRT
<213> Homo sapiens

<400> 337
Asp Ile Val Met Thr Gln Ser Pro Ala Thr Leu Ser Ala Ser Pro Gly
1               5                   10                  15

Glu Arg Val Thr Leu Ser Cys Arg Ala Ser Pro Ser Val Arg Asn Asn
            20                  25                  30

Leu Ala Trp Tyr Gln Gln Lys Pro Gly Gln Pro Pro Arg Leu Leu Ile
        35                  40                  45

Tyr Gly Ala Ser Thr Lys Val Met Gly Thr Pro Thr Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Ser Trp Pro Pro
                85                  90                  95

Tyr Thr Phe Ser Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 338
<211> 1323
<212> DNA
<213> Homo sapiens

<400> 338
gaggtgcagc tggtggagtc ggggggaggc ctggtcaggc ctggggagtc cctgagactc      60

tcctgtgcag gctctggatt caccttcagc atgtttaccc tgaactgggt ccgccaggct     120

ccagggaagg gactggagtg ggtcgcatct attagtggta gtagcagttt agtgtttcat     180

gaagactcag tgaagggccg attcaccatc tccagagaca acgccaagaa ctcagtatat     240

ctgcaaatga acagcctgag agccgaagac acggctattt attactgtgt gagaggagat     300

agtcgcgact attggggcca gggaaccctg gtcaccgtct cgagtgcctc caccaagggc     360

ccatcggtct tccccctggc gccctgctcc aggagcacct ccgagagcac agccgccctg     420

ggctgcctgg tcaaggacta cttccccgaa ccggtgacgg tgtcgtggaa ctcaggcgct     480

ctgaccagcg gcgtgcacac cttcccagct gtcctacagt cctcaggact ctactccctc     540

agcagcgtgg tgaccgtgcc ctccagcaac ttcggcaccc agacctacac ctgcaacgta     600

gatcacaagc ccagcaacac caaggtggac aagacagttg agcgcaaatg ttgtgtcgag     660

tgcccaccgt gcccagcacc acctgtggca ggaccgtcag tcttcctctt ccccccaaaa     720

cccaaggaca ccctcatgat ctcccggacc cctgaggtca cgtgcgtggt ggtggacgtg     780

agccacgaag accccgaggt ccagttcaac tggtacgtgg acggcgtgga ggtgcataat     840

gccaagacaa agccacggga ggagcagttc aacagcacgt tccgtgtggt cagcgtcctc     900

accgttgtgc accaggactg gctgaacggc aaggagtaca agtgcaaggt ctccaacaaa     960

ggcctcccag cccccatcga gaaaaccatc tccaaaacca aagggcagcc ccgagaacca    1020

caggtgtaca ccctgccccc atcccgggag gagatgacca agaaccaggt cagcctgacc    1080

tgcctggtca aaggcttcta ccccagcgac atcgccgtgg agtgggagag caatgggcag 1140

ccggagaaca actacaagac cacacctccc atgctggact ccgacggctc cttcttcctc 1200

tacagcaagc tcaccgtgga caagagcagg tggcagcagg ggaacgtctt ctcatgctcc 1260

gtgatgcatg aggctctgca caaccactac acgcagaaga gcctctccct gtctccgggt 1320

aaa 1323

<210> 339
<211> 440
<212> PRT
<213> Homo sapiens

<400> 339
Glu Val Gln Leu Val Glu Ser Gly Gly Gly Leu Val Arg Pro Gly Glu
1               5                   10                  15

Ser Leu Arg Leu Ser Cys Ala Gly Ser Gly Phe Thr Phe Ser Met Phe
            20                  25                  30

Thr Leu Asn Trp Val Arg Gln Ala Pro Gly Lys Gly Leu Glu Trp Val
        35                  40                  45

Ala Ser Ile Ser Gly Ser Ser Ser Leu Val Phe His Glu Asp Ser Val
    50                  55                  60

Lys Gly Arg Phe Thr Ile Ser Arg Asp Asn Ala Lys Asn Ser Val Tyr
65                  70                  75                  80

Leu Gln Met Asn Ser Leu Arg Ala Glu Asp Thr Ala Ile Tyr Tyr Cys
                85                  90                  95

Val Arg Gly Asp Ser Arg Asp Tyr Trp Gly Gln Gly Thr Leu Val Thr
            100                 105                 110

Val Ser Ser Ser Thr Lys Gly Pro Ser Val Phe Pro Leu Ala Pro Cys
        115                 120                 125

Ser Arg Ser Thr Ser Glu Ser Thr Ala Ala Leu Gly Cys Leu Val Lys
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Asn Phe Gly Thr
            180                 185                 190

Gln Thr Tyr Thr Cys Asn Val Asp His Lys Pro Ser Asn Thr Lys Val
        195                 200                 205

Asp Lys Thr Val Glu Arg Lys Cys Cys Val Glu Cys Pro Pro Cys Pro
    210                 215                 220

Ala Pro Pro Val Ala Gly Pro Ser Val Phe Leu Phe Pro Pro Lys Pro
225                 230                 235                 240

Lys Asp Thr Leu Met Ile Ser Arg Thr Pro Glu Val Thr Cys Val Val
                245                 250                 255

Val Asp Val Ser His Glu Asp Pro Glu Val Gln Phe Asn Trp Tyr Val
            260                 265                 270

Asp Gly Val Glu Val His Asn Ala Lys Thr Lys Pro Arg Glu Glu Gln
        275                 280                 285

Phe Asn Ser Thr Phe Arg Val Val Ser Val Leu Thr Val Val His Gln
    290                 295                 300

Asp Trp Leu Asn Gly Lys Glu Tyr Lys Cys Lys Val Ser Asn Lys Gly
305                 310                 315                 320

Leu Pro Ala Pro Ile Glu Lys Thr Ile Ser Lys Thr Lys Gly Gln Pro
                325                 330                 335

Arg Glu Pro Gln Val Tyr Thr Leu Pro Pro Ser Arg Glu Glu Met Thr
            340                 345                 350

Lys Asn Gln Val Ser Leu Thr Cys Leu Val Lys Gly Phe Tyr Pro Ser
        355                 360                 365

Asp Ile Ala Val Glu Trp Glu Ser Asn Gly Gln Pro Glu Asn Asn Tyr
    370                 375                 380

Lys Thr Thr Pro Pro Met Leu Asp Ser Asp Gly Ser Phe Phe Leu Tyr
385                 390                 395                 400

Ser Lys Leu Thr Val Asp Lys Ser Arg Trp Gln Gln Gly Asn Val Phe
                405                 410                 415

Ser Cys Ser Val Met His Glu Ala Leu His Asn His Tyr Thr Gln Lys
            420                 425                 430

Ser Leu Ser Leu Ser Pro Gly Lys

435                    440

<210> 340
<211> 648
<212> DNA
<213> Homo sapiens

<400> 340
cagtctgtgt tgacgcagcc gccctcagtg tctgcggccc caggacagaa ggtcaccatc      60

tcctgctctg gaaggacctc caatattgga aataaccatg tatcctggta ccagcaattc     120

ccaggaacag cccccaaagt cctcatttat cgcaatgata ggcgaccctc agggattcct     180

gatcgattct ctggctccaa gtctgacacg tcagccaccc tgggcatcac cgggctccag     240

actggggacg aggccgatta ttactgcgga acatgggaca acagcctgag tggttgggtg     300

ttcggcggag ggaccaagct gaccgtccta ggtcagccca aggctgcccc ctcggtcact     360

ctgttcccgc cctcctctga ggagcttcaa gccaacaagg ccacactggt gtgtctcata     420

agtgacttct acccgggagt cgtgacagtg gcctggaagg cagatagcag ccccgtcaag     480

gcgggagtgg agaccaccac accctccaaa caaagcaaca acaagtacgc ggccagcagc     540

tacctgagcc tgacgcctga gcagtggaag tcccacaaaa gctacagctg ccaggtcacg     600

catgaaggga gcaccgtgga gaagacagtg gcccctgcag aatgctca                  648

<210> 341
<211> 216
<212> PRT
<213> Homo sapiens

<400> 341
Gln Ser Val Leu Thr Gln Pro Pro Ser Val Ser Ala Ala Pro Gly Gln
1               5                   10                  15

Lys Val Thr Ile Ser Cys Ser Gly Arg Thr Ser Asn Ile Gly Asn Asn
            20                  25                  30

His Val Ser Trp Tyr Gln Gln Phe Pro Gly Thr Ala Pro Lys Val Leu
        35                  40                  45

Ile Tyr Arg Asn Asp Arg Arg Pro Ser Gly Ile Pro Asp Arg Phe Ser
    50                  55                  60

Gly Ser Lys Ser Asp Thr Ser Ala Thr Leu Gly Ile Thr Gly Leu Gln
65                  70                  75                  80

Thr Gly Asp Glu Ala Asp Tyr Tyr Cys Gly Thr Trp Asp Asn Ser Leu
                85                  90                  95

Ser Gly Trp Val Phe Gly Gly Gly Thr Lys Leu Thr Val Leu Gly Gln

100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Val Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Lys Ser Tyr Ser Cys Gln Val Thr His Glu Gly Ser Thr Val Glu Lys
        195                 200                 205

Thr Val Ala Pro Ala Glu Cys Ser
    210                 215

<210> 342
<211> 1356
<212> DNA
<213> Homo sapiens

<400> 342
caggtgcagc tggtgcagtc tggggctgag gtgaagaggc ctggggcctc agtgaaggtc      60

tcctgtgaga cttctggata tagcttcacc actcatgatt tgaactgggt gcgacaggcc     120

gctggacagg ggcttgagtg gatgggatgg atgaacccta acactggtga cacatcctac     180

gcacagaggt tccagggcag agtcaccatg atcagggacg tctccataag tacagcctac     240

ttggaattga caggcctgac atttgatgac acggccgtct acttctgtgc gaggtcgaat     300

tctgcacctg tttcaactct tttacccgct gatgcttttg atgtctgggg ccaagggaca     360

atggtcaccg tctcgagtgc ctccaccaag ggcccatcgg tcttccccct ggcgccctgc     420

tccaggagca cctccgagag cacagccgcc ctgggctgcc tggtcaagga ctacttcccc     480

gaaccggtga cggtgtcgtg gaactcaggc gctctgacca gcggcgtgca caccttccca     540

gctgtcctac agtcctcagg actctactcc ctcagcagcg tggtgaccgt gccctccagc     600

aacttcggca cccagaccta cacctgcaac gtagatcaca agcccagcaa caccaaggtg     660

gacaagacag ttgagcgcaa atgttgtgtc gagtgcccac cgtgcccagc accacctgtg     720

gcaggaccgt cagtcttcct cttcccccca aaacccaagg acaccctcat gatctcccgg     780

acccctgagg tcacgtgcgt ggtggtggac gtgagccacg aagaccccga ggtccagttc 840

aactggtacg tggacggcgt ggaggtgcat aatgccaaga caaagccacg ggaggagcag 900

ttcaacagca cgttccgtgt ggtcagcgtc ctcaccgttg tgcaccagga ctggctgaac 960

ggcaaggagt acaagtgcaa ggtctccaac aaaggcctcc cagcccccat cgagaaaacc 1020

atctccaaaa ccaaagggca gccccgagaa ccacaggtgt acaccctgcc cccatcccgg 1080

gaggagatga ccaagaacca ggtcagcctg acctgcctgg tcaaaggctt ctaccccagc 1140

gacatcgccg tggagtggga gagcaatggg cagccggaga acaactacaa gaccacacct 1200

cccatgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 343
<211> 446
<212> PRT
<213> Homo sapiens

<400> 343
Gln Val Gln Leu Val Gln Ser Gly Ala Glu Val Lys Arg Pro Gly Ala
1               5                   10                  15

Ser Val Lys Val Ser Cys Glu Thr Ser Gly Tyr Ser Phe Thr Thr Asp
            20                  25                  30

Leu Asn Trp Val Arg Gln Ala Ala Gly Gln Gly Leu Glu Trp Met Gly
        35                  40                  45

Trp Met Asn Pro Asn Thr Gly Asp Thr Ser Tyr Ala Gln Arg Phe Gln
    50                  55                  60

Gly Arg Val Thr Met Ile Arg Asp Val Ser Ile Ser Thr Ala Tyr Leu
65                  70                  75                  80

Glu Leu Thr Gly Leu Thr Phe Asp Asp Thr Ala Val Tyr Phe Cys Ala
                85                  90                  95

Arg Ser Asn Ser Ala Pro Val Ser Thr Leu Leu Pro Ala Asp Ala Phe
            100                 105                 110

Asp Val Trp Gly Gln Gly Thr Met Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn Lys Pro Ser Asn Thr Lys Val Asp Lys Lys Val Glu Pro Lys
    210                 215                 220

Ser Cys Asp Lys Thr Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

Glu Asp Pro Glu Val Lys Phe Asn Trp Tyr Val Asp Gly Val Glu Val
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met Glu Ala
            420                 425                 430

Leu Asn Tyr Thr Gln Lys Ser Leu Ser Leu Ser Pro Gly Lys
        435                 440                 445

<210> 344
<211> 645
<212> DNA
<213> Homo sapiens

<400> 344
gacatccaga tgacccagtc tccatcctcc ctgtctgcat ctataggaga cacagtcacc      60

atcacttgcc gggcaagtca gatcattaac agctatttaa attggtatca gcaaaaacca     120

ggggaagccc ctaaactcct gatctatgct gtatccaatt tgcaccgtgg ggtcccctca     180

aggttcagtg gcagtgggtc tgcggcagat ttcactctca ctatcagtgg tctgcaacct     240

gaagattttg caacttacta ctgtcaacag acttacacta ccgttgcgat caccttcggc     300

caagggacac gactggacat taaacgaact gtggctgcac catctgtctt catcttcccg     360

ccatctgatg agcagttgaa atctggaact gcctctgttg tgtgcctgct gaataacttc     420

tatcccagag aggccaaagt acagtggaag gtggataacg ccctccaatc gggtaactcc     480

caggagagtg tcacagagca ggacagcaag gacagcacct acagcctcag cagcaccctg     540

acgctgagca aagcagacta cgagaaacac aaagtctacg cctgcgaagt cacccatcag     600

ggcctgagct cgcccgtcac aaagagcttc aacaggggag agtgt                     645

<210> 345
<211> 215
<212> PRT
<213> Homo sapiens

<400> 345
Asp Ile Gln Met Thr Gln Ser Pro Ser Ser Leu Ser Ala Ser Ile Gly
1               5                   10                  15

Asp Thr Val Thr Ile Thr Cys Arg Ala Ser Gln Ile Ile Asn Ser Tyr
            20                  25                  30

Leu Asn Trp Tyr Gln Gln Lys Pro Gly Glu Ala Pro Lys Leu Leu Ile
        35                  40                  45

Tyr Ala Val Ser Asn Leu His Arg Gly Val Pro Ser Arg Phe Ser Gly
    50                  55                  60

Ser Gly Ser Ala Ala Asp Phe Thr Leu Thr Ile Ser Gly Leu Gln Pro
65                  70                  75                  80

Glu Asp Phe Ala Thr Tyr Tyr Cys Gln Gln Thr Tyr Thr Thr Val Ala
                85                  90                  95

Ile Thr Phe Gly Gln Gly Thr Arg Leu Asp Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 346
<211> 1349
<212> DNA
<213> Homo sapiens

<400> 346
caggtgcagc tggtgcagtc tggggcggag gtgaagaagc ctgggtcctc ggtgaacgtc      60

tcctgtaagg cctccgcagg gaccctcagc ggttatacca tcaactggtt gcgccaggcc     120

cctggacgag ggtttgagtg gctgggaagg atcatccctc accttggaat aggaaacaac     180

gcacataact gggaggccag agtcaccctt accgccgaca agtccacgga cactacctac     240

ctggagttgc gcgacctgag atctgacgac gtggccattt attactgcgc ggtggagttt     300

taccggctgg caacggtcac aaccccaacc cttgacttct ggggccaggg aaccctggtc     360

accgtctcga gtgcctccac caagggccca tcggtcttcc ccctggcgcc ctgctccagg     420

agcacctccg agagcacagc cgccctgggc tgcctggtca aggactactt ccccgaaccg     480

gtgacggtgt cgtggaactc aggcgctctg accagcggcg tgcacacctt cccagctgtc 540

ctacagtcct caggactcta ctccctcagc agcgtggtga ccgtgccctc cagcaacttc 600

ggcacccaga cctacacctg caacgtagat cacaagccca gcaacaccaa ggtggacaag 660

acagttgagc gcaaatgttg tgtcgagtgc ccaccgtgcc cagcaccacc tgtggcagga 720

ccgtcagtct tcctcttccc cccaaaaccc aaggacaccc tcatgatctc ccggacccct 780

gaggtcacgt gcgtggtggt ggacgtgagc cacgaagacc ccgaggtcca gttcaactgg 840

tacgtggacg gcgtggaggt gcataatgcc aagacaaagc cacgggagga gcagttcaac 900

agcacgttcc gtgtggtcag cgtcctcacc gttgtgcacc aggactggct gaacggcaag 960

gagtacaagt gcaaggtctc caacaaaggc ctcccagccc ccatcgagaa aaccatctcc 1020

aaaaccaaag ggcagccccg agaaccacag gtgtacaccc tgcccccatc ccgggaggag 1080

atgaccaaga accaggtcag cctgacctgc ctggtcaaag gcttctaccc cagcgacatc 1140

gccgtggagt gggagagcaa tgggcagccg gagaacaact acaagaccac acctcccatg 1200

ctggactccg acggctcctt cttcctctaa gcaagctcac cgtggacaag agcaggtggc 1260

agcaggggaa cgtcttctca tgctccgtga tgcatgaggc tctgcacaac cactacacgc 1320

agaagagcct ctccctgtct ccgggtaaa 1349

<210> 347
<211> 443
<212> PRT
<213> Homo sapiens

<400> 347
Gln Val Gln Leu Val Gln Ser Gly Ala Glu Val Lys Lys Pro Gly Ser
1               5                   10                  15

Ser Val Asn Val Ser Cys Lys Ala Ser Ala Gly Thr Leu Ser Gly Tyr
            20                  25                  30

Thr Ile Asn Trp Leu Arg Gln Ala Pro Gly Arg Gly Phe Glu Trp Leu
        35                  40                  45

Gly Arg Ile Ile Pro Leu Gly Ile Gly Asn Asn Ala Asn Trp Glu Ala
    50                  55                  60

Arg Val Thr Leu Thr Ala Asp Lys Ser Thr Asp Thr Thr Tyr Leu Glu
65                  70                  75                  80

Leu Arg Asp Leu Arg Ser Asp Asp Val Ala Ile Tyr Tyr Cys Ala Val
                85                  90                  95

Glu Phe Tyr Arg Leu Ala Thr Val Thr Thr Pro Thr Leu Asp Phe Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn Lys
        195                 200                 205

Pro Ser Asn Thr Lys Val Asp Lys Lys Val Glu Pro Lys Ser Cys Asp
    210                 215                 220

Lys Thr Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Glu Asp Pro
            260                 265                 270

Glu Val Lys Phe Asn Trp Tyr Val Asp Gly Val Glu Val Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Asp Glu Leu Thr Lys Asn Gln Val Ser Leu Thr Cys Leu Val

355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met Glu Ala Leu Asn Tyr
            420                 425                 430

Thr Gln Lys Ser Leu Ser Leu Ser Pro Gly Lys
        435                 440

<210> 348
<211> 642
<212> DNA
<213> Homo sapiens

<400> 348
tcctatgtgc tgactcagcc accctcggtg tccgtggccc caggaacgac ggccaggatt      60

acctgtgagg gggaccacat tggagctaaa agtgtgcact ggtaccagag gaagccaggc     120

caggcccctg tgttggtcgt ctatatgaat agtgaccggc cctcagggat ccctgagcga     180

ttgtctggct ccaactctgg gaacacggcc accctgagta tcagcagggt cgaggccgag     240

gatgaggccg actattattg tcaggtttgg gatagttttc gcgatcatca ggtattcggc     300

ggagggacca agttgaccgt cctgagtcag cccaaggctg ccccctcggt cactctgttc     360

ccgccctcct ctgaggagct tcaagccaac aaggccacac tggtgtgtct cataagtgac     420

ttctacccgg gagccgtgac agtggcctgg aaggcagata gcagccccgt caaggcggga     480

gtggagacca ccacaccctc caaacaaggc aacaacaagt acgcggccag cagctatctg     540

agcctgacgc ctgagcagtg gaagtcccac agaagctaca gctgccaggt cacgcatgaa     600

gggagcaccg tggagaagac agtggcccct acagaatgtt ca                        642

<210> 349
<211> 214
<212> PRT
<213> Homo sapiens

<400> 349
Ser Tyr Val Leu Thr Gln Pro Pro Ser Val Ser Val Ala Pro Gly Thr
1               5                   10                  15

Thr Ala Arg Ile Thr Cys Glu Gly Asp His Ile Gly Ala Lys Ser Val

20                  25                  30

His Trp Tyr Gln Arg Lys Pro Gly Gln Ala Pro Val Leu Val Val Tyr
        35                  40                  45

Met Asn Ser Asp Arg Pro Ser Gly Ile Pro Glu Arg Leu Ser Gly Ser
    50                  55                  60

Asn Ser Gly Asn Thr Ala Thr Leu Ser Ile Ser Arg Val Glu Ala Glu
65                  70                  75                  80

Asp Glu Ala Asp Tyr Tyr Cys Gln Val Trp Asp Ser Phe Arg Asp His
                85                  90                  95

Gln Val Phe Gly Gly Gly Thr Lys Leu Thr Val Leu Ser Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Gly Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210> 350
<211> 1365
<212> DNA
<213> Homo sapiens

<400> 350
caggtcacct tgaaggagtc tggtcctgct atagtgaaac ccacacagac cctcacgctg      60

acctgcacct tctctgggtt ctcactccga actagtggag tgggtgtggg ctggatccgt     120

cagcccccag gaaaggccct tgagtggctt gcactcattt actgggatga tgataagcgc     180

tacaacccat ctctgaagac cagactcacc atcaccaagg actcctccag aaaccaggtg 240

ttcctcacaa tgaccaacat ggaccctgtg gacacagcca catacttctg tgcacaccga 300

caacatatta caaacagttt tggattggtt attgcgaatg atgcttttga catttggggc 360

caagggacaa tggtcaccgt ctcgagtgcc tccaccaagg gcccatcggt cttccccctg 420

gcgccctgct ccaggagcac ctccgagagc acagccgccc tgggctgcct ggtcaaggac 480

tacttccccg aaccggtgac ggtgtcgtgg aactcaggcg ctctgaccag cggcgtgcac 540

accttcccag ctgtcctaca gtcctcagga ctctactccc tcagcagcgt ggtgaccgtg 600

ccctccagca acttcggcac ccagacctac acctgcaacg tagatcacaa gcccagcaac 660

accaaggtgg acaagacagt tgagcgcaaa tgttgtgtcg agtgcccacc gtgcccagca 720

ccacctgtgg caggaccgtc agtcttcctc ttccccccaa aacccaagga caccctcatg 780

atctcccgga cccctgaggt cacgtgcgtg gtggtggacg tgagccacga agaccccgag 840

gtccagttca actggtacgt ggacggcgtg gaggtgcata atgccaagac aaagccacgg 900

gaggagcagt tcaacagcac gttccgtgtg gtcagcgtcc tcaccgttgt gcaccaggac 960

tggctgaacg gcaaggagta caagtgcaag gtctccaaca aaggcctccc agcccccatc 1020

gagaaaacca tctccaaaac caaagggcag ccccgagaac cacaggtgta caccctgccc 1080

ccatcccggg aggagatgac caagaaccag gtcagcctga cctgcctggt caaaggcttc 1140

taccccagcg acatcgccgt ggagtgggag agcaatgggc agccggagaa caactacaag 1200

accacacctc ccatgctgga ctccgacggc tccttcttcc tctacagcaa gctcaccgtg 1260

gacaagagca ggtggcagca ggggaacgtc ttctcatgct ccgtgatgca tgaggctctg 1320

cacaaccact acacgcagaa gagcctctcc ctgtctccgg gtaaa 1365

<210> 351
<211> 448
<212> PRT
<213> Homo sapiens

<400> 351
Gln Val Thr Leu Lys Glu Ser Gly Pro Ala Ile Val Lys Pro Thr Gln
1               5                   10                  15

Thr Leu Thr Leu Thr Cys Thr Phe Ser Gly Phe Ser Leu Arg Thr Ser
            20                  25                  30

Gly Val Gly Val Gly Trp Ile Arg Gln Pro Pro Gly Lys Ala Leu Glu
        35                  40                  45

Trp Leu Ala Leu Ile Tyr Trp Asp Asp Asp Lys Arg Tyr Asn Pro Ser
    50                  55                  60

Leu Lys Thr Arg Leu Thr Ile Thr Lys Asp Ser Ser Arg Asn Gln Val
65                  70                  75                  80

Phe Leu Thr Met Thr Asn Met Asp Pro Val Asp Thr Ala Thr Tyr Phe
                85                  90                  95

Cys Ala Arg Gln Ile Thr Asn Ser Phe Gly Leu Val Ile Ala Asn Asp
            100                 105                 110

Ala Phe Asp Ile Trp Gly Gln Gly Thr Met Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val Thr Phe Pro Ala Val Leu Gln Ser Ser Gly Leu Tyr Ser Leu Ser
            180                 185                 190

Ser Val Val Thr Val Pro Ser Ser Ser Leu Gly Thr Gln Thr Tyr Ile
        195                 200                 205

Cys Asn Val Asn Lys Pro Ser Asn Thr Lys Val Asp Lys Lys Val Glu
    210                 215                 220

Pro Lys Ser Cys Asp Lys Thr Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser Glu Asp Pro Glu Val Lys Phe Asn Trp Tyr Val Asp Gly Val
        275                 280                 285

Glu Val Asn Ala Lys Thr Lys Pro Arg Glu Glu Gln Tyr Asn Ser Thr
    290                 295                 300

Tyr Arg Val Val Ser Val Leu Thr Val Leu Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

Glu Ala Leu Asn Tyr Thr Gln Lys Ser Leu Ser Leu Ser Pro Gly Lys
        435                 440                 445

<210> 352
<211> 651
<212> DNA
<213> Homo sapiens

<400> 352
cagtctgccc tgactcagcc tccgtccgcg tccgggtctc ctggacagtc agtcaccatc      60

tcctgcactg gaaccagcag tgacgttggt cgttataatt atgtcgcctg gtatcaacaa     120

cacccaggca gagcccccag actcataatt tatggggtca ctaagcggcc ctcaggggtc     180

cctgatcgct tctctggctc caagtctggc aacacggcct ccctgaccgt ctctgggctc     240

caggctgaag atgaggctga ttattattgc agttcatatg caggcagcaa ctttttggag     300

gtgttcggcg gagggaccaa gctgaccgtc ctaggtcagc ccaaggctgc cccctcggtc     360

actctgttcc cgccctcctc tgaggagctt caagccaaca aggccacact ggtgtgtctc     420

ataagtgact tctacccggg agccgtgaca gtggcctgga aggcagatag cagccccgtc     480

aaggcgggag tggagaccac cacaccctcc aaacaaagca acaacaagta cgcggccagc     540

agctacctga gcctgacgcc tgagcagtgg aagtcccaca gaagctacag ctgccaggtc     600

acgcatgaag ggagcaccgt ggagaagaca gttgccccta cggaatgttc a              651

<210> 353

<211> 217
<212> PRT
<213> Homo sapiens

<400> 353
Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Arg Tyr
            20                  25                  30

Asn Tyr Val Ala Trp Tyr Gln Gln His Pro Gly Arg Ala Pro Arg Leu
        35                  40                  45

Ile Ile Tyr Gly Val Thr Lys Arg Pro Ser Gly Val Pro Asp Arg Phe
    50                  55                  60

Ser Gly Ser Lys Ser Gly Asn Thr Ala Ser Leu Thr Val Ser Gly Leu
65                  70                  75                  80

Gln Ala Glu Asp Glu Ala Asp Tyr Tyr Cys Ser Ser Tyr Ala Gly Ser
                85                  90                  95

Asn Phe Leu Glu Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210> 354
<211> 1356

<212> DNA
<213> Homo sapiens

<400> 354
caggtgcagc tggtgcagtc tggggctgag gtgaagaagc ctgggtcctc ggtgaaggtc 60

tcctgcaagg cttctggagg caccttcagc agctatgcaa tcaactgggt gcgacaggcc 120

cctggacaag gtcttgagtg gatgggaggg atcatcccta tgtttgatac agcaatctac 180

gcacagatgt tccagggcag agtcaccatc accgcggacg aatccacgag cacagcctac 240

atggagctga gcagcctgag atctgacgac acggccgtat attactgtgc gagagatcga 300

aacgattatt atgacagtag tggttattca ggcgcctttg accagtgggg ccagggaacc 360

ctggtcaccg tctcgagtgc ctccaccaag ggcccatcgg tcttccccct ggcgccctgc 420

tccaggagca cctccgagag cacagccgcc ctgggctgcc tggtcaagga ctacttcccc 480

gaaccggtga cggtgtcgtg gaactcaggc gctctgacca gcggcgtgca caccttccca 540

gctgtcctac agtcctcagg actctactcc ctcagcagcg tggtgaccgt gccctccagc 600

aacttcggca cccagaccta cacctgcaac gtagatcaca agcccagcaa caccaaggtg 660

gacaagacag ttgagcgcaa atgttgtgtc gagtgcccac cgtgcccagc accacctgtg 720

gcaggaccgt cagtcttcct cttcccccca aaacccaagg acaccctcat gatctcccgg 780

acccctgagg tcacgtgcgt ggtggtggac gtgagccacg aagaccccga ggtccagttc 840

aactggtacg tggacggcgt ggaggtgcat aatgccaaga caaagccacg ggaggagcag 900

ttcaacagca cgttccgtgt ggtcagcgtc ctcaccgttg tgcaccagga ctggctgaac 960

ggcaaggagt acaagtgcaa ggtctccaac aaaggcctcc cagcccccat cgagaaaacc 1020

atctccaaaa ccaaagggca gccccgagaa ccacaggtgt acaccctgcc cccatcccgg 1080

gaggagatga ccaagaacca ggtcagcctg acctgcctgg tcaaaggctt ctaccccagc 1140

gacatcgccg tggagtggga gagcaatggg cagccggaga acaactacaa gaccacacct 1200

cccatgctgg actccgacgg ctccttcttc ctctacagca agctcaccgt ggacaagagc 1260

aggtggcagc aggggaacgt cttctcatgc tccgtgatgc atgaggctct gcacaaccac 1320

tacacgcaga agagcctctc cctgtctccg ggtaaa 1356

<210> 355
<211> 447
<212> PRT
<213> Homo sapiens

<400> 355
Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Asn Trp Val Arg Gln Ala Pro Gly Gln Gly Leu Glu Trp Met
        35                  40                  45

Gly Gly Ile Ile Pro Met Phe Asp Thr Ala Ile Tyr Ala Gln Met Phe
    50                  55                  60

Gln Gly Arg Val Thr Ile Thr Ala Asp Glu Ser Thr Ser Thr Ala Tyr
65                  70                  75                  80

Met Glu Leu Ser Ser Leu Arg Ser Asp Asp Thr Ala Val Tyr Tyr Cys
                85                  90                  95

Ala Arg Asp Arg Asn Asp Tyr Tyr Asp Ser Ser Gly Tyr Ser Gly Ala
            100                 105                 110

Phe Asp Gln Trp Gly Gln Gly Thr Leu Val Thr Val Ser Ser Ala Ser
        115                 120                 125

Thr Lys Gly Pro Ser Val Phe Pro Leu Ala Pro Ser Ser Lys Ser Thr
    130                 135                 140

Ser Gly Gly Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn Lys Pro Ser Asn Thr Lys Val Asp Lys Lys Val Glu Pro
    210                 215                 220

Lys Ser Cys Asp Lys Thr Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser Glu Asp Pro Glu Val Lys Phe Asn Trp Tyr Val Asp Gly Val Glu

275                 280                 285

Val Asn Ala Lys Thr Lys Pro Arg Glu Glu Gln Tyr Asn Ser Thr Tyr
    290                 295                 300

Arg Val Val Ser Val Leu Thr Val Leu Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met Glu
            420                 425                 430

Ala Leu Asn Tyr Thr Gln Lys Ser Leu Ser Leu Ser Pro Gly Lys
        435                 440                 445

<210> 356
<211> 656
<212> DNA
<213> Homo sapiens

<400> 356
gaaattgtgt tgacgcagtc tccaggcgcc ctgtctttgt ctccagggga aagagccacc      60

ctctcctgca gggccagtca gagtgttagc agcagctact tagcctggta ccagcagaaa     120

cctggccagg ctcccaggct cctcatctat ggtgcatcca gcagggccac tggcatccca     180

gacaggttca gtggcagtgg gtctgggaca gacttcactc tcacaatcag cagactggag     240

cctgaggatt ttgcagtgta ttactgtcag cagtatggta gctcacctcg cacttttggc     300

caggggacca agctggagat caaacgaact gtggctgcac catctgtctt catcttcccg     360

ccatctgatg agcagttgaa atctggaact gcctctgttg tgtgcctgct gaataacttc     420

tatcccagag aggccaaagt acagtggaag gtggataacg ccctccaatc gggtaactcc 480

caggagagtg tcacagagca ggacagcaag gacagcacct acagcctcag cagcaccctg 540

acgctgagca aagcagacta cgagaaacac aaagtctacg cctgcgaagt cacccatcag 600

ggcctgagct cgcccgtcac aaagagcttc aacaggggag agtgttagaa cgcgtg 656

<210> 357
<211> 215
<212> PRT
<213> Homo sapiens

<400> 357
Glu Ile Val Leu Thr Gln Ser Pro Gly Ala Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Pro
                85                  90                  95

Arg Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210> 358
<211> 1344
<212> DNA
<213> Homo sapiens

<400> 358
gaggtgcagc tggtggagtc cgggggaggc ttggtccagc ctggggggtc cctgaaactc      60

tcctgtgcag cctctgggtt cagcttcagt ggctctgctg tggaatgggt ccgccaggct     120

tccgggagag ggctggagtg gattggccat ataagaacca agaccaaaaa ttacgcgaca     180

gcgtatgctg cgtcggtgca aggcaggttc accatcttca gagatgattc aaggaacacg     240

gcgtatctgc agatgaacag tcttaaaacc gaggacacgg ccgtgtatta ttgttccaga     300

cacatcgaat ttagcagttc gtccgggccc cagatgggcc agggaaccct ggtcaccgtc     360

tcgagtgcct ccaccaaggg cccatcggtc ttccccctgg cgccctgctc caggagcacc     420

tccgagagca cagccgccct gggctgcctg gtcaaggact acttccccga accggtgacg     480

gtgtcgtgga actcaggcgc tctgaccagc ggcgtgcaca ccttcccagc tgtcctacag     540

tcctcaggac tctactccct cagcagcgtg gtgaccgtgc cctccagcaa cttcggcacc     600

cagacctaca cctgcaacgt agatcacaag cccagcaaca ccaaggtgga caagacagtt     660

gagcgcaaat gttgtgtcga gtgcccaccg tgcccagcac cacctgtggc aggaccgtca     720

gtcttcctct tccccccaaa acccaaggac accctcatga tctcccggac ccctgaggtc     780

acgtgcgtgg tggtggacgt gagccacgaa gaccccgagg tccagttcaa ctggtacgtg     840

gacggcgtgg aggtgcataa tgccaagaca aagccacggg aggagcagtt caacagcacg     900

ttccgtgtgg tcagcgtcct caccgttgtg caccaggact ggctgaacgg caaggagtac     960

aagtgcaagg tctccaacaa aggcctccca gcccccatcg agaaaaccat ctccaaaacc    1020

aaagggcagc cccgagaacc acaggtgtac accctgcccc catcccggga ggagatgacc    1080

aagaaccagg tcagcctgac ctgcctggtc aaaggcttct accccagcga catcgccgtg    1140

gagtgggaga gcaatgggca gccggagaac aactacaaga ccacacctcc catgctggac    1200

tccgacggct ccttcttcct ctacagcaag ctcaccgtgg acaagagcag gtggcagcag    1260

gggaacgtct tctcatgctc cgtgatgcat gaggctctgc acaaccacta cacgcagaag    1320

agcctctccc tgtctccggg taaa                                           1344

<210> 359
<211> 441

<212> PRT
<213> Homo sapiens

<400> 359
Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Ser Phe Ser Gly Ser
            20                  25                  30

Ala Val Glu Trp Val Arg Gln Ala Ser Gly Arg Gly Leu Glu Trp Ile
        35                  40                  45

Gly Ile Arg Thr Lys Thr Lys Asn Tyr Ala Thr Ala Tyr Ala Ala Ser
    50                  55                  60

Val Gln Gly Arg Phe Thr Ile Phe Arg Asp Asp Ser Arg Asn Thr Ala
65                  70                  75                  80

Tyr Leu Gln Met Asn Ser Leu Lys Thr Glu Asp Thr Ala Val Tyr Tyr
                85                  90                  95

Cys Ser Arg Ile Glu Phe Ser Ser Ser Ser Gly Pro Gln Met Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn Lys Pro Ser
        195                 200                 205

Asn Thr Lys Val Asp Lys Lys Val Glu Pro Lys Ser Cys Asp Lys Thr
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Glu Asp Pro Glu Val
            260                 265                 270

Lys Phe Asn Trp Tyr Val Asp Gly Val Glu Val Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu Gln Asp Trp Leu Asn Gly Lys Glu Tyr Lys Cys Lys Val
305                 310                 315                 320

Ser Asn Lys Ala Leu Pro Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met Glu Ala Leu Asn Tyr Thr Gln
            420                 425                 430

Lys Ser Leu Ser Leu Ser Pro Gly Lys
        435                 440

<210> 360
<211> 642
<212> DNA
<213> Homo sapiens

<400> 360
gacatccaga tgacccagtc tccttccacc ctgtctgcat ctgtaggcga cacagtcacc      60

atcacttgcc aggccagtca gaatattatt acttggttgg cctggtatca gcagaaacca     120

gggaaagccc ctaacatcct catctataag gcgtctggtt tagagagtgg ggtcccaccg 180

aggttcagcg gcagtgggtc ggggacagaa ttcactctca ccatcagcag cctgcagcct 240

gatgatgttg caacttatta ttgcctacaa tataaaactg agccgtggac gttcggccta 300

gggaccaagg tggagatcag acgaactgtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gt 642

<210> 361
<211> 214
<212> PRT
<213> Homo sapiens

<400> 361
Asp Ile Gln Met Thr Gln Ser Pro Ser Thr Leu Ser Ala Ser Val Gly
1               5                   10                  15

Asp Thr Val Thr Ile Thr Cys Gln Ala Ser Gln Asn Ile Ile Thr Trp
            20                  25                  30

Leu Ala Trp Tyr Gln Gln Lys Pro Gly Lys Ala Pro Asn Ile Leu Ile
        35                  40                  45

Tyr Lys Ala Ser Gly Leu Glu Ser Gly Val Pro Pro Arg Phe Ser Gly
    50                  55                  60

Ser Gly Ser Gly Thr Glu Phe Thr Leu Thr Ile Ser Ser Leu Gln Pro
65                  70                  75                  80

Asp Asp Val Ala Thr Tyr Tyr Cys Leu Gln Tyr Lys Thr Glu Pro Trp
                85                  90                  95

Thr Phe Gly Leu Gly Thr Lys Val Glu Ile Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 362
<211> 1335
<212> DNA
<213> Homo sapiens

<400> 362
gaggtgcagc tggtggagtc tgggggaggc ttggcccagc cgggggagtc cctgagactc      60

tcctgtgcag cctccggatt cacctttagt gcctattgga tgagctgggt ccgccagact     120

ccagggaagg ggctggagtg ggtggccaac attaaccagg gtggagataa gacagactat     180

ttggactctg tgaagggccg attcaccgtc tccagagaca atgccaagaa cttagtgttt     240

ctgcaaatgg ccagcctgag agccgacgac acggccgtgt attactgtgg gagaggtaca     300

aaccaagact actggggcca gggaaccctg gtcaccgtct cgagtgcctc caccaagggc     360

ccatcggtct tccccctggc accctcctcc aagagcacct ctgggggcac agcggccctg     420

ggctgcctgg tcaaggacta cttccccgaa ccggtgacgg tgtcgtggaa ctcaggcgcc     480

ctgaccagcg gcgtgcacac cttcccggct gtcctacagt cctcaggact ctactccctc     540

agcagcgtgg tgaccgtgcc ctccagcagc ttgggcaccc agacctacat ctgcaacgtg     600

aatcacaagc ccagcaacac caaggtggac aagaaagttg agcccaaatc ttgtgacaaa     660

actcacacat gcccaccgtg cccagcacct gaactcctgg ggggaccgtc agtcttcctc     720

ttccccccaa aacccaagga caccctcatg atctcccgga cccctgaggt cacatgcgtg     780

gtggtggacg tgagccacga agaccctgag gtcaagttca actggtacgt ggacggcgtg     840

gaggtgcata atgccaagac aaagccgcgg gaggagcagt acaacagcac gtaccgtgtg     900

gtcagcgtcc tcaccgtcct gcaccaggac tggctgaatg gcaaggagta caagtgcaag     960

gtctccaaca aagccctccc agcccccatc gagaaaacca tctccaaagc caaagggcag    1020

ccccgagaac cacaggtgta caccctgccc ccatcccggg atgagctgac caagaaccag    1080

gtcagcctga cctgcctggt caaaggcttc tatcccagcg acatcgccgt ggagtgggag    1140

agcaatgggc agccggagaa caactacaag accacgcctc ccgtgctgga ctccgacggc    1200

tccttcttcc tctacagcaa gctcaccgtg gacaagagca ggtggcagca ggggaacgtc 1260

ttctcatgct ccgtgatgca tgaggctctg cacaaccact acacgcagaa gagcctctcc 1320

ctgtctccgg gtaaa 1335

<210> 363
<211> 445
<212> PRT
<213> Homo sapiens

<400> 363
Glu Val Gln Leu Val Glu Ser Gly Gly Gly Leu Ala Gln Pro Gly Glu
1               5                   10                  15

Ser Leu Arg Leu Ser Cys Ala Ala Ser Gly Phe Thr Phe Ser Ala Tyr
            20                  25                  30

Trp Met Ser Trp Val Arg Gln Thr Pro Gly Lys Gly Leu Glu Trp Val
        35                  40                  45

Ala Asn Ile Asn Gln Gly Gly Asp Lys Thr Asp Tyr Leu Asp Ser Val
    50                  55                  60

Lys Gly Arg Phe Thr Val Ser Arg Asp Asn Ala Lys Asn Leu Val Phe
65                  70                  75                  80

Leu Gln Met Ala Ser Leu Arg Ala Asp Asp Thr Ala Val Tyr Tyr Cys
                85                  90                  95

Gly Arg Gly Thr Asn Gln Asp Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys

195                 200                 205

Val Asp Lys Lys Val Glu Pro Lys Ser Cys Asp Lys Thr His Thr Cys
    210                 215                 220

Pro Pro Cys Pro Ala Pro Glu Leu Leu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Asp Glu Leu Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Lys Ser Leu Ser Leu Ser Pro Gly Lys
        435                 440                 445

<210> 364
<211> 648
<212> DNA
<213> Homo sapiens

<400> 364
cagtctgtgc tgactcagcc accctcagcg tctgggaccc ccgggcagag ggtcaccatc 60

tcttgttctg gaagcagctc caatatcgga agtaataatg tatactggta ccaacaggtc 120

ccaggaacgg cccccaaatt tctcatctat ccgaatagtg agcggccctc aggggtccct 180

gaccgattct ctgcctccaa gtctggcacc tcagcctccc tggccatcag tggactccgg 240

tccgaggatg aggctactta ttactgtgca gcatgggatg acagcctaag aacttatgtc 300

ttcgcatctg ggaccaaggt caccgtccta ggtcagccca aggccaaccc cactgtcact 360

ctgttcccgc cctcctctga ggagctccaa gccaacaagg ccacactagt gtgtctgatc 420

agtgacttct acccgggagc tgtgacagtg gcctggaagg cagatggcag ccccgtcaag 480

gcgggagtgg agaccaccaa accctccaaa cagagcaaca acaagtacgc ggccagcagc 540

tacctgagcc tgacgcccga gcagtggaag tcccacagaa gctacagctg ccaggtcacg 600

catgaaggga gcaccgtgga gaagacagtg gcccctacag aatgttca 648

<210> 365
<211> 216
<212> PRT
<213> Homo sapiens

<400> 365
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Asn Val Tyr Trp Tyr Gln Gln Val Pro Gly Thr Ala Pro Lys Phe Leu
        35                  40                  45

Ile Tyr Pro Asn Ser Glu Arg Pro Ser Gly Val Pro Asp Arg Phe Ser
    50                  55                  60

Ala Ser Lys Ser Gly Thr Ser Ala Ser Leu Ala Ile Ser Gly Leu Arg
65                  70                  75                  80

Ser Glu Asp Glu Ala Thr Tyr Tyr Cys Ala Ala Trp Asp Asp Ser Leu
                85                  90                  95

Arg Thr Tyr Val Phe Ala Ser Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210> 366
<211> 1350
<212> DNA
<213> Homo sapiens

<400> 366
caggtgcagc tggtggagtc tgggggaggc gtggtccagc ctgggaggtc cctgagactc      60

tcctgtgcag cctccggatt caccctcagg agttatggca tgcactgggt ccgccaggct     120

ccaggcaagg gcctggagtg ggtggcagtt atatcatatg atggaagtaa gacatattat     180

agagactccg tgaagggccg attcaccatc tccagagaca attctaagaa cacactgtat     240

ttgcaaatga acagcctgcg agttgaggac acggctgtat attattgtgc gaaagaggat     300

aatttgggat ctgatgtttt tgatagatgg ggccaaggga caatggtcac cgtctcgagt     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacccagacc     600

tacatctgca acgtgaatca caagcccagc aacaccaagg tggacaagaa agttgagccc     660

aaatcttgtg acaaaactca cacatgccca ccgtgcccag cacctgaact cctgggggga     720

ccgtcagtct tcctcttccc cccaaaaccc aaggacaccc tcatgatctc ccggacccct     780

gaggtcacat gcgtggtggt ggacgtgagc cacgaagacc ctgaggtcaa gttcaactgg     840

tacgtggacg gcgtggaggt gcataatgcc aagacaaagc cgcgggagga gcagtacaac     900

agcacgtacc gtgtggtcag cgtcctcacc gtcctgcacc aggactggct gaatggcaag 960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 367
<211> 450
<212> PRT
<213> Homo sapiens

<400> 367
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Leu Arg Ser Tyr
            20                  25                  30

Gly Met His Trp Val Arg Gln Ala Pro Gly Lys Gly Leu Glu Trp Val
        35                  40                  45

Ala Val Ile Ser Tyr Asp Gly Ser Lys Thr Tyr Tyr Arg Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Val Glu Asp Thr Ala Val Tyr Tyr Cys
                85                  90                  95

Ala Lys Glu Asp Asn Leu Gly Ser Asp Val Phe Asp Arg Trp Gly Gln
            100                 105                 110

Gly Thr Met Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 368
<211> 643
<212> DNA
<213> Homo sapiens

<400> 368
gacatcgtga tgacccagtc tccttccacc ctgtctgcat ctgtaggaga cagagttacc      60

atcacttgtc gggccagtca gagtgccagt cagtggttgg cctggtatca gcaaaaacca     120

gggaaagccc ctaatctcct aatctataag gcgtctaatt tacatagtgg ggtcccatca     180

aggttcagcg gcagtggatc tgggaccgaa ttcactctca ccatcagcag cctacagcct     240

gatgattttg caacttatta ctgccaacaa tacaacattt atccgtggac gttcggccaa     300

gggaccaagg tggaagtgag acgaactgtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg     540

ctgagcaaag cagactacga gaaacacaaa gtctacgcct gcgaagtcac ccatcagggc     600

ctgagctcgc ccgtcacaaa gagcttcaac aggggagagt gtt                       643

<210> 369
<211> 214
<212> PRT
<213> Homo sapiens

<400> 369
Asp Ile Val Met Thr Gln Ser Pro Ser Thr Leu Ser Ala Ser Val Gly
1               5                   10                  15

Asp Arg Val Thr Ile Thr Cys Arg Ala Ser Gln Ser Ala Ser Gln Trp
            20                  25                  30

Leu Ala Trp Tyr Gln Gln Lys Pro Gly Lys Ala Pro Asn Leu Leu Ile
        35                  40                  45

Tyr Lys Ala Ser Asn Leu His Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Asp Asp Phe Ala Thr Tyr Tyr Cys Gln Gln Tyr Asn Ile Tyr Pro Trp
                85                  90                  95

Thr Phe Gly Gln Gly Thr Lys Val Glu Val Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 370
<211> 1350
<212> DNA
<213> Homo sapiens

<400> 370
caggtccagc tggtgcagtc tggggctgag gtgaaggagc cagggtcctc ggtgagggtc      60

tcctgcaagg cttctggacg catcttccgg aattatgctg tcagctgggt gcgacaggcc     120

cctggacaag ggcttgagtg ggtgggccgg ttcatccctt cggaaggaat aacaaactac     180

attccgacat tccgggacag actcaccttt actgcggaca agtccacgaa cacagccttc     240

attgacctga ctaacctgaa atatgaggac acggccatat attactgtgt gggtggcagg     300

ccaggcgctt tcgtcccctt tgactcctgg ggccagggaa ccctggtcac cgtctcgagt     360

gcctccacca agggcccatc ggtcttcccc ctggcaccct cctccaagag cacctctggg     420

ggcacagcgg ccctgggctg cctggtcaag gactacttcc ccgaaccggt gacggtgtcg     480

tggaactcag gcgccctgac cagcggcgtg cacaccttcc cggctgtcct acagtcctca 540

ggactctact ccctcagcag cgtggtgacc gtgccctcca gcagcttggg cacccagacc 600

tacatctgca acgtgaatca caagcccagc aacaccaagg tggacaagaa agttgagccc 660

aaatcttgtg acaaaactca cacatgccca ccgtgcccag cacctgaact cctgggggga 720

ccgtcagtct tcctcttccc cccaaaaccc aaggacaccc tcatgatctc ccggacccct 780

gaggtcacat gcgtggtggt ggacgtgagc cacgaagacc ctgaggtcaa gttcaactgg 840

tacgtggacg gcgtggaggt gcataatgcc aagacaaagc cgcgggagga gcagtacaac 900

agcacgtacc gtgtggtcag cgtcctcacc gtcctgcacc aggactggct gaatggcaag 960

gagtacaagt gcaaggtctc caacaaagcc ctcccagccc ccatcgagaa aaccatctcc 1020

aaagccaaag ggcagccccg agaaccacag gtgtacaccc tgcccccatc ccgggatgag 1080

ctgaccaaga accaggtcag cctgacctgc ctggtcaaag gcttctatcc cagcgacatc 1140

gccgtggagt gggagagcaa tgggcagccg gagaacaact acaagaccac gcctcccgtg 1200

ctggactccg acggctcctt cttcctctac agcaagctca ccgtggacaa gagcaggtgg 1260

cagcagggga acgtcttctc atgctccgtg atgcatgagg ctctgcacaa ccactacacg 1320

cagaagagcc tctccctgtc tccgggtaaa 1350

<210> 371
<211> 450
<212> PRT
<213> Homo sapiens

<400> 371
Gln Val Gln Leu Val Gln Ser Gly Ala Glu Val Lys Glu Pro Gly Ser
1               5                   10                  15

Ser Val Arg Val Ser Cys Lys Ala Ser Gly Arg Ile Phe Arg Asn Tyr
            20                  25                  30

Ala Val Ser Trp Val Arg Gln Ala Pro Gly Gln Gly Leu Glu Trp Val
        35                  40                  45

Gly Arg Phe Ile Pro Ser Glu Gly Ile Thr Asn Tyr Ile Pro Thr Phe
    50                  55                  60

Arg Asp Arg Leu Thr Phe Thr Ala Asp Lys Ser Thr Asn Thr Ala Phe
65                  70                  75                  80

Ile Asp Leu Thr Asn Leu Lys Tyr Glu Asp Thr Ala Ile Tyr Tyr Cys
                85                  90                  95

Val Gly Gly Arg Pro Gly Ala Phe Val Pro Phe Asp Ser Trp Gly Gln

100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210> 372
<211> 643
<212> DNA
<213> Homo sapiens

<400> 372
gaaatagtga tgacgcagtc tcctgccacc ctgtctgtgt ctccagggga aagagccacc 60

ctctcctgca gggccagtca gagtgtcagg ggcaacttag cctggtacca gcaaaaacct 120

ggccaggctc ccaggcttct catgtatgat gcttctacca gggccactag tttcccagcc 180

cggttcagtg gcagtgggtc tgggacagag ttcactctca ccatcagcag tgtgcagtct 240

gaagattttg cactttatta ctgtcagcag tataatgact ggccccggac ttttggccag 300

gggaccaagg tggagatcaa acgaactgtg gctgcaccat ctgtcttcat cttcccgcca 360

tctgatgagc agttgaaatc tggaactgcc tctgttgtgt gcctgctgaa taacttctat 420

cccagagagg ccaaagtaca gtggaaggtg gataacgccc tccaatcggg taactcccag 480

gagagtgtca cagagcagga cagcaaggac agcacctaca gcctcagcag caccctgacg 540

ctgagcaaag cagactacga gaaacacaaa gtctacgcct gcgaagtcac ccatcagggc 600

ctgagctcgc ccgtcacaaa gagcttcaac aggggagagt gtt 643

<210> 373
<211> 214
<212> PRT
<213> Homo sapiens

<400> 373

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Arg Gly Asn
            20                  25                  30

Leu Ala Trp Tyr Gln Gln Lys Pro Gly Gln Ala Pro Arg Leu Leu Met
        35                  40                  45

Tyr Asp Ala Ser Thr Arg Ala Thr Ser Phe Pro Ala Arg Phe Ser Gly
    50                  55                  60

Ser Gly Ser Gly Thr Glu Phe Thr Leu Thr Ile Ser Ser Val Gln Ser
65                  70                  75                  80

Glu Asp Phe Ala Leu Tyr Tyr Cys Gln Gln Tyr Asn Asp Trp Pro Arg
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210> 374
<211> 1362
<212> DNA
<213> Homo sapiens

<400> 374
caggtgcagc tggtgcagtc tggggctgag gtgaagaagc ctgggtcctc ggtgagggtc        60

tcctgcaggg tttctggagg cagtttctcc agttatgacg tacactgggt gcgacaggcc 120

cctggacaag ggcttgagtg ggtgggaagg agcgtctcac gtgctgacag ctcaagtcac 180

acacgggagt tcggggacag agtcacgatt atcgcggaca aatccacgag tacagtttat 240

ttgacactga ccagtctgac ctctgacgac acggccatct attactgtgc ggcaggggat 300

tgtagtgatg gcgggtgctt ccgcacaccc ctgcacatct ggggccaggg caccctggtc 360

accgtctcga gtgcctccac caagggccca tcggtcttcc ccctggcacc ctcctccaag 420

agcacctctg ggggcacagc ggccctgggc tgcctggtca aggactactt ccccgaaccg 480

gtgacggtgt cgtggaactc aggcgccctg accagcggcg tgcacacctt cccggctgtc 540

ctacagtcct caggactcta ctccctcagc agcgtggtga ccgtgccctc cagcagcttg 600

ggcacccaga cctacatctg caacgtgaat cacaagccca gcaacaccaa ggtggacaag 660

aaagttgagc ccaaatcttg tgacaaaact cacacatgcc caccgtgccc agcacctgaa 720

ctcctggggg gaccgtcagt cttcctcttc cccccaaaac ccaaggacac cctcatgatc 780

tcccggaccc ctgaggtcac atgcgtggtg gtggacgtga gccacgaaga ccctgaggtc 840

aagttcaact ggtacgtgga cggcgtggag gtgcataatg ccaagacaaa gccgcgggag 900

gagcagtaca acagcacgta ccgtgtggtc agcgtcctca ccgtcctgca ccaggactgg 960

ctgaatggca aggagtacaa gtgcaaggtc tccaacaaag ccctcccagc ccccatcgag 1020

aaaaccatct ccaaagccaa agggcagccc cgagaaccac aggtgtacac cctgccccca 1080

tcccgggatg agctgaccaa gaaccaggtc agcctgacct gcctggtcaa aggcttctat 1140

cccagcgaca tcgccgtgga gtgggagagc aatgggcagc cggagaacaa ctacaagacc 1200

acgcctcccg tgctggactc cgacggctcc ttcttcctct acagcaagct caccgtggac 1260

aagagcaggt ggcagcaggg gaacgtcttc tcatgctccg tgatgcatga ggctctgcac 1320

aaccactaca cgcagaagag cctctccctg tctccgggta aa 1362

<210> 375
<211> 454
<212> PRT
<213> Homo sapiens

<400> 375
Gln Val Gln Leu Val Gln Ser Gly Ala Glu Val Lys Lys Pro Gly Ser
1               5                   10                  15

Ser Val Arg Val Ser Cys Arg Val Ser Gly Gly Ser Phe Ser Ser Tyr
            20                  25                  30

Asp Val His Trp Val Arg Gln Ala Pro Gly Gln Gly Leu Glu Trp Val
        35                  40                  45

Gly Arg Ser Val Ser Arg Ala Asp Ser Ser Ser His Thr Arg Glu Phe
    50                  55                  60

Gly Asp Arg Val Thr Ile Ile Ala Asp Lys Ser Thr Ser Thr Val Tyr
65                  70                  75                  80

Leu Thr Leu Thr Ser Leu Thr Ser Asp Asp Thr Ala Ile Tyr Tyr Cys
                85                  90                  95

Ala Ala Gly Asp Cys Ser Asp Gly Gly Cys Phe Arg Thr Pro Leu His
            100                 105                 110

Ile Trp Gly Gln Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Asp Glu Leu Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Asn His Tyr Thr Gln Lys Ser Leu
        435                 440                 445

Ser Leu Ser Pro Gly Lys
    450

<210> 376
<211> 645
<212> DNA
<213> Homo sapiens

<400> 376
gaaatagtga tgacgcagtc tccagccact ctgtctctgt ctccagggga aagagccacc      60

ctctcctgca gggccagtca gagtctgaga accaacttag cctggtacca gcagcgacct     120

ggccaggctc ccaggctcct catctctggg gcatccacga gggccactgg tatcccagcc     180

aggttcagtg gcagtgggtc tgggacagaa ttcactctca ccattaggag cctgcagcct     240

gaagattttg cagtttatta ctgtcaacag tatcatgact ggcctcggac gttcggccaa     300

gggaccaagg tggatttcaa acgaactgtg gctgcaccat ctgtcttcat cttcccgcca     360

tctgatgagc agttgaaatc tggaactgcc tctgttgtgt gcctgctgaa taacttctat     420

cccagagagg ccaaagtaca gtggaaggtg gataacgccc tccaatcggg taactcccag     480

gagagtgtca cagagcagga cagcaaggac agcacctaca gcctcagcag caccctgacg 540

ctgagcaaag cagactacga gaaacacaaa ctctacgcct gcgaagtcac ccatcagggc 600

ctgagctcgc ccgtcacaaa gagcttcaac aggggagagt gttag 645

<210> 377
<211> 214
<212> PRT
<213> Homo sapiens

<400> 377
Glu Ile Val Met Thr Gln Ser Pro Ala Thr Leu Ser Leu Ser Pro Gly
1               5                   10                  15

Glu Arg Ala Thr Leu Ser Cys Arg Ala Ser Gln Ser Leu Arg Thr Asn
            20                  25                  30

Leu Ala Trp Tyr Gln Gln Arg Pro Gly Gln Ala Pro Arg Leu Leu Ile
        35                  40                  45

Ser Gly Ala Ser Thr Arg Ala Thr Gly Ile Pro Ala Arg Phe Ser Gly
    50                  55                  60

Ser Gly Ser Gly Thr Glu Phe Thr Leu Thr Ile Arg Ser Leu Gln Pro
65                  70                  75                  80

Glu Asp Phe Ala Val Tyr Tyr Cys Gln Gln Tyr His Asp Trp Pro Arg
                85                  90                  95

Thr Phe Gly Gln Gly Thr Lys Val Asp Phe Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Leu Tyr
            180                 185                 190

Ala Cys Glu Val Thr His Gln Gly Leu Ser Ser Pro Val Thr Lys Ser

195                 200                 205

Phe Asn Arg Gly Glu Cys
    210

<210> 378
<211> 1359
<212> DNA
<213> Homo sapiens

<400> 378
gaggtgcagc tggtgcagtc tggggcagaa gtgaaaaagg ccggggagtc tctgaggatc      60

tcctgtaagg gttctggata caactttaat agctattgga ttggctgggt gcgccagatg     120

cccgggaaag gcctggagtg gatggcaatc atctatcctg ctgactctga caggagatat     180

ggcccgtcct tccaaggcca ggtcaccatc tcagtcgaca agtctattaa caccgcgtac     240

ttgcagtgga gcagcctgaa ggcctcggac accgccatgt attattgtgc cagactcctc     300

agagttagta ctggttggga agatgctttt gatctgtggg gccaagggac aatggtcacc     360

gtctcgagtg cctccaccaa gggcccatcg gtcttccccc tggcaccctc ctccaagagc     420

acctctgggg gcacagcggc cctgggctgc ctggtcaagg actacttccc cgaaccggtg     480

acggtgtcgt ggaactcagg cgccctgacc agcggcgtgc acaccttccc ggctgtccta     540

cagtcctcag gactctactc cctcagcagc gtggtgaccg tgccctccag cagcttgggc     600

acccagacct acatctgcaa cgtgaatcac aagcccagca acaccaaggt ggacaagaaa     660

gttgagccca aatcttgtga caaaactcac acatgcccac cgtgcccagc acctgaactc     720

ctggggggac cgtcagtctt cctcttcccc ccaaaaccca aggacaccct catgatctcc     780

cggacccctg aggtcacatg cgtggtggtg gacgtgagcc acgaagaccc tgaggtcaag     840

ttcaactggt acgtggacgg cgtggaggtg cataatgcca agacaaagcc gcgggaggag     900

cagtacaaca gcacgtaccg tgtggtcagc gtcctcaccg tcctgcacca ggactggctg     960

aatggcaagg agtacaagtg caaggtctcc aacaaagccc tcccagcccc catcgagaaa    1020

accatctcca aagccaaagg gcagccccga gaaccacagg tgtacaccct gcccccatcc    1080

cgggatgagc tgaccaagaa ccaggtcagc ctgacctgcc tggtcaaagg cttctatccc    1140

agcgacatcg ccgtggagtg ggagagcaat gggcagccgg agaacaacta caagaccacg    1200

cctcccgtgc tggactccga cggctccttc ttcctctaca gcaagctcac cgtggacaag    1260

agcaggtggc agcaggggaa cgtcttctca tgctccgtga tgcatgaggc tctgcacaac    1320

cactacacgc agaagagcct ctccctgtct ccgggtaaa                           1359

<210> 379
<211> 453
<212> PRT

<213> Homo sapiens

<400> 379
Glu Val Gln Leu Val Gln Ser Gly Ala Glu Val Lys Lys Ala Gly Glu
1               5                   10                  15

Ser Leu Arg Ile Ser Cys Lys Gly Ser Gly Tyr Asn Phe Asn Ser Tyr
            20                  25                  30

Trp Ile Gly Trp Val Arg Gln Met Pro Gly Lys Gly Leu Glu Trp Met
        35                  40                  45

Ala Ile Ile Tyr Pro Ala Asp Ser Asp Arg Arg Tyr Gly Pro Ser Phe
    50                  55                  60

Gln Gly Gln Val Thr Ile Ser Val Asp Lys Ser Ile Asn Thr Ala Tyr
65                  70                  75                  80

Leu Gln Trp Ser Ser Leu Lys Ala Ser Asp Thr Ala Met Tyr Tyr Cys
                85                  90                  95

Ala Arg Leu Leu Arg Val Ser Thr Gly Trp Glu Asp Ala Phe Asp Leu
            100                 105                 110

Trp Gly Gln Gly Thr Met Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210> 380
<211> 663
<212> DNA
<213> Homo sapiens

<400> 380
gacatcgtga tgacccagtc tccagactcc ctggttgtgt ctctgggcga gagggccacc        60

atcaagtgcc agtccagcca gagtgttttg tacagctcca ccaataagaa ctatttagcc 120

tggtaccagc agaaatcggg acagccacct aagctactca tttactgggc ctccacccgg 180

gaatccgggg tccctgaccg attcattggt agcggctcta ggacagattt cactctcacc 240

atcagcgacc tgcagcctga ggatgcggca gtttattact gtcagcagta ttatagtgat 300

tatctcactt tcggcggagg gaccaaggtg gagatcaaac gaactgtggc tgcaccatct 360

gtcttcatct tcccgccatc tgatgagcag ttgaaatctg gaactgcctc tgttgtgtgc 420

ctgctgaata acttctatcc cagagaggcc aaagtacagt ggaaggtgga taacgccctc 480

caatcgggta actcccagga gagtgtcaca gagcaggaca gcaaggacag cacctacagc 540

ctcagcagca ccctgacgct gagcaaagca gactacgaga aacacaaagt ctacgcctgc 600

gaagtcaccc atcagggcct gagctcgccc gtcacaaaga gcttcaacag gggagagtgt 660

tag 663

<210> 381
<211> 220
<212> PRT
<213> Homo sapiens

<400> 381
Asp Ile Val Met Thr Gln Ser Pro Asp Ser Leu Val Val Ser Leu Gly
1               5                   10                  15

Glu Arg Ala Thr Ile Lys Cys Gln Ser Ser Gln Ser Val Leu Tyr Ser
            20                  25                  30

Ser Thr Asn Lys Asn Tyr Leu Ala Trp Tyr Gln Gln Lys Ser Gly Gln
        35                  40                  45

Pro Pro Lys Leu Leu Ile Tyr Trp Ala Ser Thr Arg Glu Ser Gly Val
    50                  55                  60

Pro Asp Arg Phe Ile Gly Ser Gly Ser Arg Thr Asp Phe Thr Leu Thr
65                  70                  75                  80

Ile Ser Asp Leu Gln Pro Glu Asp Ala Ala Val Tyr Tyr Cys Gln Gln
                85                  90                  95

Tyr Tyr Ser Asp Tyr Leu Thr Phe Gly Gly Gly Thr Lys Val Glu Ile
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn

130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

## Description

### CROSS-REFERENCE TO RELATED APPLICATIONS

This application claims the benefit of and priority to U.S. Provisional Application No. 61/379,580, filed September 2, 2010 and U.S. Provisional Application No. 61/380,934, filed September 8, 2010; the entire contents of each application are incorporated herein by reference.

### FIELD OF THE INVENTION

The field of the invention is molecular biology, immunology and infectious disease. More particularly, the field is anti*-Staphylococcus aureus* antibodies and therapeutic human recombinant polyclonal antibodies.

### BACKGROUND

Antibiotic-resistant strains of *Staphylococcus aureus (S. aureus)* are on the rise and are a major health concern, both in the hospital setting and, increasingly, in the community at large. Methicillin-resistant *S*. *aureus* (MRSA) is currently the leading cause of death due to infectious disease in the United States, surpassing deaths from HIV/AIDS and tuberculosis combined. The death rate from invasive MRSA infection is now 20% (Heron, M.P. *et al.,* 2008. **56**: p. 1-52). Resistant forms of *S*. *aureus* have emerged within a few years of the introduction of each new class of antibiotics (Kirby, W.M., SCIENCE (1944) 99:452-453; Boucher, H.W. et al., CLIN INFECT DIS (2009) 48:1-12). For example, in the last decade, the first cases of vancomycin-resistant S. *aureus* (VRSA), and vancomycin-intermediate resistant S. *aureus* (VISA) have been reported. Given the current status in research and development of new antibiotics, resistance in bacteria is increasing faster than our ability to develop novel antibiotics to treat resistant strains (Boucher, H.W. and G. Sakoulas, CLIN INFECT DIS (2007) 45:601-8; Walsh, C., NAT REV MICROBIOL (2003) 1:65-70). Alternative treatments are therefore needed to replace those that are increasingly ineffective.

Recent research has shown that humans infected with *S. aureus* have a diverse humoral immune reaction to the organism (Verkaik, N.J. et al., EUR J CLIN MICROBIOL INFECT DIS (2010) 29:509-18; Verkaik, N.J. et al., CLIN INFECT DIS (2010) 50:61-8). Likewise, studies on the S. *aureus* exoproteome indicate that few strains express the same spectrum of antigens, and even antigens expressed in common between strains may be expressed at widely varying levels and in dissimilar temporal expression patterns (von Eiff, C. et al., DIAGN MICROBIOL INFECT DIS (2004) 49:157-62; Bania, J. et al., INT J FOOD MICROBIOL (2006) 108: 36-41; Ziebandt, A.K. et al., PROTEOMICS (2010) 10:1634-44).

One peptide that appears to be conserved among *S. aureus* strains and plays a role in the hemolytic activity of *S. aureus* is delta-hemolysin (δ-HL; also known as delta-toxin). Recent studies have shown that the hemolytic activity of delta-toxin may be modulated by interaction with lipid raft regions of the cell membrane (Pokorny, A. and P.F. Almeida, BIOCHEMISTRY (2005) 44(27): 9538-44). In addition to delta toxin, there have been six more small cytolytic peptides identified. These peptides were identified by genome analysis (Wang, R. et al., NATURE MEDICINE (2007) 13: 1510-1514). The peptides are represented in all strains tested by Wang *et al.* (2007) and had higher levels of expression in community acquired (CA) MRSA strains. The expression of these peptides are theorized to be one means by which CA-MRSA strains have a greater virulence compared to hospital acquired (HA) strains. Deletion of any of the six cytolytic peptides decreases virulence of MRSA strains in various *in vitro* and *in vivo* models (Wang, R. et al., NATURE MEDICINE (2007) 13: 1510-1514).

Thus, there is a need for improved therapeutic agents that can be used to treat and/or prevent *S. aureus* infections.

### SUMMARY

The invention is based, in part, upon the discovery of human polyclonal antibody compositions that bind one or more strains of *S. aureus.* In one aspect, the disclosed polyclonal antibody compositions comprise at least three different human antibodies that individually bind one or more *S. aureus* strains. In certain embodiments, the disclosed polyclonal antibody compositions bind to at least three different strains of *S. aureus.* Exemplary polyclonal antibodies described herein contain specific *S. aureus* binding sites based on the CDRs of individual antibodies of the polyclonal antibody composition. The individual antibodies of the polyclonal antibody composition may bind proteins that are known or likely to be involved in S. *aureus* virulence (*e.g.,* delta-toxin) or, alternatively, they may bind previously uncharacterized S. *aureus* target proteins. The disclosed human polyclonal antibody compositions can neutralize the activity of *S. aureus* toxins, cell surface antigens and/or immunomodulating antigens. In an exemplary embodiment, the disclosed human polyclonal antibody compositions are broad-spectrum therapeutic antibodies with neutralizing activity against multiple *S. aureus* proteins that are present on one or more *S. aureus* strains. In certain embodiments, the disclosed recombinant polyclonal antibody compositions mimic the natural human immune response. It is contemplated herein that the disclosed recombinant polyclonal antibody compositions provide protection against a range of virulence factors. Such polyclonal antibodies can be used as therapeutic agents to treat S. *aureus* infections including antibiotic-resistant S. *aureus* strains.

Also disclosed herein are isolated, individual human antibodies, or antigen binding fragments thereof, that specifically bind one or more strains of *S. aureus,* including antibiotic resistant strains (*e.g.,* MRSA). In one embodiment, an isolated human antibody as described herein binds (*e.g.,* specifically binds) to *S. aureus* delta-toxin *(e.g.,* antibody 5.6.H9 and antibody 5.55.D2). In another embodiment, an isolated human antibody described herein binds *(e.g.,* specifically binds) to the toxin phenol soluble modulin beta-1 (*e.g.,* antibody 22.18A.E9).

These and other aspects and advantages of the invention will become apparent upon consideration of the following figures, detailed description, and claims. As used herein, "including" means without limitation, and examples cited are non-limiting.

### DESCRIPTION OF THE DRAWINGS

The invention can be more completely understood with reference to the following drawings.

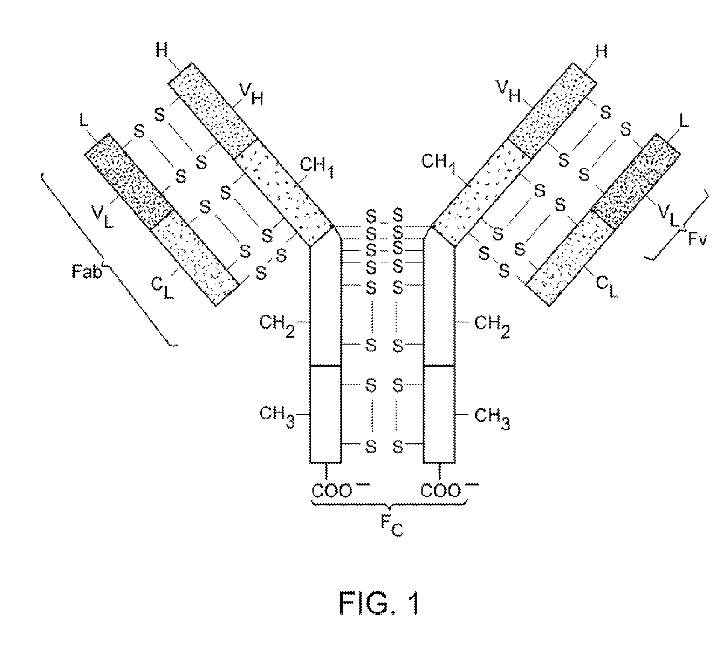

Fig. 1 (prior art) is a schematic representation of a typical naturally-occurring antibody. Naturally occurring antibodies are multimeric proteins that contain four polypeptide chains. Two of the polypeptide chains are called heavy chains (H chains), and two of the polypeptide chains are called light chains (L chains). The immunoglobulin heavy and light chains are connected by an interchain disulfide bond. The immunoglobulin heavy chains are connected by interchain disulfide bonds. A light chain consists of one variable region (V_{L}) and one constant region (C_{L}). The heavy chain consists of one variable region (V_{H}) and at least three constant regions (CH₁, CH₂ and CH₃). The variable regions determine the specificity of the antibody. Each variable region comprises three hypervariable regions also known as complementarity determining regions (CDRs) flanked by four relatively conserved framework regions (FRs). The three CDRs, referred to as CDR₁, CDR₂, and CDR₃, contribute to the antibody binding specificity.

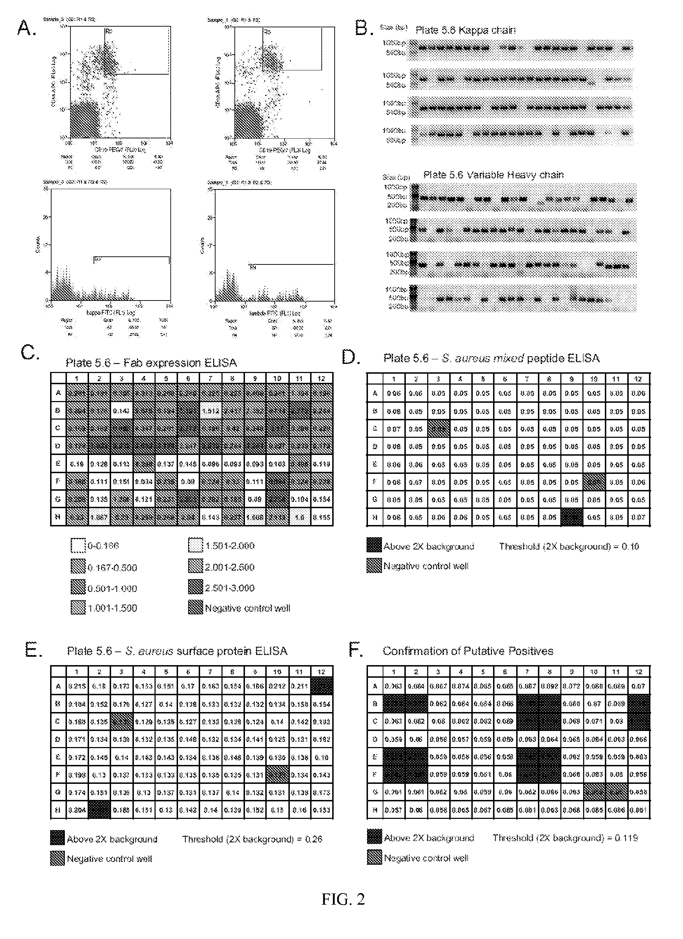

Fig. 2 is a series of panels showing data from a representative plate (plate 5.6) of isolated fully human antibodies. Fig. 2A is a representative FACS analysis showing the population of CD38+/CD19+ plasma cells targeted for cloning; Fig. 2B shows the amplified variable heavy and light antibody genes from individual plasma cells in a 96-well plate; Fig. 2C shows representative data from an antibody expression ELISA showing that the majority of amplified antibody cognate pairs are cloned and are able to express antibody; Figs. 2D and E show data from screening ELISA assays in which a well H9 was scored as a positive against *S. aureus* protein in Fig. 2D and wells H2 and A12 were scored as positives in Fig. 2E. The hits were confirmed with multiple replicates in the ELISA shown in Fig. 2F.

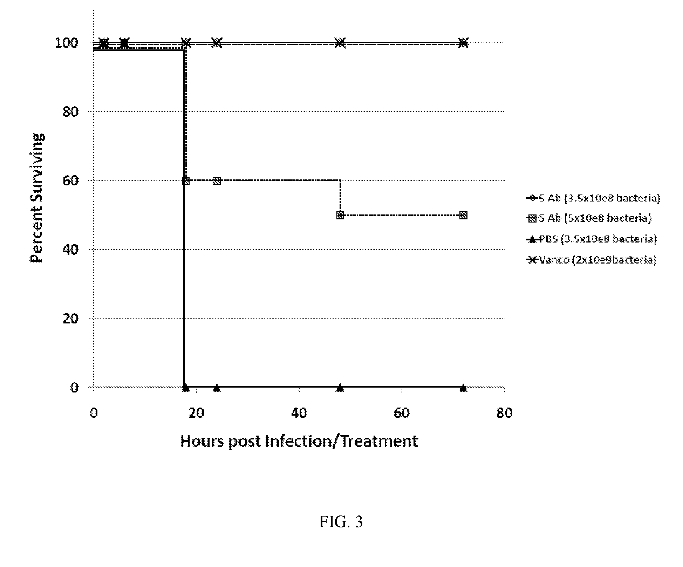

Fig. 3 is a graph showing that an anti-*S*. *aureus* recombinant polyclonal antibody mixture of five antibodies can protect mice from a challenge with live S. *aureus* (community acquired-MRSA strain, USA300) at 3.5 x 10⁸ CFU or 5 x 10⁸ CFU (◊, 5 antibody composition at 3.5 x 10⁸ CFU; □, 5 antibody composition at 5 x 10⁸ CFU; A, PBS at 3.5 x 10⁸ CFU (negative control); and x, vancomycin at 2 x 10⁹ CFU (positive control).

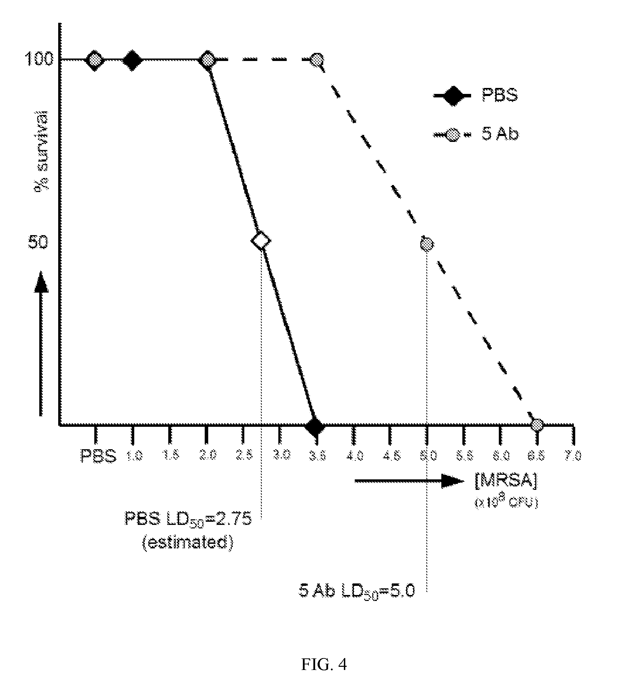

Fig. 4 is a graph showing that anti-*S*. *aureus* recombinant polyclonal antibody mixtures of five antibodies at a dose of 1 mg/kg at 72 hours shift the LD₅₀ curve (*e.g.,* increase resistance to *S. aureus* infection) in a live *S. aureus* bacterial challenge (community acquired-MRSA strain, USA300) in mice (◆, PBS LD₅₀ = 2.75 x 10⁸ CFU; ○, five antibody composition LD₅₀ = 5.0 x 10⁸ CFU.

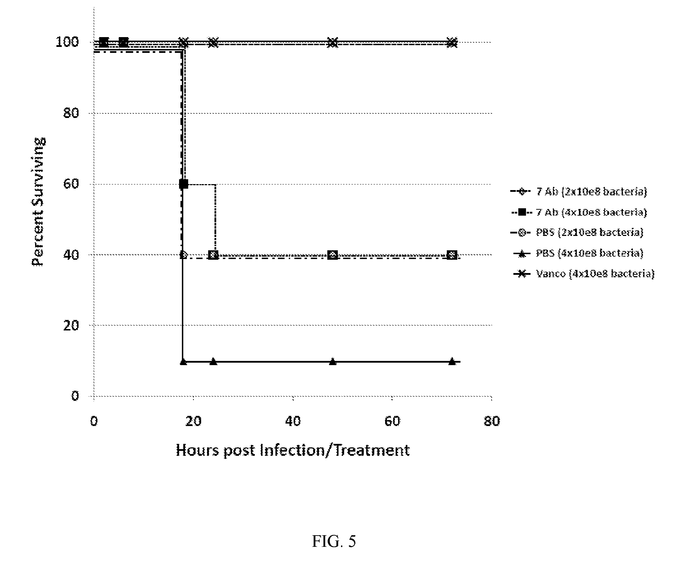

Fig. 5 is a graph showing that an anti-*S*. *aureus* recombinant polyclonal antibody mixture of seven antibodies can protect mice from a challenge with live S. *aureus* (methicillin-sensitive S. *aureus* strain, Wood-46) at 2 x 10⁸ CFU or 4 x 10⁸ CFU (◊, 7 antibody composition at 2 x 10⁸ CFU; ■, 7 antibody composition at 4 x 10⁸ CFU; ○, PBS at 2 x 10⁸ CFU (negative control); A, PBS at 4 x 10⁸ CFU (negative control); and x, vancomycin at 4 x 10⁸ CFU (positive control).

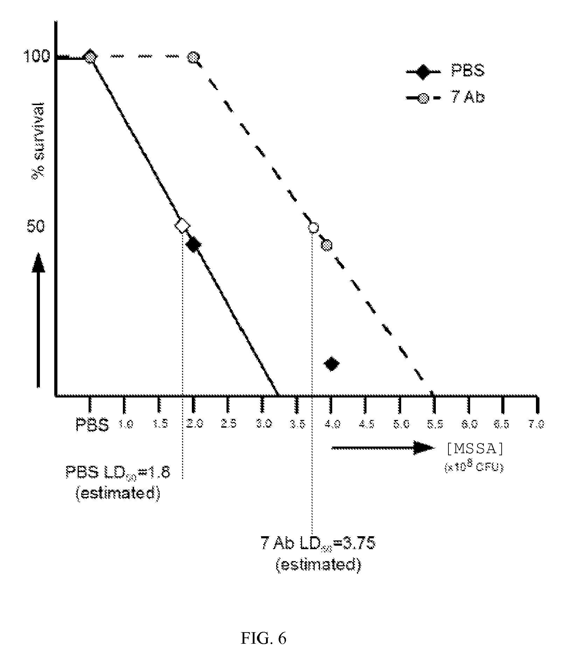

Fig. 6 is a graph showing that anti-S. *aureus* recombinant polyclonal antibody mixtures of seven antibodies at a dose of 1 mg/kg at 72 hours shift the LD₅₀ curve (*e.g.,* increase resistance to S. *aureus* infection) in a live *S. aureus* bacterial challenge (methicillin-sensitive *S. aureus* strain, Wood-46) in mice (◆, PBS LD₅₀ = 1.8 x 10⁸ CFU; -○, seven antibody composition LD₅₀ = 3.75 x 10⁸ CFU.

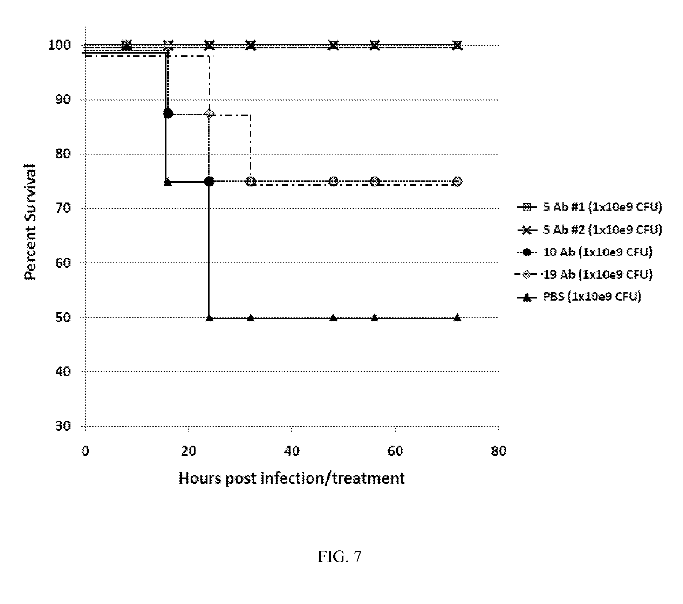

Fig. 7 is a graph showing that anti-S. *aureus* recombinant polyclonal antibody mixtures of five, 10 and 19 antibodies can protect mice from a challenge with live *S. aureus* (USA300) at 1 x 10⁹ CFU (□, 5 antibody composition #2 at 1 x 10⁹ CFU; x, 5 antibody composition #3 at 1 x 10⁹ CFU; ●, 10 antibody composition at 1 x 10⁹ CFU; 0, 19 antibody composition at 1 x 10⁹ CFU; and A, PBS at 1 x 10⁹ CFU (negative control).

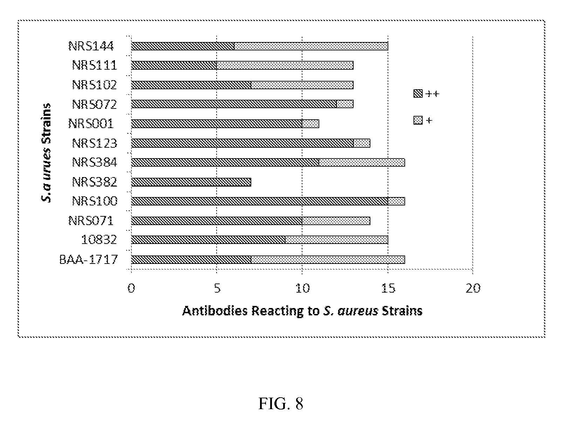

Fig. 8 is a graph showing the number of anti-*S*. *aureus* antibodies that react with various strains of *S. aureus.*

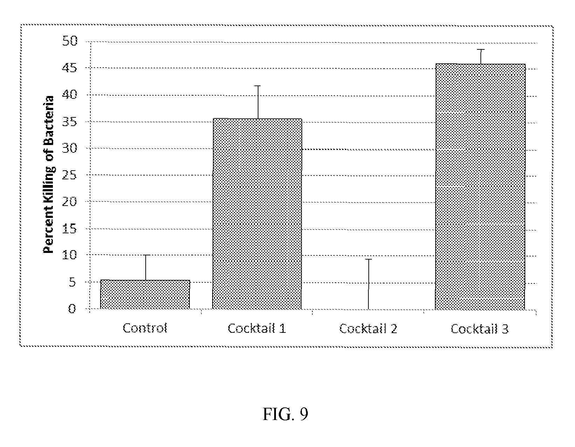

Fig. 9 is a graph showing that anti-S. *aureus* recombinant polyclonal antibody mixtures with the capacity ofbinding to the cell surface of the Wood-46 S. *aureus* strain can enhance the ability of white blood cells to opsonize the bacteria.

### DETAILED DESCRIPTION

The human recombinant polyclonal anti-*S*. *aureus* antibody compositions disclosed herein are based, in part, on the antigen binding sites of certain human antibodies that have been selected on the basis of binding and neutralizing activity of one or more *S. aureus* strains. The terms "anti-*S*. *aureus* polyclonal antibody" and "anti-*S*. *aureus* recombinant polyclonal antibody" describe a composition of recombinantly produced diverse antibody molecules, where the individual members of the polyclonal composition are capable of binding to at least one epitope on *S. aureus* or an *S. aureus* secreted protein *(e.g.,* a toxin or immunomodulator) or a cell surface antigen and where the polyclonal composition as a whole is capable of neutralizing *S. aureus.* In an exemplary embodiment, an anti-*S*. *aureus* polyclonal antibody neutralizes *S. aureus* and/or one or more antibiotic-resistant *S. aureus* strains. It is contemplated that the disclosed anti-*S*. *aureus* polyclonal antibodies are essentially free from immunoglobulin molecules that do not bind to *S. aureus* or variant strains thereof *(e.g.,* at least 70%, 75%, 80%, 85%, 90%, 95%, 98%, 99%, or 100% of the antibodies contained in the polyclonal composition bind to one or more strains of *S. aureus).*

As described herein, the diversity of antibodies included in an anti-*S*. *aureus* recombinant polyclonal antibody composition provide a surprising benefit over monoclonal and biclonal (*e.g.,* a mixture of two monoclonal antibodies) antibodies because lower dosages of the polyclonal antibody composition may be administered to prevent or treat *S. aureus* infection. For example, it is contemplated herein that the synergistic action of the individual component antibodies in the polyclonal composition allow the polyclonal composition to be effective at lower doses than is possible with conventional monoclonal antibody therapy. In addition, because a polyclonal antibody composition binds to multiple different proteins, the composition as a whole can use lower amounts of each individual antibody to prevent or treat *S. aureus* infections. Further, unlike monoclonal or biclonal antibody compositions, polyclonal antibody compositions do not present the same concerns regarding the generation of drug resistance to a single or small number of agents (*e.g.,* development of resistant *S. aureus* strains due to the monovalent nature of a monoclonal antibody's mode of action). It is also contemplated herein that the use of human antibodies in the polyclonal antibody composition are less likely to evoke an immune response compared to monoclonal antibodies, even fully human monoclonal antibodies, due to the complex nature of the polyclonal composition.

In view of the neutralizing activity of the disclosed polyclonal antibodies, they are useful for modulating the growth and/or colonization of one or more *S. aureus* strains including antibiotic-resistant *S. aureus* strains in a host cell; reducing or killing one or more strains of *S*. *aureus* including antibiotic-resistant *S. aureus* strains; and/or treating or preventing a *S*. *aureus* infection including infection with an antibiotic-resistant strain in a mammal. An anti-*S*. *aureus* polyclonal antibody may bind to *S. aureus* antigens in a multivalent manner, which may result in synergistic neutralization, improved phagocytosis of infected cells by macrophages, improved antibody-dependent cellular cytotoxicity (ADCC) against infected cells, and/or increased complement activity. It is contemplated herein that *S. aureus* is a multifaceted pathogen that may be neutralized using a multifaceted antibody approach that targets various antigens thereby enhancing the capacity of the immune system (*e.g.,* opsonization) to eliminate these bacteria.

In one embodiment, the diversity of the recombinant polyclonal antibody is located in the variable regions (*e.g.,* V_{H} and V_{L} regions) of the individual antibodies in the polyclonal antibody composition, in particular, in the CDR₁, CDR₂, and CDR₃ regions of the immunoglobulin heavy and/or light chains. For example, the individual antibodies of the polyclonal antibody composition contain (a) an immunoglobulin heavy chain variable region comprising the structure CDR_{H1}-CDR_{H2}-CDR_{H3} and (b) an immunoglobulin light chain variable region comprising the structure CDR_{L1}-CDR_{L2}-CDR_{L3}, wherein the heavy chain variable region and the light chain variable region together define a single binding site for binding to one or more S. *aureus* strains by binding to an antigen of *S*. *aureus* or an *S. aureus* antigenic epitope. An individual antibody of the polyclonal composition may also bind an *S. aureus* secreted protein or an antigenic epitope on the secreted protein. In some embodiments, each individual antibody of the polyclonal composition binds to an epitope, which is not bound by any other member of the polyclonal composition. In other embodiments, one or more S. *aureus* antigens or antigenic epitopes may be bound by more than one individual antibody of the polyclonal composition.

An individual antibody molecule of the recombinant polyclonal antibody composition may be characterized by its variable region sequences *(i.e.,* V_{H} and V_{L} region), or by its CDR₁, CDR₂, and CDR₃ regions of immunoglobulin heavy and light chains. When the individual antibodies of the polyclonal composition are defined by their heavy and light chain CDR regions, it is contemplated that the CDR regions are interposed between human immunoglobulin framework regions (FRs).

It is understood that each of the antibodies discussed above can be an intact tetrameric antibody. Alternatively, the antibody can be an antigen-binding fragment of an antibody. Antibody fragments include Fab, Fab', F(ab')₂, and Fv fragments, and single chain antibodies (*e.g.,* scFv).

An antibody, or antigen binding fragment thereof, may also be conjugated to an effector agent such as a small molecule toxin, a drug, or a radionuclide using standard *in vitro* conjugation chemistries. If the effector agent is a polypeptide, the antibody can be chemically conjugated to the effector or joined to the effector as a fusion protein. Construction of fusion proteins is within ordinary skill in the art.

Human antibodies are selected based on binding to one or more *S. aureus* strains. Individual antibodies of the polyclonal composition may bind a protein associated with *S. aureus* virulence, such as, but not limited to coagulase, leukocidin (Luk), panton-valentine leukocidin (PVL), aureolysin, staphylokinase (SAK), beta-hemolysin (β-HL), delta-hemolysin (δ-HL), gamma-hemolysin (γ-HL), alpha-toxin (α-toxin), staphylococcal complement inhibitor (SCIN), enterotoxins, and adhesions (*e.g*., clumping factor A (ClfA), clumping factor B (ClfB), fibronectin binding protein (FnbpA), and fibronectin binding protein B (FnbpB). Additional *S. aureus* target proteins for binding by a *S*. *aureus* antibody include SdrD (a cell surface protein containing serine-aspartate (SD) repeats with organization and sequence similarity to fibrinogen-binding clumping factors ClfA and ClfB), IsaA, Aux1 (a transmembrane protein phosphatase), and LP309 (a lipoprotein). The disclosed antibodies may also bind to uncharacterized *S. aureus* proteins.

In certain embodiments, an anti-*S*. *aureus* polyclonal antibody binds to at least two, at least three, at least four, at least five, at least six, at least seven, at least eight, at least nine, at least ten, at least 15, at least 20, at least 25 or more *S. aureus* proteins. In an exemplary embodiment, an anti-*S*. *aureus* polyclonal antibody binds to at least three *S. aureus* proteins. In certain embodiments, the three proteins may include a cell surface antigen, a toxin and/or an immunomodulator produced by *S. aureus.* In one embodiment, an anti-*S*. *aureus* polyclonal antibody binds at least a cell surface antigen on one or more strains of *S. aureus* and a toxin produced by one or more strains of *S*. *aureus.* In another embodiment, an anti-*S*. *aureus* polyclonal antibody binds at least a cell surface antigen on one or more strains of *S. aureus* and an immunomodulator produced by one or more strains of *S. aureus.* In yet another embodiment, an anti-*S*. *aureus* polyclonal antibody binds at least a toxin produced by one or more strains of *S. aureus* and an immunomodulator produced by one or more strains of S. *aureus.* In certain embodiments, the at least 2, 3, 4, 5, 10, 15, 20, 25 or more proteins are on more than one strain of *S. aureus.* It is contemplated herein that the broad spectrum nature of the disclosed anti-*S*. *aureus* antibody compositions may be attributed to the inclusion of individuals antibodies that bind a protein present on more than one strain of S. *aureus (e.g.,* conserved proteins). In addition, the broad spectrum efficacy of the disclosed polyclonal antibodies may be attributed to the inclusion of certain antibodies that bind antigens associated with only one or two *S. aureus* strains.

An anti-*S*. *aureus* polyclonal antibody composition may also be composed of individual antibodies raised by the immune response of a donor (*e.g.,* a human), which has been vaccinated or infected with *S. aureus.* Further, if antibodies to a particular antigen are known to be relevant and/or effective in the protection, neutralization and/or elimination of *S*. *aureus* infection, such antibodies may be raised by immunization of a donor with that particular antigen.

It is contemplated herein that the disclosed recombinant polyclonal antibodies are not naturally occurring antibodies isolated from human blood. Exemplary recombinant polyclonal antibodies described herein are a mixture of antibodies, wherein each individual antibody may be expressed from a cell or cell line transfected with an expression vector comprising the coding sequence of the antibody, which is not naturally associated with the cell.

A human recombinant polyclonal anti-*S*. *aureus* antibody disclosed herein may comprise at least three, at least 4, at least 5, at least 7, at least 10, at least 15, at least 20, at least 25 or more antibodies. In exemplary embodiments, the recombinant polyclonal antibodies disclosed herein may comprise about 3 to about 30 antibodies, about 3 to about 25 antibodies, about 3 to about 20 antibodies, about 3 to about 15 antibodies, about 3 to about 10 antibodies, about 3 to about 5 antibodies, about 5 to about 30 antibodies, about 5 to about 25 antibodies, about 5 to about 20 antibodies, about 5 to about 15 antibodies, about 5 to about 10 antibodies, about 8 to about 30 antibodies, about 8 to about 25 antibodies, about 8 to about 20 antibodies, about 8 to about 15 antibodies, about 8 to about 10 antibodies, about 10 to about 30 antibodies, about 10 to about 25 antibodies, about 10 to about 20 antibodies, about 10 to about 15 antibodies, about 15 to about 25 antibodies, about 15 to about 20 antibodies, about 20 to about 25 antibodies, and about 25 to about 30 antibodies.

A polyclonal anti-*S*. *aureus* antibody may comprise immunoglobulin heavy and light chain variable regions or, heavy and light chain CDR regions, from two, three, four, five, ten or more of the following antibodies as disclosed herein: 1.62.B9, 5.11.H10, 5.6.H2, 5.6.H9, 5.17.F8, 5.19.F12, 5.23.C9, 5.23.C12, 5.27.A11, 8.51.G11, 9.51.H9, 18.43.D8, 22.22.E7, 8.51.G10, 5.24A.A7, 5.24A.F3, 5.8B.H4, 26.51.E1, 22.21.A7, 22.18A.E9, 5.52.H10, 5.15.C1, 5.54.E6, 5.55.D2, 22.14.A1, 26.53.B4, 5.63.E2, 5.64.G4, 43.52.A11, 43.52.E12 and 43.62.E2, wherein each of the disclosed antibodies comprise the immunoglobulin heavy and light chain CDR1, CDR2, CDR3 sequences set forth in Table 3. For example, antibody 5.6.H9 comprises the CDR1, CDR2, and CDR3 sequences of an immunogloblulin heavy chain amino acid sequence of SEQ ID NO: 38 and the CDR1, CDR2, and CDR3 of an immunoglobulin light chain amino acid sequence of SEQ ID NO: 40. Also, as disclosed in Table 3, for example, antibody 5.6.H9 comprises an immunoglobulin heavy chain comprising a CDR_{H1} comprising the amino acid sequence of SEQ ID NO: 41, a CDR_{H2} comprising amino acid sequence of SEQ ID NO: 42 and a CDR_{H3} comprising an amino acid sequence of SEQ ID NO: 43; and an immunoglobulin light chain comprising a CDR_{L1} comprising the amino acid sequence of SEQ ID NO: 44, a CDR_{L2} comprising the amino acid sequence of DAS and a CDR_{L3} comprising an amino acid sequence of SEQ ID NO: 45.

Exemplary recombinant polyclonal antibody compositions including two antibodies disclosed herein are shown in Table 1.

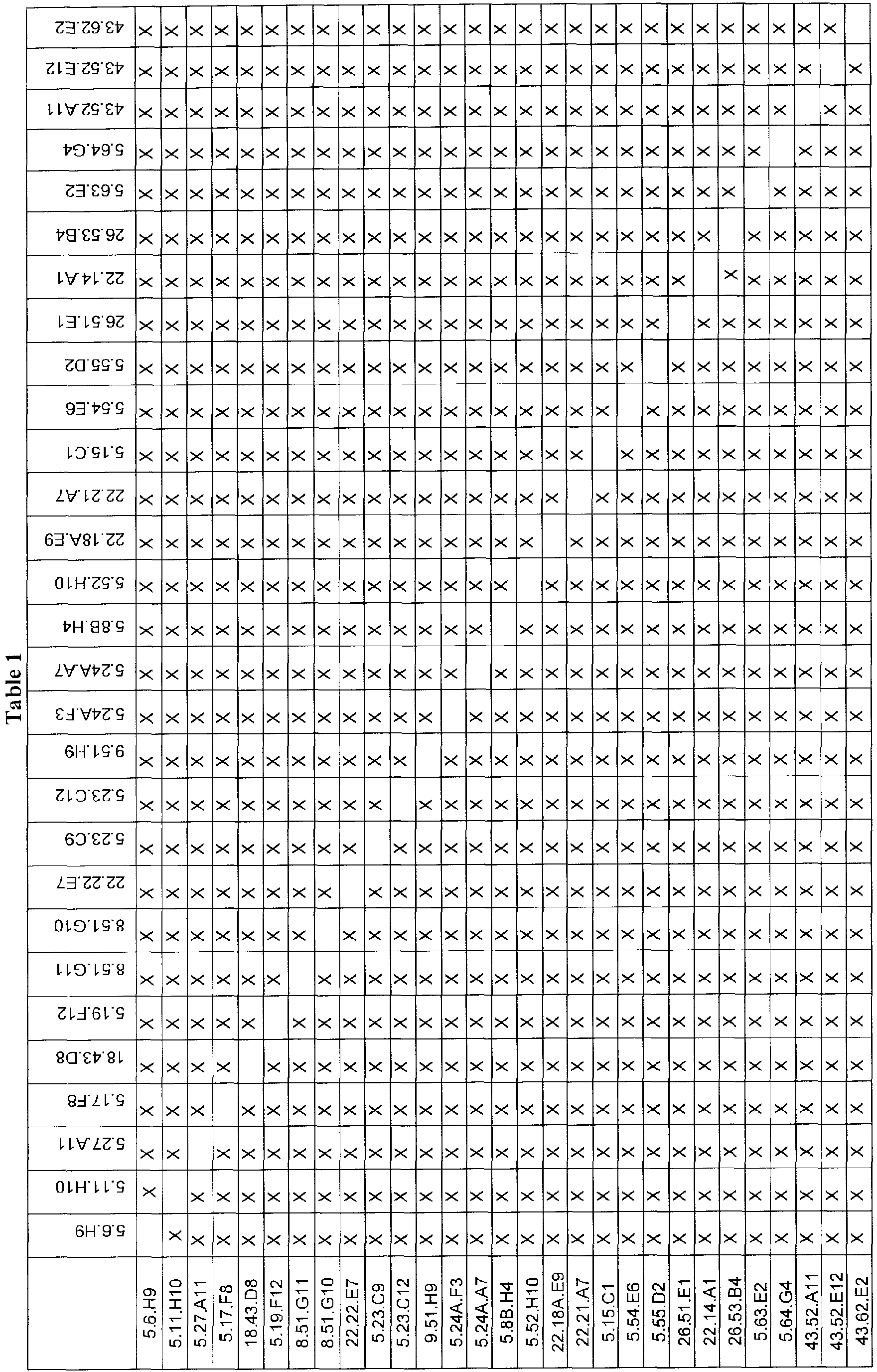

Exemplary recombinant polyclonal antibody compositions including three antibodies disclosed herein include: 5.6.H9, 22.18A.E9 and 5.55.D2; 5.6.H9, 22.18A.E9 and 9.51.H9; 5.6 H9, 22.18A.E9 and 5.11.H10; 5.6 H9, 22.18A.E9 and 5.23.C12; 5.6.H9, 22.18A.E9 and 5.52.H10; 5.6.H9, 22.18A.E9 and 18.43.D8; 5.6 H9, 22.18A.E9 and 8.51 G10; 5.6.H9, 22.18A.E9 and 8.51.G11.

It is also contemplated herein that the disclosed recombinant human polyclonal antibody compositions may include one or more antibodies that compete with one of the disclosed antibodies for binding to one or more *S. aureus* strains, for example, under the conditions described in Example 4. Exemplary *S. aureus* strains for determining whether an antibody competes with binding to a disclosed antibody include ATCC Strain BAA-1717 (also known as USA300), ATCC Strain 10832 (also known as Wood-46), NRS071 (also known as Sanger 252), NRS100 (also known as COL), NRS382 (also known as strain 626), NRS384 (also known as LAC), NRS123 (also known as MW2), NRS001 (also known as Mu50), NRS072 (also known as Sanger 476), NRS 102 (also known as Reynolds), NRS111 (also known as FRI913), NRS 144 (also known as RN4220) and USA300. Other exemplary *S. aureus* stains include USA100, USA200, USA400 and USA500 types.

It is contemplated herein that the disclosed polyclonal antibody compositions bind at least 3 different S. *aureus* strains. For example, in exemplary embodiments, the disclosed polyclonal antibody compositions bind at least 4, at least 5, at least 6, at least 7, at least 8, at least 9, at least 10, at least 11, or at least 12 different *S. aureus* strains, *e.g.,* the strains identified in Table 4.

In one embodiment, an antibody provided herein competes with 5.6.H9 comprising CDR1, CDR2 and CDR3 of heavy chain amino acid sequence SEQ ID NO: 38 and CDR1, CDR2, CDR3 of light chain amino acid sequence SEQ ID NO: 40 for binding to one or more *S. aureus* strains. Exemplary strains for a competitive binding assay with 5.6.H9, for example, under the conditions set forth in Example 4, are set forth in Table 4.

In another embodiment, an antibody provided herein competes with 22.18A.E9 comprising CDR1, CDR2 and CDR3 of heavy chain amino acid sequence SEQ ID NO: 173 and CDR1, CDR2, CDR3 of light chain amino acid sequence SEQ ID NO: 175 for binding to one or more S. *aureus.* Exemplary strains for a competitive binding assay with 22.18A.E9, for example, under the conditions set forth in Example 4, are set forth in Table 4.

In another embodiment, an antibody provided herein competes with 5.11.H10 comprising CDR1, CDR2 and CDR3 of heavy chain amino acid sequence SEQ ID NO: 11 and CDR1, CDR2, CDR3 of light chain amino acid sequence SEQ ID NO: 13 for binding to one or more S. *aureus* strains. Exemplary strains for a competitive binding assay with 5.11.H10, for example, under the conditions set forth in Example 4, are set forth in Table 4.

In another embodiment, an antibody provided herein competes with 5.27.A11 comprising CDR1, CDR2 and CDR3 of heavy chain amino acid sequence SEQ ID NO: 20 and CDR1, CDR2, CDR3 of light chain amino acid sequence SEQ ID NO: 22 for binding to one or more *S. aureus* strains. Exemplary strains for a competitive binding assay with 5.27.A11, for example, under the conditions set forth in Example 4, are set forth in Table 4.

In another embodiment, an antibody provided herein competes with 5.17.F8 comprising CDR1, CDR2 and CDR3 of heavy chain amino acid sequence SEQ ID NO: 47 and CDR1, CDR2, CDR3 of light chain amino acid sequence SEQ ID NO: 49 for binding to one or more *S. aureus* strains. Exemplary strains for a competitive binding assay with 5.17.F8, for example, under the conditions set forth in Example 4, are set forth in Table 4.

In another embodiment, an antibody provided herein competes with 5.19.F12 comprising CDR1, CDR2 and CDR3 of heavy chain amino acid sequence SEQ ID NO: 56 and CDR1, CDR2, CDR3 of light chain amino acid sequence SEQ ID NO: 58 for binding to one or more S. *aureus* strains. Exemplary strains for a competitive binding assay with 5.19.F12, for example, under the conditions set forth in Example 4, are set forth in Table 4.

In another embodiment, an antibody provided herein competes with 5.23.C9 comprising CDR1, CDR2 and CDR3 of heavy chain amino acid sequence SEQ ID NO: 65 and CDR1, CDR2, CDR3 of light chain amino acid sequence SEQ ID NO: 67 for binding to one or more S. *aureus* strains. Exemplary strains for a competitive binding assay with 5.23.C9, for example, under the conditions set forth in Example 4, are set forth in Table 4.

In another embodiment, an antibody provided herein competes with 5.23.C12 comprising CDR1, CDR2 and CDR3 of heavy chain amino acid sequence SEQ ID NO: 74 and CDR1, CDR2, CDR3 of light chain amino acid sequence SEQ ID NO: 76 for binding to one or more *S. aureus* strains. Exemplary strains for a competitive binding assay with 5.23.C12, for example, under the conditions set forth in Example 4, are set forth in Table 4.

In another embodiment, an antibody provided herein competes with 8.51. G11 comprising CDR1, CDR2 and CDR3 of heavy chain amino acid sequence SEQ ID NO: 83 and CDR1, CDR2, CDR3 of light chain amino acid sequence SEQ ID NO: 85 for binding to one or more *S. aureus* strains. Exemplary strains for a competitive binding assay with 8.51 .G11, for example, under the conditions set forth in Example 4, are set forth in Table 4.

In another embodiment, an antibody provided herein competes with 9.51.H9 comprising CDR1, CDR2 and CDR3 of heavy chain amino acid sequence SEQ ID NO: 92 and CDR1, CDR2, CDR3 of light chain amino acid sequence SEQ ID NO: 94 for binding to one or more *S. aureus* strains. Exemplary strains for a competitive binding assay with 9.51.H9, for example, under the conditions set forth in Example 4, are set forth in Table 4.

In another embodiment, an antibody provided herein competes with 18.43.D8 comprising CDR1, CDR2 and CDR3 of heavy chain amino acid sequence SEQ ID NO: 101 and CDR1, CDR2, CDR3 of light chain amino acid sequence SEQ ID NO: 103 for binding to one or more *S. aureus* strains. Exemplary strains for a competitive binding assay with 18.43.D8, for example, under the conditions set forth in Example 4, are set forth in Table 4.

In another embodiment, an antibody provided herein competes with 22.22.E7 comprising CDR1, CDR2 and CDR3 of heavy chain amino acid sequence SEQ ID NO: 110 and CDR1, CDR2, CDR3 of light chain amino acid sequence SEQ ID NO: 112 for binding to one or more *S. aureus* strains. Exemplary strains for a competitive binding assay with 22.22.E7, for example, under the conditions set forth in Example 4, are set forth in Table 4.

In another embodiment, an antibody provided herein competes with 8.51.G10 comprising CDR1, CDR2 and CDR3 of heavy chain amino acid sequence SEQ ID NO: 149 and CDR1, CDR2, CDR3 of light chain amino acid sequence SEQ ID NO: 151 for binding to one or more *S. aureus* strains. Exemplary strains for a competitive binding assay with 8.51.G10, for example, under the conditions set forth in Example 4, are set forth in Table 4.

In another embodiment, an antibody provided herein competes with 5.24A.A7 comprising CDR1, CDR2 and CDR3 of heavy chain amino acid sequence SEQ ID NO: 153 and CDR1, CDR2, CDR3 of light chain amino acid sequence SEQ ID NO: 155 for binding to one or more *S. aureus* strains. Exemplary strains for a competitive binding assay with 5.24A.A7, for example, under the conditions set forth in Example 4, are set forth in Table 4.

In another embodiment, an antibody provided herein competes with 5.24A.F3 comprising CDR1, CDR2 and CDR3 of heavy chain amino acid sequence SEQ ID NO: 157 and CDR1, CDR2, CDR3 of light chain amino acid sequence SEQ ID NO: 159 for binding to one or more *S. aureus* strains. Exemplary strains for a competitive binding assay with 5.24A.F3, for example, under the conditions set forth in Example 4, are set forth in Table 4.

In another embodiment, an antibody provided herein competes with 5.8B.H4 comprising CDR1, CDR2 and CDR3 of heavy chain amino acid sequence SEQ ID NO: 161 and CDR1, CDR2, CDR3 of light chain amino acid sequence SEQ ID NO: 163 for binding to one or more *S. aureus* strains. Exemplary strains for a competitive binding assay with 5.8B.H4, for example, under the conditions set forth in Example 4, are set forth in Table 4.

In another embodiment, an antibody provided herein competes with 26.51.E1 comprising CDR1, CDR2 and CDR3 of heavy chain amino acid sequence SEQ ID NO: 165 and CDR1, CDR2, CDR3 of light chain amino acid sequence SEQ ID NO: 167 for binding to one or more *S. aureus* strains. Exemplary strains for a competitive binding assay with 26.51.E1, for example, under the conditions set forth in Example 4, are set forth in Table 4.

In another embodiment, an antibody provided herein competes with 22.21.A7 comprising CDR1, CDR2 and CDR3 of heavy chain amino acid sequence SEQ ID NO: 169 and CDR1, CDR2, CDR3 of light chain amino acid sequence SEQ ID NO: 171 for binding to one or more *S. aureus* strains. Exemplary strains for a competitive binding assay with 22.21.A7, for example, under the conditions set forth in Example 4, are set forth in Table 4.

In another embodiment, an antibody provided herein competes with 5.52.H1 comprising CDR1, CDR2 and CDR3 of heavy chain amino acid sequence SEQ ID NO: 177 and CDR1, CDR2, CDR3 of light chain amino acid sequence SEQ ID NO: 179 for binding to one or more *S. aureus* strains. Exemplary strains for a competitive binding assay with 5.52.H1, for example, under the conditions set forth in Example 4 are set forth in Table 4.

In another embodiment, an antibody provided herein competes with 5.15.C1 comprising CDR1, CDR2 and CDR3 of heavy chain amino acid sequence SEQ ID NO: 181 and CDR1, CDR2, CDR3 of light chain amino acid sequence SEQ ID NO: 183 for binding to one or more *S. aureus* strains. Exemplary strains for a competitive binding assay with 5.15.C1, for example, under the conditions set forth in Example 4, are set forth in Table 4.

In another embodiment, an antibody provided herein competes with 5.54.E6 comprising CDR1, CDR2 and CDR3 of heavy chain amino acid sequence SEQ ID NO: 185 and CDR1, CDR2, CDR3 of light chain amino acid sequence SEQ ID NO: 187 for binding to one or more S. *aureus* strains. Exemplary strains for a competitive binding assay with 5.54.E6, for example, under the conditions set forth in Example 4, are set forth in Table 4.

In another embodiment, an antibody provided herein competes with 5.55.D2 comprising CDR1, CDR2 and CDR3 of heavy chain amino acid sequence SEQ ID NO: 189 and CDR1, CDR2, CDR3 of light chain amino acid sequence SEQ ID NO: 191 for binding to one or more *S. aureus* strains. Exemplary strains for a competitive binding assay with 5.55.D2, for example, under the conditions set forth in Example 4, are set forth in Table 4.

In another embodiment, an antibody provided herein competes with 22.14.A1 comprising CDR1, CDR2 and CDR3 of heavy chain amino acid sequence SEQ ID NO: 193 and CDR1, CDR2, CDR3 of light chain amino acid sequence SEQ ID NO: 195 for binding to one or more *S. aureus* strains. Exemplary strains for a competitive binding assay with 22.14.A1, for example, under the conditions set forth in Example 4 are set forth in Table 4.

In another embodiment, an antibody provided herein competes with 26.53.B4 comprising CDR1, CDR2 and CDR3 of heavy chain amino acid sequence SEQ ID NO: 197 and CDR1, CDR2, CDR3 of light chain amino acid sequence SEQ ID NO: 199 for binding to one or more *S. aureus* strains. Exemplary strains for a competitive binding assay with 26.53.B4, for example, under the conditions set forth in Example 4, are set forth in Table 4.

In another embodiment, an antibody provided herein competes with 5.63.E2 comprising CDR1, CDR2 and CDR3 of heavy chain amino acid sequence SEQ ID NO: 201 and CDR1, CDR2, CDR3 of light chain amino acid sequence SEQ ID NO: 203 for binding to one or more *S. aureus* strains. Exemplary strains for a competitive binding assay with 5.63.E2, for example, under the conditions set forth in Example 4, are set forth in Table 4.

In another embodiment, an antibody provided herein competes with 5.64.G4 comprising CDR1, CDR2 and CDR3 of heavy chain amino acid sequence SEQ ID NO: 205 and CDR1, CDR2, CDR3 of light chain amino acid sequence SEQ ID NO: 207 for binding to one or more *S. aureus* strains. Exemplary strains for a competitive binding assay with 5.64.G4, for example, under the conditions set forth in Example 4, are set forth in Table 4.

In another embodiment, an antibody provided herein competes with 43.52.A11 comprising CDR1, CDR2 and CDR3 of heavy chain amino acid sequence SEQ ID NO: 209 and CDR1, CDR2, CDR3 of light chain amino acid sequence SEQ ID NO: 211 for binding to one or more *S. aureus* strains. Exemplary strains for a competitive binding assay with 43.52.A11, for example, under the conditions set forth in Example 4, are set forth in Table 4.

In another embodiment, an antibody provided herein competes with 43.52.E12 comprising CDR1, CDR2 and CDR3 of heavy chain amino acid sequence SEQ ID NO: 213 and CDR1, CDR2, CDR3 of light chain amino acid sequence SEQ ID NO: 215 for binding to one or more *S. aureus* strains. Exemplary strains for a competitive binding assay with 43.52.E12, for example, under the conditions set forth in Example 4, are set forth in Table 4.

In another embodiment, an antibody provided herein competes with 43.62.E2 comprising CDR1, CDR2 and CDR3 of heavy chain amino acid sequence SEQ ID NO: 217 and CDR1, CDR2, CDR3 of light chain amino acid sequence SEQ ID NO: 219 for binding to one or more *S. aureus* strains. Exemplary strains for a competitive binding assay with 43.62.E2, for example, under the conditions set forth in Example 4, are set forth in Table 4.

In another aspect, isolated human antibodies are also disclosed (*e.g.,* isolated, individual antibodies or antigen binding fragments thereof). Exemplary human antibodies include an isolated human antibodies that bind *S*. *aureus* delta-toxin. One exemplary antibody that binds *S. aureus* delta-toxin comprises CDR1, CDR2 and CDR3 of heavy chain amino acid sequence SEQ ID NO: 38 and CDR1, CDR2, CDR3 of light chain amino acid sequence SEQ ID NO: 40 of 5.6.H9. Another exemplary antibody that bind *S*. *aureus* delta-toxin comprises CDR1, CDR2 and CDR3 of heavy chain amino acid sequence SEQ ID NO: 189 and CDR1, CDR2, CDR3 of light chain amino acid sequence SEQ ID NO: 191 of 5.55.D2.

Also provided herein is an isolated human antibody that binds *S. aureus* phenol soluble modulin beta-1. The antibody comprises CDR1, CDR2 and CDR3 of heavy chain amino acid sequence SEQ ID NO: 173 and CDR1, CDR2, CDR3 of light chain amino acid sequence SEQ ID NO: 175 of 22.18A.E9.

Additional exemplary isolated human antibodies disclosed herein include: a human antibody that binds one or more *S. aureus* strains comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9;

a human antibody that binds one or more *S. aureus* strains comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10;

a human antibody that binds one or more *S. aureus* strains comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12;

a human antibody that binds one or more *S. aureus* strains comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10;

a human antibody that binds one or more *S. aureus* strains comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8;

a human antibody that binds one or more *S. aureus* strains comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10; and

a human antibody that binds one or more *S. aureus* strains comprising CDR1, CDR2 and CDR3 of heavy chain amino acid sequence SEQ ID NO: 83 and CDR1, CDR2, CDR3 of light chain amino acid sequence SEQ ID NO: 85 of 8.51.G11.

### Production of Antibodies

Methods for producing antibodies of the invention are known in the art. In certain embodiments, an anti-*S*. *aureus* polyclonal antibody composition may be produced from a single manufacturing cell line or a mixture of cell lines producing individual monoclonal antibodies. In other embodiments, DNA molecules encoding light chain variable regions and heavy chain variable regions can be chemically synthesized using the sequence information provided herein. Synthetic DNA molecules can be ligated to other appropriate nucleotide sequences, including, e.g., constant region coding sequences, and expression control sequences, to produce conventional gene expression constructs encoding the desired antibodies. Production of defined gene constructs is within routine skill in the art. Alternatively, the sequences provided herein can be cloned out of hybridomas or B-cells by conventional hybridization techniques or polymerase chain reaction (PCR) techniques, using synthetic nucleic acid probes whose sequences are based on sequence information provided herein, or prior art sequence information regarding genes encoding the heavy and light chains of human antibodies in hybridoma cells.

As disclosed herein, individual anti-*S*. *aureus* antibodies may be characterized by their variable region (V_{H} and V_{L} sequences) or by their heavy and light chain CDR sequences. Each antibody will have a pair of sequences if defined by its variable region sequences (*i.e*., V_{H} and V_{L} cognate pairs) or a set of sequences if defined by its CDR sequences *(i.e.,* three heavy chain CDRs and three light chain CDRs). V_{H} and V_{L} pairs can be expressed as full-length antibodies, Fab fragments or other antibody fragments that have binding specificity to a *S*. *aureus* associated antigen. Specific V_{H} and V_{L} pairs are identified in Table 2 in Example 1. Specific heavy and light chain CDR sets are identified in Table 3 in Example 1.

Nucleic acids encoding desired antibodies (*e.g.,* V_{H} and V_{L} pairs) can be incorporated (ligated) into expression vectors, which can be introduced into host cells through conventional transfection or transformation techniques. Exemplary host cells are *E.coli* cells, Chinese hamster ovary (CHO) cells, human embryonic kidney 293 (HEK 293) cells, HeLa cells, baby hamster kidney (BHK) cells, monkey kidney cells (COS), human hepatocellular carcinoma cells (*e.g.,* Hep G2), and myeloma cells that do not otherwise produce IgG protein. Transformed host cells can be grown under conditions that permit the host cells to express the genes that encode the immunoglobulin light and/or heavy chain variable regions.

Specific expression and purification conditions will vary depending upon the expression system employed. For example, if a gene is to be expressed in *E. coli,* it is first cloned into an expression vector by positioning the engineered gene downstream from a suitable bacterial promoter, e.g., Trp or Tac, and a prokaryotic signal sequence. The expressed secreted protein accumulates in refractile or inclusion bodies, and can be harvested after disruption of the cells by French press or sonication. The refractile bodies then are solubilized, and the proteins refolded and cleaved by methods known in the art.

If the engineered gene is to be expressed in eukayotic host cells, e.g., CHO cells, it is first inserted into an expression vector containing a suitable eukaryotic promoter, a secretion signal, IgG enhancers, and various introns. This expression vector optionally contains sequences encoding all or part of a constant region, enabling an entire, or a part of, a heavy or light chain to be expressed. The gene construct can be introduced into eukaryotic host cells using convention techniques. The host cells express V_{L} or V_{H} fragments, V_{L}-V_{H} heterodimers, V_{H}-V_{L} or V_{L}-V_{H} single chain polypeptides, complete heavy or light immunoglobulin chains, or portions thereof, each of which may be attached to a moiety having another function (*e.g.,* cytotoxicity). In some embodiments, a host cell is transfected with a single vector expressing a polypeptide expressing an entire, or part of, a heavy chain *(e.g.,* a heavy chain variable region) or a light chain *(e.g.,* a light chain variable region). In other embodiments, a host cell is transfected with a single vector encoding (a) a polypeptide comprising a heavy chain variable region and a polypeptide comprising a light chain variable region, or (b) an entire immunoglobulin heavy chain and an entire immunoglobulin light chain. In still other embodiments, a host cell is co-transfected with more than one expression vector (*e.g.,* one expression vector expressing a polypeptide comprising an entire, or part of, a heavy chain or heavy chain variable region, and another expression vector expressing a polypeptide comprising an entire, or part of, a light chain or light chain variable region).

The expression vector may also include constant regions for the heavy and/or light chain. It is contemplated that the choice of the constant region may vary for the individual antibodies included in the polyclonal composition. For example, it may be desirous to have IgG1 constant regions for certain antibodies and IgG2 constant regions for other antibodies depending on the desired effector function to clear or destroy antigen (*e.g.,* ADCC, phagocytosis, increased complement activity (*e.g.,* via the classic and/or alternative complement pathways), binding to mass cells and/or basinophiles). Heavy chain constant regions may be selected from the isotypes IgG1, IgG2, IgG3, IgG4, IgA1, IgA2, IgM, IgD, and IgE. Light chain constant regions may be either kappa or lambda.

A polypeptide comprising an immunoglobulin heavy chain variable region or light chain variable region can be produced by growing a host cell transfected with an expression vector encoding such variable region, under conditions that permit expression of the polypeptide. Following expression, the polypeptide can be harvested and purified using techniques well known in the art, *e.g.,* affinity tags such as glutathione-S-transferase (GST) and histidine tags.

A human antibody that binds to one or more *S. aureus* strains, or an antigen-binding fragment of the antibody, can be produced by growing a host cell transfected with: (a) an expression vector that encodes a complete or partial immunoglobulin heavy chain, and a separate expression vector that encodes a complete or partial immunoglobulin light chain; or (b) a single expression vector that encodes both chains, under conditions that permit expression of both chains. The intact antibody (or antigen-binding fragment) can be harvested and purified using techniques well known in the art, *e.g.,* Protein A, Protein G, affinity tags such as glutathione-S-transferase (GST) and histidine tags. It is within ordinary skill in the art to express the heavy chain and the light chain from a single expression vector or from two separate expression vectors.

### Use of Antibodies

Anti-*S*. *aureus* polyclonal antibodies as described herein can be used to treat one or more strains of *S. aureus* and/or prevent infection of one or more strains of *S. aureus,* including antibiotic resistant strains of *S. aureus, e.g.,* penicillin-resistant strains, methicillin-resistant strains (MRSA) (*e.g.,* community acquired MRSA (CA-MRSA), hospital-acquired MRSA (HA-MRSA)), vancomycin-resistant strains (VRSA), and vancomycin-intermediate resistant strains (VISA). The disclosed antibodies may also be used to treat methicillin-sensitive strains (*e.g.,* MSSA). *S. aureus* infected host cells (*e.g.,* mammalian host cells, *e.g.,* human host cells) are exposed to a therapeutically effective amount of the antibody so as to inhibit infectivity of *S. aureus* by inhibiting growth and/or colonization, and/or by inducing phagocytosis, and/or killing the S. *aureus.* In some embodiments, the antibodies inhibit infectivity of *S. aureus* by at least 30%, 40%, 50%, 60%, 70%, 80%, 90%, 95%, 98%, 99%, or 100%.

In certain embodiments, individual human anti-*S*. *aureus* antibodies may be used to treat one or more strains of *S. aureus* and/or prevent infection of one or more strains of *S. aureus.*

As used herein, "treat", "treating" and "treatment" mean the treatment of a disease in a mammal, *e.g.,* in a human. This includes: (a) inhibiting the disease or infection, *i.e.,* arresting its development or progression; (b) relieving the disease or infection, *i.e.,* causing regression of the disease state or infection; and/or (c) curing the disease or infection.

Exemplary diseases that can be treated or prevented using the disclosed antibodies include, but are not limited to, invasive or toxigenic diseases associated with pathogenic *S. aureus* strains. Invasive diseases include pneumonia (e.g., S. aureus pneumonia), meningitis, Bacteremia, osteomyelitis, sepsis *(e.g.,* septic arthritis, septic thrombophlebitis), and endocarditis (*e.g.,* acute bacterial endocarditis). Toxic diseases include, but are not limited to, Staphylococcal food poisoning, scalded skin syndrome, and toxic shock syndrome (TSS). Additional examples of diseases and disorders that may be treated include skin abscesses, cellulitis, upper respiratory tract infections (*e.g.,* otis media, bacterial trachetis, acute epiglottitis, thyroiditis), lower respiratory tract infections (*e.g.,* empyema, lung abscess), heart, gastrointestinal *(e.g.,* secretory diarrhea, splenic abscess, retroperitoneal abscess), CNS *(e.g.,* cerebral abscess), eye (*e.g.,* blepharitis, conjunctivitis, keratitis, endophthalmitis, preseptal and orbital cellulitis, darcryocystitis), kidney, urinary, skin (*e.g.,* impetigo, folloculitis, cutaneous abscesses, cellulitis, wound infection, bacterial myositis), and bone and joint infections. It is contemplated herein that certain disorders associated with polymicrobial infections including a *S*. *aureus* infection may be treated or prevented using the disclosed antibodies. Exemplary disorders associated with polymicrobial infections (including *S. aureus* infections) include cystic fibrosis *(e.g.,* infections with *S. aureus* and *Pseudomonas),* upper and lower respiratory tract infections, pneumonia, septicemia and skin infections.

Generally, a therapeutically effective amount of active component is in the range of 0.1 mg/kg to 100 mg/kg, *e.g.,* 1 mg/kg to 100 mg/kg, 1 mg/kg to 10 mg/kg. In an exemplary embodiment, a human recombinant polyclonal antibody may be administered at 1 mg/kg. The amount administered will depend on variables such as the type and extent of disease or indication to be treated, the overall health of the patient, the *in vivo* potency of the antibody, the pharmaceutical formulation, and the route of administration. The initial dosage can be increased beyond the upper level in order to rapidly achieve the desired blood-level or tissue-level. Alternatively, the initial dosage can be smaller than the optimum, and the dosage may be progressively increased during the course of treatment. Human dosage can be optimized, *e.g.,* in a conventional Phase I dose escalation study designed to run from 0.5 mg/kg to 20 mg/kg. Dosing frequency can vary, depending on factors such as route of administration, dosage amount and the disease being treated. Exemplary dosing frequencies are once per day, once every 2 days, once every three days, once every four days, once every five days, once every six days, once per week, once every two weeks, once every month, once every six months, and once a year. In some embodiments of the invention, dosing is once every two weeks. A preferred route of administration is parenteral, *e.g.,* intravenous or subcutaneous. Formulation of antibody-based drugs is within ordinary skill in the art. In some embodiments of the invention, the antibody is lyophilized and reconstituted in buffered saline at the time of administration.

For therapeutic use, one or more disclosed antibodies, or an antigen binding fragments thereof, can be combined with a pharmaceutically acceptable carrier. As used herein, "pharmaceutically acceptable carrier" means buffers, carriers, and excipients suitable for use in contact with the tissues of human beings and animals without excessive toxicity, irritation, allergic response, or other problem or complication, commensurate with a reasonable benefit/risk ratio. The carrier(s) should be "acceptable" in the sense of being compatible with the other ingredients of the formulations and not deleterious to the recipient. Pharmaceutically acceptable carriers include buffers, solvents, dispersion media, coatings, isotonic and absorption delaying agents, and the like, that are compatible with pharmaceutical administration. The use of such media and agents for pharmaceutically active substances is known in the art.

Pharmaceutical compositions containing one or more of the disclosed antibodies can be presented in a dosage unit form and can be prepared by any suitable method. A pharmaceutical composition should be formulated to be compatible with its intended route of administration. Examples of routes of administration are intravenous (IV), intradermal, inhalation, transdermal, topical, transmucosal, and rectal administration. An exemplary route of administration for monoclonal antibodies is IV infusion. Useful formulations can be prepared by methods well known in the pharmaceutical art. For example, see Remington's Pharmaceutical Sciences, 18th ed. (Mack Publishing Company, 1990). Formulation components suitable for parenteral administration include a sterile diluent such as water for injection, saline solution, fixed oils, polyethylene glycols, glycerine, propylene glycol or other synthetic solvents; antibacterial agents such as benzyl alcohol or methyl paraben; antioxidants such as ascorbic acid or sodium bisulfite; chelating agents such as EDTA; buffers such as acetates, citrates or phosphates; and agents for the adjustment of tonicity such as sodium chloride or dextrose.

For intravenous administration, suitable carriers include physiological saline, bacteriostatic water, Cremophor ELTM (BASF, Parsippany, NJ) or phosphate buffered saline (PBS). The carrier should be stable under the conditions of manufacture and storage, and should be preserved against microorganisms. The carrier can be a solvent or dispersion medium containing, for example, water, ethanol, polyol (for example, glycerol, propylene glycol, and liquid polyetheylene glycol), and suitable mixtures thereof.

Pharmaceutical formulations preferably are sterile. Sterilization can be accomplished, for example, by filtration through sterile filtration membranes. Where the composition is lyophilized, filter sterilization can be conducted prior to or following lyophilization and reconstitution.

### EXAMPLES

The following Examples are merely illustrative and are not intended to limit the scope or content of the invention in any way.

### Example 1: Sequence Analysis of Anti-S. aureus Antibodies

This example describes the sequence analysis of the anti-*S*. *aureus* antibodies disclosed herein.

Individual anti-*S*. *aureus* antibodies were isolated from human donor individuals (*e.g.,* healthy individuals) who had been exposed to *S. aureus* including the *S. aureus* strains methicillin sensitive *S. aureus* (MSSA), hospital-acquired-MRSA, and community-acquired-MRSA. The individual antibodies (*e.g.,* V_{H} and V_{L} regions) were sequenced by Sanger dideoxy-sequencing and were analyzed using IMGT/V-Quest software (Montpellier, France) to identify and confirm variable region sequences.

The nucleic acid sequences encoding and the protein sequences defining heavy and light chain variable regions of the anti-*S*. *aureus* antibodies are shown below (amino terminal signal peptide sequences are not shown). CDR sequences (IMGT definition) are indicated by bold font and underlining in the nucleic acid and amino acid sequences.

Nucleic Acid Sequence Encoding the 1.62.B9 Heavy Chain Variable Region (SEQ ID NO: 1)

GAGGTGCAGCTGGTGGAGTCTGGGGGAGGCTTGGTAAAGCCTGGGGGGTCCCTTAGACTCTCCTGT
GCAGCCTCTGGATTCACTTTCAGTAACGCCTGGATGACCTGGGTCCGCCAGGCTCCAGGGAAGGGG
CTGGAGTGGGTTGGCCGTATTAAGAGCAAAACTGATGGTGGGACAACAGACTACGCTGCACCCGTG
AAAGGCAGATTCACCATCTCAAGAGATGATTCAATAAACACACTGTATCTGCAAATGAACAGCCTG
AAAACCGAGGACACAGCCGTGTATTATTGTAGGACGGGGGGCTACTGGGGCCAGGGAACCCTGGTC
ACCGTCTCGAGTG

Protein Sequence Defining the 1.62.B9 Heavy Chain Variable Region (SEQ ID NO: 2)

EVQLVESGGGLVKPGGSLRLSCAASGFTFSNAWMTWVRQAPGKGLEWVGRIKSKTDGGTTDYAAPV
KGRFTISRDDSINTLYLQMNSLKTEDTAVYYCRTGGYWGQGTLVTVSS

Nucleic Acid Sequence Encoding the 1.62.B9 Lambda Chain Variable Region (SEQ ID NO: 3)

CAGGCTGTGGTGACTCAGGAGCCCTCACTGACTGTGTCCCCAGGAGGGACAGTCACTCTCACCTGT
GGCTCCAGCACTGGAGCTGTCACCAGTGGTCATTATCCCTACTGGTTCCAGCAGAAGCCTGGCCAA
GCCCCCAGGACACTGATTTATGATACAAGCAAGAAACATTCCTGGACACCTGCCCGGTTCTCAGGC
TCCCTCCTTGGGGGCAAAGCTGCCCTGACCCTTTCGGGTGCGCAGCCTGAGGATGAGGCTGAGTAT
TACTGCTTGGTCTCCGATAGTGGTGCTCGGATCTTCGGACCTGGGACCAAGGTCACCGTCCTAG

Protein Sequence Defining the 1.62.B9 Lambda Chain Variable Region (SEQ ID NO: 4)

QAVVTQEPSLTVSPGGTVTLTCGSSTGAVTSGHYPYWFQQKPGQAPRTLIYDTSKKHSWTPARFSG
SLLGGKAALTLSGAQPEDEAEYYCLVSDSGARIFGPGTKVTVL

Nucleic Acid Sequence Encoding the 5.11.H10 Heavy Chain Variable Region (SEQ ID NO: 10)

CAGGTTCAGCTGGTGCAGTCTGGACCTGAAATGAAGAAGCCTGGGGCCTCAGTGAAGGTCTCCTGT
CAGGCCTCTGGTTACACCTTTACCATTAATGGAATTAGTTGGGTGCGACAGGCCCCTGGACAACGG
CCTGAGTGGATGGGGTGGATCAGCACTAGTAATGGTCAGACAAACTTTGCACAGAATTTCCAGGGC
AGACTCACCTTGACCACAGACACATCCACGACCACGGCCTTCATGGAGTTGATGAGCCTCACATCT
GCCGACACGGCCGTATACTACTGTGCGAGAAATCCATACGGCAATTCGCGCAGATTCTTTGACTAC
TGGGGCCAGGGCACCCTGGTCACCGTCTCGAGTG

Protein Sequence Defining the 5.11.H10 Heavy Chain Variable Region (SEQ ID NO: 11)

QVQLVQSGPEMKKPGASVKVSCQASGYTFTINGISWVRQAPGQRPEWMGWISTSNGQTNFAQNFQG
RLTLTTDTSTTTAFMELMSLTSADTAVYYCARNPYGNSRRFFDYWGQGTLVTVSS

Nucleic Acid Sequence Encoding the 5.11.H10 Kappa Chain Variable Region (SEQ ID NO: 12)

GACATCCAGATGACCCAGTCTCCATCCTCCCTGTCTGCCTCTGTTGGCGACAGAGTCATCATCACT
TGCCGGACAAGTCAGACCATCACCAATTACATAAATTGGTATCAGCAAAAACCAGGGAAAGCCCCG
CAGCTCCTGATCTATGGTGTATCCAGTTTGCAAAGTGGGGTCCCATCAAGGTTCAGTGGCGGTGGA
TCTGGGACAGAATTCACTCTCACCATCAGCAGTCTGCAACCTGAAGACTTTGCAACTTATTACTGT
CAACAGAGTTATAGGGCCCCTCAAACATTCGGCCAAGGGACCAAGCTGGAAATCAAAC

Protein Sequence Defining the 5.11.H10 Kappa Chain Variable Region (SEQ ID NO: 13)

DIQMTQSPSSLSASVGDRVIITCRTSQTITNYINWYQQKPGKAPQLLIYGVSSLQSGVPSRFSGGG
SGTEFTLTISSLQPEDFATYYCQQSYRAPQTFGQGTKLEIK

Nucleic Acid Sequence Encoding the 5.27.A11 Heavy Chain Variable Region (SEQ ID NO: 19)

GAGGTGCAGCTGGTGGAGTCTGGGGGAGGCTTGGTGCAGCCGGGGGGGTCTCTGAGACTCTCTTGT
GCAGCCTCTGGTTTCGCCTTCAGCAGCCACGAGATGCACTGGGTTCGTCAAGGATAGGACACAGT
TTGGAGTGGGTCTCAGCTATTGGCCTTGCTGGTGACACATATTATTCAGACTCCGTGAAGGGCCGC
TTCACCATCTCCAGAGAAAACGCCAAGAACACTTTGTATCTTGAAATGAACAATTTGAGAGTCGGC
GACACGGCTGTCTATTACTGTGTCAAAGAAGGGGCTCATTGGAATTTGACTCCTGGGGCCAGGGA
ACCCTGGTCACCGTCTCGAGTG

Protein Sequence Defining the 5.27.A11 Heavy Chain Variable Region (SEQ ID NO: 20)

EVQLVESGGGLVQPGGSLRLSCAASGFAFSSHEMHWVRQGIGHSLEWVSAIGLAGDTYYSDSVKGR
FTISRENAKNTLYLEMNNLRVGDTAVYYCVKEGAHWEFDSWGQGTLVTVSS

Nucleic Acid Sequence Encoding the 5.27.A11 Lambda Chain Variable Region (SEQ ID NO: 21)

CAGTCTGTGCTGACGCAGCCGCCCTCAGTGTCTGGGGCCCCTGGACAGAGGGTCATCATCTCCTGC
ACTGGGAGCAGCTCCAACCTCGGGGCAAGTCTTGATGTACACTGGTACCAGCGCCTTCCAGGACA
GGCCCCCGACTCCTCATCTATGCTAACAACAATCGGCCCTCAGGGGTCCCTGACCGATTCTCTGGC
TCCAAGTCTGGCACCTCAGCCTCCCTGGCCATCACCGGGCTCCAGGCTGAAGATGAGGGTGATTAT
TACTGCCAGTCCTATGACGGCAGCCTGAGGGTGTTCGGCGGAGGGACCAGGCTGACCGTCCTAG

Protein Sequence Defining the 5.27.A11 Lambda Chain Variable Region (SEQ ID NO: 22)

QSVLTQPPSVSGAPGQRVIISCTGSSSNLGASLDVHWYQRLPGTGPRLLIYANNNRPSGVPDRFSG
SKSGTSASLAITGLQAEDEGDYYCQSYDGSLRVFGGGTRLTVL

Nucleic Acid Sequence Encoding the 5.6.H2 Heavy Chain Variable Region (SEQ ID NO: 28)

CAGGTGCAGCTGGTGGAGTCTGGGGGAGGCTTGTTCAAGCCTGGAGGGTCCCTGAGACTGTCCTGT
GCAGCCTCTGGATTCACCTTCAGTGACTACTACATGAGCTGGATCCGGCAGGCTCCAGGGAAGGGG
CTGGAGTGGATTTCATACATTAGTAGTAGTGGTAGGACCATAAAGTACGCAGACTCTGTGAAGGGC
CGATTCACCATCTCCAGGGACAACGCCAAGAACTCACTTTATCTGCAAATGAACAGCCTGAGAGCC
GAGGACACGGCCGTGTATTATTGTGCGAGAGATGGAGGATATTGTGGAAGTGCAAACTGCCTTCAT
GACGCTTTTGATATCTGGGGCCAAGGGACAATGGTCACCGTCTCGAGTG

Protein Sequence Defining the 5.6.H2 Heavy Chain Variable Region (SEQ ID NO: 29)

QVQLVESGGGLFKPGGSLRLSCAASGFTFSDYYMSWIRQAPGKGLEWISYISSSGRTIKYADSVKG
RFTISRDNAKNSLYLQMNSLRAEDTAVYYCARDGGYCGSANCLHDAFDIWGQGTMVTVSS

Nucleic Acid Sequence Encoding the 5.6.H2 Kappa Chain Variable Region (SEQ ID NO: 30)

GACATCCAGATGACCCAGTCTCCTTCCACCCTGTCTGCATCTGTAGGAGACAGAGTCACCATCACT
TGCCGGGCCAGTCAGAGTATTAGTAGCTGGTTGGCCTGGTATCAGCAGAAACCAGGGAAAGCCCCT
AAGCTCCTGATCTATAAGGCGTCTAGTTTAGAAAGTGGGGTCCCATCAAGGTTCAGCGGCAGTGGA
TCTGGGACAGAATTCACTCTCACCATCAGCAGCCTGCAGCCTGATGATTTTGCAACTTATTACTGC
CAACAGTATAATAGTTATTCGACGTTCGGCCAAGGGACCAAGGTGGAAATCAAAC

Protein Sequence Defining the 5.6.H2 Kappa Chain Variable Region (SEQ ID NO: 31)

DIQMTQSPSTLSASVGDRVTITCRASQSISSWLAWYQQKPGKAPKLLIYKASSLESGVPSRFSGSG
SGTEFTLTISSLQPDDFATYYCQQYNSYSTFGQGTKVEIK

Nucleic Acid Sequence Encoding the 5.6.H9 Heavy Chain Variable Region (SEQ ID NO: 37)

CAGGTCACCTTGAAGGAGTCTGGTCCCACGCTGGTGAAACCCACACAGACCCTCACGCTGACGTGC
ACCTTCTCTGGGTTTTCGCTCAGCACTAGTGGAGAGGGTGTGGCCTGGATCCGTCAGGCCCCAGGA
AAGGCCCTGGAGTGGCTTGCACTCATTTATTGGGATGATGATAAGCGCTACAACCCATCTCTGAAG
AGCAGGCTCACCATCACCAAGGACAACTCCAGAAACCAAGTGGTCCTTACAATGACCAACATGGAC
CCTGTGGACACAGCCACATATTACTGTGTACACAGGGGGATGAATAGGGTTTTTGGGGTAGTTTAT
AATTCGGGCTGGTTCGACCCCTGGGGCCAGGGAACCCTGGTCACCGTCTCGAGTG

Protein Sequence Defining the 5.6.H9 Heavy Chain Variable Region (SEQ ID NO: 38)

QVTLKESGPTLVKPTQTLTLTCTFSGFSLSTSGEGVAWIRQAPGKALEWLALIYWDDDKRYNPSLK
SRLTITKDNSRNQVVLTMTNMDPVDTATYYCVHRGMNRVFGVVYNSGWFDPWGQGTLVTVSS

Nucleic Acid Sequence Encoding the 5.6.H9 Kappa Chain Variable Region (SEQ ID NO: 39)

GAAATTGTGTTGACGCAGTCTCCAGGCACCCTGTCTTTGTCTCCAGGGGAAAGAGCCACCCTCTCC
TGTAGGGCCAGTCAGAGCGTTAGAAGCAGCCAGCTAGCCTGGTACCAGCAGAAGCCTGGCCAGGCT
CCCAGCCTCCTCATGTATGACGCATCTAAGAGGGCCACTGGCATCCCAGACAGGTTCAGTGGCAGT
GGGTCTGGGACAGACTTCACTCTCACCATCAGCAGACTGGAGCCTGAAGATTTTGTAGTGTATTTC
TGTCAGCAGTATGATACATCACCGTGGACGTTCGGCCAAGGGACCAAGGTGGAAATCAAAC

Protein Sequence Defining the 5.6.H9 Kappa Chain Variable Region (SEQ ID NO: 40)

EIVLTQSPGTLSLSPGERATLSCRASQSVRSSQLAWYQQKPGQAPSLLMYDASKRATGIPDRFSGS
GSGTDFTLTISRLEPEDFVVYFCQQYDTSPWTFGQGTKVEIK

Nucleic Acid Sequence Encoding the 5.17.F8 Heavy Chain Variable Region (SEQ ID NO: 46)

CAGGTGCAGCTACAGCAGTGGGGCGCAGGACTGTTGAAGCCTTCGGAGACCCTGTCCCTCAGTTGC
GCTGTCTCCGGTACGTCCTTCAGTGATTACTACTGGGCCTGGATCCGTCAGCCCCCAGGGAAGGGG
CTGGAGTGGATTGGGGAAATCAATCAGAGTGGACACACTAACGACAACCCGTCCTTCAAGAGTCGA
GTCACCATGTTAGTAGACAAGTCCAAGAACCAGTTCTCCCTGAGGATGACTTCTGTGACCGCCGCG
GACACGGCTGTATATTACTGTGCGAGATTTCCTTGGGTGGACCCGAGGGTTTTGACCACTGGGGC
CAGGGAACCCTGGTCACCGTCTCGAGTG

Protein Sequence Defining the 5.17.F8 Heavy Chain Variable Region (SEQ ID NO: 47)

QVQLQQWGAGLLKPSETLSLSCAVSGTSFSDYYWAWIRQPPGKGLEWIGEINQSGHTNDNPSFKSR
VTMLVDKSKNQFSLRMTSVTAADTAVYYCARFPWVGPEGFDHWGQGTLVTVSS

Nucleic Acid Sequence Encoding the 5.17.F8 Kappa Chain Variable Region (SEQ ID NO: 48)

GAAATTGTGCTGACTCAGTCTCCAGACTTTCAGTCTGGGACTCCAAAGGAGATGGTCACCATCACC
TGCCGGGCCAGTCAGACCATTGGTAACAGCTTACACTGGTACCAACAGAAACCAGGTCAGTCTCCA
AAGCTCCTCATCAAATATGGTTCCCAGTCCATGTCAGGGGTCCCCTCGCGGTTCAGTGGCAGTGGA

TTTGGGACAGATTTCACCCTGACCATCAACAGCCTGGAAGCTGAAGATGCTGCAGTGTATTACTGT
CTTCAGAGTGGTGGTTTTCCGTGGACGTTCGGCCAAGGGACCAAGGTGGAGATCAGACGAACTGTG
GCTGCACCATCTGTCTTCATCTTCCCGCCATCTGATGAGCAGTTGAAATCTG

Protein Sequence Defining the 5.17.F8 Kappa Chain Variable Region (SEQ ID NO: 49)

EIVLTQSPDFQSGTPKEMVTITCRASQTIGNSLHWYQQKPGQSPKLLIKYGSQSMSGVPSRFSGSG
FGTDFTLTINSLEAEDAAVYYCLQSGGFPWTFGQGTKVEIR

Nucleic Acid Sequence Encoding the 5.19.F12 Heavy Chain Variable Region (SEQ ID NO: 55)

CAGGTCCAGCTGGTACAGTCTGGGGCTGAGATGAAGAAGCCTGGGGCCTCAGTGAAGGTCTCCTGC
AAGGGTTCCGGATCCAGTCTCCCTTATCTTTCGATACAATGGGTGCGACAGGCCCCTGGAAAAGGC
CTTGAGTGGATGGGAGGCTTTGATCCTGAAGACGGTGAAACAATCTACAACGAGAAATTCGAGGGC
AGAATCACCATGGCCGAGGACACATCGACAGACACAGCCTACATGGAACTCAGCAGCCTCACATCT
GACGACACGGCCGTCTATTATTGCGCAACAAGCCTCATCCGTAACGGTCTTTATGAAGCGTTCGAA
CACTGGGGCCAGGGAACCCTGGTCACCGTCTCGAGTG

Protein Sequence Defining the 5.19.F12 Heavy Chain Variable Region (SEQ ID NO: 56)

QVQLVQSGAEMKKPGASVKVSCKGSGSSLPYLSIQWVRQAPGKGLEWMGGFDPEDGETIYNEKFEG
RITMAEDTSTDTAYMELSSLTSDDTAVYYCATSLIRNGLYEAFEHWGQGTLVTVSS

Nucleic Acid Sequence Encoding the 5.19.F12 Kappa Chain Variable Region (SEQ ID NO: 57)

GACATCCAGATGACCCAGTCTCCATCCTCCCTGTCTGCATCTGTAGGAGACAGAGTCACCATCACT
TGCCAGGCGAGTGATGACATTAAAAAGAATTTAAATTGGTATCAACAAAAACAGGGACAGCCCCT
AAGCTCCTGGTCTACGACGCATCCAATTTGGAGACAGGGGTCCCATCGAGGTTCCGTGGACGTGCA
TCTGGGACACAATATACTTTCACCATCACGAATCTGCAGCCTCAAGATTCTGGAATATATTACTGT
CAACAATATGACAATGTCCCGCTCAGTTTTGGCGGAGGGACCAAGGTGGAGGTCAGAC

Protein Sequence Defining the 5.19.F12 Kappa Chain Variable Region (SEQ ID NO: 58)

DIQMTQSPSSLSASVGDRVTITCQASDDIKKNLNWYQQKTGTAPKLLVYDASNLETGVPSRFRGRA
SGTQYTFTITNLQPQDSGIYYCQQYDNVPLSFGGGTKVEVR

Nucleic Acid Sequence Encoding the 5.23.C9 Heavy Chain Variable Region (SEQ ID NO: 64)

CAGGTGCAGCTGGTGCAGTCTGGGGCTGAGGTGAAGATGCCTGAGTCCTCGGTGAGGGTCTCCTGC
AGGATTTCTGGAGGCAACTTCAAAAATTATATTTTTAACTGGGTGCGACAGGCCCCCGGACAAGGG
CTTGAGTGGGTTGGAAGGATCAACCCAAAAGTTGGAAATGAAAATCACATACAGAGATATGGAAC
AGACTCACCCTCACCGCGGACGATTCCAGGAGAACAGTTTACATGGAGTTGAGCAGCCTGACAACA
GACGACACGGCCGTATATTATTGTGCGACATTACGTGTTATTGGTAACCGGAGACTACGACCGGTG
GGCGGAATTTTTGATATCTGGGGCCAAGGGACAATGGTCACCGTCTCGAGTG

Protein Sequence Defining the 5.23.C9 Heavy Chain Variable Region (SEQ ID NO: 65)

QVQLVQSGAEVKMPESSVRVSCRISGGNFKNYIFNWVRQAPGQGLEWVGRINPKVGNENHIQRYGN
RLTLTADDSRRTVYMELSSLTTDDTAVYYCATLRVIGNRRLRPVGGIFDIWGQGTMVTVSS

Nucleic Acid Sequence Encoding the 5.23.C9 Kappa Chain Variable Region (SEQ ID NO: 66)

GACATCCAGATGACCCAGTCTCCATCCTCCCTGTCTGCGTCTGTGGGAGACAGAGTCACCATCACT
TGCCAGGCGAGTCAGGACGTGAGTCACTATGTAAATTGGTATCAACAAAAACCAGGGAAAGCCCCA
AAGCCCTGGTCTACGAGGCATCCAATTTGGAAACAGGGGTCCCATCAAGATTCAGTGGAAGTGGA
TCTGGGACACATTTCACCTTCACCATCAACAGCCTGCAGCCTGAAGATATCGCGACTTATTTCTGT
CAACATGATGCAGAGCTGCCTCCCTCTTTCGGAGGAGGGACCAAGGTGGAGATCAAAC

Protein Sequence Defining the 5.23.C9 Kappa Chain Variable Region (SEQ ID NO: 67)

DIQMTQSPSSLSASVGDRVTITCQASQDVSHYVNWYQQKPGKAPKALVYEASNLETGVPSRFSGSG
SGTHFTFTINSLQPEDIATYFCQHDAELPPSFGGGTKVEIK

Nucleic Acid Sequence Encoding the 5.23.C12 Heavy Chain Variable Region (SEQ ID NO: 73)

CAGGTTCAGCTGGTGCAGTCTGGAGCTGAAGTGAAGAAGCCTGGGGCCTCAGTGAAGGTCTCCTGC
AAGTCTTCTGGTCACTCCTTTACCGCCTATGGCATCAGCTGGGTGCGACAGGCCCCTGGACAAGGG
CTTGAGTGGATGGGGTGGATCAGCACTAACAATGGCAACACAAACTATAGAGAGAAGTTCCAGGAC
AGACTCTCCTTGACCACGGACACATCCACGAGCACAGTCTACATGGAACTGAGGAGCCTGACATCT

GACGACACGGCCGTGTATTACTGTGCGAGAAATCCTTATGGCAATTCGCGGAGATTCTTAGACTAC
TGGGGCCAGGGAACCCTGGTCACCGTCTCGAGTG

Protein Sequence Defining the 5.23.C12 Heavy Chain Variable Region (SEQ ID NO: 74)

QVQLVQSGAEVKKPGASVKVSCKSSGHSFTAYGISWVRQAPGQGLEWMGWISTNNGNTNYREKFQD
RLSLTTDTSTSTVYMELRSLTSDDTAVYYCARNPYGNSRRFLDYWGQGTLVTVSS

Nucleic Acid Sequence Encoding the 5.23.C12 Kappa Chain Variable Region (SEQ ID NO: 75)

GACATCCAGATGACCCAGTCTCCATCCTCCCTGTCTGCATCTGTCGGAGACAGAGTCATCATCACT
TGCCGGGCAAGTCAGAACATTAGCCCCTATTTAAATTGGTATCAGCAGAAGCCAGGGAGAGCCCCT
GAGCTCCTGATCTATGGTACGACCACTGTACAAACTGGGGTCCCGTCACGGTTCAGTGGCATTGGA
TCTGGGACAGATTTCACTCTCACCATCAGCAGTCTGCAACCTGAAGATTTTGCAACTTATTACTGT
CAACAGAGCTACAGGGCCCCTCAAACGTTCGGCCAAGGGACCAAGGTGGAAGTCAAGC

Protein Sequence Defining the 5.23.C12 Kappa Chain Variable Region (SEQ ID NO: 76)

DIQMTQSPSSLSASVGDRVIITCRASQNISPYLNWYQQKPGRAPELLIYGTTTVQTGVPSRFSGIG
SGTDFTLTISSLQPEDFATYYCQQSYRAPQTFGQGTKVEVK

Nucleic Acid Sequence Encoding the 8.51. G11Heavy Chain Variable Region (SEQ ID NO: 82)

CAGGTCACCTTGAAGGAGTCTGGTCCTCCGCTGGTGAATCCCACAGAGACCCTCACGGTGACCTGC
AATTTCCCTGGCTTCTCATTCACCTCTAGTGGAGAGGGTGTGGGCTGGATCCGTCAGCCCCCAGGA
AAGGCCCTGGAATGGCTTGCTCACATTTATTGGGATGGCGATAAGCGGTTCAACACATCTCTGAAA
AACAGACTCAACATCACCAAGGACACCTCTAAGAATCAGGTGGTCCTCACTTTGACCAACGTGGCC
CCTGTGGACACAGCCACGTATTTCTGTGTCCACCTGACACGCCCATCCTTTTGGAGTCCCTATTAC
CTTTTTGACCTCTGGGGCCAGGGAACCCTGGTCACCGTCTCGAGTG

Protein Sequence Defining the 8.51. G11Heavy Chain Variable Region (SEQ ID NO: 83)

QVTLKESGPPLVNPTETLTVTCNFPGFSFTSSGEGVGWIRQPPGKALEWLAHIYWDGDKRFNTSLK
NRLNITKDTSKNQVVLTLTNVAPVDTATYFCVHLTRPSFWSPYYLFDLWGQGTLVTVSS

Nucleic Acid Sequence Encoding the 8.51. G11Kappa Chain Variable Region (SEQ ID NO: 84)

GACATCCAGTTGACCCAGTCTCCGCCCTCCCTGTCTGCATCTGTCGGAGACAGTGTCACCATAACT
TGCCGGGCAAGTCAATATATTAGTACGTATTTAAATTGGTATCAGCAGAAACCGGGAAAGCCCCC
AGACTCTTGATCTATGGTGCATCCAATTTGCACAGTGGGGTCTCATCAAGGTTCAGTGGCAGTAGA
TCTGGGACAGATTTCACTCTCACCATAACCAGTCTACAGCCTGAAGATTTTGCGACTTACTACTGC
CAACAGAGTTCCACTTTGTGGTCGTTCGGCCAAGGGACCAAGGTGGAGATGCACC

Protein Sequence Defining the 8.51. G11Kappa Chain Variable Region (SEQ ID NO: 85)

DIQLTQSPPSLSASVGDSVTITCRASQYISTYLNWYQQKPGKAPRLLIYGASNLHSGVSSRFSGSR
SGTDFTLTITSLQPEDFATYYCQQSSTLWSFGQGTKVEMH

Nucleic Acid Sequence Encoding the 9.51.H9 Heavy Chain Variable Region (SEQ ID NO: 91)

CAGGTTCAGCTGGTGCAGTCTGGAGGTGAAATGAGGAAACCTGGGGCCTCATTGAAGGTCTCCTGC
AAGGCCTCTAATTACACCTTCACCAGTTTTGGTCTGACCTGGCTGCGACAGGCCCCTGGCAAGGC
CCTGAGTGGCTGGGCTGGATCAGTCCTTATAATGGAAACACCAACTATGCACAGAACTTCCGCGAC
AGACTCACCATGACCACAGACACGTCCACGAGCACAGCCTACATGGAGCTGAAGAGCCTGACATCT
GACGACACGGCCGTCTATTACTGTGCCAGAACTAACTGGGGGAATTGGTACCTCGATCTCTGGGGC
CCTGGCACCCTGGTCACTGTCTCGAGTG

Protein Sequence Defining the 9.51.H9 Heavy Chain Variable Region (SEQ ID NO: 92)

QVQLVQSGGEMRKPGASLKVSCKASNYTFTSFGLTWLRQAPGQGPEWLGWISPYNGNTNYAQNFRD
RLTMTTDTSTSTAYMELKSLTSDDTAVYYCARTNWGNWYLDLWGPGTLVTVSS

Nucleic Acid Sequence Encoding the 9.51.H9 Kappa Chain Variable Region (SEQ ID NO: 93)

GACATCGTGATGACCCAGTCTCCAGACTCCCTGGCTGTGGCTCTGGGCGAGCGGGCCACCATCAGC
TGCAAGTCCAGCCAGACTATCTTGTTCGGCTCCAACAACAAGAACTACTTACAGTGGTTTCAGCAG
AAACCCGGACAGCCTCCTAAGTTACTCATTTACTGGGCATCTACCCGGGAATCCGGGGTCCCTGAC
CGATTCAGTGGCGACGGGTCTGGGACAGACTTCACTCTCTCCATCGACAACCTACAGGCTGAAGAT
GTGGCACTTTATTATTGCCAGCAATATTTTAGTAATCCGTATACTTTCGGCCAGGGGACCAAATTG
GAGATCAAAC

Protein Sequence Defining the 9.51.H9 Kappa Chain Variable Region (SEQ ID NO: 94)

DIVMTQSPDSLAVALGERATISCKSSQTILFGSNNKNYLQWFQQKPGQPPKLLIYWASTRESGVPD
RFSGDGSGTDFTLSIDNLQAEDVALYYCQQYFSNPYTFGQGTKLEIK

Nucleic Acid Sequence Encoding the 18.43.D8 Heavy Chain Variable Region (SEQ ID NO: 100)

CAGGTCCAGCTTGTGCAGTCTGGGGCTGAGGTGAAGAAGCCTGGGGCCTCAGTGAAAATTGCCTGC
AAGACCTCTGGATACATCTTCGTTGACTATGCTGTACAATGGGTGCGCCAGGCCCCCGGACAGAGG
CTTGAGTGGATGGGGTGGCTCCAGTCAGCCAGTGGCAAGACGAGCTCGTCACATAGTCTCCAAGAC
AGAGTCTCCATTACCCGGGACACATCCGCGAACACAGCCTACTTGGAACTGAGGAGTCTGACTTCT
GAAGATACGGCTGTCTACTACTGTGCGAGAGGGAGTATAAGTCATTACGCCCCCTTTGACTACTGG
GGCCAGGGAACCCTGGTCACCGTCTCGAGTG

Protein Sequence Defining the 18.43.D8 Heavy Chain Variable Region (SEQ ID NO: 101)

QVQLVQSGAEVKKPGASVKIACKTSGYIFVDYAVQWVRQAPGQRLEWMGWLQSASGKTSSSHSLQD
RVSITRDTSANTAYLELRSLTSEDTAVYYCARGSISHYAPFDYWGQGTLVTVSS

Nucleic Acid Sequence Encoding the 18.43.D8 Kappa Chain Variable Region (SEQ ID NO: 102)

GACATCGTGATGACCCAGTCTCCAGACTCCCTGGCTGTGTCTCTGGGCGAGAGGGCCACCATCGAC
TGCAAGTCCAGTCAGAATATTTTATATACTTCCAACAATAAGAGTTTCTTAGCTTGGTACCAACAG
AAACCAGGACAGCCTCCTAAATTGCTCATTTACTGGGCGTCTACCCGAGAGTTCGGGGTCCCTGCG
CGATTCAGTGGCAGCGGGTCTGGGACAGATTTCACTCTCACCATCAACAACCTGCAGGGTGAAGAT

GTGGCCATTTATTACTGTCAACAGTATTTTTCTACTCCATACACTTTTGGCCAGGGGACCAAGCTG
GAGATCAAAC

Protein Sequence Defining the 18.43.D8 Kappa Chain Variable Region (SEQ ID NO: 103)

DIVMTQSPDSLAVSLGERATIDCKSSQNILYTSNNKSFLAWYQQKPGQPPKLLIYWASTREFGVPA
RFSGSGSGTDFTLTINNLQGEDVAIYYCQQYFSTPYTFGQGTKLEIK

Nucleic Acid Sequence Encoding the 22.22.E7 Heavy Chain Variable Region (SEQ ID NO: 109)

CAGGTGCAGCTGCAGGAGTCGGGCCCAGGACTGGTGAAGCCCTCGGAGACCCTGTCCCTCACGTGC
ACTGTCTCTGGTGCCTACATCACTAATGACTACTGGAGCTGGATCCGGCAGCCCGCCGGGAAGGGA
CTGGCGTGGATTGGGCGTCTCTATACCAGTGGGAGCACCAACTACAACCCCTCCCTCAAGAGTCGA
GTCACCATGTCAGTAGACACGTCCAAAAACCAGTTCTCCCTGAGGCTCACCTCTGTGACCGCCGCG
GACACGGCCGTTTATTATTGTGCGAGAGATGAGTGTAGTGGTGGTGGGTGCTTCCATGAGAACTGG
GGCCAGGGAACCCTGGTCACCGTCTCGAGTG

Protein Sequence Defining the 22.22.E7 Heavy Chain Variable Region (SEQ ID NO: 110)

QVQLQESGPGLVKPSETLSLTCTVSGAYITNDYWSWIRQPAGKGLAWIGRLYTSGSTNYNPSLKSR
VTMSVDTSKNQFSLRLTSVTAADTAVYYCARDECSGGGCFHENWGQGTLVTVSS

Nucleic Acid Sequence Encoding the 22.22.E7 Kappa Chain Variable Region (SEQ ID NO: 111)

GAAATTGTGTTGACGCAGTCTCCAGGCACCCTGTCTTTGTCTCCAGGGGAAAGAGCCACCCTCTCC
TGCAGGGCCAGCCAGAGTATTAGCAGCAGCTACTTGGCCTGGTACCAGCAGAAGCGTGGCCAGGCT
CCCAGGCTCCTCATCTATGGTGCATCCAGTAGGGCCACTGGCATCCCAGACAGGTTCAGTGGCAGT
GGGACTGGGACAGACTTCACTCTCACCATCAGCAGACTGGAGCCTGACGATTTTGCACTTTATTAC
TGTCAGCAGTATGATAGGTCACCGCTCACTTTCGGCGGAGGGACCAAGGTGGAGATCAAAC

Protein Sequence Defining the 22.22.E7 Kappa Chain Variable Region (SEQ ID NO: 112)

EIVLTQSPGTLSLSPGERATLSCRASQSISSSYLAWYQQKRGQAPRLLIYGASSRATGIPDRFSGS
GTGTDFTLTISRLEPDDFALYYCQQYDRSPLTFGGGTKVEIK

Nucleic Acid Sequence Encoding the 8.51.G10 Heavy Chain Variable Region (SEQ ID NO: 148)

CAGATCACCTTGAAGGAGTCTGGTCCTACGCTGGTGAACCCACACAGACCCTCACGCTGACCTGC
ACCTTTTCTGGGTTCTCATTCACCTCTAGTGGAGAGGCTGTGGGCTGGATCCGTCAGCCCCCAGGA
AAGGCCCTGGAATGGCTTGCTCACATTTATTGGGATGATGATCCGCGTTATAGTACATCTCTGAAG
AGCAGACTCACCATCACCAAGGACACCTCCAAAAATCAGGTGGTCCTCTCTATGACCGACATTGAC
CCTGCGGACACAGGCACATATTACTGTGCACACCTGAGACGCCCATCCCTTTGGAGTCCTTATTAT
CTTTTTGACTGCTGGGGCCAGGGAACCCTGGTCACCGTCTCGAGTG

Protein Sequence Defining the 8.51.G10 Heavy Chain Variable Region (SEQ ID NO: 149)

QITLKESGPTLVNPTQTLTLTCTFSGFSFTSSGEAVGWIRQPPGKALEWLAHIYWDDDPRYSTSLK
SRLTITKDTSKNQVVLSMTDIDPADTGTYYCAHLRRPSLWSPYYLFDCWGQGTLVTVSS

Nucleic Acid Sequence Encoding the 8.51.G10 Kappa Chain Variable Region (SEQ ID NO: 150)

GACATCCAGATGACCCAGTCTCCATCCTCCCTGTCTGCATCTGTTGGAGACAGAGTCACCATCACT
TGCCGGGCAAGTCAGAGTATTAGTAGCTATTTAAATTGGTATCAACTGAAACCAGGACAAGCCCCT
AAGCTCCTGATCTATGGTGCATCCAGTTTGGAAAATGGGGTCCCTTCACGGTTCAGTGGCAGTGGC
TCTGGGCCAGATTTCACTCTCACCATTAGCAGTCTGCAACCTGAAGATTTTGCAACTTACTACTGT
CAACAGAGTTACAAAATGTGGTCATTCGGCCAAGGGACCAAGGTGGAAATCCAAC

Protein Sequence Defining the 8.51.G10 Kappa Chain Variable Region (SEQ ID NO: 151)

DIQMTQSPSSLSASVGDRVTITCRASQSISSYLNWYQLKPGQAPKLLIYGASSLENGVPSRFSGSG
SGPDFTLTISSLQPEDFATYYCQQSYKMWSFGQGTKVEIQ

Nucleic Acid Sequence Encoding the 5.24A.A7 Heavy Chain Variable Region (SEQ ID NO: 152)

CAGGTTCAGCTGGTGCAGTCTGGAGTTGAGGTGAAGAAGCCTGGGGCCTCAGTGAAGGTC
TCCTGCAAGGCTTCTGGCTACACCTTTACCAACTTTGGCTTCAGTTGGGTGCGGCAGGCC
CCAGGACAAGGGCTTGAGTGGCTGGGATGGATCAACACTCATAACGGTAACACAAACTAT
GCACCGAGATTCCAAGACAGAGTCACCCTGACCACAGACACATCCACGACCACAGCCTAC
ATGGAGCTGAGGAGCCTGAGATCTGAGGACACGGCCGTGTATTATTGTGCGAGAGATCTT
GGGGGAAGTAGTTCCGCCTACTGGGGACAGGGAACCCTGGTCACCGTCTCGAGTG

Protein Sequence Defining the 5.24A.A7 Heavy Chain Variable Region (SEQ ID NO: 153)

QVQLVQSGVEVKKPGASVKVSCKASGYTFTNFGFSWVRQAPGQGLEWLGWINTHNGNTNYAPRFQD
RVTLTTDTSTTTAYMELRSLRSEDTAVYYCARDLGGSSSAYWGQGTLVTVSS

Nucleic Acid Sequence Encoding the 5.24A.A7 Kappa Chain Variable Region (SEQ ID NO: 154)

GAAATTGTGTTGACGCAGTCTCCAGGCACCCTGTCTTTGTCTCCAGGGGAGAGAGCCACC
CTCTCTTGCAGGGCCAGTCACAGTGGTACCAACAACTACATCGCCTGGTACCAGCAGAAA
CCTGGCCAGCCTCCCAGGCTCCTCATCTATGGTGCATCCAGCAGGGCCCCTGGCATCTCA
GAGAGGTTCAGCGGCAGTGGGTCTGGGACAGACTTCACTCTCATCATCAGCAGACTGGAG
CCTGAAGACTTTGGAGTATATTACTGTGAGCAGTATGGGAACTCACCTCTGACGTTCGGC
CAAGGGACCAAGGTGGAAATCAAAC

Protein Sequence Defining the 5.24A.A7 Kappa Chain Variable Region (SEQ ID NO: 155)

EIVLTQSPGTLSLSPGERATLSCRASHSGTNNYIAWYQQKPGQPPRLLIYGASSRAPGISERFSGS
GSGTDFTLIISRLEPEDFGVYYCEQYGNSPLTFGQGTKVEIK

Nucleic Acid Sequence Encoding the 5.24A.F3 Heavy Chain Variable Region (SEQ ID NO: 156)

CAGGTGCAGCTGCAGGAGTCGGGCCCAGGACTGGTGGAGTCTTCGGACGTCCTGTCCCTC
ACCTGCACTGTCTCTGGTGGCTCCATCAGTAATGACTATTGGGCCTGGATCCGGCAGACC
CCAGGGAAGGGACTGGAGTGGATTGGGTCTGTCTATTACAGTGGCAGAACCAGTTTCAGC
CCCTCCCTCAAGAGTCGAGTCACTATGTCAGTGGACACGACCAAGAACCAATTCTTCCTG

AACCTGGGCTCTCTGACCGTTGCGGACACGGCCGTGTATTACTGTGCGAGAACTACAAGG
TTGTCCTTTGACTGGTTAGTACACTACTATGGAATGGACGTCTGGGGCCAGGGGACCACG
GTCACCGTCTCGAGTG

Protein Sequence Defining the 5.24A.F3 Heavy Chain Variable Region (SEQ ID NO: 157)

QVQLQESGPGLVESSDVLSLTCTVSGGSISNDYWAWIRQTPGKGLEWIGSVYYSGRTSFSPSLKSR
VTMSVDTTKNQFFLNLGSLTVADTAVYYCARTTRLSFDWLVHYYGMDVWGQGTTVTVSS

Nucleic Acid Sequence Encoding the 5.24A.F3 Kappa Chain Variable Region (SEQ ID NO: 158)

GACATCCAGATGACCCAGTCTCCATCGTCCCTGACTGCATCTGTAGGAGACAGAGTCACC
ATTATTTGTCGGGCAAGTCAGCCCATTGGCAGCTATTTGAATTGGTATCAGCAGAGTCCA
GGGAAAGCCCCTAAACTCCTCATCTATGCTGCATCCACTTTGCAGAGTGGGGTCCCATCA
AGGTTCAGTGGCAGTGGATCTGGGACAGATTTCACTCTCAACATCAGTGGTCTGCAACCT
GAAGATTTTGCAACTTACTACTGTCAACAGAGTTACAATAACCCTTTGTTCACCTTCGGC
CCTGGGACCAAAGTGGATATCAAACGAACTGTGGCTGCACCATCTGTCTTCATCTTCCCG
CCATCTGATGAGCAGTTGAAATCTG

Protein Sequence Defining the 5.24A.F3 Kappa Chain Variable Region (SEQ ID NO: 159)

DIQMTQSPSSLTASVGDRVTIICRASQPIGSYLNWYQQSPGKAPKLLIYAASTLQSGVPSRFSGSG
SGTDFTLNISGLQPEDFATYYCQQSYNNPLFTFGPGTKVDIK

Nucleic Acid Sequence Encoding the 5.8B.H4 Heavy Chain Variable Region (SEQ ID NO: 160)

GAGGTGCAGCTGGTGGAGTCTGGGGGAAGTCTGGTACGGCCGGGGGGGTCCCTGCGACTC
TCCTGTGCAGCCTCTGGATTCATCTTTGATGATTATGGCATGAGTTGGGTCCGCCAAGTT
CCAGGGGAGGGCCTGGAGTGGGTCGCCGGCATTACTTGGGATGGCGTGAACACAGATTAT
GCAGACTCCGTGAAGGGCCGCTTCACCATCTCCAGAGACAACGCCAAGAACTCCCTGTTT
CTCCTAATGAACAGTCTCAGAGCCGAGGACACGGCCCTGTATCACTGTGCGAGAGCCAGA
TACTACCAAGGCACTGGCCTCCGGTCCTACCTCGACGAGTGGGGCCAGGGAACCCTGGTC
ACCGTCTCGAGTG

Protein Sequence Defining the 5.8B.H4 Heavy Chain Variable Region (SEQ ID NO: 161)

EVQLVESGGSLVRPGGSLRLSCAASGFIFDDYGMSWVRQVPGEGLEWVAGITWDGVNTDYADSVKG
RFTISRDNAKNSLFLLMNSLRAEDTALYHCARARYYQGTGLRSYLDEWGQGTLVTVSS

Nucleic Acid Sequence Encoding the 5.8B.H4 Kappa Chain Variable Region (SEQ ID NO: 162)

GACATCCAGATGACCCAGTCTCCATCCTCCGTGTCTGCATCTGTAGGAGAAAGAGTCACT
ATCACTTGTCGGGCGACTCAGGGTATTGACAACTGGTTGGCCTGGTATCAGCATAAACCA
GGGAAAGCCCCGAAGCTCCTGATTTATGCTGCATCCACTTTGCAAAGTGGGGTCCCGTCC
AGGTTCAGCGGCAGTGGATCTGGGACAGATTTCACTCTCACCATCAGCAGCGTGCAGCCT
GAAGATGTTGCAACTTACTTTTGTCAACAGGCTAAGAATTTCCCTCGGGGAGGATTCACT
TTCGGCCCTGGGACCAAAGTCGCTCTCAAACGAACTGTGGCTGCACCATCTGTCTTCATC
TTCCCGCCATCTGATGAGCAGTT

Protein Sequence Defining the 5.8B.H4 Kappa Chain Variable Region (SEQ ID NO: 163)

DIQMTQSPSSVSASVGERVTITCRATQGIDNWLAWYQHKPGKAPKLLIYAASTLQSGVPSRFSGSG
SGTDFTLTISSVQPEDVATYFCQQAKNFPRGGFTFGPGTKVALK

Nucleic Acid Sequence Encoding the 26.51.E1 Heavy Chain Variable Region (SEQ ID NO: 164)

CAGGTGCAGCTGGTGGAGTCTGGGGGAGGCGTGGTCCAGCCTGGGAGGTCCCTGAGACTC
TCCTGTGCAGCCTCTGGATTCACCTTCAGTAAATTTGCCATGCACTGGGTCCGCCAGGCT
CCAGGCAAGGGGCTGGAGTGGGTGGCAGCTATAGCGTCTGATGGATATAATAAATACTAT
GCAGACTCCGTGAAGGGCCAATTCACCGTCTCCAGAGACACTTCCAGGAACACGCTGTAT
CTCCAAATGGACAGCCTGAGACCTGATGACACGGCTATTTATTACTGTGCGTACGGTCAC
TTGTCTTTTTGGTTCGACCCCTGGGGCCAGGGAACCCTGGTCACCGTCTCGAGTG

Protein Sequence Defining the 26.51.E1 Heavy Chain Variable Region (SEQ ID NO: 165)

QVQLVESGGGVVQPGRSLRLSCAASGFTFSKFAMHWVRQAPGKGLEWVAAIASDGYNKYYADSVKG
QFTVSRDTSRNTLYLQMDSLRPDDTAIYYCAYGHLSFWFDPWGQGTLVTVSS

Nucleic Acid Sequence Encoding the 26.51.E1 Kappa Chain Variable Region (SEQ ID NO: 166)

GACATCCAGATGACCCAGTCTCCATCCTCCCTGTCCGCATCTGTAGGAGACAGAGTCACCA
TCACTTGCCGGGCAAGTCAGGACATTGGAAATGGCTTAGGCTGGTATCAACAGATACCAGG
GAAAGCCCCTAAGCGCCTGATCTATGCTGCATCCTCTTTGCAAAGTGGGGTCCCATCAAGG
TTCAGCGGCAGTGGTTCTGGGACAGAGTTCACTCTCACAATCCACAGCCTGCAGCCTGAAG
ATTTTGCAACTTATTACTGTCTACAGCATAATTTTTACCCTCGCACTTTTGGCCAGGGGAC
CAAGCTGGAGATCAAGCGAACTGTGGCTGCACCATCTGTCTTCATCTTCCCGCCATCTGAT
GAGCAGTTGAAATCTG

Protein Sequence Defining the 26.51.E1 Kappa Chain Variable Region (SEQ ID NO: 167)

DIQMTQSPSSLSASVGDRVTITCRASQDIGNGLGWYQQIPGKAPKRLIYAASSLQSGVPSRFSGSG
SGTEFTLTIHSLQPEDFATYYCLQHNFYPRTFGQGTKLEIK

Nucleic Acid Sequence Encoding the 22.21.A7 Heavy Chain Variable Region (SEQ ID NO: 168)

CAGGTGCAGCTGGTGCAGTCTGGGGCTGAGGTGAAGAAGCCTGGGGCCTCAGTGAAGGTC
TCCTGCAAGGCTTCTGGATACACCTTCACCGACTACTATATACACTGGGTGCGACAGGCC
CCTGGACAAGGGCTTGAGTGGATGGGATGGATCAACCCTTATAGTGGTGGCACAAACTAT
GCACCGAAGTTTCAGGGCAGGTTCACCATGGCCAGGGACACGTCCATCACCACAACCTAC
TTGGACCTGGGCAGGCTGACGTCTAGCGACACGGCCATTTATTTCTGTGCGAGAGATTTC
TTCAGAGCAGCAGCTGGCACGGCCGGAGACTACTGGGGCCAGGGAACCCTGGTCACCGTC
TCGAGTG

Protein Sequence Defining the 22.21.A7 Heavy Chain Variable Region (SEQ ID NO: 169)

QVQLVQSGAEVKKPGASVKVSCKASGYTFTDYYIHWVRQAPGQGLEWMGWINPYSGGTNYAPKFQG
RFTMARDTSITTTYLDLGRLTSSDTAIYFCARDFFRAAAGTAGDYWGQGTLVTVSS

Nucleic Acid Sequence Encoding the 22.21.A7 Kappa Chain Variable Region (SEQ ID NO: 170)

GACATCGTGATGACCCAGTCTCCAGAAGACTCCCTGGCTGTGTCTCTGGGCGAGAGGGCC
ACCATCAACTGCTCCAGCCAGAGTGCTTTATACAGCCCCAGCAGTAAGACCTACTTAGCT

TGGTACCAGCAGAGACCGGACACCCTCCTAAGCTTCTCATTTACTGGGCATCTACCCGG
GTATCCGGGGTCCCTGACCGATTCAGTGGCAGCGGGTCTGGGACAGATTTCACTCTCACC
ATCGGCAGCCTGCAGGCTGAAGATGTGGCAGTTTATTACTGTCAGTACTATTATGGTACT
GTCACTTTCGGCGGAGGGACCAAGGTGGAGATCAAACGAACTGTGGCTGCACCATCTGTC
TTCATCTTCCCGCCATCTGATGAGCAGTTGAAATCTG

Protein Sequence Defining the 22.21.A7 Kappa Chain Variable Region (SEQ ID NO: 171)

ADIVMTQSPDSLAVSLGERATINCSSQSALYSPSSKTYLAWYQQRPGHPPKLLIYWASTRVSGVPD
RFSGSGSGTDFTLTIGSLQAEDVAVYYCQYYYGTVTFGGGTKVEIK

Nucleic Acid Sequence Encoding the 22.18A.E9 Heavy Chain Variable Region (SEQ ID NO: 172)

CAGGTGCAGCTGCAGGAGTCGGGCCCAAGACTGGTGAAGCCTTCGGAGACCCTGTCCCTC
ACCTGCACTGTCTCTGGTGGGTCCATCAATAGTTATTATTGGAGTTGGATCCGGCAGCCC
GTCGGGAAGGGACTGGAGTGGATTGGGCGTATGTATCCCGGTGGTACCTCCAGTTACAAT
TCATCCCTCAAGAGTCGAGTCATCATGTCGGTAGACACGTCGAAGAACCAGTTTTCTCTG
AACCTCACTTCTGTGACCGCCGCAGACACGGCCGTATATTTCTGTGCGAGAGATCCAACA
TATCACCATGGCACTAGTGGTTATGTGGGGACTTTTGATCTCTGGGGCCAAGGGACAATG
GTCACCGTCTCGAGTG

Protein Sequence Defining the 22.18A.E9 Heavy Chain Variable Region (SEQ ID NO: 173)

QVQLQESGPRLVKPSETLSLTCTVSGGSINSYYWSWIRQPVGKGLEWIGRMYPGGTSSYNSSLKSR
VIMSVDTSKNQFSLNLTSVTAADTAVYFCARDPTYHHGTSGYVGTFDLWGQGTMVTVSS

Nucleic Acid Sequence Encoding the 22.18A.E9 Kappa Chain Variable Region (SEQ ID NO: 174)

GAAATAGTGATGACGCAGTCTCCAGCCACCCTGTCTGCGTCTCCAGGGGAAAGAGTCACC
CTCTCCTGCAGGGCCAGTCCGAGTGTTCGCAACAATTTAGCCTGGTACCAGCAGAAACCT
GGCCAGCCTCCTAGGCTCCTCATCTATGGTGCATCCACCAAGGTCATGGGTACTCCAACC
AGGTTCAGTGGCAGTGGGTCTGGGACAGAGTTCACTCTCACCATCAGCAGCCTGCAGTCT
GAAGATTTTGCAGTTTATTACTGTCAGCAGTATAATAGCTGGCCTCCGTACACTTTTAGC
CAGGGGACCAAACTGGAGATCAAACGAACTGTGGCTGCACCATCTGTCTTCATCTTCCCG
CCATCTGATGAGCAGTTGAAATCTG

Protein Sequence Defining the 22.18A.E9 Kappa Chain Variable Region (SEQ ID NO: 175)

DIVMTQSPATLSASPGERVTLSCRASPSVRNNLAWYQQKPGQPPRLLIYGASTKVMGTPTRFSGSGSGTEFTLTISSLQ
SEDFAVYYCQQYNSWPPYTFSQGTKLEIK

Nucleic Acid Sequence Encoding the 5.52.H10 Heavy Chain Variable Region (SEQ ID NO: 176) 
GAGGTGCAGCTGGTGGAGTCGGGGGGAGGCCTGGTCAGGCCTGGGGAGTCCCTGAGACTC
TCCTGTGCAGGCTCTGGATTCACCTTCAGCATGTTTACCCTGAACTGGGTCCGCCAGGCT
CCAGGGAAGGGACTGGAGTGGGTCGCATCTATTAGTGGTAGTAGCAGTTTAGTGTTTCAT
GAAGACTCAGTGAAGGGCCGATTCACCATCTCCAGAGACAACGCCAAGAACTCAGTATAT
CTGCAAATGAACAGCCTGAGAGCCGAAGACACGGCTATTTATTACTGTGTGAGAGGAGAT
AGTCGCGACTATTGGGGCCAGGGAACCCTGGTCACCGTCTCGAGTG
 Protein Sequence Defining the 5.52.H10 Heavy Chain Variable Region (SEQ ID NO: 177)

EVQLVESGGGLVRPGESLRLSCAGSGFTFSMFTLNWVRQAPGKGLEWVASISGSSSLVFHEDSVKG
RFTISRDNAKNSVYLQMNSLRAEDTAIYYCVRGDSRDYWGQGTLVTVSS

Nucleic Acid Sequence Encoding the 5.52.H10 Kappa Chain Variable Region (SEQ ID NO: 178)

CAGTCTGTGTTGACGCAGCCGCCCTCAGTGTCTGCGGCCCCAGGACAGAAGGTCACCATC
TCCTGCTCTGGAAGGACCTCCAATATTGGAAATAACCATGTATCCTGGTACCAGCAATTC
CCAGGAACAGCCCCCAAAGTCCTCATTTATCGCAATGATAGGCGACCCTCAGGGATTCCT
GATCGATTCTCTGGCTCCAAGTCTGACACGTCAGCCACCCTGGGCATCACCGGGCTCCAG
ACTGGGGACGAGGCCGATTATTACTGCGGAACATGGGACAACAGCCTGAGTGGTTGGGTG
TTCGGCGGAGGGACCAAGCTGACCGTCCTAG

Protein Sequence Defining the 5.52.H10 Kappa Chain Variable Region (SEQ ID NO: 179)

QSVLTQPPSVSAAPGQKVTISCSGRTSNIGNNHVSWYQQFPGTAPKVLIYRNDRRPSGIPDRFSGS
KSDTSATLGITGLQTGDEADYYCGTWDNSLSGWVFGGGTKLTVL

Nucleic Acid Sequence Encoding the 5.15.C1 Heavy Chain Variable Region (SEQ ID NO: 180)

CAGGTGCAGCTGGTGCAGTCTGGGGCTGAGGTGAAGAGGCCTGGGGCCTCAGTGAAGGTCTCCTGT
GAGACTTCTGGATATAGCTTCACCACTCATGATTTGAACTGGGTGCGACAGGCCGCTGGACAGGGG
CTTGAGTGGATGGGATGGATGAACCCTAACACTGGTGACACATCCTACGCACAGAGGTTCCAGGGC
AGAGTCACCATGATCAGGGACGTCTCCATAAGTACAGCCTACTTGGAATTGACAGGCCTGACATTT
GATGACACGGCCGTCTACTTCTGTGCGAGGTCGAATTCTGCACCTGTTTCAACTCTTTTACCCGCT
GATGCTTTTGATGTCTGGGGCCAAGGGACAATGGTCACCGTCTCGAGTG

Protein Sequence Defining the 5.15.C1 Heavy Chain Variable Region (SEQ ID NO: 181)

QVQLVQSGAEVKRPGASVKVSCETSGYSFTTDLNWVRQAAGQGLEWMGWMNPNTGDTSYAQRFQGR
VTMIRDVSISTAYLELTGLTFDDTAVYFCARSNSAPVSTLLPADAFDVWGQGTMVTVSS

Nucleic Acid Sequence Encoding the 5.15.C1 Kappa Chain Variable Region (SEQ ID NO: 182)

GACATCCAGATGACCCAGTCTCCATCCTCCCTGTCTGCATCTATAGGAGACACAGTCACCATCACT
TGCCGGGCAAGTCAGATCATTAACAGCTATTTAAATTGGTATCAGCAAAAACCAGGGAAGCCCCT
AAACTCCTGATCTATGCTGTATCCAATTTGCACCGTGGGGTCCCCTCAAGGTTCAGTGGCAGTGGG
TCTGCGGCAGATTTCACTCTCACTATCAGTGGTCTGCAACCTGAAGATTTTGCAACTTACTACTGT
CAACAGACTTACACTACCGTTGCGATCACCTTCGGCCAAGGGACACGACTGGACATTAAAC

Protein Sequence Defining the 5.15.C1 Kappa Chain Variable Region (SEQ ID NO: 183)

DIQMTQSPSSLSASIGDTVTITCRASQIINSYLNWYQQKPGEAPKLLIYAVSNLHRGVPSRFSGSG
SAADFTLTISGLQPEDFATYYCQQTYTTVAITFGQGTRLDIK

Nucleic Acid Sequence Encoding the 5.54.E6 Heavy Chain Variable Region (SEQ ID NO: 184)

CAGGTGCAGCTGGTGCAGTCTGGGGCGGAGGTGAAGAAGCCTGGGTCCTCGGTGAACGTCTCCTGT
AAGGCCTCCGCAGGGACCCTCAGCGGTTATACCATCAACTGGTTGCGCCAGGCCCCTGGACGAGGG
TTTGAGTGGCTGGGAAGGATCATCCCTCACCTTGGAATAGGAAACAACGCACATAACTGGGAGGCC
AGAGTCACCCTTACCGCCGACAAGTCCACGGACACTACCTACCTGGAGTTGCGCGACCTGAGATCT

GACGACGTGGCCATTTATTACTGCGCGGTGGAGTTTTACCGGCTGGCAACGGTCACAACCCCAACC
CTTGACTTCTGGGGCCAGGGAACCCTGGTCACCGTCTCGAGTG

Protein Sequence Defining the 5.54.E6 Heavy Chain Variable Region (SEQ ID NO: 185)

QVQLVQSGAEVKKPGSSVNVSCKASAGTLSGYTINWLRQAPGRGFEWLGRIIPLGIGNNANWEARV
TLTADKSTDTTYLELRDLRSDDVAIYYCAVEFYRLATVTTPTLDFWGQGTLVTVSS

Nucleic Acid Sequence Encoding the 5.54.E6 Lambda Chain Variable Region (SEQ ID NO: 186)

TCCTATGTGCTGACTCAGCCACCCTCGGTGTCCGTGGCCCCAGGAACGACGGCCAGGATTACCTGT
GAGGGGGACCACATTGGAGCTAAAAGTGTGCACTGGTACCAGAGGAAGCCAGGCCAGGCCCCTGTG
TTGGTCGTCTATATGAATAGTGACCGGCCCTCAGGGATCCCTGAGCGATTGTCTGGCTCCAACTCT
GGGAACACGGCCACCCTGAGTATCAGCAGGGTCGAGGCCGAGGATGAGGCCGACTATTATTGTCAG
GTTTGGGATAGTTTTCGCGATCATCAGGTATTCGGCGGAGGGACCAAGTTGACCGTCCTGA

Protein Sequence Defining the 5.54.E6 Lambda Chain Variable Region (SEQ ID NO: 187)

SYVLTQPPSVSVAPGTTARITCEGDHIGAKSVHWYQRKPGQAPVLVVYMNSDRPSGIPERLSGSNS
GNTATLSISRVEAEDEADYYCQVWDSFRDHQVFGGGTKLTVL

Nucleic Acid Sequence Encoding the 5.55.D2Heavy Chain Variable Region (SEQ ID NO: 188)

CAGGTCACCTTGAAGGAGTCTGGTCCTGCTATAGTGAAACCCACACAGACCCTCACGCTGACCTGC
ACCTTCTCTGGGTTCTCACTCCGAACTAGTGGAGTGGGTGTGGGCTGGATCCGTCAGCCCCCAGGA
AAGGCCCTTGAGTGGCTTGCACTCATTTACTGGGATGATGATAAGCGCTACAACCCATCTCTGAAG
ACCAGACTCACCATCACCAAGGACTCCTCCAGAAACCAGGTGTTCCTCACAATGACCAACATGGAC
CCTGTGGACACAGCCACATACTTCTGTGCACACCGACAACATATTACAAACAGTTTTGGATTGGTT
ATTGCGAATGATGCTTTTGACATTTGGGGCCAAGGGACAATGGTCACCGTCTCGAGTG

Protein Sequence Defining the 5.55.D2 Heavy Chain Variable Region (SEQ ID NO: 189)

QVTLKESGPAIVKPTQTLTLTCTFSGFSLRTSGVGVGWIRQPPGKALEWLALIYWDDDKRYNPSLK
TRLTITKDSSRNQVFLTMTNMDPVDTATYFCARQITNSFGLVIANDAFDIWGQGTMVTVSS

Nucleic Acid Sequence Encoding the 5.55.D2Lambda Chain Variable Region (SEQ ID NO: 190)

CAGTCTGCCCTGACTCAGCCTCCGTCCGCGTCCGGGTCTCCTGGACAGTCAGTCACCATCTCCTGC
ACTGGAACCAGCAGTGACGTTGGTCGTTATAATTATGTCGCCTGGTATCAACAACACCCAGGCAGA
GCCCCCAGACTCATAATTTATGGGGTCACTAAGCGGCCCTCAGGGGTCCCTGATCGCTTCTCTGGC
TCCAAGTCTGGCAACACGGCCTCCCTGACCGTCTCTGGGCTCCAGGCTGAAGATGAGGCTGATTAT
TATTGCAGTTCATATGCAGGCAGCAACTTTTTGGAGGTGTTCGGCGGAGGGACCAAGCTGACCGTC
CTAG

Protein Sequence Defining the 5.55.D2Lambda Chain Variable Region (SEQ ID NO: 191)

QSALTQPPSASGSPGQSVTISCTGTSSDVGRYNYVAWYQQHPGRAPRLIIYGVTKRPSGVPDRFSG
SKSGNTASLTVSGLQAEDEADYYCSSYAGSNFLEVFGGGTKLTVL

Nucleic Acid Sequence Encoding the 22.14.A1 Heavy Chain Variable Region (SEQ ID NO: 192)

CAGGTGCAGCTGGTGCAGTCTGGGGCTGAGGTGAAGAAGCCTGGGTCCTCGGTGAAGGTCTCCTGC
AAGGCTTCTGGAGGCACCTTCAGCAGCTATGCAATCAACTGGGTGCGACAGGCCCCTGGACAAGGT
CTTGAGTGGATGGGAGGGATCATCCCTATGTTTGATACAGCAATCTACGCACAGATGTTCCAGGGC
AGAGTCACCATCACCGCGGACGAATCCACGAGCACAGCCTACATGGAGCTGAGCAGCCTGAGATCT
GACGACACGGCCGTATATTACTGTGCGAGAGATCGAAACGATTATTATGACAGTAGTGGTTATTCA
GGCGCCTTTGACCAGTGGGGCCAGGGAACCCTGGTCACCGTCTCGAGTG

Protein Sequence Defining the 22.14.A1 Heavy Chain Variable Region (SEQ ID NO: 193)

QVQLVQSGAEVKKPGSSVKVSCKASGGTFSSYAINWVRQAPGQGLEWMGGIIPMFDTAIYAQMFQG
RVTITADESTSTAYMELSSLRSDDTAVYYCARDRNDYYDSSGYSGAFDQWGQGTLVTVSS

Nucleic Acid Sequence Encoding the 22.14.A1 Kappa Chain Variable Region (SEQ ID NO: 194)

GAAATTGTGTTGACGCAGTCTCCAGGCGCCCTGTCTTTGTCTCCAGGGGAAAGAGCCACCCTCTCC
TGCAGGGCCAGTCAGAGTGTTAGCAGCAGCTACTTAGCCTGGTACCAGCAGAAACCTGGCCAGGCT
CCCAGGCTCCTCATCTATGGTGCATCCAGCAGGGCCACTGGCATCCCAGACAGGTTCAGTGGCAGT
GGGTCTGGGACAGACTTCACTCTCACAATCAGCAGACTGGAGCCTGAGGATTTTGCAGTGTATTAC
TGTCAGCAGTATGGTAGCTCACCTCGCACTTTTGGCCAGGGGACCAAGCTGGAGATCAAAC

Protein Sequence Defining the 22.14.A1 Kappa Chain Variable Region (SEQ ID NO: 195)

EIVLTQSPGALSLSPGERATLSCRASQSVSSSYLAWYQQKPGQAPRLLIYGASSRATGIPDRFSGS
GSGTDFTLTISRLEPEDFAVYYCQQYGSSPRTFGQGTKLEIK

Nucleic Acid Sequence Encoding the 26.53.B4 Heavy Chain Variable Region (SEQ ID NO: 196)

GAGGTGCAGCTGGTGGAGTCCGGGGGAGGCTTGGTCCAGCCTGGGGGGTCCCTGAAACTCTCCTGT
GCAGCCTCTGGGTTCAGCTTCAGTGGCTCTGCTGTGGAATGGGTCCGCCAGGCTTCCGGGAGAGGG
CTGGAGTGGATTGGCCATATAAGAACCAAGACCAAAAATTACGCGACAGCGTATGCTGCGTCGGTG
CAAGGCAGGTTCACCATCTTCAGAGATGATTCAAGGAACACGGCGTATCTGCAGATGAACAGTCTT
AAAACCGAGGACACGGCCGTGTATTATTGTTCCAGACACATCGAATTTAGCAGTTCGTCCGGGCCC
CAGATGGGCCAGGGAACCCTGGTCACCGTCTCGAGTG

Protein Sequence Defining the 26.53.B4 Heavy Chain Variable Region (SEQ ID NO: 197)

EVQLVESGGGLVQPGGSLKLSCAASGFSFSGSAVEWVRQASGRGLEWIGIRTKTKNYATAYAASVQ
GRFTIFRDDSRNTAYLQMNSLKTEDTAVYYCSRIEFSSSSGPQMGQGTLVTVSS

Nucleic Acid Sequence Encoding the 26.53.B4 Kappa Chain Variable Region (SEQ ID NO: 198)

GACATCCAGATGACCCAGTCTCCTTCCACCCTGTCTGCATCTGTAGGCGACACAGTCACCATCACT
TGCCAGGCCAGTCAGAATATTATTACTTGGTTGGCCTGGTATCAGCAGAAACCAGGGAAAGCCCCT
AACATCCTCATCTATAAGGCGTCTGGTTTAGAGAGTGGGGTCCCACCGAGGTTCAGCGGCAGTGGG
TCGGGACAGAATTCACTCTCACCATCAGCAGCCTGCAGCCTGATGATGTTGCAACTTATTATTGC
CTACAATATAAAACTGAGCCGTGGACGTTCGGCCTAGGGACCAAGGTGGAGATCAGAC

Protein Sequence Defining the 26.53.B4 Kappa Chain Variable Region (SEQ ID NO: 199)

DIQMTQSPSTLSASVGDTVTITCQASQNIITWLAWYQQKPGKAPNILIYKASGLESGVPPRFSGSG
SGTEFTLTISSLQPDDVATYYCLQYKTEPWTFGLGTKVEIR

Nucleic Acid Sequence Encoding the 5.63.E2 Heavy Chain Variable Region (SEQ ID NO: 200)

GAGGTGCAGCTGGTGGAGTCTGGGGGAGGCTTGGCCCAGCCGGGGGAGTCCCTGAGACTCTCCTGT
GCAGCCTCCGGATTCACCTTTAGTGCCTATTGGATGAGCTGGGTCCGCCAGACTCCAGGGAAGGGG
CTGGAGTGGGTGGCCAACATTAACCAGGGTGGAGATAAGACAGACTATTTGGACTCTGTGAAGGGC
CGATTCACCGTCTCCAGAGACAATGCCAAGAACTTAGTGTTTCTGCAAATGGCCAGCCTGAGAGCC
GACGACACGGCCGTGTATTACTGTGGAGAGGTACAAACCAAGACTACTGGGGCCAGGGAACCCTG
GTCACCGTCTCGAGTG

Protein Sequence Defining the 5.63.E2 Heavy Chain Variable Region (SEQ ID NO: 201)

EVQLVESGGGLAQPGESLRLSCAASGFTFSAYWMSWVRQTPGKGLEWVANINQGGDKTDYLDSVKG
RFTVSRDNAKNLVFLQMASLRADDTAVYYCGRGTNQDYWGQGTLVTVSS

Nucleic Acid Sequence Encoding the 5.63.E2 Lambda Chain Variable Region (SEQ ID NO: 202)

CAGTCTGTGCTGACTCAGCCACCCTCAGCGTCTGGGACCCCCGGGCAGAGGGTCACCATCTCTTGT
TCTGGAAGCAGCTCCAATATCGGAAGTAATAATGTATACTGGTACCAACAGGTCCCAGGAACGGCC
CCCAAATTTCTCATCTATCCGAATAGTGAGCGGCCCTCAGGGGTCCCTGACCGATTCTCTGCCTCC
AAGTCTGGCACCTCAGCCTCCCTGGCCATCAGTGGACTCCGGTCCGAGGATGAGGCTACTTATTAC
TGTGCAGCATGGGATGACAGCCTAAGAACTTATGTCTTCGCATCTGGGACCAAGGTCAC

Protein Sequence Defining the 5.63.E2 Lambda Chain Variable Region (SEQ ID NO: 203)

QSVLTQPPSASGTPGQRVTISCSGSSSNIGSNNVYWYQQVPGTAPKFLIYPNSERPSGVPDRFSAS
KSGTSASLAISGLRSEDEATYYCAAWDDSLRTYVFASGTKVTVL

Nucleic Acid Sequence Encoding the 5.64.G4 Heavy Chain Variable Region (SEQ ID NO: 204)

CAGGTGCAGCTGGTGGAGTCTGGGGGAGGCGTGGTCCAGCCTGGGAGGTCCCTGAGACTCTCCTGT
GCAGCCTCCGGATTCACCCTCAGGAGTTATGGCATGCACTGGGTCCGCCAGGCTCCAGGCAAGGGC
CTGGAGTGGGTGGCAGTTATATCATATGATGGAAGTAAGACATATTATAGAGACTCCGTGAAGGGC
CGATTCACCATCTCCAGAGACAATTCTAAGAACACACTGTATTTGCAAATGAACAGCCTGCGAGTT
GAGGACACGGCTGTATATTATTGTGCGAAAGAGGATAATTTGGGATCTGATGTTTTTGATAGATGG
GGCCAAGGGACAATGGTCACCGTCTCGAGTG

Protein Sequence Defining the 5.64.G4 Heavy Chain Variable Region (SEQ ID NO: 205)

QVQLVESGGGVVQPGRSLRLSCAASGFTLRSYGMHWVRQAPGKGLEWVAVISYDGSKTYYRDSVKG
RFTISRDNSKNTLYLQMNSLRVEDTAVYYCAKEDNLGSDVFDRWGQGTMVTVSS

Nucleic Acid Sequence Encoding the 5.64.G4 Kappa Chain Variable Region (SEQ ID NO: 206)

GACATCGTGATGACCCAGTCTCCTTCCACCCTGTCTGCATCTGTAGGAGACAGAGTTACCATCACT
TGTCGGGCCAGTCAGAGTGCCAGTCAGTGGTTGGCCTGGTATCAGCAAAAACCAGGGAAAGCCCCT
AATCTCCTAATCTATAAGGCGTCTAATTTACATAGTGGGGTCCCATCAAGGTTCAGCGGCAGTGGA
TCTGGGACCGAATTCACTCTCACCATCAGCAGCCTACAGCCTGATGATTTTGCAACTTATTACTGC
CAACAATACAACATTTATCCGTGGACGTTCGGCCAAGGGACCAAGGTGGAAGTGAGAC

Protein Sequence Defining the 5.64.G4 Chain Variable Region (SEQ ID NO: 207)

DIVMTQSPSTLSASVGDRVTITCRASQSASQWLAWYQQKPGKAPNLLIYKASNLHSGVPSRFSGSG
SGTEFTLTISSLQPDDFATYYCQQYNIYPWTFGQGTKVEVR

Nucleic Acid Sequence Encoding the 43.52.A11 Heavy Chain Variable Region (SEQ ID NO: 208)

CAGGTCCAGCTGGTGCAGTCTGGGGCTGAGGTGAAGGAGCCAGGGTCCTCGGTGAGGGTCTCCTGC
AAGGCTTCTGGACGCATCTTCCGGAATTATGCTGTCAGCTGGGTGCGACAGGCCCCTGGACAAGGG
CTTGAGTGGGTGGGCCGGTTCATCCCTTCGGAAGGAATAACAAACTACATTCCGACATTCCGGGAC
AGACTCACCTTTACTGCGGACAAGTCCACGAACACAGCCTTCATTGACCTGACTAACCTGAAATAT
GAGGACACGGCCATATATTACTGTGTGGGTGGCAGGCCAGGCGCTTTCGTCCCCTTTGACTCCTGG
GGCCAGGGAACCCTGGTCACCGTCTCGAGTG

Protein Sequence Defining the 43.52.A11 Heavy Chain Variable Region (SEQ ID NO: 209)

QVQLVQSGAEVKEPGSSVRVSCKASGRIFRNYAVSWVRQAPGQGLEWVGRFIPSEGITNYIPTFRD
RLTFTADKSTNTAFIDLTNLKYEDTAIYYCVGGRPGAFVPFDSWGQGTLVTVSS

Nucleic Acid Sequence Encoding the 43.52.A11 Kappa Chain Variable Region (SEQ ID NO: 210)

GAAATAGTGATGACGCAGTCTCCTGCCACCCTGTCTGTGTCTCCAGGGGAAAGAGCCACCCTCTCC
TGCAGGGCCAGTCAGAGTGTCAGGGGCAACTTAGCCTGGTACCAGCAAAAACCTGGCCAGGCTCCC
AGGCTTCTCATGTATGATGCTTCTACCAGGGCCACTAGTTTCCCAGCCCGGTTCAGTGGCAGTGGG
TCTGGGACAGAGTTCATCTCACCATCAGCAGTGTGCAGTCTGAAGATTTTGCACTTTATTACTGTC
AGCAGTATAATGACTGGCCCCGGACTTTTGGCCAGGGGACCAAGGTGGAGATCAAAC

Protein Sequence Defining the 43.52.A11 Chain Variable Region (SEQ ID NO: 211)

EIVMTQSPATLSVSPGERATLSCRASQSVRGNLAWYQQKPGQAPRLLMYDASTRATSFPARFSGSG
SGTEFTLTISSVQSEDFALYYCQQYNDWPRTFGQGTKVEIK

Nucleic Acid Sequence Encoding the 43.52.E12 Heavy Chain Variable Region (SEQ ID NO: 212)

CAGGTGCAGCTGGTGCAGTCTGGGGCTGAGGTGAAGAAGCCTGGGTCCTCGGTGAGGGTCTCCTGC
AGGGTTTCTGGAGGCAGTTTCTCCAGTTATGACGTACACTGGGTGCGACAGGCCCCTGGACAAGGG
CTTGAGTGGGTGGGAAGGAGCGTCTCACGTGCTGACAGCTCAAGTCACACACGGGAGTTCGGGGAC
AGAGTCACGATTATCGCGGACAAATCCACGAGTACAGTTTATTTGACACTGACCAGTCTGACCTCT
GACGACACGGCCATCTATTACTGTGCGGCAGGGGATTGTAGTGATGGCGGGTGCTTCCGCACACCC
CTGCACATCTGGGGCCAGGGCACCCTGGTCACCGTCTCGAGTG

Protein Sequence Defining the 43.52.E12 Heavy Chain Variable Region (SEQ ID NO: 213)

QVQLVQSGAEVKKPGSSVRVSCRVSGGSFSSYDVHWVRQAPGQGLEWVGRSVSRADSSSHTREFGD
RVTIIADKSTSTVYLTLTSLTSDDTAIYYCAAGDCSDGGCFRTPLHIWGQGTLVTVSS

Nucleic Acid Sequence Encoding the 43.52.E12 Kappa Chain Variable Region (SEQ ID NO: 214)

GAAATAGTGATGACGCAGTCTCCAGCCACTCTGTCTCTGTCTCCAGGGGAAAGAGCCACCCTCTCC
TGCAGGGCCAGTCAGAGTCTGAGAACCAACTTAGCCTGGTACCAGCAGCGACCTGGCCAGGCTCCC
AGGCTCCTCATCTCTGGGGCATCCACGAGGGCCACTGGTATCCCAGCCAGGTTCAGTGGCAGTGGG

TCTGGGACAGAATTCACTCTCACCATTAGGAGCCTGCAGCCTGAAGATTTTGCAGTTTATTACTGT
CAACAGTATCATGACTGGCCTCGGACGTTCGGCCAAGGGACCAAGGTGGATTTCAAAC

Protein Sequence Defining the 43.52.E12 Kappa Chain Variable Region (SEQ ID NO: 215)

EIVMTQSPATLSLSPGERATLSCRASQSLRTNLAWYQQRPGQAPRLLISGASTRATGIPARFSGSG
SGTEFTLTIRSLQPEDFAVYYCQQYHDWPRTFGQGTKVDFK

Nucleic Acid Sequence Encoding the 43.62.E2 Heavy Chain Variable Region (SEQ ID NO: 216)

GAGGTGCAGCTGGTGCAGTCTGGGGCAGAAGTGAAAAAGGCCGGGGAGTCTCTGAGGATCTCCTGT
AAGGGTTCTGGATACAACTTTAATAGCTATTGGATTGGCTGGGTGCGCCAGATGCCCGGGAAAGGC
CTGGAGTGGATGGCAATCATCTATCCTGCTGACTCTGACAGGAGATATGGCCCGTCCTTCCAAGGC
CAGGTCACCATCTCAGTCGACAAGTCTATTAACACCGCGTACTTGCAGTGGAGCAGCCTGAAGGCC
TCGGACACCGCCATGTATTATTGTGCCAGACTCCTCAGAGTTAGTACTGGTTGGGAAGATGCTTTT
GATCTGTGGGGCCAAGGGACAATGGTCACCGTCTCGAGTG

Protein Sequence Defining the 43.62.E2 Heavy Chain Variable Region (SEQ ID NO: 217)

EVQLVQSGAEVKKAGESLRISCKGSGYNFNSYWIGWVRQMPGKGLEWMAIIYPADSDRRYGPSFQG
QVTISVDKSINTAYLQWSSLKASDTAMYYCARLLRVSTGWEDAFDLWGQGTMVTVSS

Nucleic Acid Sequence Encoding the 43.62.E2 Kappa Chain Variable Region (SEQ ID NO: 218)

GACATCGTGATGACCCAGTCTCCAGACTCCCTGGTTGTGTCTCTGGGCGAGAGGGCCACCATCAAG
TGCCAGTCCAGCCAGAGTGTTTTGTACAGCTCCACCAATAAGAACTATTTAGCCTGGTACCAGCAG
AAATCGGACAGCCACCTAAGCTACTCATTTACTGGGCCTCCACCCGGGAATCCGGGGTCCCTGAC
CGATTCATTGGTAGCGGCTCTAGGACAGATTTCACTCTCACCATCAGCGACCTGCAGCCTGAGGAT
GCGGCAGTTTATTACTGTCAGCAGTATTATAGTGATTATCTCACTTTCGGCGGAGGGACCAAGGTG
GAGATCAAAC

Protein Sequence Defining the 43.62.E2 Kappa Chain Variable Region (SEQ ID NO: 219)

DIVMTQSPDSLVVSLGERATIKCQSSQSVLYSSTNKNYLAWYQQKSGQPPKLLIYWASTRESGVPD
RFIGSGSRTDFTLTISDLQPEDAAVYYCQQYYSDYLTFGGGTKVEIK

Table 2 is a concordance table showing the SEQ ID NO. of the heavy chain and light chain variable region amino acid sequences as a fully human cognate pairs for each anti-*S*. *aureus* antibody described herein.

**Table 2**

| Antibody | Heavy Chain Variable Region (SEQ ID NO:) | Light Chain Variable Region (SEQ ID NO:) |
|---|---|---|
| 1.62.B9 | 2 | 4 |
| 5.11.H10 | 11 | 13 |
| 5.27.A11 | 20 | 22 |
| 5.6.H2 | 29 | 31 |
| 5.6.H9 | 38 | 40 |
| 5.17.F8 | 47 | 49 |
| 5.19.F12 | 56 | 58 |
| 5.23.C9 | 65 | 67 |
| 5.23.C12 | 74 | 76 |
| 8.51.G11 | 83 | 85 |
| 9.51.H9 | 92 | 94 |
| 18.43.D8 | 101 | 103 |
| 22.22.E7 | 110 | 112 |
| 8.51.G10 | 149 | 151 |
| 5.24A.A7 | 153 | 155 |
| 5.24A.F3 | 157 | 159 |
| 5.8B.H4 | 161 | 163 |
| 26.51.E1 | 165 | 167 |
| 22.21.A7 | 169 | 171 |
| 22.18A.E9 | 173 | 175 |
| 5.52.H10 | 177 | 179 |
| 5.15.C1 | 181 | 183 |
| 5.54.E6 | 185 | 187 |
| 5.55.D2 | 189 | 191 |
| 22.14.A1 | 193 | 195 |
| 26.53.B4 | 197 | 199 |
| 5.63.E2 | 201 | 203 |
| 5.64.G4 | 205 | 207 |
| 43.52.A11 | 209 | 211 |
| 43.52.E12 | 213 | 215 |
| 43.62.E2 | 217 | 219 |

Table 3 summarizes the heavy chain and light chain CDR sequences (IMGT definition) of the disclosed *S. aureus* antibodies.

**Table 3**

| | Heavy Chain | | | Light Chain | | |
|---|---|---|---|---|---|---|
| Antibody | CDR1 | CDR2 | CDR3 | CDR1 | CDR2 | CDR3 |
| 1.62.B9 | GFTFSNAW (SEQ ID NO: 5) | IKSKTDGGTT (SEQ ID NO: 6) | CRTGGYW (SEQ ID NO: 7) | TGAVTSGHY (SEQ ID NO: 8) | DTS | CLVSDSGARIF (SEQ ID NO: 9) |
| 5.11.H10 | GYTFTING (SEQ ID NO: 14) | ISTSNGQT (SEQ ID NO: 15) | CARNPYGNSRRFFDYW (SEQ ID NO: 16) | QTITNY (SEQ ID NO: 17) | GVS | CQQSYRAPQTF (SEQ ID NO: 18) |
| 5.27.A11 | GFAFSSHE (SEQ ID NO: 23) | IGLAGDT (SEQ ID NO: 24) | CVKEGAHWEFDSW (SEQ ID NO: 25) | SSNLGASLD (SEQ ID NO: 26) | ANN | CQSYDGSLRVF (SEQ ID NO: 27) |
| 5.6.H2 | GFTFSDYY (SEQ ID NO: 32) | ISYISSSGRTI (SEQ ID NO: 33) | CARDGGYCGSANCLHDAFDIW (SEQ ID NO: 34) | QSISSW (SEQ ID NO: 35) | KAS | CQQYNSYSTF (SEQ ID NO: 36) |
| 5.6.H9 | GFSLSTSGEG (SEQ ID NO: 41) | IYWDDDK (SEQ ID NO: 42) | CVHRGMNRVFGVVYNSGWFDPW (SEQ ID NO: 43) | QSVRSSQ (SEQ ID NO: 44) | DAS | CQQYDTSPWTF (SEQ ID NO: 45) |
| 5.17.F8 | GTSFSDYY (SEQ ID NO: 50) | INQSGHT (SEQ ID NO: 51) | CARFPWVGPEGFDHW (SEQ ID NO: 52) | QTIGNS (SEQ ID NO: 53) | YGS | CLQSGGFPWTF (SEQ ID NO: 54) |
| 5.19.F12 | GSSLPYLS (SEQ ID NO: 59) | FDPEDGET (SEQ ID NO: 60) | CATSLIRNGL YEAFEHW (SEQ ID NO: 61) | DDIKKN (SEQ ID NO: 62) | DAS | CQQYDNVPLSF (SEQ ID NO: 63) |
| 5.23.C9 | GGNFKNYI (SEQ ID NO: 68) | INPKVGNE (SEQ ID NO: 69) | CATLRVIGNRRLRPVGGIFDIW (SEQ ID NO: 70) | QDVSHY (SEQ ID NO: 71) | EAS | CQHDAELPPSF (SEQ ID NO: 72) |
| 5.23.C12 | GHSFTAYG (SEQ ID NO: 77) | ISTNNGNT (SEQ ID NO: 78) | CARNPYGNSRRFLDYW (SEQ ID NO: 79) | QNISPY (SEQ ID NO: 80) | GTT | CQQSYRAPQTF (SEQ ID NO: 81) |
| 8.51.G11 | GFSFTSSGEG (SEQ ID NO: 86) | IYWDGDK (SEQ ID NO: 87) | CVHLTRPSFWSPYYLFDLW (SEQ ID NO: 88) | QYISTYL (SEQ ID NO: 89) | GAS | CQQSSTLWSF (SEQ ID NO: 90) |
| 9.51.H9 | NYTFTSFG (SEQ ID NO: 95) | ISPYNGNT (SEQ ID NO: 96) | CARTNWGNWYLDLW (SEQ ID NO: 97) | QTILFGSNNKNY (SEQ ID NO: 98) | WAS | CQQYFSNPYTF (SEQ ID NO: 99) |
| 18.43.D8 | GYIFVDYA (SEQ ID NO: 104) | LQSASGKT (SEQ ID NO: 105) | CARGSISHYAPFDYW (SEQ ID NO: 106) | QNILYTSNNKSF (SEQ ID NO: 107) | WAS | CQQYFSTPYTF (SEQ ID NO: 108) |
| 22.22.E7 | GAYITNDY (SEQ ID NO: 113) | LYTSGST (SEQ ID NO: 114) | CARDECSGGGCFHENW (SEQ ID NO: 115) | QSISSSY (SEQ ID NO: 116) | GAS | CQQYDRSPLTF (SEQ ID NO: 117) |
| 8.51.G10 | GFSFTSSGEA (SEQ ID NO: 220) | IYWDDDP (SEQ ID NO: 221) | CAHLRRPSLWSPYYLFDCW (SEQ ID NO: 222) | QSISS (SEQ ID NO: 223) | GAS | CQQSYKMWSF (SEQ ID NO: 224) |

| HEAVY CHAIN | | | | LIGHT CHAIN | | |
|---|---|---|---|---|---|---|
| Antibody | CDR1 | CDR2 | CDR3 | CDR1 | CDR2 | CDR3 |
| 5.24A.A7 | GYTFTNFG (SEQ ID NO: 225) | INTHNGNT (SEQ ID NO: 226) | CARDFFRAAAGTAGDYW (SEQ ID NO: 227) | HSGTNNY (SEQ ID NO: 228) | GAS | CQQSYKMWSF (SEQ ID NO: 229) |
| 5.24A.F3 | GGSISNDY (SEQ ID NO: 230) | VYYSGRT (SEQ ID NO: 231) | CARTTRLSFDWLVHYYGMDVW (SEQ ID NO: 232) | QPIGSY (SEQ ID NO: 233) | AAS | CQQSYNNPLFTF (SEQ ID NO: 234) |
| 5.8B.H4 | GFIFDDYG (SEQ ID NO: 235) | ITWDGVNT (SEQ ID NO: 236) | CARARYYQGTGLRSYLDEW (SEQ ID NO: 237) | QGIDNW (SEQ ID NO: 238) | AAS | CQQAKNFPRGGFTF (SEQ ID NO: 239) |
| 26.51.E1 | GFTFSKFA (SEQ ID NO: 240) | IASDGYNK (SEQ ID NO: 241) | CAYGHLSFWFDPW (SEQ ID NO: 242) | QDIGNG (SEQ ID NO:243) | AAS | CLQHNFYPRTF (SEQ ID NO: 244) |
| 22.21.A7 | GYTFTDYY (SEQ ID NO: 245) | INPYSGGT (SEQ ID NO: 246) | CARDFFRAAAGTAGDYW (SEQ ID NO: 247) | QSALYSPSSKTY (SEQ ID NO: 248) | WAS | CQYYYGTVTF(SEQ ID NO: 249) |
| 22.18A.E9 | GGSINSYY (SEQ ID NO:250) | MYPGGTS (SEQ ID NO: 251) | CARDPTYHHGTSGYVGTFDLW (SEQ ID NO: 252) | PSVRNN (SEQ ID NO: 253) | GAS | CQQYNSWPPYTF (SEQ ID NO: 254) |
| 5.52.H10 | GFTFSMFT (SEQ ID NO: 255) | ISGSSSLV (SEQ ID NO: 256) | CVRGDSRDYW (SEQ ID NO: 257) | TSNIGNNH (SEQ ID NO: 258) | RND | CGTWDNSLSGWVF (SEQ ID NO:259) |
| 5.15.C1 | GYSFTTD (SEQ ID NO: 260) | MNPNTGDT (SEQ ID NO: 261) | CARSNSAPVSTLLPADAFDVW (SEQ ID NO: 262) | QIINSY (SEQ ID NO: 263) | AVS | CQQTYTTVAITF (SEQ ID NO: 264) |
| 5.54.E6 | AGTLSGYT (SEQ ID NO: 265) | IIPLGIG (SEQ ID NO: 266) | CAVEFYRLATVTTPTLDFW (SEQ ID NO: 267) | HIGAKS (SEQ ID NO: 268) | MNS | CQVWDSFRDHQVF (SEQ ID NO: 269) |
| 5.55.D2 | GFSLRTSGVG (SEQ ID NO: 270) | IYWDDDK (SEQ ID NO: 271) | CARQITNSFGLVIANDAFDIW (SEQ ID NO: 272) | SSDVGRYNY (SEQ ID NO: 273) | GVT | CSSYAGSNFLEVF (SEQ ID NO: 274) |
| 22.14.A1 | GGTFSSYA (SEQ ID NO: 275) | IIPMFDTA (SEQ ID NO: 276) | CARDRNDYYDSSGYSGAFDQW (SEQ ID NO: 277) | QSVSSSY (SEQ ID NO: 278) | GAS | CQQYGSSPRTF (SEQ ID NO: 279) |
| 26.53.B4 | GFSFSGSA (SEQ ID NO: 280) | IRTKTKNYAT (SEQ ID NO: 281) | CSRIEFSSSSGPQM (SEQ ID NO: 282) | QNIITW (SEQ ID NO: 283) | KAS | CLQYKTEPWTF (SEQ ID NO: 284) |
| 5.63.E2 | GFTFSAYW (SEQ ID NO: 285) | INQGGDKT (SEQ ID NO: 286) | CGRGTNQDYW (SEQ ID NO: 287) | SSNIGSNN (SEQ ID NO: 288) | PNS | CAAWDDSLRTYVF (SEQ ID NO: 289) |
| 5.64.G4. | GFTLRSYG (SEQ ID NO: 290) | ISYDGSKT (SEQ ID NO: 291) | CAKEDNLGSDVFDRW (SEQ ID NO: 292) | QSASQW (SEQ ID NO: 293) | KAS | CQQYNIYPWTF (SEQ ID NO: 294) |
| 43.52.A11 | GRIFRNYA (SEQ ID NO: 295) | FIPSEGIT (SEQ ID NO: 296) | CVGGRPGAFVPFDSW (SEQ ID NO: 297) | QSVRGN (SEQ ID NO: 298) | DAS | CQQYNDWPRTF (SEQ ID NO: 299) |
| 43.52.E12 | GGSFSSYD (SEQ ID NO: 300) | SVSRADSS (SEQ ID NO: 301) | CAAGDCSDGGCFRTPLHIW (SEQ ID NO: 302) | QSLRTN (SEQ ID NO: 303) | GAS | CQQYHDWPRTF (SEQ ID NO: 304) |
| 43.62.E2 | GYNFNSYW (SEQ ID NO: 305) | IYPADSDR (SEQ ID NO: 306) | CARLLRVSTGWEDAFDLW (SEQ ID NO: 307) | QSVLYSSTNKNY (SEQ ID NO: 308) | WAS | CQQYYSDYLTF (SEQ ID NO: 309) |

To create complete heavy and/or light chain antibody sequences, each variable sequence above can be combined with a constant region. Human constant regions for heavy chain, kappa chain, and lambda chain are known in the art. For example, a complete heavy chain comprises a heavy variable sequence followed by a human heavy chain constant sequence such as IgG1, IgG2, IgG3, IgG4, IgA1, IgA2, IgM, IgD, or IgE. A complete kappa chain comprises a kappa variable sequence followed by the human kappa light chain constant sequence, and a complete lambda chain comprises a lambda variable sequence followed by the human lambda light chain constant sequence. Exemplary human heavy chain, kappa chain, and lambda chains are shown below.

Nucleic Acid Sequence Encoding the genomic Human IgG1 Heavy Chain Constant Region (SEQ ID NO: 118)

CCTCCACCAAGGGCCCATCGGTCTTCCCCCTGGCACCCTCCTCCAAGAGCACCTCTGGGG
GCACAGCGGCCCTGGGCTGCCTGGTCAAGGACTACTTCCCCGAACCGGTGACGGTGTCGT
GGAACTCAGGCGCCCTGACCAGCGGCGTGCACACCTTCCCGGCTGTCCTACAGTCCTCAG
GACTCTACTCCCTCAGCAGCGTGGTGACCGTGCCCTCCAGCAGCTTGGGCACCCAGACCT
ACATCTGCAACGTGAATCACAAGCCCAGCAACACCAAGGTGGACAAGAAAGTTGGTGAGA
GGCCAGCACAGGGAGGGAGGGTGTCTGCTGGAAGCAGGCTCAGCGCTCCTGCCTGGACGC
ATCCCGGCTATGCAGCCCCAGTCCAGGGCAGCAAGGCAGGCCCCGTCTGCCTCTTCACCC
GGAGCCTCTGCCCGCCCCACTCATGCTCAGGGAGAGGGTCTTCTGGCTTTTTCCAGGCT
CTGGGCAGGCACAGGCTAGGTGCCCCTAACCCAGGCCCTGCACACAAAGGGGCAGGTGCT
GGGCTCAGACCTGCCAAGAGCCATATCCGGGAGGACCCTGCCCCTGACCTAAGCCCACCC
CAAAGGCCAAACTCTCCACTCCCTCAGCTCGGACACCTTCTCTCCTCCCAGATTCCAGTA
ACTCCCAATCTTCTCTCTGCAGAGCCCAAATCTTGTGACAAAACTCACACATGCCCACCG
TGCCCAGGTAAGCCAGCCCAGGCCTCGCCCTCCAGCTCAAGGCGGGACAGGTGCCCTAGA
GTAGCCTGCATCCAGGGACAGGCCCCAGCCGGGTGCTGACACGTCCACCTCCATCTCTTC
CTCAGCACCTGAACTCCTGGGGGGACCGTCAGTCTTCCTCTTCCCCCCAAAACCCAAGGA
CACCCTCATGATCTCCCGGACCCCTGAGGTCACATGCGTGGTGGTGGACGTGAGCCACGA
AGACCCTGAGGTCAAGTTCAACTGGTACGTGGACGGCGTGGAGGTGCATAATGCCAAGAC
AAAGCCGCGGGAGGAGCAGTACAACAGCACGTACCGGGTGGTCAGCGTCCTCACCGTCCT
GCACCAGGACTGGCTGAATGGCAAGGAGTACAAGTGCAAGGTCTCCAACAAAGCCCTCCC
AGCCCCCATCGAGAAAACCATCTCCAAAGCCAAAGGTGGGACCCGTGGGGTGCGAGGGCC
ACATGGACAGAGGCCGGCTCGGCCCACCCTCTGCCCTGAGAGTGACCGCTGTACCAACCT
CTGTCCTACAGGGCAGCCCCGAGAACCACAGGTGTACACCCTGCCCCCATCCCGGGATGA
GCTGACCAAGAACCAGGTCAGCCTGACCTGCCTGGTCAAAGGCTTCTATCCCAGCGACAT
CGCCGTGGAGTGGGAGAGCAATGGGCAGCCGGAGAACAACTACAAGACCACGCCTCCCGT
GCTGGACTCCGACGGCTCCTTCTTCCTCTACAGCAAGCTCACCGTGGACAAGAGCAGGTG
GCAGCAGGGGAACGTCTTCTCATGCTCCGTGATGCATGAGGCTCTGCACAACCACTACAC
GCAGAAGAGCCTCTCCCTGTCTCCGGGTAAA

Nucleic Acid Sequence Encoding the Human IgG1 Heavy Chain Constant Region cDNA (SEQ ID NO: 119)

CCTCCACCAAGGGCCCATCGGTCTTCCCCCTGGCACCCTCCTCCAAGAGCACCTCTGGGGGCACAG
CGGCCCTGGGCTGCCTGGTCAAGGACTACTTCCCCGAACCGGTGACGGTGTCGTGGAACTCAGGCG
CCCTGACCAGCGGCGTGCACACCTTCCCGGCTGTCCTACAGTCCTCAGGACTCTACTCCCTCAGCA
GCGTGGTGACCGTGCCCTCCAGCAGCTTGGGCACCCAGACCTACATCTGCAACGTGAATCACAAGC
CCAGCAACACCAAGGTGGACAAGAAAGTTGAGCCCAAATCTTGTGACAAAACTCACACATGCCCAC
CGTGCCCAGCACCTGAACTCCTGGGGGGACCGTCAGTCTTCCTCTTCCCCCCAAAACCCAAGGACA
CCCTCATGATCTCCCGGACCCCTGAGGTCACATGCGTGGTGGTGGACGTGAGCCACGAAGACCCTG
AGGTCAAGTTCAACTGGTACGTGGACGGCGTGGAGGTGCATAATGCCAAGACAAAGCCGCGGGAGG
AGCAGTACAACAGCACGTACCGTGTGGTCAGCGTCCTCACCGTCCTGCACCAGGACTGGCTGAATG
GCAAGGAGTACAAGTGCAAGGTCTCCAACAAAGCCCTCCCAGCCCCCATCGAGAAAACCATCTCCA
AAGCCAAAGGGCAGCCCCGAGAACCACAGGTGTACACCCTGCCCCCATCCCGGGATGAGCTGACCA
AGAACCAGGTCAGCCTGACCTGCCTGGTCAAAGGCTTCTATCCCAGCGACATCGCCGTGGAGTGGG
AGAGCAATGGGCAGCCGGAGAACAACTACAAGACCACGCCTCCCGTGCTGGACTCCGACGGCTCCT
TCTTCCTCTACAGCAAGCTCACCGTGGACAAGAGCAGGTGGCAGCAGGGGAACGTCTTCTCATGCT
CCGTGATGCATGAGGCTCTGCACAACCACTACACGCAGAAGAGCCTCTCCCTGTCTCCGGGTAAA

Protein Sequence Defining the Human IgG1 Heavy Chain Constant Region (SEQ ID NO: 120)

STKGPSVFPLAPSSKSTSGGTAALGCLVKDYFPEPVTVSWNSGALTSGVHTFPAVLQSSGLYSLSS
VVTVPSSSLGTQTYICNVNHKPSNTKVDKKVEPKSCDKTHTCPPCPAPELLGGPSVFLFPPKPKDT
LMISRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHNHYTQKSLSLSPGK

Nucleic Acid Sequence Encoding the genomic Human IgG2 Heavy Chain Constant Region (SEQ ID NO: 121)

CCTCCACCAAGGGCCCATCGGTCTTCCCCCTGGCGCCCTGCTCCAGGAGCACCTCCGAGA
GCACAGCCGCCCTGGGCTGCCTGGTCAAGGACTACTTCCCCGAACCGGTGACGGTGTCGT
GGAACTCAGGCGCTCTGACCAGCGGCGTGCACACCTTCCCAGCTGTCCTACAGTCCTCAG
GACTCTACTCCCTCAGCAGCGTGGTGACCGTGCCCTCCAGCAACTTCGGCACCCAGACCT
ACACCTGCAACGTAGATCACAAGCCCAGCAACACCAAGGTGGACAAGACAGTTGGTGAGA
GGCCAGCTCAGGGAGGGAGGGTGTCTGCTGGAAGCCAGGCTCAGCCCTCCTGCCTGGACG
CACCCCGGCTGTGCAGCCCCAGCCCAGGGCAGCAAGGCAGGCCCCATCTGTCTCCTCACC
CGGAGGCCTCTGCCCGCCCCACTCATGCTCAGGGAGAGGGTCTTCTGGCTTTTTCCACCA
GGCTCCAGGCAGGCACAGGCTGGGTGCCCCTACCCCAGGCCCTTCACACACAGGGGCAGG
TGCTTGGCTCAGACCTGCCAAAAGCCATATCCGGGAGGACCCTGCCCCTGACCTAAGCCG
ACCCCAAAGGCCAAACTGTCCACTCCCTCAGCTCGGACACCTTCTCTCCTCCCAGATCCG
AGTAACTCCCAATCTTCTCTCTGCAGAGCGCAAATGTTGTGTCGAGTGCCCACCGTGCCC

AGGTAAGCCAGCCCAGGCCTCGCCCTCCAGCTCAAGGCGGGACAGGTGCCCTAGAGTAGC
CTGCATCCAGGGACAGGCCCCAGCTGGGTGCTGACACGTCCACCTCCATCTCTTCCTCAG
CACCACCTGTGGCAGGACCGTCAGTCTTCCTCTTCCCCCCAAAACCCAAGGACACCCTCA
TGATCTCCCGGACCCCTGAGGTCACGTGCGTGGTGGTGGACGTGAGCCACGAAGACCCCG
AGGTCCAGTTCAACTGGTACGTGGACGGCGTGGAGGTGCATAATGCCAAGACAAAGCCAC
GGGAGGAGCAGTTCAACAGCACGTTCCGTGTGGTCAGCGTCCTCACCGTTGTGCACCAGG
ACTGGCTGAACGGCAAGGAGTACAAGTGCAAGGTCTCCAACAAAGGCCTCCCAGCCCCCA
TCGAGAAAACCATCTCCAAAACCAAAGGTGGGACCCGCGGGGTATGAGGGCCACATGGAC
AGAGGCCGGCTCGGCCCACCCTCTGCCCTGGGAGTGACCGCTGTGCCAACCTCTGTCCCT
ACAGGGCAGCCCCGAGAACCACAGGTGTACACCCTGCCCCCATCCCGGGAGGAGATGACC
AAGAACCAGGTCAGCCTGACCTGCCTGGTCAAAGGCTTCTACCCCAGCGACATCGCCGTG
GAGTGGGAGAGCAATGGGCAGCCGGAGAACAACTACAAGACCACACCTCCCATGCTGGAC
TCCGACGGCTCCTTCTTCCTCTACAGCAAGCTCACCGTGGACAAGAGCAGGTGGCAGCAG
GGGAACGTCTTCTCATGCTCCGTGATGCATGAGGCTCTGCACAACCACTACACGCAGAAG
AGCCTCTCCCTGTCTCCGGGTAAA

Nucleic Acid Sequence Encoding the Human IgG2 Heavy Chain Constant Region cDNA (SEQ ID NO: 122)

CCTCCACCAAGGGCCCATCGGTCTTCCCCCTGGCGCCCTGCTCCAGGAGCACCTCCGAGAGCACAG
CCGCCCTGGGCTGCCTGGTCAAGGACTACTTCCCCGAACCGGTGACGGTGTCGTGGAACTCAGGCG
CTCTGACCAGCGGCGTGCACACCTTCCCAGCTGTCCTACAGTCCTCAGGACTCTACTCCCTCAGCA
GCGTGGTGACCGTGCCCTCCAGCAACTTCGGCACCCAGACCTACACCTGCAACGTAGATCACAAGC
CCAGCAACACCAAGGTGGACAAGACAGTTGAGCGCAAATGTTGTGTCGAGTGCCCACCGTGCCCAG
CACCACCTGTGGCAGGACCGTCAGTCTTCCTCTTCCCCCCAAAACCCAAGGACACCCTCATGATCT
CCCGGACCCCTGAGGTCACGTGCGTGGTGGTGGACGTGAGCCACGAAGACCCCGAGGTCCAGTTCA
ACTGGTACGTGGACGGCGTGGAGGTGCATAATGCCAAGACAAAGCCACGGGAGGAGCAGTTCAACA
GCACGTTCCGTGTGGTCAGCGTCCTCACCGTTGTGCACCAGGACTGGCTGAACGGCAAGGAGTACA
AGTGCAAGGTCTCCAACAAAGGCCTCCCAGCCCCCATCGAGAAAACCATCTCCAAAACCAAAGGGC
AGCCCCGAGAACCACAGGTGTACACCCTGCCCCCATCCCGGGAGGAGATGACCAAGAACCAGGTCA
GCCTGACCTGCCTGGTCAAAGGCTTCTACCCCAGCGACATCGCCGTGGAGTGGGAGAGCAATGGGC
AGCCGGAGAACAACTACAAGACCACACCTCCCATGCTGGACTCCGACGGCTCCTTCTTCCTCTACA
GCAAGCTCACCGTGGACAAGAGCAGGTGGCAGCAGGGGAACGTCTTCTCATGCTCCGTGATGCATG
AGGCTCTGCACAACCACTACACGCAGAAGAGCCTCTCCCTGTCTCCGGGTAAA

Protein Sequence Defining the Human IgG2 Heavy Chain Constant Region (SEQ ID NO: 123)

STKGPSVFPLAPCSRSTSESTAALGCLVKDYFPEPVTVSWNSGALTSGVHTFPAVLQSSGLYSLSS
VVTVPSSNFGTQTYTCNVDHKPSNTKVDKTVERKCCVECPPCPAPPVAGPSVFLFPPKPKDTLMIS
RTPEVTCVVVDVSHEDPEVQFNWYVDGVEVHNAKTKPREEQFNSTFRVVSVLTVVHQDWLNGKEYK
CKVSNKGLPAPIEKTISKTKGQPREPQVYTLPPSREEMTKNQVSLTCLVKGFYPSDIAVEWESNGQ
PENNYKTTPPMLDSDGSFFLYSKLTVDKSRWQQGNVFSCSVMHEALHNHYTQKSLSLSPGK

Nucleic Acid Sequence Encoding the Human Kappa Light Chain Constant Region (SEQ ID NO: 124)

GAACTGTGGCTGCACCATCTGTCTTCATCTTCCCGCCATCTGATGAGCAGTTGAAATCTGGAACTG
CCTCTGTTGTGTGCCTGCTGAATAACTTCTATCCCAGAGAGGCCAAAGTACAGTGGAAGGTGGATA
ACGCCCTCCAATCGGGTAACTCCCAGGAGAGTGTCACAGAGCAGGACAGCAAGGACAGCACCTACA
GCCTCAGCAGCACCCTGACGCTGAGCAAAGCAGACTACGAGAAACACAAAGTCTACGCCTGCGAAG
TCACCCATCAGGGCCTGAGCTCGCCCGTCACAAAGAGCTTCAACAGGGGAGAGTGT

Protein Sequence Defining the Human Kappa Light Chain Constant Region (SEQ ID NO: 125)

Nucleic Acid Sequence Encoding the Human Lambda Light Chain Constant Region (SEQ ID NO: 126)

GTCAGCCCAAGGCCAACCCCACTGTCACTCTGTTCCCGCCCTCCTCTGAGGAGCTCCAAGCCAACA
AGGCCACACTAGTGTGTCTGATCAGTGACTTCTACCCGGGAGCTGTGACAGTGGCCTGGAAGGCAG
ATGGCAGCCCCGTCAAGGCGGGAGTGGAGACCACCAAACCCTCCAAACAGAGCAACAACAAGTACG
CGGCCAGCAGCTACCTGAGCCTGACGCCCGAGCAGTGGAAGTCCCACAGAAGCTACAGCTGCCAGG
TCACGCATGAAGGGAGCACCGTGGAGAAGACAGTGGCCCCTACAGAATGTTCA

Protein Sequence Defining the Human Lambda Light Chain Constant Region (SEQ ID NO: 127)

GQPKANPTVTLFPPSSEELQANKATLVCLISDFYPGAVTVAWKADGSPVKAGVETTKPSKQSNNKY
AASSYLSLTPEQWKSHRSYSCQVTHEGSTVEKTVAPTECS

It is appreciated, however, that the variable region sequences described herein can be ligated to each of a number of other constant region sequences known to those skilled in the art to produce active full length immunoglobulin heavy and light chains.

The following sequences represent the actual or contemplated full length immunoglobulin heavy and light chain sequences (*i.e.,* containing both the variable and constant regions sequences) for the anti-*S*. *aureus* antibodies described herein. The immunoglobulin heavy chain and light chain variable regions are shown as uppercase letters and the human IgG1, IgG2, kappa and lambda constant regions are shown in bold uppercase letters in the full length heavy and light chain sequences. CDR sequences are underlined.

Nucleic Acid Sequence Encoding the Full Length Heavy Chain Sequence of 1.62.B9 (SEQ ID NO: 128)

GAGGTGCAGCTGGTGGAGTCTGGGGGAGGCTTGGTAAAGCCTGGGGGGTCCCTTAGACTCTCCTGT
GCAGCCTCTGGATTCACTTTCAGTAACGCCTGGATGACCTGGGTCCGCCAGGCTCCAGGGAAGGGG
CTGGAGTGGGTTGGCCGTATTAAGAGCAAAACTGATGGTGGGACAACAGACTACGCTGCACCCGTG
AAAGGCAGATTCACCATCTCAAGAGATGATTCAATAAACACACTGTATCTGCAAATGAACAGCCTG
AAAACCGAGGACACAGCCGTGTATTATTGTAGGACGGGGGGCTACTGGGGCCAGGGAACCCTGGTC
ACCGTCTCGAGTGCCTCCACCAAGGGCCCATCGGTCTTCCCCCTGGCGCCCTGCTCCAGGAGCACC
TCCGAGAGCACAGCCGCCCTGGGCTGCCTGGTCAAGGACTACTTCCCCGAACCGGTGACGGTGTCG
TGGAACTCAGGCGCTCTGACCAGCGGCGTGCACACCTTCCCAGCTGTCCTACAGTCCTCAGGACTC
TACTCCCTCAGCAGCGTGGTGACCGTGCCCTCCAGCAACTTCGGCACCCAGACCTACACCTGCAAC
GTAGATCACAAGCCCAGCAACACCAAGGTGGACAAGACAGTTGAGCGCAAATGTTGTGTCGAGTGC
CCACCGTGCCCAGCACCACCTGTGGCAGGACCGTCAGTCTTCCTCTTCCCCCCAAAACCCAAGGAC
ACCCTCATGATCTCCCGGACCCCTGAGGTCACGTGCGTGGTGGTGGACGTGAGCCACGAAGACCCC
GAGGTCCAGTTCAACTGGTACGTGGACGGCGTGGAGGTGCATAATGCCAAGACAAAGCCACGGGAG
GAGCAGTTCAACAGCACGTTCCGTGTGGTCAGCGTCCTCACCGTTGTGCACCAGGACTGGCTGAAC
GGCAAGGAGTACAAGTGCAAGGTCTCCAACAAAGGCCTCCCAGCCCCCATCGAGAAAACCATCTCC
AAAACCAAAGGGCAGCCCCGAGAACCACAGGTGTACACCCTGCCCCCATCCCGGGAGGAGATGACC
AAGAACCAGGTCAGCCTGACCTGCCTGGTCAAAGGCTTCTACCCCAGCGACATCGCCGTGGAGTGG
GAGAGCAATGGGCAGCCGGAGAACAACTACAAGACCACACCTCCCATGCTGGACTCCGACGGCTCC
TTCTTCCTCTACAGCAAGCTCACCGTGGACAAGAGCAGGTGGCAGCAGGGGAACGTCTTCTCATGC
TCCGTGATGCATGAGGCTCTGCACAACCACTACACGCAGAAGAGCCTCTCCCTGTCTCCGGGTAAA
TGA

Protein Sequence Defining the Full Length Heavy Chain Sequence of 1.62.B9 (SEQ ID NO: 129)

EVQLVESGGGLVKPGGSLRLSCAASGFTFSNAWMTWVRQAPGKGLEWVGRIKSKTDGGTTDYAAPV
KGRFTISRDDSINTLYLQMNSLKTEDTAVYYCRTGGYWGQGTLVTVSSASTKGPSVFPLAPCSRST
SESTAALGCLVKDYFPEPVTVSWNSGALTSGVHTFPAVLQSSGLYSLSSVVTVPSSNFGTQTYTCN
VDHKPSNTKVDKTVERKCCVECPPCPAPPVAGPSVFLFPPKPKDTLMISRTPEVTCVVVDVSHEDP
EVQFNWYVDGVEVHNAKTKPREEQFNSTFRVVSVLTVVHQDWLNGKEYKCKVSNKGLPAPIEKTIS
KTKGQPREPQVYTLPPSREEMTKNQVSLTCLVKGFYPSDIAVEWESNGQPENNYKTTPPMLDSDGS
FFLYSKLTVDKSRWQQGNVFSCSVMHEALHNHYTQKSLSLSPGK

Nucleic Acid Sequence Encoding the Full Length Lambda Light Chain Sequence of 1.62.B9 (SEQ ID NO: 130)

CAGGCTGTGGTGACTCAGGAGCCCTCACTGACTGTGTCCCCAGGAGGGACAGTCACTCTCACCTGT
GGCTCCAGCACTGGAGCTGTCACCAGTGGTCATTATCCCTACTGGTTCCAGCAGAAGCCTGGCCAA
GCCCCCAGGACACTGATTTATGATACAAGCAAGAAACATTCCTGGACACCTGCCCGGTTCTCAGGC
TCCCTCCTTGGGGGCAAAGCTGCCCTGACCCTTTCGGGTGCGCAGCCTGAGGATGAGGCTGAGTAT
TACTGCTTGGTCTCCGATAGTGGTGCTCGGATCTTCGGACCTGGGACCAAGGTCACCGTCCTAGGT
CAGCCCAAGGCCAACCCCACTGTCACTCTGTTCCCGCCCTCCTCTGAGGAGCTCCAAGCCAACAAG
GCCACACTAGTGTGTCTGATCAGTGACTTCTACCCGGGAGCTGTGACAGTGGCCTGGAAGGCAGAT
GGCAGCCCCGTCAAGGCGGGAGTGGAGACCACCAAACCCTCCAAACAGAGCAACAACAAGTACGCG
GCCAGCAGCTACCTGAGCCTGACGCCCGAGCAGTGGAAGTCCCACAGAAGCTACAGCTGCCAGGTC
ACGCATGAAGGGAGCACCGTGGAGAAGACAGTGGCCCCTACAGAATGTTCA

Protein Sequence Defining the Full Length Lambda Light Chain Sequence of 1.62.B9 (SEQ ID NO: 131)

QAVVTQEPSLTVSPGGTVTLTCGSSTGAVTSGHYPYWFQQKPGQAPRTLIYDTSKKHSWTPARFSG
SLLGGKAALTLSGAQPEDEAEYYCLVSDSGARIFGPGTKVTVLGQPKANPTVTLFPPSSEELQANK
ATLVCLISDFYPGAVTVAWKADGSPVKAGVETTKPSKQSNNKYAASSYLSLTPEQWKSHRSYSCQV
THEGSTVEKTVAPTECS

Nucleic Acid Sequence Encoding the Full Length Heavy Chain Sequence of 5.11.H10 (SEQ ID NO: 132)

CAGGTTCAGCTGGTGCAGTCTGGACCTGAAATGAAGAAGCCTGGGGCCTCAGTGAAGGTCTCCTGT
CAGGCCTCTGGTTACACCTTTACCATTAATGGAATTAGTTGGGTGCGACAGGCCCCTGGACAACGG
CCTGAGTGGATGGGGTGGATCAGCACTAGTAATGGTCAGACAAACTTTGCACAGAATTTCCAGGGC
AGACTCACCTTGACCACAGACACATCCACGACCACGGCCTTCATGGAGTTGATGAGCCTCACATCT
GCCGACACGGCCGTATACTACTGTGCGAGAAATCCATACGGCAATTCGCGCAGATTCTTTGACTAC
TGGGGCCAGGGAACCCTGGTCACCGTCTCGAGTGCCTCCACCAAGGGCCCATCGGTCTTCCCCCTG
GCGCCCTGCTCCAGGAGCACCTCCGAGAGCACAGCCGCCCTGGGCTGCCTGGTCAAGGACTACTTC
CCCGAACCGGTGACGGTGTCGTGGAACTCAGGCGCTCTGACCAGCGGCGTGCACACCTTCCCAGCT
GTCCTACAGTCCTCAGGACTCTACTCCCTCAGCAGCGTGGTGACCGTGCCCTCCAGCAACTTCGGC
ACCCAGACCTACACCTGCAACGTAGATCACAAGCCCAGCAACACCAAGGTGGACAAGACAGTTGAG
CGCAAATGTTGTGTCGAGTGCCCACCGTGCCCAGCACCACCTGTGGCAGGACCGTCAGTCTTCCTC
TTCCCCCCAAAACCCAAGGACACCCTCATGATCTCCCGGACCCCTGAGGTCACGTGCGTGGTGGTG
GACGTGAGCCACGAAGACCCCGAGGTCCAGTTCAACTGGTACGTGGACGGCGTGGAGGTGCATAAT
GCCAAGACAAAGCCACGGGAGGAGCAGTTCAACAGCACGTTCCGTGTGGTCAGCGTCCTCACCGTT
GTGCACCAGGACTGGCTGAACGGCAAGGAGTACAAGTGCAAGGTCTCCAACAAAGGCCTCCCAGCC

CCCATCGAGAAAACCATCTCCAAAACCAAAGGGCAGCCCCGAGAACCACAGGTGTACACCCTGCCC
CCATCCCGGGAGGAGATGACCAAGAACCAGGTCAGCCTGACCTGCCTGGTCAAAGGCTTCTACCCC
AGCGACATCGCCGTGGAGTGGGAGAGCAATGGGCAGCCGGAGAACAACTACAAGACCACACCTCCC
ATGCTGGACTCCGACGGCTCCTTCTTCCTCTACAGCAAGCTCACCGTGGACAAGAGCAGGTGGCAG
CAGGGGAACGTCTTCTCATGCTCCGTGATGCATGAGGCTCTGCACAACCACTACACGCAGAAGAGC
CTCTCCCTGTCTCCGGGTAAATGA

Protein Sequence Defining the Full Length Heavy Chain Sequence of 5.11.H10 (SEQ ID NO: 133)

Nucleic Acid Sequence Encoding the Full Length Kappa Light Chain Sequence of 5.11.H10 (SEQ ID NO: 134)

GACATCCAGATGACCCAGTCTCCATCCTCCCTGTCTGCCTCTGTTGGCGACAGAGTCATCATCACT
TGCCGGACAAGTCAGACCATCACCAATTACATAAATTGGTATCAGCAAAAACCAGGGAAAGCCCCG
CAGCTCCTGATCTATGGTGTATCCAGTTTGCAAAGTGGGGTCCCATCAAGGTTCAGTGGCGGTGGA
TCTGGGACAGAATTCACTCTCACCATCAGCAGTCTGCAACCTGAAGACTTTGCAACTTATTACTGT
CAACAGAGTTATAGGGCCCCTCAAACATTCGGCCAAGGGACCAAGCTGGAAATCAAACGAACTGTG
GCTGCACCATCTGTCTTCATCTTCCCGCCATCTGATGAGCAGTTGAAATCTGGAACTGCCTCTGTT
GTGTGCCTGCTGAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACCTACAGCCTCAGC
AGCACCCTGACGCTGAGCAAAGCAGACTACGAGAAACACAAAGTCTACGCCTGCGAAGTCACCCAT
CAGGGCCTGAGCTCGCCCGTCACAAAGAGCTTCAACAGGGGAGAGTGT

Protein Sequence Defining t the Full Length Kappa Light Chain Sequence of 5.11.H10 (SEQ ID NO: 135)

DIQMTQSPSSLSASVGDRVIITCRTSQTITNYINWYQQKPGKAPQLLIYGVSSLQSGVPSRFSGGG
SGTEFTLTISSLQPEDFATYYCQQSYRAPQTFGQGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC

Nucleic Acid Sequence Encoding the Full Length Heavy Chain Sequence of 5.27.A11 (SEQ ID NO: 136)

GAGGTGCAGCTGGTGGAGTCTGGGGGAGGCTTGGTGCAGCCGGGGGGGTCTCTGAGACTCTCTTGT
GCAGCCTCTGGTTTCGCCTTCAGCAGCCACGAGATGCACTGGGTTCGTCAAGGATAGGACACAGT
TTGGAGTGGGTCTCAGCTATTGGCCTTGCTGGTGACACATATTATTCAGACTCCGTGAAGGGCCGC
TTCACCATCTCCAGAGAAAACGCCAAGAACACTTTGTATCTTGAAATGAACAATTTGAGAGTCGGC
GACACGGCTGTCTATTACTGTGTCAAAGAAGGGGCTCATTGGAATTTGACTCCTGGGGCCAGGGA
ACCCTGGTCACCGTCTCGAGTGCCTCCACCAAGGGCCCATCGGTCTTCCCCCTGGCGCCCTGCTCC
AGGAGCACCTCCGAGAGCACAGCCGCCCTGGGCTGCCTGGTCAAGGACTACTTCCCCGAACCGGTG
ACGGTGTCGTGGAACTCAGGCGCTCTGACCAGCGGCGTGCACACCTTCCCAGCTGTCCTACAGTCC
TCAGGACTCTACTCCCTCAGCAGCGTGGTGACCGTGCCCTCCAGCAACTTCGGCACCCAGACCTAC
ACCTGCAACGTAGATCACAAGCCCAGCAACACCAAGGTGGACAAGACAGTTGAGCGCAAATGTTGT
GTCGAGTGCCCACCGTGCCCAGCACCACCTGTGGCAGGACCGTCAGTCTTCCTCTTCCCCCCAAAA
CCCAAGGACACCCTCATGATCTCCCGGACCCCTGAGGTCACGTGCGTGGTGGTGGACGTGAGCCAC
GAAGACCCCGAGGTCCAGTTCAACTGGTACGTGGACGGCGTGGAGGTGCATAATGCCAAGACAAAG
CCACGGGAGGAGCAGTTCAACAGCACGTTCCGTGTGGTCAGCGTCCTCACCGTTGTGCACCAGGAC
TGGCTGAACGGCAAGGAGTACAAGTGCAAGGTCTCCAACAAAGGCCTCCCAGCCCCCATCGAGAAA
ACCATCTCCAAAACCAAAGGGCAGCCCCGAGAACCACAGGTGTACACCCTGCCCCCATCCCGGGAG
GAGATGACCAAGAACCAGGTCAGCCTGACCTGCCTGGTCAAAGGCTTCTACCCCAGCGACATCGCC
GTGGAGTGGGAGAGCAATGGGCAGCCGGAGAACAACTACAAGACCACACCTCCCATGCTGGACTCC
GACGGCTCCTTCTTCCTCTACAGCAAGCTCACCGTGGACAAGAGCAGGTGGCAGCAGGGGAACGTC
TTCTCATGCTCCGTGATGCATGAGGCTCTGCACAACCACTACACGCAGAAGAGCCTCTCCCTGTCT
CCGGGTAAATGA

Protein Sequence Defining the Full Length Heavy Chain Sequence of 5.27.A11 (SEQ ID NO: 137)

EVQLVESGGGLVQPGGSLRLSCAASGFAFSSHEMHWVRQGIGHSLEWVSAIGLAGDTYYSDSVKGR
FTISRENAKNTLYLEMNNLRVGDTAVYYCVKEGAHWEFDSWGQGTLVTVSSASTKGPSVFPLAPCS
RSTSESTAALGCLVKDYFPEPVTVSWNSGALTSGVHTFPAVLQSSGLYSLSSVVTVPSSNFGTQTY
TCNVDHKPSNTKVDKTVERKCCVECPPCPAPPVAGPSVFLFPPKPKDTLMISRTPEVTCVVVDVSH
EDPEVQFNWYVDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYKTTPPMLDS
DGSFFLYSKLTVDKSRWQQGNVFSCSVMHEALHNHYTQKSLSLSPGK

Nucleic Acid Sequence Encoding the Full Length Lambda Light Chain Sequence of 5.27.A11 (SEQ ID NO: 138)

CAGTCTGTGCTGACGCAGCCGCCCTCAGTGTCTGGGGCCCCTGGACAGAGGGTCATCATCTCCTGC
ACTGGGAGCAGCTCCAACCTCGGGGCAAGTCTTGATGTACACTGGTACCAGCGCCTTCCAGGGACA
GGCCCCCGACTCCTCATCTATGCTAACAACAATCGGCCCTCAGGGGTCCCTGACCGATTCTCTGGC

TCCAAGTCTGGCACCTCAGCCTCCCTGGCCATCACCGGGCTCCAGGCTGAAGATGAGGGTGATTAT
TACTGCCAGTCCTATGACGGCAGCCTGAGGGTGTTCGGCGGAGGGACCAGGCTGACCGTCCTAGGT
CAGCCCAAGGCTGCCCCCTCGGTCACTCTGTTCCCGCCCTCCTCTGAGGAGCTTCAAGCCAACAAG
GCCACACTGGTGTGTCTCATAAGTGACTTCTACCCGGGAGCCGTGACAGTGGCCTGGAAGGCAGAT
AGCAGCCCCGTCAAGGCGGGAGTGGAGACCACCACACCCTCCAAACAAAGCAACAACAAGTACGCG
GCCAGCAGCTACCTGAGCCTGACGCCTGAGCAGTGGAAGTCCCACAAAAGCTACAGCTGCCAGGTC
ACGCATGAAGGGAGCACCGTGGAGAAGACAGTGGCCCCTACAGAATGTTCA

Protein Sequence Defining the Full Length Lambda Light Chain Sequence of 5.27.A11 (SEQ ID NO: 139)

QSVLTQPPSVSGAPGQRVIISCTGSSSNLGASLDVHWYQRLPGTGPRLLIYANNNRPSGVPDRFSG
SKSGTSASLAITGLQAEDEGDYYCQSYDGSLRVFGGGTRLTVLGQPKAAPSVTLFPPSSEELQANK
ATLVCLISDFYPGAVTVAWKADSSPVKAGVETTTPSKQSNNKYAASSYLSLTPEQWKSHKSYSCQV
THEGSTVEKTVAPTECS

Nucleic Acid Sequence Encoding the Full Length Heavy Chain Sequence of 5.6.H2 (SEQ ID NO: 140)

CAGGTGCAGCTGGTGGAGTCTGGGGGAGGCTTGTTCAAGCCTGGAGGGTCCCTGAGACTGTCCTGT
GCAGCCTCTGGATTCACCTTCAGTGACTACTACATGAGCTGGATCCGGCAGGCTCCAGGGAAGGGG
CTGGAGTGGATTTCATACATTAGTAGTAGTGGTAGGACCATAAAGTACGCAGACTCTGTGAAGGGC
CGATTCACCATCTCCAGGGACAACGCCAAGAACTCACTTTATCTGCAAATGAACAGCCTGAGAGCC
GAGGACACGGCCGTGTATTATTGTGCGAGAGATGGAGGATATTGTGGAAGTGCAAACTGCCTTCAT
GACGCTTTTGATATCTGGGGCCAAGGGACAATGGTCACCGTCTCGAGTGCCTCCACCAAGGGCCCA
TCGGTCTTCCCCCTGGCGCCCTGCTCCAGGAGCACCTCCGAGAGCACAGCCGCCCTGGGCTGCCTG
GTCAAGGACTACTTCCCCGAACCGGTGACGGTGTCGTGGAACTCAGGCGCTCTGACCAGCGGCGTG
CACACCTTCCCAGCTGTCCTACAGTCCTCAGGACTCTACTCCCTCAGCAGCGTGGTGACCGTGCCC
TCCAGCAACTTCGGCACCCAGACCTACACCTGCAACGTAGATCACAAGCCCAGCAACACCAAGGTG
GACAAGACAGTTGAGCGCAAATGTTGTGTCGAGTGCCCACCGTGCCCAGCACCACCTGTGGCAGGA
CCGTCAGTCTTCCTCTTCCCCCCAAAACCCAAGGACACCCTCATGATCTCCCGGACCCCTGAGGTC
ACGTGCGTGGTGGTGGACGTGAGCCACGAAGACCCCGAGGTCCAGTTCAACTGGTACGTGGACGGC
GTGGAGGTGCATAATGCCAAGACAAAGCCACGGGAGGAGCAGTTCAACAGCACGTTCCGTGTGGTC
AGCGTCCTCACCGTTGTGCACCAGGACTGGCTGAACGGCAAGGAGTACAAGTGCAAGGTCTCCAAC
AAAGGCCTCCCAGCCCCCATCGAGAAAACCATCTCCAAAACCAAAGGGCAGCCCCGAGAACCACAG
GTGTACACCCTGCCCCCATCCCGGGAGGAGATGACCAAGAACCAGGTCAGCCTGACCTGCCTGGTC
AAAGGCTTCTACCCCAGCGACATCGCCGTGGAGTGGGAGAGCAATGGGCAGCCGGAGAACAACTAC
AAGACCACACCTCCCATGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGAGGCTCTGCACAACCAC
TACACGCAGAAGAGCCTCTCCCTGTCTCCGGGTAAATGA

Protein Sequence Defining the Full Length Heavy Chain Sequence of 5.6.H2 (SEQ ID NO: 141)

QVQLVESGGGLFKPGGSLRLSCAASGFTFSDYYMSWIRQAPGKGLEWISYISSSGRTIKYADSVKG
RFTISRDNAKNSLYLQMNSLRAEDTAVYYCARDGGYCGSANCLHDAFDIWGQGTMVTVSSASTKGP
SVFPLAPCSRSTSESTAALGCLVKDYFPEPVTVSWNSGALTSGVHTFPAVLQSSGLYSLSSVVTVP
SSNFGTQTYTCNVDHKPSNTKVDKTVERKCCVECPPCPAPPVAGPSVFLFPPKPKDTLMISRTPEV
TCVVVDVSHEDPEVQFNWYVDGVEVHNAKTKPREEQFNSTFRVVSVLTVVHQDWLNGKEYKCKVSN
KGLPAPIEKTISKTKGQPREPQVYTLPPSREEMTKNQVSLTCLVKGFYPSDIAVEWESNGQPENNY
KTTPPMLDSDGSFFLYSKLTVDKSRWQQGNVFSCSVMHEALHNHYTQKSLSLSPGK

Nucleic Acid Sequence Encoding the Full Length Kappa Light Chain Sequence of 5.6.H2 (SEQ ID NO: 142)

GACATCCAGATGACCCAGTCTCCTTCCACCCTGTCTGCATCTGTAGGAGACAGAGTCACCATCACT
TGCCGGGCCAGTCAGAGTATTAGTAGCTGGTTGGCCTGGTATCAGCAGAAACCAGGGAAAGCCCCT
AAGCTCCTGATCTATAAGGCGTCTAGTTTAGAAAGTGGGGTCCCATCAAGGTTCAGCGGCAGTGGA
TCTGGGACAGAATTCACTCTCACCATCAGCAGCCTGCAGCCTGATGATTTTGCAACTTATTACTGC
CAACAGTATAATAGTTATTCGACGTTCGGCCAAGGGACCAAGGTGGAAATCAAACGAACTGTGGCT
GCACCATCTGTCTTCATCTTCCCGCCATCTGATGAGCAGTTGAAATCTGGAACTGCCTCTGTTGTG
TGCCTGCTGAATAACTTCTATCCCAGAGAGGCCAAAGTACAGTGGAAGGTGGATAACGCCCTCCAA
TCGGGTAACTCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGT

Protein Sequence Defining the Full Length Kappa Light Chain Sequence of 5.6.H2 (SEQ ID NO: 143)

DIQMTQSPSTLSASVGDRVTITCRASQSISSWLAWYQQKPGKAPKLLIYKASSLESGVPSRFSGSG
SGTEFTLTISSLQPDDFATYYCQQYNSYSTFGQGTKVEIKRTVAAPSVFIFPPSDEQLKSGTASVV
CLLNNFYPREAKVQWKVDNALQSGNSQESVTEQDSKDSTYSLSSTLTLSKADYEKHKVYACEVTHQ
GLSSPVTKSFNRGEC

Nucleic Acid Sequence Encoding the Full Length Heavy Chain Sequence of 5.6.H9 (SEQ ID NO: 144)

CAGGTCACCTTGAAGGAGTCTGGTCCCACGCTGGTGAAACCCACACAGACCCTCACGCTGACGTGC
ACCTTCTCTGGGTTTTCGCTCAGCACTAGTGGAGAGGGTGTGGCCTGGATCCGTCAGGCCCCAGGA
AAGGCCCTGGAGTGGCTTGCACTCATTTATTGGGATGATGATAAGCGCTACAACCCATCTCTGAAG
AGCAGGCTCACCATCACCAAGGACAACTCCAGAAACCAAGTGGTCCTTACAATGACCAACATGGAC

CCTGTGGACACAGCCACATATTACTGTGTACACAGGGGATGAATAGGGTTTTGGGGTAGTTTAT
AATTCGGCTGGTTCGACCCCTGGGGCCAGGGAACCCTGGTCACCGTCTCGAGTGCCTCCACCAAG
GGCCCATCGGTCTTCCCCCTGGCGCCCTGCTCCAGGAGCACCTCCGAGAGCACAGCCGCCCTGGGC
TGCCTGGTCAAGGACTACTTCCCCGAACCGGTGACGGTGTCGTGGAACTCAGGCGCTCTGACCAGC
GGCGTGCACACCTTCCCAGCTGTCCTACAGTCCTCAGGACTCTACTCCCTCAGCAGCGTGGTGACC
GTGCCCTCCAGCAACTTCGGCACCCAGACCTACACCTGCAACGTAGATCACAAGCCCAGCAACACC
AAGGTGGACAAGACAGTTGAGCGCAAATGTTGTGTCGAGTGCCCACCGTGCCCAGCACCACCTGTG
GCAGGACCGTCAGTCTTCCTCTTCCCCCCAAAACCCAAGGACACCCTCATGATCTCCCGGACCCCT
GAGGTCACGTGCGTGGTGGTGGACGTGAGCCACGAAGACCCCGAGGTCCAGTTCAACTGGTACGTG
GACGGCGTGGAGGTGCATAATGCCAAGACAAAGCCACGGGAGGAGCAGTTCAACAGCACGTTCCGT
GTGGTCAGCGTCCTCACCGTTGTGCACCAGGACTGGCTGAACGGCAAGGAGTACAAGTGCAAGGTC
TCCAACAAAGGCCTCCCAGCCCCCATCGAGAAAACCATCTCCAAAACCAAAGGGCAGCCCCGAGAA
CCACAGGTGTACACCCTGCCCCCATCCCGGGAGGAGATGACCAAGAACCAGGTCAGCCTGACCTGC
CTGGTCAAAGGCTTCTACCCCAGCGACATCGCCGTGGAGTGGGAGAGCAATGGGCAGCCGGAGAAC
AACTACAAGACCACACCTCCCATGCTGGACTCCGACGGCTCCTTCTTCCTCTACAGCAAGCTCACC
GTGGACAAGAGCAGGTGGCAGCAGGGGAACGTCTTCTCATGCTCCGTGATGCATGAGGCTCTGCAC
AACCACTACACGCAGAAGAGCCTCTCCCTGTCTCCGGGTAAATGA

Protein Sequence Defining the Full Length Heavy Chain Sequence of 5.6.H9 (SEQ ID NO: 145)

QVTLKESGPTLVKPTQTLTLTCTFSGFSLSTSGEGVAWIRQAPGKALEWLALIYWDDDKRYNPSLK
SRLTITKDNSRNQVVLTMTNMDPVDTATYYCVHRGMNRVFGVVYNSGWFDPWGQGTLVTVSSASTK
GPSVFPLAPCSRSTSESTAALGCLVKDYFPEPVTVSWNSGALTSGVHTFPAVLQSSGLYSLSSVVT
VPSSNFGTQTYTCNVDHKPSNTKVDKTVERKCCVECPPCPAPPVAGPSVFLFPPKPKDTLMISRTP
EVTCVVVDVSHEDPEVQFNWYVDGVEVHNAKTKPREEQFNSTFRVVSVLTVVHQDWLNGKEYKCKV
SNKGLPAPIEKTISKTKGQPREPQVYTLPPSREEMTKNQVSLTCLVKGFYPSDIAVEWESNGQPEN
NYKTTPPMLDSDGSFFLYSKLTVDKSRWQQGNVFSCSVMHEALHNHYTQKSLSLSPGK

Nucleic Acid Sequence Encoding the Full Length Kappa Light Chain Sequence of 5.6.H9 (SEQ ID NO: 146)

GAAATTGTGTTGACGCAGTCTCCAGGCACCCTGTCTTTGTCTCCAGGGGAAAGAGCCACCCTCTCC
TGTAGGGCCAGTCAGAGCGTTAGAAGCAGCCAGCTAGCCTGGTACCAGCAGAAGCCTGGCCAGGCT
CCCAGCCTCCTCATGTATGACGCATCTAAGAGGGCCACTGGCATCCCAGACAGGTTCAGTGGCAGT
GGGTCTGGGACAGACTTCACTCTCACCATCAGCAGACTGGAGCCTGAAGATTTTGTAGTGTATTTC
TGTCAGCAGTATGATACATCACCGTGGACGTTCGGCCAAGGGACCAAGGTGGAAATCAAACGAACT
GTGGCTGCACCATCTGTCTTCATCTTCCCGCCATCTGATGAGCAGTTGAAATCTGGAACTGCCTCT
GTTGTGTGCCTGCTGAATAACTTCTATCCCAGAGAGGCCAAAGTACAGTGGAAGGTGGATAACGCC
CTCCAATCGGGTAACTCCCAGGAGAGTGTCACAGAGCAGGACAGCAAGGACAGCACCTACAGCCTC
AGCAGCACCCTGACGCTGAGCAAAGCAGACTACGAGAAACACAAAGTCTACGCCTGCGAAGTCACC
CATCAGGGCCTGAGCTCGCCCGTCACAAAGAGCTTCAACAGGGGAGAGTGT

Protein Sequence Defining the Full Length Kappa Light Chain Sequence of 5.6.H9 (SEQ ID NO: 147)

EIVLTQSPGTLSLSPGERATLSCRASQSVRSSQLAWYQQKPGQAPSLLMYDASKRATGIPDRFSGS
GSGTDFTLTISRLEPEDFVVYFCQQYDTSPWTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 8.51.G10 (SEQ ID NO: 310)

CAGATCACCTTGAAGGAGTCTGGTCCTACGCTGGTGAACCCACACAGACCCTCACGCTGACCTGC
ACCTTTTCTGGGTTCTCATTCACCTCTAGTGGAGAGGCTGTGGCTGGATCCGTCAGCCCCCAGGA
AAGGCCCTGGAATGGCTTGCTCACATTTATTGGATGATGATCCGCGTTATAGTACATCTCTGAAG
AGCAGACTCACCATCACCAAGGACACCTCCAAAAATCAGGTGGTCCTCTCTATGACCGACATTGAC
CCTGCGGACACAGGCACATATTACTGTGCACACCTGAGACGCCCATCCCTTTGGAGTCCTTATTAT
CTTTTTGACTGCTGGGGCCAGGGAACCCTGGTCACCGTCTCGAGTGCCTCCACCAAGGGCCCATCG
GTCTTCCCCCTGGCGCCCTGCTCCAGGAGCACCTCCGAGAGCACAGCCGCCCTGGGCTGCCTGGTC
AAGGACTACTTCCCCGAACCGGTGACGGTGTCGTGGAACTCAGGCGCTCTGACCAGCGGCGTGCAC
ACCTTCCCAGCTGTCCTACAGTCCTCAGGACTCTACTCCCTCAGCAGCGTGGTGACCGTGCCCTCC
AGCAACTTCGGCACCCAGACCTACACCTGCAACGTAGATCACAAGCCCAGCAACACCAAGGTGGAC
AAGACAGTTGAGCGCAAATGTTGTGTCGAGTGCCCACCGTGCCCAGCACCACCTGTGGCAGGACCG
TCAGTCTTCCTCTTCCCCCCAAAACCCAAGGACACCCTCATGATCTCCCGGACCCCTGAGGTCACG
TGCGTGGTGGTGGACGTGAGCCACGAAGACCCCGAGGTCCAGTTCAACTGGTACGTGGACGGCGTG
GAGGTGCATAATGCCAAGACAAAGCCACGGGAGGAGCAGTTCAACAGCACGTTCCGTGTGGTCAGC
GTCCTCACCGTTGTGCACCAGGACTGGCTGAACGGCAAGGAGTACAAGTGCAAGGTCTCCAACAAA
GGCCTCCCAGCCCCCATCGAGAAAACCATCTCCAAAACCAAAGGGCAGCCCCGAGAACCACAGGTG
TACACCCTGCCCCCATCCCGGGAGGAGATGACCAAGAACCAGGTCAGCCTGACCTGCCTGGTCAAA
GGCTTCTACCCCAGCGACATCGCCGTGGAGTGGGAGAGCAATGGGCAGCCGGAGAACAACTACAAG
ACCACACCTCCCATGCTGGACTCCGACGGCTCCTTCTTCCTCTACAGCAAGCTCACCGTGGACAAG
AGCAGGTGGCAGCAGGGGAACGTCTTCTCATGCTCCGTGATGCATGAGGCTCTGCACAACCACTAC
ACGCAGAAGAGCCTCTCCCTGTCTCCGGGTAAA

Protein Sequence Defining the Full Length Heavy Chain Sequence of 8.51.G10 (SEQ ID NO: 311)

QITLKESGPTLVNPTQTLTLTCTFSGFSFTSSGEAVGWIRQPPGKALEWLAHIYWDDDPRYSTSLK
SRLTITKDTSKNQVVLSMTDIDPADTGTYYCAHLRRPSLWSPYYLFDCWGQGTLVTVSSSTKGPSV
FPLAPCSRSTSESTAALGCLVKDYFPEPVTVSWNSGALTSGVHTFPAVLQSSGLYSLSSVVTVPSS
NFGTQTYTCNVDHKPSNTKVDKTVERKCCVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKG
LPAPIEKTISKTKGQPREPQVYTLPPSREEMTKNQVSLTCLVKGFYPSDIAVEWESNGQPENNYKT
TPPMLDSDGSFFLYSKLTVDKSRWQQGNVFSCSVMHEALHNHYTQKSLSLSPGK

Nucleic Acid Sequence Encoding the Full Length Kappa Light Chain Sequence of 8.51.G10 (SEQ ID NO: 312)

GACATCCAGATGACCCAGTCTCCATCCTCCCTGTCTGCATCTGTTGGAGACAGAGTCACCATCACT
TGCCGGGCAAGTCAGAGTATTAGTAGCTATTTAAATTGGTATCAACTGAAACCAGGACAGCCCCT
AAGCTCCTGATCTATGGTGCATCCAGTTTGGAAAATGGGGTCCCTTCACGGTTCAGTGGCAGTGGC
TCTGGGCCAGATTTCACTCTCACCATTAGCAGTCTGCAACCTGAAGATTTTGCAACTTACTACTGT
CAACAGAGTTACAAAATGTGGTCATTCGGCCAAGGGACCAAGGTGGAAATCCAACGAACTGTGGCT
GCACCATCTGTCTTCATCTTCCCGCCATCTGATGAGCAGTTGAAATCTGGAACTGCCTCTGTTGTG
TGCCTGCTGAATAACTTCTATCCCAGAGAGGCCAAAGTACAGTGGAAGGTGGATAACGCCCTCCAA
TCGGGTAACTCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGT

Protein Sequence Defining the Full Length Kappa Light Chain Sequence of 8.51.G10 (SEQ ID NO: 313)

DIQMTQSPSSLSASVGDRVTITCRASQSISSYLNWYQLKPGQAPKLLIYGASSLENGVPSRFSGSG
SGPDFTLTISSLQPEDFATYYCQQSYKMWSFGQGTKVEIQRTVAAPSVFIFPPSDEQLKSGTASVV
CLLNNFYPREAKVQWKVDNALQSGNSQESVTEQDSKDSTYSLSSTLTLSKADYEKHKVYACEVTHQ
GLSSPVTKSFNRGEC

Nucleic Acid Sequence Encoding the Full Length Heavy Chain Sequence of 5.24A.A7 (SEQ ID NO: 314)

CAGGTTCAGCTGGTGCAGTCTGGAGTTGAGGTGAAGAAGCCTGGGGCCTCAGTGAAGGTC
TCCTGCAAGGCTTCTGGCTACACCTTTACCAACTTTGGCTTCAGTTGGGTGCGGCAGGCC
CCAGGACAAGGGCTTGAGTGGCTGGGATGGATCAACACTCATAACGGTAACACAAACTAT
GCACCGAGATTCCAAGACAGAGTCACCCTGACCACAGACACATCCACGACCACAGCCTAC
ATGGAGCTGAGGAGCCTGAGATCTGAGGACACGGCCGTGTATTATTGTGCGAGAGATCTT
GGGGGAAGTAGTTCCGCCTACTGGGGACAGGGAACCCTGGTCACCGTCTCGAGTGCCTCCACCAAG
GGCCCATCGGTCTTCCCCCTGGCGCCCTGCTCCAGGAGCACCTCCGAGAGCACAGCCGCCCTGGGC
TGCCTGGTCAAGGACTACTTCCCCGAACCGGTGACGGTGTCGTGGAACTCAGGCGCTCTGACCAGC
GGCGTGCACACCTTCCCAGCTGTCCTACAGTCCTCAGGACTCTACTCCCTCAGCAGCGTGGTGACC
GTGCCCTCCAGCAACTTCGGCACCCAGACCTACACCTGCAACGTAGATCACAAGCCCAGCAACACC
AAGGTGGACAAGACAGTTGAGCGCAAATGTTGTGTCGAGTGCCCACCGTGCCCAGCACCACCTGTG
GCAGGACCGTCAGTCTTCCTCTTCCCCCCAAAACCCAAGGACACCCTCATGATCTCCCGGACCCCT
GAGGTCACGTGCGTGGTGGTGGACGTGAGCCACGAAGACCCCGAGGTCCAGTTCAACTGGTACGTG
GACGGCGTGGAGGTGCATAATGCCAAGACAAAGCCACGGGAGGAGCAGTTCAACAGCACGTTCCGT
GTGGTCAGCGTCCTCACCGTTGTGCACCAGGACTGGCTGAACGGCAAGGAGTACAAGTGCAAGGTC

TCCAACAAAGGCCTCCCAGCCCCCATCGAGAAAACCATCTCCAAAACCAAAGGGCAGCCCCGAGAA
CCACAGGTGTACACCCTGCCCCCATCCCGGGAGGAGATGACCAAGAACCAGGTCAGCCTGACCTGC
CTGGTCAAAGGCTTCTACCCCAGCGACATCGCCGTGGAGTGGGAGAGCAATGGGCAGCCGGAGAAC
AACTACAAGACCACACCTCCCATGCTGGACTCCGACGGCTCCTTCTTCCTCTACAGCAAGCTCACC
GTGGACAAGAGCAGGTGGCAGCAGGGGAACGTCTTCTCATGCTCCGTGATGCATGAGGCTCTGCAC
AACCACTACACGCAGAAGAGCCTCTCCCTGTCTCCGGGTAAA

Protein Sequence Defining the Full Length Heavy Chain Sequence of 5.24A.A7 (SEQ ID NO: 315)

QVQLVQSGVEVKKPGASVKVSCKASGYTFTNFGFSWVRQAPGQGLEWLGWINTHNGNTNYAPRFQD
RVTLTTDTSTTTAYMELRSLRSEDTAVYYCARDLGGSSSAYWGQGTLVTVSSSTKGPSVFPLAPCS
RSTSESTAALGCLVKDYFPEPVTVSWNSGALTSGVHTFPAVLQSSGLYSLSSVVTVPSSNFGTQTY
TCNVDHKPSNTKVDKTVERKCCVECPPCPAPPVAGPSVFLFPPKPKDTLMISRTPEVTCVVVDVSH
EDPEVQFNWYVDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYKTTPPMLDS
DGSFFLYSKLTVDKSRWQQGNVFSCSVMHEALHNHYTQKSLSLSPGK

Nucleic Acid Sequence Encoding the Full Length Kappa Light Chain Sequence of 5.24A.A7 (SEQ ID NO: 316)

GAAATTGTGTTGACGCAGTCTCCAGGCACCCTGTCTTTGTCTCCAGGGGAGAGAGCCACC
CTCTCTTGCAGGGCCAGTCACAGTGGTACCAACAACTACATCGCCTGGTACCAGCAGAAA
CCTGGCCAGCCTCCCAGGCTCCTCATCTATGGTGCATCCAGCAGGGCCCCTGGCATCTCA
GAGAGGTTCAGCGGCAGTGGGTCTGGGACAGACTTCACTCTCATCATCAGCAGACTGGAG
CCTGAAGACTTTGGAGTATATTACTGTGAGCAGTATGGGAACTCACCTCTGACGTTCGGC
CAAGGGACCAAGGTGGAAATCAAACGAACTGTGGCTGCACCATCTGTCTTCATCTTCCCG
CCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCC
CAGGAGAGTGTCACAGAGCAGGACAGCAAGGACAGCACCTACAGCCTCAGCAGCACCCTG
ACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGT

Protein Sequence Defining the Full Length Kappa Light Chain Sequence of 5.24A.A7 (SEQ ID NO: 317)

EIVLTQSPGTLSLSPGERATLSCRASHSGTNNYIAWYQQKPGQPPRLLIYGASSRAPGISERFSGS
GSGTDFTLIISRLEPEDFGVYYCEQYGNSPLTFGQGTKVEIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 5.24A.F3 (SEQ ID NO: 318)

CAGGTGCAGCTGCAGGAGTCGGGCCCAGGACTGGTGGAGTCTTCGGACGTCCTGTCCCTC
ACCTGCACTGTCTCTGGTGGCTCCATCAGTAATGACTATTGGGCCTGGATCCGGCAGACC
CCAGGGAAGGGACTGGAGTGGATTGGGTCTGTCTATTACAGTGGCAGAACCAGTTTCAGC
CCCTCCCTCAAGAGTCGAGTCACTATGTCAGTGGACACGACCAAGAACCAATTCTTCCTG
AACCTGGGCTCTCTGACCGTTGCGGACACGGCCGTGTATTACTGTGCGAGAACTACAAGG
TTGTCCTTTGACTGGTTAGTACACTACTATGGAATGGACGTCTGGGGCCAGGGGACCACG
GTCACCGTCTCGAGTGCCTCCACCAAGGGCCCATCGGTCTTCCCCCTGGCGCCCTGCTCCAGGAGC
ACCTCCGAGAGCACAGCCGCCCTGGGCTGCCTGGTCAAGGACTACTTCCCCGAACCGGTGACGGTG
TCGTGGAACTCAGGCGCTCTGACCAGCGGCGTGCACACCTTCCCAGCTGTCCTACAGTCCTCAGGA
CTCTACTCCCTCAGCAGCGTGGTGACCGTGCCCTCCAGCAACTTCGGCACCCAGACCTACACCTGC
AACGTAGATCACAAGCCCAGCAACACCAAGGTGGACAAGACAGTTGAGCGCAAATGTTGTGTCGAG
TGCCCACCGTGCCCAGCACCACCTGTGGCAGGACCGTCAGTCTTCCTCTTCCCCCCAAAACCCAAG
GACACCCTCATGATCTCCCGGACCCCTGAGGTCACGTGCGTGGTGGTGGACGTGAGCCACGAAGAC
CCCGAGGTCCAGTTCAACTGGTACGTGGACGGCGTGGAGGTGCATAATGCCAAGACAAAGCCACGG
GAGGAGCAGTTCAACAGCACGTTCCGTGTGGTCAGCGTCCTCACCGTTGTGCACCAGGACTGGCTG
AACGGCAAGGAGTACAAGTGCAAGGTCTCCAACAAAGGCCTCCCAGCCCCCATCGAGAAAACCATC
TCCAAAACCAAAGGGCAGCCCCGAGAACCACAGGTGTACACCCTGCCCCCATCCCGGGAGGAGATG
ACCAAGAACCAGGTCAGCCTGACCTGCCTGGTCAAAGGCTTCTACCCCAGCGACATCGCCGTGGAG
TGGGAGAGCAATGGGCAGCCGGAGAACAACTACAAGACCACACCTCCCATGCTGGACTCCGACGGC
TCCTTCTTCCTCTACAGCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTCTCCCTGTCTCCGGGT
AAA

Protein Sequence Defining the Full Length Heavy Chain Sequence of 5.24A.F3 (SEQ ID NO: 319)

QVQLQESGPGLVESSDVLSLTCTVSGGSISNDYWAWIRQTPGKGLEWIGSVYYSGRTSFSPSLKSR
VTMSVDTTKNQFFLNLGSLTVADTAVYYCARTTRLSFDWLVHYYGMDVWGQGTTVTVSSSTKGPSV
FPLAPCSRSTSESTAALGCLVKDYFPEPVTVSWNSGALTSGVHTFPAVLQSSGLYSLSSVVTVPSS
NFGTQTYTCNVDHKPSNTKVDKTVERKCCVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKG
LPAPIEKTISKTKGQPREPQVYTLPPSREEMTKNQVSLTCLVKGFYPSDIAVEWESNGQPENNYKT
TPPMLDSDGSFFLYSKLTVDKSRWQQGNVFSCSVMHEALHNHYTQKSLSLSPGK

Nucleic Acid Sequence Encoding the Full Length Kappa Light Chain Sequence of 5.24A.F3 (SEQ ID NO: 320)

GACATCCAGATGACCCAGTCTCCATCGTCCCTGACTGCATCTGTAGGAGACAGAGTCACC
ATTATTTGTCGGGCAAGTCAGCCCATTGGCAGCTATTTGAATTGGTATCAGCAGAGTCCA
GGGAAAGCCCCTAAACTCCTCATCTATGCTGCATCCACTTTGCAGAGTGGGGTCCCATCA
AGGTTCAGTGGCAGTGGATCTGGGACAGATTTCACTCTCAACATCAGTGGTCTGCAACCT
GAAGATTTTGCAACTTACTACTGTCAACAGAGTTACAATAACCCTTTGTTCACCTTCGGC
CCTGGGACCAAAGTGGATATCAAACGAACTGTGGCTGCACCATCTGTCTTCATCTTCCCG
CCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCC
CAGGAGAGTGTCACAGAGCAGGACAGCAAGGACAGCACCTACAGCCTCAGCAGCACCCTG
ACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGT

Protein Sequence Defining the Full Length Kappa Light Chain Sequence of 5.24A.F3 (SEQ ID NO: 321)

DIQMTQSPSSLTASVGDRVTIICRASQPIGSYLNWYQQSPGKAPKLLIYAASTLQSGVPSRFSGSG
SGTDFTLNISGLQPEDFATYYCQQSYNNPLFTFGPGTKVDIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 5.8B.H4 (SEQ ID NO: 322)

GAGGTGCAGCTGGTGGAGTCTGGGGGAAGTCTGGTACGGCCGGGGGGTCCCTGCGACTC
TCCTGTGCAGCCTCTGGATTCATCTTTGATGATTATGGCATGAGTTGGGTCCGCCAAGTT
CCAGGGGAGGGCCTGGAGTGGGTCGCCGGCATTACTTGGATGGCGTGAACACAGATTAT
GCAGACTCCGTGAAGGGCCGCTTCACCATCTCCAGAGACAACGCCAAGAACTCCCTGTTT
CTCCTAATGAACAGTCTCAGAGCCGAGGACACGGCCCTGTATCACTGTGCGAGAGCCAGA
TACTACCAAGGCACTGGCCTCCGGTCCTACCTCGACGAGTGGGGCCAGGGAACCCTGGTC
ACCGTCTCGAGTGCCTCCACCAAGGGCCCATCGGTCTTCCCCCTGGCGCCCTGCTCCAGGAGCACC
TCCGAGAGCACAGCCGCCCTGGGCTGCCTGGTCAAGGACTACTTCCCCGAACCGGTGACGGTGTCG
TGGAACTCAGGCGCTCTGACCAGCGGCGTGCACACCTTCCCAGCTGTCCTACAGTCCTCAGGACTC
TACTCCCTCAGCAGCGTGGTGACCGTGCCCTCCAGCAACTTCGGCACCCAGACCTACACCTGCAAC
GTAGATCACAAGCCCAGCAACACCAAGGTGGACAAGACAGTTGAGCGCAAATGTTGTGTCGAGTGC
CCACCGTGCCCAGCACCACCTGTGGCAGGACCGTCAGTCTTCCTCTTCCCCCCAAAACCCAAGGAC
ACCCTCATGATCTCCCGGACCCCTGAGGTCACGTGCGTGGTGGTGGACGTGAGCCACGAAGACCCC
GAGGTCCAGTTCAACTGGTACGTGGACGGCGTGGAGGTGCATAATGCCAAGACAAAGCCACGGGAG
GAGCAGTTCAACAGCACGTTCCGTGTGGTCAGCGTCCTCACCGTTGTGCACCAGGACTGGCTGAAC
GGCAAGGAGTACAAGTGCAAGGTCTCCAACAAAGGCCTCCCAGCCCCCATCGAGAAAACCATCTCC

AAAACCAAAGGGCAGCCCCGAGAACCACAGGTGTACACCCTGCCCCCATCCCGGGAGGAGATGACC
AAGAACCAGGTCAGCCTGACCTGCCTGGTCAAAGGCTTCTACCCCAGCGACATCGCCGTGGAGTGG
GAGAGCAATGGGCAGCCGGAGAACAACTACAAGACCACACCTCCCATGCTGGACTCCGACGGCTCC
TTCTTCCTCTACAGCAAGCTCACCGTGGACAAGAGCAGGTGGCAGCAGGGGAACGTCTTCTCATGC
TCCGTGATGCATGAGGCTCTGCACAACCACTACACGCAGAAGAGCCTCTCCCTGTCTCCGGGTAAA

Protein Sequence Defining the Full Length Heavy Chain Sequence of 5.8B.H4 (SEQ ID NO: 323)

EVQLVESGGSLVRPGGSLRLSCAASGFIFDDYGMSWVRQVPGEGLEWVAGITWDGVNTDYADSVKG
RFTISRDNAKNSLFLLMNSLRAEDTALYHCARARYYQGTGLRSYLDEWGQGTLVTVSSSTKGPSVF
PLAPCSRSTSESTAALGCLVKDYFPEPVTVSWNSGALTSGVHTFPAVLQSSGLYSLSSVVTVPSSN
FGTQTYTCNVDHKPSNTKVDKTVERKCCVECPPCPAPPVAGPSVFLFPPKPKDTLMISRTPEVTCV
VVDVSHEDPEVQFNWYVDGVEVHNAKTKPREEQFNSTFRVVSVLTVVHQDWLNGKEYKCKVSNKGL
PAPIEKTISKTKGQPREPQVYTLPPSREEMTKNQVSLTCLVKGFYPSDIAVEWESNGQPENNYKTT
PPMLDSDGSFFLYSKLTVDKSRWQQGNVFSCSVMHEALHNHYTQKSLSLSPGK

Nucleic Acid Sequence Encoding the Full Length Kappa Light Chain Sequence of 5.8B.H4 (SEQ ID NO: 324)

GACATCCAGATGACCCAGTCTCCATCCTCCGTGTCTGCATCTGTAGGAGAAAGAGTCACT
ATCACTTGTCGGGCGACTCAGGGTATTGACAACTGGTTGGCCTGGTATCAGCATAAACCA
GGGAAAGCCCCGAAGCTCCTGATTTATGCTGCATCCACTTTGCAAAGTGGGGTCCCGTCC
AGGTTCAGCGGCAGTGGATCTGGGACAGATTTCACTCTCACCATCAGCAGCGTGCAGCCT
GAAGATGTTGCAACTTACTTTTGTCAACAGGCTAAGAATTTCCCTCGGGGAGGATTCACT
TTCGGCCCTGGGACCAAAGTCGCTCTCAAACGAACTGTGGCTGCACCATCTGTCTTCATC
TTCCCGCCATCTGATGAGCAGTTGAAATCTGGAACTGCCTCTGTTGTGTGCCTGCTGAAT
AACTTCTATCCCAGAGAGGCCAAAGTACAGTGGAAGGTGGATAACGCCCTCCAATCGGGT
AACTCCCAGGAGAGTGTCACAGAGCAGGACAGCAAGGACAGCACCTACAGCCTCAGCAGC
ACCCTGACGCTGAGCAAAGCAGACTACGAGAAACACAAAGTCTACGCCTGCGAAGTCACC
CATCAGGGCCTGAGCTCGCCCGTCACAAAGAGCTTCAACAGGGGAGAGTGT

Protein Sequence Defining the Full Length Kappa Light Chain Sequence of 5.8B.H4 (SEQ ID NO: 325)

DIQMTQSPSSVSASVGERVTITCRATQGIDNWLAWYQHKPGKAPKLLIYAASTLQSGVPSRFSGSG
SGTDFTLTISSVQPEDVATYFCQQAKNFPRGGFTFGPGTKVALKRTVAAPSVFIFPPSDEQLKSGT
ASVVCLLNNFYPREAKVQWKVDNALQSGNSQESVTEQDSKDSTYSLSSTLTLSKADYEKHKVYACE
VTHQGLSSPVTKSFNRGEC

Nucleic Acid Sequence Encoding the Full Length Heavy Chain Sequence of 26.51.E1 (SEQ ID NO: 326)

CAGGTGCAGCTGGTGGAGTCTGGGGGAGGCGTGGTCCAGCCTGGGAGGTCCCTGAGACTCTCCTGT
GCAGCCTCTGGATTCACCTTCAGTAAATTTGCCATGCACTGGGTCCGCCAGGCTCCAGGCAAGGGG
CTGGAGTGGGTGGCAGCTATAGCGTCTGATGGATATAATAAATACTATGCAGACTCCGTGAAGGGC
CAATTCACCGTCTCCAGAGACACTTCCAGGAACACGCTGTATCTCCAAATGGACAGCCTGAGACCT
GATGACACGGCTATTTATTACTGTGCGTACGGTCACTTGTCTTTTTGGTTCGACCCCTGGGGCCAG
GGAACCCTGGTCACCGTCTCGAGTGCCTCCACCAAGGGCCCATCGGTCTTCCCCCTGGCGCCCTGC
TCCAGGAGCACCTCCGAGAGCACAGCCGCCCTGGGCTGCCTGGTCAAGGACTACTTCCCCGAACCG
GTGACGGTGTCGTGGAACTCAGGCGCTCTGACCAGCGGCGTGCACACCTTCCCAGCTGTCCTACAG
TCCTCAGGACTCTACTCCCTCAGCAGCGTGGTGACCGTGCCCTCCAGCAACTTCGGCACCCAGACC
TACACCTGCAACGTAGATCACAAGCCCAGCAACACCAAGGTGGACAAGACAGTTGAGCGCAAATGT
TGTGTCGAGTGCCCACCGTGCCCAGCACCACCTGTGGCAGGACCGTCAGTCTTCCTCTTCCCCCCA
AAACCCAAGGACACCCTCATGATCTCCCGGACCCCTGAGGTCACGTGCGTGGTGGTGGACGTGAGC
CACGAAGACCCCGAGGTCCAGTTCAACTGGTACGTGGACGGCGTGGAGGTGCATAATGCCAAGACA
AAGCCACGGGAGGAGCAGTTCAACAGCACGTTCCGTGTGGTCAGCGTCCTCACCGTTGTGCACCAG
GACTGGCTGAACGGCAAGGAGTACAAGTGCAAGGTCTCCAACAAAGGCCTCCCAGCCCCCATCGAG
AAAACCATCTCCAAAACCAAAGGGCAGCCCCGAGAACCACAGGTGTACACCCTGCCCCCATCCCGG
GAGGAGATGACCAAGAACCAGGTCAGCCTGACCTGCCTGGTCAAAGGCTTCTACCCCAGCGACATC
GCCGTGGAGTGGGAGAGCAATGGGCAGCCGGAGAACAACTACAAGACCACACCTCCCATGCTGGAC
TCCGACGGCTCCTTCTTCCTCTACAGCAAGCTCACCGTGGACAAGAGCAGGTGGCAGCAGGGGAAC
GTCTTCTCATGCTCCGTGATGCATGAGGCTCTGCACAACCACTACACGCAGAAGAGCCTCTCCCTG
TCTCCGGGTAAA

Protein Sequence Defining the Full Length Heavy Chain Sequence of 26.51.E1 (SEQ ID NO: 327)

QVQLVESGGGVVQPGRSLRLSCAASGFTFSKFAMHWVRQAPGKGLEWVAAIASDGYNKYYADSVKG
QFTVSRDTSRNTLYLQMDSLRPDDTAIYYCAYGHLSFWFDPWGQGTLVTVSSSTKGPSVFPLAPCS
RSTSESTAALGCLVKDYFPEPVTVSWNSGALTSGVHTFPAVLQSSGLYSLSSVVTVPSSNFGTQTY
TCNVDHKPSNTKVDKTVERKCCVECPPCPAPPVAGPSVFLFPPKPKDTLMISRTPEVTCVVVDVSH
EDPEVQFNWYVDGVEVHNAKTKPREEQFNSTFRVVSVLTVVHQDWLNGKEYKCKVSNKGLPAPIEK
TISKTKGQPREPQVYTLPPSREEMTKNQVSLTCLVKGFYPSDIAVEWESNGQPENNYKTTPPMLDS
DGSFFLYSKLTVDKSRWQQGNVFSCSVMHEALHNHYTQKSLSLSPGK

Nucleic Acid Sequence Encoding the Full Length Kappa Light Chain Sequence of 26.51.E1 (SEQ ID NO: 328)

GACATCCAGATGACCCAGTCTCCATCCTCCCTGTCCGCATCTGTAGGAGACAGAGTCACCA
TCACTTGCCGGGCAAGTCAGGACATTGGAAATGGCTTAGGCTGGTATCAACAGATACCAGG
GAAAGCCCCTAAGCGCCTGATCTATGCTGCATCCTCTTTGCAAAGTGGGGTCCCATCAAGG

TTCAGCGGCAGTGGTTCTGGGACAGAGTTCACTCTCACAATCCACAGCCTGCAGCCTGAAG
ATTTTGCAACTTATTACTGTCTACAGCATAATTTTACCCTCGCACTTTTGGCCAGGGGAC
CAAGCTGGAGATCAAGCGAACTGTGGCTGCACCATCTGTCTTCATCTTCCCGCCATCTGAT
GAGCAGTTGAAATCTGGAACTGCCTCTGTTGTGTGCCTGCTGAATAACTTCTATCCCAGAG
AGGCCAAAGTACAGTGGAAGGTGGATAACGCCCTCCAATCGGGTAACTCCCAGGAGAGTGT
CACAGAGCAGGACAGCAAGGACAGCACCTACAGCCTCAGCAGCACCCTGACGCTGAGCAAA
GCAGACTACGAGAAACACAAAGTCTACGCCTGCGAAGTCACCCATCAGGGCCTGAGCTCGC
CCGTCACAAAGAGCTTCAACAGGGGAGAGTGT

Protein Sequence Defining the Full Length Kappa Light Chain Sequence of 26.51.E1 (SEQ ID NO: 329)

DIQMTQSPSSLSASVGDRVTITCRASQDIGNGLGWYQQIPGKAPKRLIYAASSLQSGVPSRFSGSG
SGTEFTLTIHSLQPEDFATYYCLQHNFYPRTFGQGTKLEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC

Nucleic Acid Sequence Encoding the Full Length Heavy Chain Sequence of 22.21.A7 (SEQ ID NO: 330)

CAGGTGCAGCTGGTGCAGTCTGGGGCTGAGGTGAAGAAGCCTGGGGCCTCAGTGAAGGTCTCCTGC
AAGGCTTCTGGATACACCTTCACCGACTACTATATACACTGGGTGCGACAGGCCCCTGGACAAGGG
CTTGAGTGGATGGGATGGATCAACCCTTATAGTGGTGGCACAAACTATGCACCGAAGTTTCAGGGC
AGGTTCACCATGGCCAGGGACACGTCCATCACCACAACCTACTTGGACCTGGGCAGGCTGACGTCT
AGCGACACGGCCATTTATTTCTGTGCGAGAGATTTCTTCAGAGCAGCAGCTGGCACGGCCGGAGAC
TACTGGGGCCAGGGAACCCTGGTCACCGTCTCGAGTGCCTCCACCAAGGGCCCATCGGTCTTCCCC
CTGGCGCCCTGCTCCAGGAGCACCTCCGAGAGCACAGCCGCCCTGGGCTGCCTGGTCAAGGACTAC
TTCCCCGAACCGGTGACGGTGTCGTGGAACTCAGGCGCTCTGACCAGCGGCGTGCACACCTTCCCA
GCTGTCCTACAGTCCTCAGGACTCTACTCCCTCAGCAGCGTGGTGACCGTGCCCTCCAGCAACTTC
GGCACCCAGACCTACACCTGCAACGTAGATCACAAGCCCAGCAACACCAAGGTGGACAAGACAGTT
GAGCGCAAATGTTGTGTCGAGTGCCCACCGTGCCCAGCACCACCTGTGGCAGGACCGTCAGTCTTC
CTCTTCCCCCCAAAACCCAAGGACACCCTCATGATCTCCCGGACCCCTGAGGTCACGTGCGTGGTG
GTGGACGTGAGCCACGAAGACCCCGAGGTCCAGTTCAACTGGTACGTGGACGGCGTGGAGGTGCAT
AATGCCAAGACAAAGCCACGGGAGGAGCAGTTCAACAGCACGTTCCGTGTGGTCAGCGTCCTCACC
GTTGTGCACCAGGACTGGCTGAACGGCAAGGAGTACAAGTGCAAGGTCTCCAACAAAGGCCTCCCA
GCCCCCATCGAGAAAACCATCTCCAAAACCAAAGGGCAGCCCCGAGAACCACAGGTGTACACCCTG
CCCCCATCCCGGGAGGAGATGACCAAGAACCAGGTCAGCCTGACCTGCCTGGTCAAAGGCTTCTAC
CCCAGCGACATCGCCGTGGAGTGGGAGAGCAATGGGCAGCCGGAGAACAACTACAAGACCACACCT
CCCATGCTGGACTCCGACGGCTCCTTCTTCCTCTACAGCAAGCTCACCGTGGACAAGAGCAGGTGG
CAGCAGGGGAACGTCTTCTCATGCTCCGTGATGCATGAGGCTCTGCACAACCACTACACGCAGAAG
AGCCTCTCCCTGTCTCCGGGTAAA

Protein Sequence Defining the Full Length Heavy Chain Sequence of 22.21.A7 (SEQ ID NO: 331)

QVQLVQSGAEVKKPGASVKVSCKASGYTFTDYYIHWVRQAPGQGLEWMGWINPYSGGTNYAPKFQG
RFTMARDTSITTTYLDLGRLTSSDTAIYFCARDFFRAAAGTAGDYWGQGTLVTVSSSTKGPSVFPL
APCSRSTSESTAALGCLVKDYFPEPVTVSWNSGALTSGVHTFPAVLQSSGLYSLSSVVTVPSSNFG
TQTYTCNVDHKPSNTKVDKTVERKCCVECPPCPAPPVAGPSVFLFPPKPKDTLMISRTPEVTCVVV
DVSHEDPEVQFNWYVDGVEVHNAKTKPREEQFNSTFRVVSVLTVVHQDWLNGKEYKCKVSNKGLPA
PIEKTISKTKGQPREPQVYTLPPSREEMTKNQVSLTCLVKGFYPSDIAVEWESNGQPENNYKTTPP
MLDSDGSFFLYSKLTVDKSRWQQGNVFSCSVMHEALHNHYTQKSLSLSPGK

Nucleic Acid Sequence Encoding the Full Length Kappa Light Chain Sequence of 22.21.A7 (SEQ ID NO: 332)

GACATCGTGATGACCCAGTCTCCAGAAGACTCCCTGGCTGTGTCTCTGGGCGAGAGGGCC
ACCATCAACTGCTCCAGCCAGAGTGCTTTATACAGCCCCAGCAGTAAGACCTACTTAGCT
TGGTACCAGCAGAGACCGGGACACCCTCCTAAGCTTCTCATTTACTGGGCATCTACCCGG
GTATCCGGGGTCCCTGACCGATTCAGTGGCAGCGGGTCTGGGACAGATTTCACTCTCACC
ATCGGCAGCCTGCAGGCTGAAGATGTGGCAGTTTATTACTGTCAGTACTATTATGGTACT
GTCACTTTCGGCGGAGGGACCAAGGTGGAGATCAAACGAACTGTGGCTGCACCATCTGTC
TTCATCTTCCCGCCATCTGATGAGCAGTTGAAATCTGGAACTGCCTCTGTTGTGTGCCTG
CTGAATAACTTCTATCCCAGAGAGGCCAAAGTACAGTGGAAGGTGGATAACGCCCTCCAA
TCGGGTAACTCCCAGGAGAGTGTCACAGAGCAGGACAGCAAGGACAGCACCTACAGCCTC
AGCAGCACCCTGACGCTGAGCAAAGCAGACTACGAGAAACACAAAGTCTACGCCTGCGAA
GTCACCCATCAGGGCCTGAGCTCGCCCGTCACAAAGAGCTTCAACAGGGGAGAGTGT

Protein Sequence Defining the Full Length Kappa Light Chain Sequence of 22.21.A7 (SEQ ID NO: 333)

ADIVMTQSPDSLAVSLGERATINCSSQSALYSPSSKTYLAWYQQRPGHPPKLLIYWASTRVSGVPD
RFSGSGSGTDFTLTIGSLQAEDVAVYYCQYYYGTVTFGGGTKVEIKRTVAAPSVFIFPPSDEQLKS
GTASVVCLLNNFYPREAKVQWKVDNALQSGNSQESVTEQDSKDSTYSLSSTLTLSKADYEKHKVYA
CEVTHQGLSSPVTKSFNRGEC

Nucleic Acid Sequence Encoding the Full Length Heavy Chain Sequence of 22.18A.E9 (SEQ ID NO: 334)

CAGGTGCAGCTGCAGGAGTCGGGCCCAAGACTGGTGAAGCCTTCGGAGACCCTGTCCCTCACCTGC
ACTGTCTCTGGTGGGTCCATCAATAGTTATTATTGGAGTTGGATCCGGCAGCCCGTCGGGAAGGGA
CTGGAGTGGATTGGGCGTATGTATCCCGGTGGTACCTCCAGTTACAATTCATCCCTCAAGAGTCGA

GTCATCATGTCGGTAGACACGTCGAAGAACCAGTTTTCTCTGAACCTCACTTCTGTGACCGCCGCA
GACACGGCCGTATATTTCTGTGCGAGAGATCCAACATATCACCATGGCACTAGTGGTTATGTGGGG
ACTTTTGATCTCTGGGGCCAAGGGACAATGGTCACCGTCTCGAGTGCCTCCACCAAGGGCCCATCG
GTCTTCCCCCTGGCGCCCTGCTCCAGGAGCACCTCCGAGAGCACAGCCGCCCTGGGCTGCCTGGTC
AAGGACTACTTCCCCGAACCGGTGACGGTGTCGTGGAACTCAGGCGCTCTGACCAGCGGCGTGCAC
ACCTTCCCAGCTGTCCTACAGTCCTCAGGACTCTACTCCCTCAGCAGCGTGGTGACCGTGCCCTCC
AGCAACTTCGGCACCCAGACCTACACCTGCAACGTAGATCACAAGCCCAGCAACACCAAGGTGGAC
AAGACAGTTGAGCGCAAATGTTGTGTCGAGTGCCCACCGTGCCCAGCACCACCTGTGGCAGGACCG
TCAGTCTTCCTCTTCCCCCCAAAACCCAAGGACACCCTCATGATCTCCCGGACCCCTGAGGTCACG
TGCGTGGTGGTGGACGTGAGCCACGAAGACCCCGAGGTCCAGTTCAACTGGTACGTGGACGGCGTG
GAGGTGCATAATGCCAAGACAAAGCCACGGGAGGAGCAGTTCAACAGCACGTTCCGTGTGGTCAGC
GTCCTCACCGTTGTGCACCAGGACTGGCTGAACGGCAAGGAGTACAAGTGCAAGGTCTCCAACAAA
GGCCTCCCAGCCCCCATCGAGAAAACCATCTCCAAAACCAAAGGGCAGCCCCGAGAACCACAGGTG
TACACCCTGCCCCCATCCCGGGAGGAGATGACCAAGAACCAGGTCAGCCTGACCTGCCTGGTCAAA
GGCTTCTACCCCAGCGACATCGCCGTGGAGTGGGAGAGCAATGGGCAGCCGGAGAACAACTACAAG
ACCACACCTCCCATGCTGGACTCCGACGGCTCCTTCTTCCTCTACAGCAAGCTCACCGTGGACAAG
AGCAGGTGGCAGCAGGGGAACGTCTTCTCATGCTCCGTGATGCATGAGGCTCTGCACAACCACTAC
ACGCAGAAGAGCCTCTCCCTGTCTCCGGGTAAA

Protein Sequence Defining the Full Length Heavy Chain Sequence of 22.18A.E9 (SEQ ID NO: 335)

QVQLQESGPRLVKPSETLSLTCTVSGGSINSYYWSWIRQPVGKGLEWIGRMYPGGTSSYNSSLKSR
VIMSVDTSKNQFSLNLTSVTAADTAVYFCARDPTYHHGTSGYVGTFDLWGQGTMVTVSSSTKGPSV
FPLAPCSRSTSESTAALGCLVKDYFPEPVTVSWNSGALTSGVHTFPAVLQSSGLYSLSSVVTVPSS
NFGTQTYTCNVDHKPSNTKVDKTVERKCCVECPPCPAPPVAGPSVFLFPPKPKDTLMISRTPEVTC
VVVDVSHEDPEVQFNWYVDGVEVHNAKTKPREEQFNSTFRVVSVLTVVHQDWLNGKEYKCKVSNKG
LPAPIEKTISKTKGQPREPQVYTLPPSREEMTKNQVSLTCLVKGFYPSDIAVEWESNGQPENNYKT
TPPMLDSDGSFFLYSKLTVDKSRWQQGNVFSCSVMHEALHNHYTQKSLSLSPGK

Nucleic Acid Sequence Encoding the Full Length Kappa Light Chain Sequence of 22.18A.E9 (SEQ ID NO: 336)

GAAATAGTGATGACGCAGTCTCCAGCCACCCTGTCTGCGTCTCCAGGGGAAAGAGTCACC
CTCTCCTGCAGGGCCAGTCCGAGTGTTCGCAACAATTTAGCCTGGTACCAGCAGAAACCT
GGCCAGCCTCCTAGGCTCCTCATCTATGGTGCATCCACCAAGGTCATGGGTACTCCAACC
AGGTTCAGTGGCAGTGGGTCTGGGACAGAGTTCACTCTCACCATCAGCAGCCTGCAGTCT
GAAGATTTTGCAGTTTATTACTGTCAGCAGTATAATAGCTGGCCTCCGTACACTTTTAGC
CAGGGGACCAAACTGGAGATCAAACGAACTGTGGCTGCACCATCTGTCTTCATCTTCCCG
CCATCTGATGAGCAGTTGAAATCTGGAACTGCCTCTGTTGTGTGCCTGCTGAATAACTTC
TATCCCAGAGAGGCCAAAGTACAGTGGAAGGTGGATAACGCCCTCCAATCGGGTAACTCC
CAGGAGAGTGTCACAGAGCAGGACAGCAAGGACAGCACCTACAGCCTCAGCAGCACCCTG

ACGCTGAGCAAAGCAGACTACGAGAAACACAAAGTCTACGCCTGCGAAGTCACCCATCAG
GGCCTGAGCTCGCCCGTCACAAAGAGCTTCAACAGGGGAGAGTGT

Protein Sequence Defining the Full Length Kappa Light Chain Sequence of 22.18A.E9 (SEQ ID NO: 337)

DIVMTQSPATLSASPGERVTLSCRASPSVRNNLAWYQQKPGQPPRLLIYGASTKVMGTPTRFSGSG
SGTEFTLTISSLQSEDFAVYYCQQYNSWPPYTFSQGTKLEIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 5.52.H10 (SEQ ID NO: 338)

GAGGTGCAGCTGGTGGAGTCGGGGGGAGGCCTGGTCAGGCCTGGGGAGTCCCTGAGACTCTCCTGT
GCAGGCTCTGGATTCACCTTCAGCATGTTTACCCTGAACTGGGTCCGCCAGGCTCCAGGGAAGGGA
CTGGAGTGGGTCGCATCTATTAGTGGTAGTAGCAGTTTAGTGTTTCATGAAGACTCAGTGAAGGGC
CGATTCACCATCTCCAGAGACAACGCCAAGAACTCAGTATATCTGCAAATGAACAGCCTGAGAGCC
GAAGACACGGCTATTTATTACTGTGTGAGAGGAGATAGTCGCGACTATTGGGGCCAGGGAACCCTG
GTCACCGTCTCGAGTGCCTCCACCAAGGGCCCATCGGTCTTCCCCCTGGCGCCCTGCTCCAGGAGC
ACCTCCGAGAGCACAGCCGCCCTGGGCTGCCTGGTCAAGGACTACTTCCCCGAACCGGTGACGGTG
TCGTGGAACTCAGGCGCTCTGACCAGCGGCGTGCACACCTTCCCAGCTGTCCTACAGTCCTCAGGA
CTCTACTCCCTCAGCAGCGTGGTGACCGTGCCCTCCAGCAACTTCGGCACCCAGACCTACACCTGC
AACGTAGATCACAAGCCCAGCAACACCAAGGTGGACAAGACAGTTGAGCGCAAATGTTGTGTCGAG
TGCCCACCGTGCCCAGCACCACCTGTGGCAGGACCGTCAGTCTTCCTCTTCCCCCCAAAACCCAAG
GACACCCTCATGATCTCCCGGACCCCTGAGGTCACGTGCGTGGTGGTGGACGTGAGCCACGAAGAC
CCCGAGGTCCAGTTCAACTGGTACGTGGACGGCGTGGAGGTGCATAATGCCAAGACAAAGCCACGG
GAGGAGCAGTTCAACAGCACGTTCCGTGTGGTCAGCGTCCTCACCGTTGTGCACCAGGACTGGCTG
AACGGCAAGGAGTACAAGTGCAAGGTCTCCAACAAAGGCCTCCCAGCCCCCATCGAGAAAACCATC
TCCAAAACCAAAGGGCAGCCCCGAGAACCACAGGTGTACACCCTGCCCCCATCCCGGGAGGAGATG
ACCAAGAACCAGGTCAGCCTGACCTGCCTGGTCAAAGGCTTCTACCCCAGCGACATCGCCGTGGAG
TGGGAGAGCAATGGGCAGCCGGAGAACAACTACAAGACCACACCTCCCATGCTGGACTCCGACGGC
TCCTTCTTCCTCTACAGCAAGCTCACCGTGGACAAGAGCAGGTGGCAGCAGGGGAACGTCTTCTCA
TGCTCCGTGATGCATGAGGCTCTGCACAACCACTACACGCAGAAGAGCCTCTCCCTGTCTCCGGGT
AAA

Protein Sequence Defining the Full Length Heavy Chain Sequence of 5.52.H10 (SEQ ID NO: 339)

EVQLVESGGGLVRPGESLRLSCAGSGFTFSMFTLNWVRQAPGKGLEWVASISGSSSLVFHEDSVKG
RFTISRDNAKNSVYLQMNSLRAEDTAIYYCVRGDSRDYWGQGTLVTVSSSTKGPSVFPLAPCSRST
SESTAALGCLVKDYFPEPVTVSWNSGALTSGVHTFPAVLQSSGLYSLSSVVTVPSSNFGTQTYTCN
VDHKPSNTKVDKTVERKCCVECPPCPAPPVAGPSVFLFPPKPKDTLMISRTPEVTCVVVDVSHEDP
EVQFNWYVDGVEVHNAKTKPREEQFNSTFRVVSVLTVVHQDWLNGKEYKCKVSNKGLPAPIEKTIS
KTKGQPREPQVYTLPPSREEMTKNQVSLTCLVKGFYPSDIAVEWESNGQPENNYKTTPPMLDSDGS
FFLYSKLTVDKSRWQQGNVFSCSVMHEALHNHYTQKSLSLSPGK

Nucleic Acid Sequence Encoding the Full Length Kappa Light Chain Sequence of 5.52.H10 (SEQ ID NO: 340)

CAGTCTGTGTTGACGCAGCCGCCCTCAGTGTCTGCGGCCCCAGGACAGAAGGTCACCATC
TCCTGCTCTGGAAGGACCTCCAATATTGGAAATAACCATGTATCCTGGTACCAGCAATTC
CCAGGAACAGCCCCCAAAGTCCTCATTTATCGCAATGATAGGCGACCCTCAGGGATTCCT
GATCGATTCTCTGGCTCCAAGTCTGACACGTCAGCCACCCTGGGCATCACCGGGCTCCAG
ACTGGGGACGAGGCCGATTATTACTGCGGAACATGGGACAACAGCCTGAGTGGTTGGGTG
TTCGGCGGAGGGACCAAGCTGACCGTCCTAGGTCAGCCCAAGGCTGCCCCCTCGGTCACT
CTGTTCCCGCCCTCCTCTGAGGAGCTTCAAGCCAACAAGGCCACACTGGTGTGTCTCATA
AGTGACTTCTACCCGGGAGTCGTGACAGTGGCCTGGAAGGCAGATAGCAGCCCCGTCAAG
GCGGGAGTGGAGACCACCACACCCTCCAAACAAAGCAACAACAAGTACGCGGCCAGCAGC
TACCTGAGCCTGACGCCTGAGCAGTGGAAGTCCCACAAAAGCTACAGCTGCCAGGTCACG
CATGAAGGGAGCACCGTGGAGAAGACAGTGGCCCCTGCAGAATGCTCA

Protein Sequence Defining the Full Length Kappa Light Chain Sequence of 5.52.H10 (SEQ ID NO: 341)

QSVLTQPPSVSAAPGQKVTISCSGRTSNIGNNHVSWYQQFPGTAPKVLIYRNDRRPSGIPDRFSGS
KSDTSATLGITGLQTGDEADYYCGTWDNSLSGWVFGGGTKLTVLGQPKAAPSVTLFPPSSEELQAN
KATLVCLISDFYPGVVTVAWKADSSPVKAGVETTTPSKQSNNKYAASSYLSLTPEQWKSHKSYSCQ
VTHEGSTVEKTVAPAECS

Nucleic Acid Sequence Encoding the Full Length Heavy Chain Sequence of 5.15.C1 with an IgG1 Constant Region (SEQ ID NO: 342)

CAGGTGCAGCTGGTGCAGTCTGGGGCTGAGGTGAAGAGGCCTGGGGCCTCAGTGAAGGTCTCCTGT
GAGACTTCTGGATATAGCTTCACCACTCATGATTTGAACTGGGTGCGACAGGCCGCTGGACAGGGG

CTTGAGTGGATGGGATGGATGAACCCTAACACTGGTGACACATCCTACGCACAGAGGTTCCAGGGC
AGAGTCACCATGATCAGGGACGTCTCCATAAGTACAGCCTACTTGGAATTGACAGGCCTGACATTT
GATGACACGGCCGTCTACTTCTGTGCGAGGTCGAATTCTGCACCTGTTTCAACTCTTTTACCCGCT
GATGCTTTTGATGTCTGGGGCCAAGGGACAATGGTCACCGTCTCGAGTGCCTCCACCAAGGGCCCA
TCGGTCTTCCCCCTGGCGCCCTGCTCCAGGAGCACCTCCGAGAGCACAGCCGCCCTGGGCTGCCTG
GTCAAGGACTACTTCCCCGAACCGGTGACGGTGTCGTGGAACTCAGGCGCTCTGACCAGCGGCGTG
CACACCTTCCCAGCTGTCCTACAGTCCTCAGGACTCTACTCCCTCAGCAGCGTGGTGACCGTGCCC
TCCAGCAACTTCGGCACCCAGACCTACACCTGCAACGTAGATCACAAGCCCAGCAACACCAAGGTG
GACAAGACAGTTGAGCGCAAATGTTGTGTCGAGTGCCCACCGTGCCCAGCACCACCTGTGGCAGGA
CCGTCAGTCTTCCTCTTCCCCCCAAAACCCAAGGACACCCTCATGATCTCCCGGACCCCTGAGGTC
ACGTGCGTGGTGGTGGACGTGAGCCACGAAGACCCCGAGGTCCAGTTCAACTGGTACGTGGACGGC
GTGGAGGTGCATAATGCCAAGACAAAGCCACGGGAGGAGCAGTTCAACAGCACGTTCCGTGTGGTC
AGCGTCCTCACCGTTGTGCACCAGGACTGGCTGAACGGCAAGGAGTACAAGTGCAAGGTCTCCAAC
AAAGGCCTCCCAGCCCCCATCGAGAAAACCATCTCCAAAACCAAAGGGCAGCCCCGAGAACCACAG
GTGTACACCCTGCCCCCATCCCGGGAGGAGATGACCAAGAACCAGGTCAGCCTGACCTGCCTGGTC
AAAGGCTTCTACCCCAGCGACATCGCCGTGGAGTGGGAGAGCAATGGGCAGCCGGAGAACAACTAC
AAGACCACACCTCCCATGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGAGGCTCTGCACAACCAC
TACACGCAGAAGAGCCTCTCCCTGTCTCCGGGTAAA

Protein Sequence Defining the Full Length Heavy Chain Sequence of 5.15.C1 with an IgG1 Constant Region (SEQ ID NO: 343)

QVQLVQSGAEVKRPGASVKVSCETSGYSFTTDLNWVRQAAGQGLEWMGWMNPNTGDTSYAQRFQGR
VTMIRDVSISTAYLELTGLTFDDTAVYFCARSNSAPVSTLLPADAFDVWGQGTMVTVSSASTKGPS
VFPLAPSSKSTSGGTAALGCLVKDYFPEPVTVSWNSGALTSGVTFPAVLQSSGLYSLSSVVTVPSS
SLGTQTYICNVNKPSNTKVDKKVEPKSCDKTTCPPCPAPELLGGPSVFLFPPKPKDTLMISRTPEV
TCVVVDVSEDPEVKFNWYVDGVEVNAKTKPREEQYNSTYRVVSVLTVLQDWLNGKEYKCKVSNKAL
PAPIEKTISKAKGQPREPQVYTLPPSRDELTKNQVSLTCLVKGFYPSDIAVEWESNGQPENNYKTT
PPVLDSDGSFFLYSKLTVDKSRWQQGNVFSCSVMEALNYTQKSLSLSPGK

Nucleic Acid Sequence Encoding the Full Length Kappa Chain Sequence of 5.15.C1 (SEQ ID NO: 344)

GACATCCAGATGACCCAGTCTCCATCCTCCCTGTCTGCATCTATAGGAGACACAGTCACCATCACT
TGCCGGGCAAGTCAGATCATTAACAGCTATTTAAATTGGTATCAGCAAAAACCAGGGAAGCCCCT
AAACTCCTGATCTATGCTGTATCCAATTTGCACCGTGGGGTCCCCTCAAGGTTCAGTGGCAGTGGG
TCTGCGGCAGATTTCACTCTCACTATCAGTGGTCTGCAACCTGAAGATTTTGCAACTTACTACTGT
CAACAGACTTACACTACCGTTGCGATCACCTTCGGCCAAGGGACACGACTGGACATTAAACGAACT
GTGGCTGCACCATCTGTCTTCATCTTCCCGCCATCTGATGAGCAGTTGAAATCTGGAACTGCCTCT
GTTGTGTGCCTGCTGAATAACTTCTATCCCAGAGAGGCCAAAGTACAGTGGAAGGTGGATAACGCC
CTCCAATCGGGTAACTCCCAGGAGAGTGTCACAGAGCAGGACAGCAAGGACAGCACCTACAGCCTC

AGCAGCACCCTGACGCTGAGCAAAGCAGACTACGAGAAACACAAAGTCTACGCCTGCGAAGTCACC
CATCAGGGCCTGAGCTCGCCCGTCACAAAGAGCTTCAACAGGGGAGAGTGT

Protein Sequence Defining the Full Length Kappa Chain Sequence of 5.15.C1 (SEQ ID NO: 345)

DIQMTQSPSSLSASIGDTVTITCRASQIINSYLNWYQQKPGEAPKLLIYAVSNLHRGVPSRFSGSG
SAADFTLTISGLQPEDFATYYCQQTYTTVAITFGQGTRLDIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 5.54.E6 with an IgG1 Constant Region (SEQ ID NO: 346)

CAGGTGCAGCTGGTGCAGTCTGGGGCGGAGGTGAAGAAGCCTGGGTCCTCGGTGAACGTCTCCTGT
AAGGCCTCCGCAGGGACCCTCAGCGGTTATACCATCAACTGGTTGCGCCAGGCCCCTGGACGAGGG
TTTGAGTGGCTGGGAAGGATCATCCCTCACCTTGGAATAGGAAACAACGCACATAACTGGGAGGCC
AGAGTCACCCTTACCGCCGACAAGTCCACGGACACTACCTACCTGGAGTTGCGCGACCTGAGATCT
GACGACGTGGCCATTTATTACTGCGCGGTGGAGTTTTACCGGCTGGCAACGGTCACAACCCCAACC
CTTGACTTCTGGGGCCAGGGAACCCTGGTCACCGTCTCGAGTGCCTCCACCAAGGGCCCATCGGTC
TTCCCCCTGGCGCCCTGCTCCAGGAGCACCTCCGAGAGCACAGCCGCCCTGGGCTGCCTGGTCAAG
GACTACTTCCCCGAACCGGTGACGGTGTCGTGGAACTCAGGCGCTCTGACCAGCGGCGTGCACACC
TTCCCAGCTGTCCTACAGTCCTCAGGACTCTACTCCCTCAGCAGCGTGGTGACCGTGCCCTCCAGC
AACTTCGGCACCCAGACCTACACCTGCAACGTAGATCACAAGCCCAGCAACACCAAGGTGGACAAG
ACAGTTGAGCGCAAATGTTGTGTCGAGTGCCCACCGTGCCCAGCACCACCTGTGGCAGGACCGTCA
GTCTTCCTCTTCCCCCCAAAACCCAAGGACACCCTCATGATCTCCCGGACCCCTGAGGTCACGTGC
GTGGTGGTGGACGTGAGCCACGAAGACCCCGAGGTCCAGTTCAACTGGTACGTGGACGGCGTGGAG
GTGCATAATGCCAAGACAAAGCCACGGGAGGAGCAGTTCAACAGCACGTTCCGTGTGGTCAGCGTC
CTCACCGTTGTGCACCAGGACTGGCTGAACGGCAAGGAGTACAAGTGCAAGGTCTCCAACAAAGGC
CTCCCAGCCCCCATCGAGAAAACCATCTCCAAAACCAAAGGGCAGCCCCGAGAACCACAGGTGTAC
ACCCTGCCCCCATCCCGGGAGGAGATGACCAAGAACCAGGTCAGCCTGACCTGCCTGGTCAAAGGC
TTCTACCCCAGCGACATCGCCGTGGAGTGGGAGAGCAATGGGCAGCCGGAGAACAACTACAAGACC
ACACCTCCCATGCTGGACTCCGACGGCTCCTTCTTCCTCTAAGCAAGCTCACCGTGGACAAGAGCA
GGTGGCAGCAGGGGAACGTCTTCTCATGCTCCGTGATGCATGAGGCTCTGCACAACCACTACACGC
AGAAGAGCCTCTCCCTGTCTCCGGGTAAA

Protein Sequence Defining the Full Length Heavy Chain Sequence of 5.54.E6 with an IgG1 Constant Region (SEQ ID NO: 347)

QVQLVQSGAEVKKPGSSVNVSCKASAGTLSGYTINWLRQAPGRGFEWLGRIIPLGIGNNANWEARV
TLTADKSTDTTYLELRDLRSDDVAIYYCAVEFYRLATVTTPTLDFWGQGTLVTVSSASTKGPSVFP
LAPSSKSTSGGTAALGCLVKDYFPEPVTVSWNSGALTSGVTFPAVLQSSGLYSLSSVVTVPSSSLG

TQTYICNVNKPSNTKVDKKVEPKSCDKTTCPPCPAPELLGGPSVFLFPPKPKDTLMISRTPEVTCV
VVDVSEDPEVKFNWYVDGVEVNAKTKPREEQYNSTYRVVSVLTVLQDWLNGKEYKCKVSNKALPAP
IEKTISKAKGQPREPQVYTLPPSRDELTKNQVSLTCLVKGFYPSDIAVEWESNGQPENNYKTTPPV
LDSDGSFFLYSKLTVDKSRWQQGNVFSCSVMEALNYTQKSLSLSPGK

Nucleic Acid Sequence Encoding the Full Length Lambda Chain Sequence of 5.54.E6 (SEQ ID NO: 348)

TCCTATGTGCTGACTCAGCCACCCTCGGTGTCCGTGGCCCCAGGAACGACGGCCAGGATTACCTGT
GAGGGGGACCACATTGGAGCTAAAAGTGTGCACTGGTACCAGAGGAAGCCAGGCCAGGCCCCTGTG
TTGGTCGTCTATATGAATAGTGACCGGCCCTCAGGGATCCCTGAGCGATTGTCTGGCTCCAACTCT
GGGAACACGGCCACCCTGAGTATCAGCAGGGTCGAGGCCGAGGATGAGGCCGACTATTATTGTCAG
GTTTGGGATAGTTTTCGCGATCATCAGGTATTCGGCGGAGGGACCAAGTTGACCGTCCTGAGTCAG
CCCAAGGCTGCCCCCTCGGTCACTCTGTTCCCGCCCTCCTCTGAGGAGCTTCAAGCCAACAAGGCC
ACACTGGTGTGTCTCATAAGTGACTTCTACCCGGGAGCCGTGACAGTGGCCTGGAAGGCAGATAGC
AGCCCCGTCAAGGCGGGAGTGGAGACCACCACACCCTCCAAACAAGGCAACAACAAGTACGCGGCC
AGCAGCTATCTGAGCCTGACGCCTGAGCAGTGGAAGTCCCACAGAAGCTACAGCTGCCAGGTCACG
CATGAAGGGAGCACCGTGGAGAAGACAGTGGCCCCTACAGAATGTTCA

Protein Sequence Defining the Full Length Lambda Chain Sequence of 5.54.E6 (SEQ ID NO: 349)

SYVLTQPPSVSVAPGTTARITCEGDHIGAKSVHWYQRKPGQAPVLVVYMNSDRPSGIPERLSGSNS
GNTATLSISRVEAEDEADYYCQVWDSFRDHQVFGGGTKLTVLSQPKAAPSVTLFPPSSEELQANKA
TLVCLISDFYPGAVTVAWKADSSPVKAGVETTTPSKQGNNKYAASSYLSLTPEQWKSHRSYSCQVT
HEGSTVEKTVAPTECS

Nucleic Acid Sequence Encoding the Full Length Heavy Chain Sequence of 5.55.D2 with an IgG1 Constant Region (SEQ ID NO: 350)

CAGGTCACCTTGAAGGAGTCTGGTCCTGCTATAGTGAAACCCACACAGACCCTCACGCTGACCTGC
ACCTTCTCTGGGTTCTCACTCCGAACTAGTGGAGTGGGTGTGGGCTGGATCCGTCAGCCCCCAGGA
AAGGCCCTTGAGTGGCTTGCACTCATTTACTGGGATGATGATAAGCGCTACAACCCATCTCTGAAG
ACCAGACTCACCATCACCAAGGACTCCTCCAGAAACCAGGTGTTCCTCACAATGACCAACATGGAC
CCTGTGGACACAGCCACATACTTCTGTGCACACCGACAACATATTACAAACAGTTTTGGATTGGTT
ATTGCGAATGATGCTTTTGACATTTGGGGCCAAGGGACAATGGTCACCGTCTCGAGTGCCTCCACC
AAGGGCCCATCGGTCTTCCCCCTGGCGCCCTGCTCCAGGAGCACCTCCGAGAGCACAGCCGCCCTG
GGCTGCCTGGTCAAGGACTACTTCCCCGAACCGGTGACGGTGTCGTGGAACTCAGGCGCTCTGACC
AGCGGCGTGCACACCTTCCCAGCTGTCCTACAGTCCTCAGGACTCTACTCCCTCAGCAGCGTGGTG
ACCGTGCCCTCCAGCAACTTCGGCACCCAGACCTACACCTGCAACGTAGATCACAAGCCCAGCAAC
ACCAAGGTGGACAAGACAGTTGAGCGCAAATGTTGTGTCGAGTGCCCACCGTGCCCAGCACCACCT
GTGGCAGGACCGTCAGTCTTCCTCTTCCCCCCAAAACCCAAGGACACCCTCATGATCTCCCGGACC

CCTGAGGTCACGTGCGTGGTGGTGGACGTGAGCCACGAAGACCCCGAGGTCCAGTTCAACTGGTAC
GTGGACGGCGTGGAGGTGCATAATGCCAAGACAAAGCCACGGGAGGAGCAGTTCAACAGCACGTTC
CGTGTGGTCAGCGTCCTCACCGTTGTGCACCAGGACTGGCTGAACGGCAAGGAGTACAAGTGCAAG
GTCTCCAACAAAGGCCTCCCAGCCCCCATCGAGAAAACCATCTCCAAAACCAAAGGGCAGCCCCGA
GAACCACAGGTGTACACCCTGCCCCCATCCCGGGAGGAGATGACCAAGAACCAGGTCAGCCTGACC
TGCCTGGTCAAAGGCTTCTACCCCAGCGACATCGCCGTGGAGTGGGAGAGCAATGGGCAGCCGGAG
AACAACTACAAGACCACACCTCCCATGCTGGACTCCGACGGCTCCTTCTTCCTCTACAGCAAGCTC
ACCGTGGACAAGAGCAGGTGGCAGCAGGGGAACGTCTTCTCATGCTCCGTGATGCATGAGGCTCTG
CACAACCACTACACGCAGAAGAGCCTCTCCCTGTCTCCGGGTAAA

Protein Sequence Defining the Full Length Heavy Chain Sequence of 5.55.D2 with an IgG1 Constant Region (SEQ ID NO: 351)

QVTLKESGPAIVKPTQTLTLTCTFSGFSLRTSGVGVGWIRQPPGKALEWLALIYWDDDKRYNPSLK
TRLTITKDSSRNQVFLTMTNMDPVDTATYFCARQITNSFGLVIANDAFDIWGQGTMVTVSSASTKG
PSVFPLAPSSKSTSGGTAALGCLVKDYFPEPVTVSWNSGALTSGVTFPAVLQSSGLYSLSSVVTVP
SSSLGTQTYICNVNKPSNTKVDKKVEPKSCDKTTCPPCPAPELLGGPSVFLFPPKPKDTLMISRTP
EVTCVVVDVSEDPEVKFNWYVDGVEVNAKTKPREEQYNSTYRVVSVLTVLQDWLNGKEYKCKVSNK
ALPAPIEKTISKAKGQPREPQVYTLPPSRDELTKNQVSLTCLVKGFYPSDIAVEWESNGQPENNYK
TTPPVLDSDGSFFLYSKLTVDKSRWQQGNVFSCSVMEALNYTQKSLSLSPGK

Nucleic Acid Sequence Encoding the Full Length Lambda Chain Sequence of 5.55.D2 (SEQ ID NO: 352)

CAGTCTGCCCTGACTCAGCCTCCGTCCGCGTCCGGGTCTCCTGGACAGTCAGTCACCATCTCCTGC
ACTGGAACCAGCAGTGACGTTGGTCGTTATAATTATGTCGCCTGGTATCAACAACACCCAGGCAGA
GCCCCCAGACTCATAATTTATGGGGTCACTAAGCGGCCCTCAGGGGTCCCTGATCGCTTCTCTGGC
TCCAAGTCTGGCAACACGGCCTCCCTGACCGTCTCTGGGCTCCAGGCTGAAGATGAGGCTGATTAT
TATTGCAGTTCATATGCAGGCAGCAACTTTTTGGAGGTGTTCGGCGGAGGGACCAAGCTGACCGTC
CTAGGTCAGCCCAAGGCTGCCCCCTCGGTCACTCTGTTCCCGCCCTCCTCTGAGGAGCTTCAAGCC
AACAAGGCCACACTGGTGTGTCTCATAAGTGACTTCTACCCGGGAGCCGTGACAGTGGCCTGGAAG
GCAGATAGCAGCCCCGTCAAGGCGGGAGTGGAGACCACCACACCCTCCAAACAAAGCAACAACAAG
TACGCGGCCAGCAGCTACCTGAGCCTGACGCCTGAGCAGTGGAAGTCCCACAGAAGCTACAGCTGC
CAGGTCACGCATGAAGGGAGCACCGTGGAGAAGACAGTTGCCCCTACGGAATGTTCA

Protein Sequence Defining the Full Length Lambda Chain Sequence of 5.55.D2 (SEQ ID NO: 353)

QSALTQPPSASGSPGQSVTISCTGTSSDVGRYNYVAWYQQHPGRAPRLIIYGVTKRPSGVPDRFSG
SKSGNTASLTVSGLQAEDEADYYCSSYAGSNFLEVFGGGTKLTVLGQPKAAPSVTLFPPSSEELQA

NKATLVCLISDFYPGAVTVAWKADSSPVKAGVETTTPSKQSNNKYAASSYLSLTPEQWKSHRSYSC
QVTHEGSTVEKTVAPTECS

Nucleic Acid Sequence Encoding the Full Length Heavy Chain Sequence of 22.14.A1 with an IgG1 Constant Region (SEQ ID NO: 354)

CAGGTGCAGCTGGTGCAGTCTGGGGCTGAGGTGAAGAAGCCTGGGTCCTCGGTGAAGGTCTCCTGC
AAGGCTTCTGGAGGCACCTTCAGCAGCTATGCAATCAACTGGGTGCGACAGGCCCCTGGACAAGGT
CTTGAGTGGATGGGAGGGATCATCCCTATGTTTGATACAGCAATCTACGCACAGATGTTCCAGGGC
AGAGTCACCATCACCGCGGACGAATCCACGAGCACAGCCTACATGGAGCTGAGCAGCCTGAGATCT
GACGACACGGCCGTATATTACTGTGCGAGAGATCGAAACGATTATTATGACAGTAGTGGTTATTCA
GGCGCCTTTGACCAGTGGGGCCAGGGAACCCTGGTCACCGTCTCGAGTGCCTCCACCAAGGGCCCA
TCGGTCTTCCCCCTGGCGCCCTGCTCCAGGAGCACCTCCGAGAGCACAGCCGCCCTGGGCTGCCTG
GTCAAGGACTACTTCCCCGAACCGGTGACGGTGTCGTGGAACTCAGGCGCTCTGACCAGCGGCGTG
CACACCTTCCCAGCTGTCCTACAGTCCTCAGGACTCTACTCCCTCAGCAGCGTGGTGACCGTGCCC
TCCAGCAACTTCGGCACCCAGACCTACACCTGCAACGTAGATCACAAGCCCAGCAACACCAAGGTG
GACAAGACAGTTGAGCGCAAATGTTGTGTCGAGTGCCCACCGTGCCCAGCACCACCTGTGGCAGGA
CCGTCAGTCTTCCTCTTCCCCCCAAAACCCAAGGACACCCTCATGATCTCCCGGACCCCTGAGGTC
ACGTGCGTGGTGGTGGACGTGAGCCACGAAGACCCCGAGGTCCAGTTCAACTGGTACGTGGACGGC
GTGGAGGTGCATAATGCCAAGACAAAGCCACGGGAGGAGCAGTTCAACAGCACGTTCCGTGTGGTC
AGCGTCCTCACCGTTGTGCACCAGGACTGGCTGAACGGCAAGGAGTACAAGTGCAAGGTCTCCAAC
AAAGGCCTCCCAGCCCCCATCGAGAAAACCATCTCCAAAACCAAAGGGCAGCCCCGAGAACCACAG
GTGTACACCCTGCCCCCATCCCGGGAGGAGATGACCAAGAACCAGGTCAGCCTGACCTGCCTGGTC
AAAGGCTTCTACCCCAGCGACATCGCCGTGGAGTGGGAGAGCAATGGGCAGCCGGAGAACAACTAC
AAGACCACACCTCCCATGCTGGACTCCGACGGCTCCTTCTTCCTCTACAGCAAGCTCACCGTGGAC
AAGAGCAGGTGGCAGCAGGGGAACGTCTTCTCATGCTCCGTGATGCATGAGGCTCTGCACAACCAC
TACACGCAGAAGAGCCTCTCCCTGTCTCCGGGTAAA

Protein Sequence Defining the Full Length Heavy Chain Sequence of 22.14.A1 with an IgG1 Constant Region (SEQ ID NO: 355)

QVQLVQSGAEVKKPGSSVKVSCKASGGTFSSYAINWVRQAPGQGLEWMGGIIPMFDTAIYAQMFQG
RVTITADESTSTAYMELSSLRSDDTAVYYCARDRNDYYDSSGYSGAFDQWGQGTLVTVSSASTKGP
SVFPLAPSSKSTSGGTAALGCLVKDYFPEPVTVSWNSGALTSGVTFPAVLQSSGLYSLSSVVTVPS
SSLGTQTYICNVNKPSNTKVDKKVEPKSCDKTTCPPCPAPELLGGPSVFLFPPKPKDTLMISRTPE
VTCVVVDVSEDPEVKFNWYVDGVEVNAKTKPREEQYNSTYRVVSVLTVLQDWLNGKEYKCKVSNKA
LPAPIEKTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMEALNYTQKSLSLSPGK

Nucleic Acid Sequence Encoding the Full Length Kappa Chain Sequence of 22.14.A1 (SEQ ID NO: 356)

GAAATTGTGTTGACGCAGTCTCCAGGCGCCCTGTCTTTGTCTCCAGGGGAAAGAGCCACCCTCTCC
TGCAGGGCCAGTCAGAGTGTTAGCAGCAGCTACTTAGCCTGGTACCAGCAGAAACCTGGCCAGGCT
CCCAGGCTCCTCATCTATGGTGCATCCAGCAGGGCCACTGGCATCCCAGACAGGTTCAGTGGCAGT
GGGTCTGGGACAGACTTCACTCTCACAATCAGCAGACTGGAGCCTGAGGATTTTGCAGTGTATTAC
TGTCAGCAGTATGGTAGCTCACCTCGCACTTTTGGCCAGGGGACCAAGCTGGAGATCAAACGAACT
GTGGCTGCACCATCTGTCTTCATCTTCCCGCCATCTGATGAGCAGTTGAAATCTGGAACTGCCTCT
GTTGTGTGCCTGCTGAATAACTTCTATCCCAGAGAGGCCAAAGTACAGTGGAAGGTGGATAACGCC
CTCCAATCGGGTAACTCCCAGGAGAGTGTCACAGAGCAGGACAGCAAGGACAGCACCTACAGCCTC
AGCAGCACCCTGACGCTGAGCAAAGCAGACTACGAGAAACACAAAGTCTACGCCTGCGAAGTCACC
CATCAGGGCCTGAGCTCGCCCGTCACAAAGAGCTTCAACAGGGGAGAGTGTTAGAACGCGTG

Protein Sequence Defining the Full Length Kappa Chain Sequence of 22.14.A1 (SEQ ID NO: 357)

EIVLTQSPGALSLSPGERATLSCRASQSVSSSYLAWYQQKPGQAPRLLIYGASSRATGIPDRFSGS
GSGTDFTLTISRLEPEDFAVYYCQQYGSSPRTFGQGTKLEIKRTVAAPSVFIFPPSDEQLKSGTAS
VVCLLNNFYPREAKVQWKVDNALQSGNSQESVTEQDSKDSTYSLSSTLTLSKADYEKHKVYACEVT
HQGLSSPVTKSFNRGEC

Nucleic Acid Sequence Encoding the Full Length Heavy Chain Sequence of 26.53.B4 with an IgG1 Constant Region (SEQ ID NO: 358)

GAGGTGCAGCTGGTGGAGTCCGGGGGAGGCTTGGTCCAGCCTGGGGGGTCCCTGAAACTCTCCTGT
GCAGCCTCTGGGTTCAGCTTCAGTGGCTCTGCTGTGGAATGGGTCCGCCAGGCTTCCGGGAGAGGG
CTGGAGTGGATTGGCCATATAAGAACCAAGACCAAAAATTACGCGACAGCGTATGCTGCGTCGGTG
CAAGGCAGGTTCACCATCTTCAGAGATGATTCAAGGAACACGGCGTATCTGCAGATGAACAGTCTT
AAAACCGAGGACACGGCCGTGTATTATTGTTCCAGACACATCGAATTTAGCAGTTCGTCCGGGCCC
CAGATGGGCCAGGGAACCCTGGTCACCGTCTCGAGTGCCTCCACCAAGGGCCCATCGGTCTTCCCC
CTGGCGCCCTGCTCCAGGAGCACCTCCGAGAGCACAGCCGCCCTGGGCTGCCTGGTCAAGGACTAC
TTCCCCGAACCGGTGACGGTGTCGTGGAACTCAGGCGCTCTGACCAGCGGCGTGCACACCTTCCCA
GCTGTCCTACAGTCCTCAGGACTCTACTCCCTCAGCAGCGTGGTGACCGTGCCCTCCAGCAACTTC
GGCACCCAGACCTACACCTGCAACGTAGATCACAAGCCCAGCAACACCAAGGTGGACAAGACAGTT
GAGCGCAAATGTTGTGTCGAGTGCCCACCGTGCCCAGCACCACCTGTGGCAGGACCGTCAGTCTTC
CTCTTCCCCCCAAAACCCAAGGACACCCTCATGATCTCCCGGACCCCTGAGGTCACGTGCGTGGTG
GTGGACGTGAGCCACGAAGACCCCGAGGTCCAGTTCAACTGGTACGTGGACGGCGTGGAGGTGCAT
AATGCCAAGACAAAGCCACGGGAGGAGCAGTTCAACAGCACGTTCCGTGTGGTCAGCGTCCTCACC
GTTGTGCACCAGGACTGGCTGAACGGCAAGGAGTACAAGTGCAAGGTCTCCAACAAAGGCCTCCCA
GCCCCCATCGAGAAAACCATCTCCAAAACCAAAGGGCAGCCCCGAGAACCACAGGTGTACACCCTG

CCCCCATCCCGGGAGGAGATGACCAAGAACCAGGTCAGCCTGACCTGCCTGGTCAAAGGCTTCTAC
CCCAGCGACATCGCCGTGGAGTGGGAGAGCAATGGGCAGCCGGAGAACAACTACAAGACCACACCT
CCCATGCTGGACTCCGACGGCTCCTTCTTCCTCTACAGCAAGCTCACCGTGGACAAGAGCAGGTGG
CAGCAGGGGAACGTCTTCTCATGCTCCGTGATGCATGAGGCTCTGCACAACCACTACACGCAGAAG
AGCCTCTCCCTGTCTCCGGGTAAA

Protein Sequence Defining the Full Length Heavy Chain Sequence of 26.53.B4 with an IgG1 Constant Region (SEQ ID NO: 359)

EVQLVESGGGLVQPGGSLKLSCAASGFSFSGSAVEWVRQASGRGLEWIGIRTKTKNYATAYAASVQ
GRFTIFRDDSRNTAYLQMNSLKTEDTAVYYCSRIEFSSSSGPQMGQGTLVTVSSASTKGPSVFPLA
PSSKSTSGGTAALGCLVKDYFPEPVTVSWNSGALTSGVTFPAVLQSSGLYSLSSVVTVPSSSLGTQ
TYICNVNKPSNTKVDKKVEPKSCDKTTCPPCPAPELLGGPSVFLFPPKPKDTLMISRTPEVTCVVV
DVSEDPEVKFNWYVDGVEVNAKTKPREEQYNSTYRVVSVLTVLQDWLNGKEYKCKVSNKALPAPIE
KTISKAKGQPREPQVYTLPPSRDELTKNQVSLTCLVKGFYPSDIAVEWESNGQPENNYKTTPPVLD
SDGSFFLYSKLTVDKSRWQQGNVFSCSVMEALNYTQKSLSLSPGK

Nucleic Acid Sequence Encoding the Full Length Kappa Chain Sequence of 26.53.B4 (SEQ ID NO: 360)

GACATCCAGATGACCCAGTCTCCTTCCACCCTGTCTGCATCTGTAGGCGACACAGTCACCATCACT
TGCCAGGCCAGTCAGAATATTATTACTTGGTTGGCCTGGTATCAGCAGAAACCAGGGAAAGCCCCT
AACATCCTCATCTATAAGGCGTCTGGTTTAGAGAGTGGGGTCCCACCGAGGTTCAGCGGCAGTGGG
TCGGGGACAGAATTCACTCTCACCATCAGCAGCCTGCAGCCTGATGATGTTGCAACTTATTATTGC
CTACAATATAAAACTGAGCCGTGGACGTTCGGCCTAGGGACCAAGGTGGAGATCAGACGAACTGTG
GCTGCACCATCTGTCTTCATCTTCCCGCCATCTGATGAGCAGTTGAAATCTGGAACTGCCTCTGTT
GTGTGCCTGCTGAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACCTACAGCCTCAGC
AGCACCCTGACGCTGAGCAAAGCAGACTACGAGAAACACAAAGTCTACGCCTGCGAAGTCACCCAT
CAGGGCCTGAGCTCGCCCGTCACAAAGAGCTTCAACAGGGGAGAGTGT

Protein Sequence Defining the Full Length Kappa Chain Sequence of 26.53.B4 (SEQ ID NO: 361)

DIQMTQSPSTLSASVGDTVTITCQASQNIITWLAWYQQKPGKAPNILIYKASGLESGVPPRFSGSG
SGTEFTLTISSLQPDDVATYYCLQYKTEPWTFGLGTKVEIRRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC

Nucleic Acid Sequence Encoding the Full Length Heavy Chain Sequence of 5.63.E2 (SEQ ID NO: 362)

GAGGTGCAGCTGGTGGAGTCTGGGGGAGGCTTGGCCCAGCCGGGGGAGTCCCTGAGACTCTCCTGT
GCAGCCTCCGGATTCACCTTTAGTGCCTATTGGATGAGCTGGGTCCGCCAGACTCCAGGGAAGGGG
CTGGAGTGGGTGGCCAACATTAACCAGGGTGGAGATAAGACAGACTATTTGGACTCTGTGAAGGGC
CGATTCACCGTCTCCAGAGACAATGCCAAGAACTTAGTGTTTCTGCAAATGGCCAGCCTGAGAGCC
GACGACACGGCCGTGTATTACTGTGGGAGAGGTACAAACCAAGACTACTGGGGCCAGGGAACCCTG
GTCACCGTCTCGAGTGCCTCCACCAAGGGCCCATCGGTCTTCCCCCTGGCACCCTCCTCCAAGAGC
ACCTCTGGGGGCACAGCGGCCCTGGGCTGCCTGGTCAAGGACTACTTCCCCGAACCGGTGACGGTG
TCGTGGAACTCAGGCGCCCTGACCAGCGGCGTGCACACCTTCCCGGCTGTCCTACAGTCCTCAGGA
CTCTACTCCCTCAGCAGCGTGGTGACCGTGCCCTCCAGCAGCTTGGGCACCCAGACCTACATCTGC
AACGTGAATCACAAGCCCAGCAACACCAAGGTGGACAAGAAAGTTGAGCCCAAATCTTGTGACAAA
ACTCACACATGCCCACCGTGCCCAGCACCTGAACTCCTGGGGGGACCGTCAGTCTTCCTCTTCCCC
CCAAAACCCAAGGACACCCTCATGATCTCCCGGACCCCTGAGGTCACATGCGTGGTGGTGGACGTG
AGCCACGAAGACCCTGAGGTCAAGTTCAACTGGTACGTGGACGGCGTGGAGGTGCATAATGCCAAG
ACAAAGCCGCGGGAGGAGCAGTACAACAGCACGTACCGTGTGGTCAGCGTCCTCACCGTCCTGCAC
CAGGACTGGCTGAATGGCAAGGAGTACAAGTGCAAGGTCTCCAACAAAGCCCTCCCAGCCCCCATC
GAGAAAACCATCTCCAAAGCCAAAGGGCAGCCCCGAGAACCACAGGTGTACACCCTGCCCCCATCC
CGGGATGAGCTGACCAAGAACCAGGTCAGCCTGACCTGCCTGGTCAAAGGCTTCTATCCCAGCGAC
ATCGCCGTGGAGTGGGAGAGCAATGGGCAGCCGGAGAACAACTACAAGACCACGCCTCCCGTGCTG
GACTCCGACGGCTCCTTCTTCCTCTACAGCAAGCTCACCGTGGACAAGAGCAGGTGGCAGCAGGGG
AACGTCTTCTCATGCTCCGTGATGCATGAGGCTCTGCACAACCACTACACGCAGAAGAGCCTCTCC
CTGTCTCCGGGTAAA

Protein Sequence Defining the Full Length Heavy Chain Sequence of 5.63.E2 (SEQ ID NO: 363)

EVQLVESGGGLAQPGESLRLSCAASGFTFSAYWMSWVRQTPGKGLEWVANINQGGDKTDYLDSVKG
RFTVSRDNAKNLVFLQMASLRADDTAVYYCGRGTNQDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYIC
NVNHKPSNTKVDKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKVSNKALPAPI
EKTISKAKGQPREPQVYTLPPSRDELTKNQVSLTCLVKGFYPSDIAVEWESNGQPENNYKTTPPVL
DSDGSFFLYSKLTVDKSRWQQGNVFSCSVMHEALHNHYTQKSLSLSPGK

Nucleic Acid Sequence Encoding the Full Length Lambda Light Chain Sequence of 5.63.E2 (SEQ ID NO: 364)

CAGTCTGTGCTGACTCAGCCACCCTCAGCGTCTGGGACCCCCGGGCAGAGGGTCACCATCTCTTGT
TCTGGAAGCAGCTCCAATATCGGAAGTAATAATGTATACTGGTACCAACAGGTCCCAGGAACGGCC
CCCAAATTTCTCATCTATCCGAATAGTGAGCGGCCCTCAGGGGTCCCTGACCGATTCTCTGCCTCC

AAGTCTGGCACCTCAGCCTCCCTGGCCATCAGTGGACTCCGGTCCGAGGATGAGGCTACTTATTAC
TGTGCAGCATGGGATGACAGCCTAAGAACTTATGTCTTCGCATCTGGGACCAAGGTCACCGTCCTA
GGTCAGCCCAAGGCCAACCCCACTGTCACTCTGTTCCCGCCCTCCTCTGAGGAGCTCCAAGCCAAC
AAGGCCACACTAGTGTGTCTGATCAGTGACTTCTACCCGGGAGCTGTGACAGTGGCCTGGAAGGCA
GATGGCAGCCCCGTCAAGGCGGGAGTGGAGACCACCAAACCCTCCAAACAGAGCAACAACAAGTAC
GCGGCCAGCAGCTACCTGAGCCTGACGCCCGAGCAGTGGAAGTCCCACAGAAGCTACAGCTGCCAG
GTCACGCATGAAGGGAGCACCGTGGAGAAGACAGTGGCCCCTACAGAATGTTCA

Protein Sequence Defining the Full Length Lambda Light Chain Sequence of 5.63.E2 (SEQ ID NO: 365)

QSVLTQPPSASGTPGQRVTISCSGSSSNIGSNNVYWYQQVPGTAPKFLIYPNSERPSGVPDRFSAS
KSGTSASLAISGLRSEDEATYYCAAWDDSLRTYVFASGTKVTVLGQPKANPTVTLFPPSSEELQAN
KATLVCLISDFYPGAVTVAWKADGSPVKAGVETTKPSKQSNNKYAASSYLSLTPEQWKSHRSYSCQ
VTHEGSTVEKTVAPTECS

Nucleic Acid Sequence Encoding the Full Length Heavy Chain Sequence of 5.64.G4 (SEQ ID NO: 366)

CAGGTGCAGCTGGTGGAGTCTGGGGGAGGCGTGGTCCAGCCTGGGAGGTCCCTGAGACTCTCCTGT
GCAGCCTCCGGATTCACCCTCAGGAGTTATGGCATGCACTGGGTCCGCCAGGCTCCAGGCAAGGGC
CTGGAGTGGGTGGCAGTTATATCATATGATGGAAGTAAGACATATTATAGAGACTCCGTGAAGGGC
CGATTCACCATCTCCAGAGACAATTCTAAGAACACACTGTATTTGCAAATGAACAGCCTGCGAGTT
GAGGACACGGCTGTATATTATTGTGCGAAAGAGGATAATTTGGGATCTGATGTTTTTGATAGATGG
GGCCAAGGGACAATGGTCACCGTCTCGAGTGCCTCCACCAAGGGCCCATCGGTCTTCCCCCTGGCA
CCCTCCTCCAAGAGCACCTCTGGGGGCACAGCGGCCCTGGGCTGCCTGGTCAAGGACTACTTCCCC
GAACCGGTGACGGTGTCGTGGAACTCAGGCGCCCTGACCAGCGGCGTGCACACCTTCCCGGCTGTC
CTACAGTCCTCAGGACTCTACTCCCTCAGCAGCGTGGTGACCGTGCCCTCCAGCAGCTTGGGCACC
CAGACCTACATCTGCAACGTGAATCACAAGCCCAGCAACACCAAGGTGGACAAGAAAGTTGAGCCC
AAATCTTGTGACAAAACTCACACATGCCCACCGTGCCCAGCACCTGAACTCCTGGGGGGACCGTCA
GTCTTCCTCTTCCCCCCAAAACCCAAGGACACCCTCATGATCTCCCGGACCCCTGAGGTCACATGC
GTGGTGGTGGACGTGAGCCACGAAGACCCTGAGGTCAAGTTCAACTGGTACGTGGACGGCGTGGAG
GTGCATAATGCCAAGACAAAGCCGCGGGAGGAGCAGTACAACAGCACGTACCGTGTGGTCAGCGTC
CTCACCGTCCTGCACCAGGACTGGCTGAATGGCAAGGAGTACAAGTGCAAGGTCTCCAACAAAGCC
CTCCCAGCCCCCATCGAGAAAACCATCTCCAAAGCCAAAGGGCAGCCCCGAGAACCACAGGTGTAC
ACCCTGCCCCCATCCCGGGATGAGCTGACCAAGAACCAGGTCAGCCTGACCTGCCTGGTCAAAGGC
TTCTATCCCAGCGACATCGCCGTGGAGTGGGAGAGCAATGGGCAGCCGGAGAACAACTACAAGACC
ACGCCTCCCGTGCTGGACTCCGACGGCTCCTTCTTCCTCTACAGCAAGCTCACCGTGGACAAGAGC
AGGTGGCAGCAGGGGAACGTCTTCTCATGCTCCGTGATGCATGAGGCTCTGCACAACCACTACACG
CAGAAGAGCCTCTCCCTGTCTCCGGGTAAA

Protein Sequence Defining the Full Length Heavy Chain Sequence of 5.64.G4 (SEQ ID NO: 367)

QVQLVESGGGVVQPGRSLRLSCAASGFTLRSYGMHWVRQAPGKGLEWVAVISYDGSKTYYRDSVKG
RFTISRDNSKNTLYLQMNSLRVEDTAVYYCAKEDNLGSDVFDRWGQGTMVTVSSASTKGPSVFPLA
PSSKSTSGGTAALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK

Nucleic Acid Sequence Encoding the Full Length Lambda Light Chain Sequence of 5.64.G4 (SEQ ID NO: 368)

GACATCGTGATGACCCAGTCTCCTTCCACCCTGTCTGCATCTGTAGGAGACAGAGTTACCATCACT
TGTCGGGCCAGTCAGAGTGCCAGTCAGTGGTTGGCCTGGTATCAGCAAAAACCAGGGAAAGCCCCT
AATCTCCTAATCTATAAGGCGTCTAATTTACATAGTGGGGTCCCATCAAGGTTCAGCGGCAGTGGA
TCTGGGACCGAATTCACTCTCACCATCAGCAGCCTACAGCCTGATGATTTTGCAACTTATTACTGC
CAACAATACAACATTTATCCGTGGACGTTCGGCCAAGGGACCAAGGTGGAAGTGAGACGAACTGTG
GCTGCACCATCTGTCTTCATCTTCCCGCCATCTGATGAGCAGTTGAAATCTGGAACTGCCTCTGTT
GTGTGCCTGCTGAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACCTACAGCCTCAGC
AGCACCCTGACGCTGAGCAAAGCAGACTACGAGAAACACAAAGTCTACGCCTGCGAAGTCACCCAT
CAGGGCCTGAGCTCGCCCGTCACAAAGAGCTTCAACAGGGGAGAGTGTT

Protein Sequence Defining the Full Length Lambda Light Chain Sequence of 5.64.G4 (SEQ ID NO: 369)

DIVMTQSPSTLSASVGDRVTITCRASQSASQWLAWYQQKPGKAPNLLIYKASNLHSGVPSRFSGSG
SGTEFTLTISSLQPDDFATYYCQQYNIYPWTFGQGTKVEVRRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC

Nucleic Acid Sequence Encoding the Full Length Heavy Chain Sequence of 43.52.A11 (SEQ ID NO: 370)

CAGGTCCAGCTGGTGCAGTCTGGGGCTGAGGTGAAGGAGCCAGGGTCCTCGGTGAGGGTCTCCTGC
AAGGCTTCTGGACGCATCTTCCGGAATTATGCTGTCAGCTGGGTGCGACAGGCCCCTGGACAAGGG
CTTGAGTGGGTGGGCCGGTTCATCCCTTCGGAAGGAATAACAAACTACATTCCGACATTCCGGGAC
AGACTCACCTTTACTGCGGACAAGTCCACGAACACAGCCTTCATTGACCTGACTAACCTGAAATAT

GAGGACACGGCCATATATTACTGTGTGGGTGGCAGGCCAGGCGCTTTCGTCCCCTTTGACTCCTGG
GGCCAGGGAACCCTGGTCACCGTCTCGAGTGCCTCCACCAAGGGCCCATCGGTCTTCCCCCTGGCA
CCCTCCTCCAAGAGCACCTCTGGGGGCACAGCGGCCCTGGGCTGCCTGGTCAAGGACTACTTCCCC
GAACCGGTGACGGTGTCGTGGAACTCAGGCGCCCTGACCAGCGGCGTGCACACCTTCCCGGCTGTC
CTACAGTCCTCAGGACTCTACTCCCTCAGCAGCGTGGTGACCGTGCCCTCCAGCAGCTTGGGCACC
CAGACCTACATCTGCAACGTGAATCACAAGCCCAGCAACACCAAGGTGGACAAGAAAGTTGAGCCC
AAATCTTGTGACAAAACTCACACATGCCCACCGTGCCCAGCACCTGAACTCCTGGGGGGACCGTCA
GTCTTCCTCTTCCCCCCAAAACCCAAGGACACCCTCATGATCTCCCGGACCCCTGAGGTCACATGC
GTGGTGGTGGACGTGAGCCACGAAGACCCTGAGGTCAAGTTCAACTGGTACGTGGACGGCGTGGAG
GTGCATAATGCCAAGACAAAGCCGCGGGAGGAGCAGTACAACAGCACGTACCGTGTGGTCAGCGTC
CTCACCGTCCTGCACCAGGACTGGCTGAATGGCAAGGAGTACAAGTGCAAGGTCTCCAACAAAGCC
CTCCCAGCCCCCATCGAGAAAACCATCTCCAAAGCCAAAGGGCAGCCCCGAGAACCACAGGTGTAC
ACCCTGCCCCCATCCCGGGATGAGCTGACCAAGAACCAGGTCAGCCTGACCTGCCTGGTCAAAGGC
TTCTATCCCAGCGACATCGCCGTGGAGTGGGAGAGCAATGGGCAGCCGGAGAACAACTACAAGACC
ACGCCTCCCGTGCTGGACTCCGACGGCTCCTTCTTCCTCTACAGCAAGCTCACCGTGGACAAGAGC
AGGTGGCAGCAGGGGAACGTCTTCTCATGCTCCGTGATGCATGAGGCTCTGCACAACCACTACACG
CAGAAGAGCCTCTCCCTGTCTCCGGGTAAA

Protein Sequence Defining the Full Length Heavy Chain Sequence of 43.52.A11 (SEQ ID NO: 371)

QVQLVQSGAEVKEPGSSVRVSCKASGRIFRNYAVSWVRQAPGQGLEWVGRFIPSEGITNYIPTFRD
RLTFTADKSTNTAFIDLTNLKYEDTAIYYCVGGRPGAFVPFDSWGQGTLVTVSSASTKGPSVFPLA
PSSKSTSGGTAALGCLVKDYFPEPVTVSWNSGALTSGVHTFPAVLQSSGLYSLSSVVTVPSSSLGT
QTYICNVNHKPSNTKVDKKVEPKSCDKTHTCPPCPAPELLGGPSVFLFPPKPKDTLMISRTPEVTC
VVVDVSHEDPEVKFNWYVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK

Nucleic Acid Sequence Encoding the Full Length Kappa Light Chain Sequence of 43.52.A11 (SEQ ID NO: 372)

GAAATAGTGATGACGCAGTCTCCTGCCACCCTGTCTGTGTCTCCAGGGGAAAGAGCCACCCTCTCC
TGCAGGGCCAGTCAGAGTGTCAGGGGCAACTTAGCCTGGTACCAGCAAAAACCTGGCCAGGCTCCC
AGGCTTCTCATGTATGATGCTTCTACCAGGGCCACTAGTTTCCCAGCCCGGTTCAGTGGCAGTGGG
TCTGGGACAGAGTTCACTCTCACCATCAGCAGTGTGCAGTCTGAAGATTTTGCACTTTATTACTGT
CAGCAGTATAATGACTGGCCCCGGACTTTTGGCCAGGGGACCAAGGTGGAGATCAAACGAACTGTG
GCTGCACCATCTGTCTTCATCTTCCCGCCATCTGATGAGCAGTTGAAATCTGGAACTGCCTCTGTT
GTGTGCCTGCTGAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACCTACAGCCTCAGC
AGCACCCTGACGCTGAGCAAAGCAGACTACGAGAAACACAAAGTCTACGCCTGCGAAGTCACCCAT
CAGGGCCTGAGCTCGCCCGTCACAAAGAGCTTCAACAGGGGAGAGTGTT

Protein Sequence Defining the Full Length Kappa Light Chain Sequence of 43.52.A11 (SEQ ID NO: 373)

EIVMTQSPATLSVSPGERATLSCRASQSVRGNLAWYQQKPGQAPRLLMYDASTRATSFPARFSGSG
SGTEFTLTISSVQSEDFALYYCQQYNDWPRTFGQGTKVEIKRTVAAPSVFIFPPSDEQLKSGTASV
VCLLNNFYPREAKVQWKVDNALQSGNSQESVTEQDSKDSTYSLSSTLTLSKADYEKHKVYACEVTH
QGLSSPVTKSFNRGEC

Nucleic Acid Sequence Encoding the Full Length Heavy Chain Sequence of 43.52.E12 (SEQ ID NO: 374)

CAGGTGCAGCTGGTGCAGTCTGGGGCTGAGGTGAAGAAGCCTGGGTCCTCGGTGAGGGTCTCCTGC
AGGGTTTCTGGAGGCAGTTTCTCCAGTTATGACGTACACTGGGTGCGACAGGCCCCTGGACAAGGG
CTTGAGTGGGTGGGAAGGAGCGTCTCACGTGCTGACAGCTCAAGTCACACACGGAGTTCGGGGAC
AGAGTCACGATTATCGCGGACAAATCCACGAGTACAGTTTATTTGACACTGACCAGTCTGACCTCT
GACGACACGGCCATCTATTACTGTGCGGCAGGGGATTGTAGTGATGGCGGGTGCTTCCGCACACCC
CTGCACATCTGGGGCCAGGGCACCCTGGTCACCGTCTCGAGTGCCTCCACCAAGGGCCCATCGGTC
TTCCCCCTGGCACCCTCCTCCAAGAGCACCTCTGGGGGCACAGCGGCCCTGGGCTGCCTGGTCAAG
GACTACTTCCCCGAACCGGTGACGGTGTCGTGGAACTCAGGCGCCCTGACCAGCGGCGTGCACACC
TTCCCGGCTGTCCTACAGTCCTCAGGACTCTACTCCCTCAGCAGCGTGGTGACCGTGCCCTCCAGC
AGCTTGGGCACCCAGACCTACATCTGCAACGTGAATCACAAGCCCAGCAACACCAAGGTGGACAAG
AAAGTTGAGCCCAAATCTTGTGACAAAACTCACACATGCCCACCGTGCCCAGCACCTGAACTCCTG
GGGGGACCGTCAGTCTTCCTCTTCCCCCCAAAACCCAAGGACACCCTCATGATCTCCCGGACCCCT
GAGGTCACATGCGTGGTGGTGGACGTGAGCCACGAAGACCCTGAGGTCAAGTTCAACTGGTACGTG
GACGGCGTGGAGGTGCATAATGCCAAGACAAAGCCGCGGGAGGAGCAGTACAACAGCACGTACCGT
GTGGTCAGCGTCCTCACCGTCCTGCACCAGGACTGGCTGAATGGCAAGGAGTACAAGTGCAAGGTC
TCCAACAAAGCCCTCCCAGCCCCCATCGAGAAAACCATCTCCAAAGCCAAAGGGCAGCCCCGAGAA
CCACAGGTGTACACCCTGCCCCCATCCCGGGATGAGCTGACCAAGAACCAGGTCAGCCTGACCTGC
CTGGTCAAAGGCTTCTATCCCAGCGACATCGCCGTGGAGTGGGAGAGCAATGGGCAGCCGGAGAAC
AACTACAAGACCACGCCTCCCGTGCTGGACTCCGACGGCTCCTTCTTCCTCTACAGCAAGCTCACC
GTGGACAAGAGCAGGTGGCAGCAGGGGAACGTCTTCTCATGCTCCGTGATGCATGAGGCTCTGCAC
AACCACTACACGCAGAAGAGCCTCTCCCTGTCTCCGGGTAAA

Protein Sequence Defining the Full Length Heavy Chain Sequence of 43.52.E12 (SEQ ID NO: 375)

QVQLVQSGAEVKKPGSSVRVSCRVSGGSFSSYDVHWVRQAPGQGLEWVGRSVSRADSSSHTREFGD
RVTIIADKSTSTVYLTLTSLTSDDTAIYYCAAGDCSDGGCFRTPLHIWGQGTLVTVSSASTKGPSV
FPLAPSSKSTSGGTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPKPKDTLMISRTP
EVTCVVVDVSHEDPEVKFNWYVDGVEVHNAKTKPREEQYNSTYRVVSVLTVLHQDWLNGKEYKCKV
SNKALPAPIEKTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSPGK

Nucleic Acid Sequence Encoding the Full Length Kappa Light Chain Sequence of 43.52.E12 (SEQ ID NO: 376)

GAAATAGTGATGACGCAGTCTCCAGCCACTCTGTCTCTGTCTCCAGGGGAAAGAGCCACCCTCTCC
TGCAGGGCCAGTCAGAGTCTGAGAACCAACTTAGCCTGGTACCAGCAGCGACCTGGCCAGGCTCCC
AGGCTCCTCATCTCTGGGGCATCCACGAGGGCCACTGGTATCCCAGCCAGGTTCAGTGGCAGTGGG
TCTGGGACAGAATTCACTCTCACCATTAGGAGCCTGCAGCCTGAAGATTTTGCAGTTTATTACTGT
CAACAGTATCATGACTGGCCTCGGACGTTCGGCCAAGGGACCAAGGTGGATTTCAAACGAACTGTG
GCTGCACCATCTGTCTTCATCTTCCCGCCATCTGATGAGCAGTTGAAATCTGGAACTGCCTCTGTT
GTGTGCCTGCTGAATAACTTCTATCCCAGAGAGGCCAAAGTACAGTGGAAGGTGGATAACGCCCTC
CAATCGGGTAACTCCCAGGAGAGTGTCACAGAGCAGGACAGCAAGGACAGCACCTACAGCCTCAGC
AGCACCCTGACGCTGAGCAAAGCAGACTACGAGAAACACAAACTCTACGCCTGCGAAGTCACCCAT
CAGGGCCTGAGCTCGCCCGTCACAAAGAGCTTCAACAGGGGAGAGTGTTAG

Protein Sequence Defining the Full Length Kappa Light Chain Sequence of 43.52.E12 (SEQ ID NO: 377)

EIVMTQSPATLSLSPGERATLSCRASQSLRTNLAWYQQRPGQAPRLLISGASTRATGIPARFSGSG
SGTEFTLTIRSLQPEDFAVYYCQQYHDWPRTFGQGTKVDFKRTVAAPSVFIFPPSDEQLKSGTASV
VCLLNNFYPREAKVQWKVDNALQSGNSQESVTEQDSKDSTYSLSSTLTLSKADYEKHKLYACEVTH
QGLSSPVTKSFNRGEC

Nucleic Acid Sequence Encoding the Full Length Heavy Chain Sequence of 43.62.E2 (SEQ ID NO: 378)

GAGGTGCAGCTGGTGCAGTCTGGGGCAGAAGTGAAAAAGGCCGGGGAGTCTCTGAGGATCTCCTGT
AAGGGTTCTGGATACAACTTTAATAGCTATTGGATTGGCTGGGTGCGCCAGATGCCCGGGAAAGGC
CTGGAGTGGATGGCAATCATCTATCCTGCTGACTCTGACAGGAGATATGGCCCGTCCTTCCAAGGC
CAGGTCACCATCTCAGTCGACAAGTCTATTAACACCGCGTACTTGCAGTGGAGCAGCCTGAAGGCC
TCGGACACCGCCATGTATTATTGTGCCAGACTCCTCAGAGTTAGTACTGGTTGGGAAGATGCTTTT
GATCTGTGGGGCCAAGGGACAATGGTCACCGTCTCGAGTGCCTCCACCAAGGGCCCATCGGTCTTC
CCCCTGGCACCCTCCTCCAAGAGCACCTCTGGGGGCACAGCGGCCCTGGGCTGCCTGGTCAAGGAC
TACTTCCCCGAACCGGTGACGGTGTCGTGGAACTCAGGCGCCCTGACCAGCGGCGTGCACACCTTC
CCGGCTGTCCTACAGTCCTCAGGACTCTACTCCCTCAGCAGCGTGGTGACCGTGCCCTCCAGCAGC
TTGGGCACCCAGACCTACATCTGCAACGTGAATCACAAGCCCAGCAACACCAAGGTGGACAAGAAA
GTTGAGCCCAAATCTTGTGACAAAACTCACACATGCCCACCGTGCCCAGCACCTGAACTCCTGGGG
GGACCGTCAGTCTTCCTCTTCCCCCCAAAACCCAAGGACACCCTCATGATCTCCCGGACCCCTGAG
GTCACATGCGTGGTGGTGGACGTGAGCCACGAAGACCCTGAGGTCAAGTTCAACTGGTACGTGGAC
GGCGTGGAGGTGCATAATGCCAAGACAAAGCCGCGGGAGGAGCAGTACAACAGCACGTACCGTGTG
GTCAGCGTCCTCACCGTCCTGCACCAGGACTGGCTGAATGGCAAGGAGTACAAGTGCAAGGTCTCC
AACAAAGCCCTCCCAGCCCCCATCGAGAAAACCATCTCCAAAGCCAAAGGGCAGCCCCGAGAACCA
CAGGTGTACACCCTGCCCCCATCCCGGGATGAGCTGACCAAGAACCAGGTCAGCCTGACCTGCCTG

GTCAAAGGCTTCTATCCCAGCGACATCGCCGTGGAGTGGGAGAGCAATGGGCAGCCGGAGAACAAC
TACAAGACCACGCCTCCCGTGCTGGACTCCGACGGCTCCTTCTTCCTCTACAGCAAGCTCACCGTG
GACAAGAGCAGGTGGCAGCAGGGGAACGTCTTCTCATGCTCCGTGATGCATGAGGCTCTGCACAAC
CACTACACGCAGAAGAGCCTCTCCCTGTCTCCGGGTAAA

Protein Sequence Defining the Full Length Heavy Chain Sequence of 43.62.E2 (SEQ ID NO: 379)

EVQLVQSGAEVKKAGESLRISCKGSGYNFNSYWIGWVRQMPGKGLEWMAIIYPADSDRRYGPSFQG
QVTISVDKSINTAYLQWSSLKASDTAMYYCARLLRVSTGWEDAFDLWGQGTMVTVSSASTKGPSVF
PLAPSSKSTSGGTAALGCLVKDYFPEPVTVSWNSGALTSGVHTFPAVLQSSGLYSLSSVVTVPSSS
LGTQTYICNVNHKPSNTKVDKKVEPKSCDKTHTCPPCPAPELLGGPSVFLFPPKPKDTLMISRTPE
VTCVVVDVSHEDPEVKFNWYVDGVEVHNAKTKPREEQYNSTYRVVSVLTVLHQDWLNGKEYKCKVS
NKALPAPIEKTISKAKGQPREPQVYTLPPSRDELTKNQVSLTCLVKGFYPSDIAVEWESNGQPENN
YKTTPPVLDSDGSFFLYSKLTVDKSRWQQGNVFSCSVMHEALHNHYTQKSLSLSPGK

Nucleic Acid Sequence Encoding the Full Length Kappa Light Chain Sequence of 43.62.E2 (SEQ ID NO: 380)

GACATCGTGATGACCCAGTCTCCAGACTCCCTGGTTGTGTCTCTGGGCGAGAGGGCCACCATCAAG
TGCCAGTCCAGCCAGAGTGTTTTGTACAGCTCCACCAATAAGAACTATTTAGCCTGGTACCAGCAG
AAATCGGGACAGCCACCTAAGCTACTCATTTACTGGGCCTCCACCCGGGAATCCGGGGTCCCTGAC
CGATTCATTGGTAGCGGCTCTAGGACAGATTTCACTCTCACCATCAGCGACCTGCAGCCTGAGGAT
GCGGCAGTTTATTACTGTCAGCAGTATTATAGTGATTATCTCACTTTCGGCGGAGGGACCAAGGTG
GAGATCAAACGAACTGTGGCTGCACCATCTGTCTTCATCTTCCCGCCATCTGATGAGCAGTTGAAA
TCTGGAACTGCCTCTGTTGTGTGCCTGCTGAATAACTTCTATCCCAGAGAGGCCAAAGTACAGTGG
AAGGTGGATAACGCCCTCCAATCGGGTAACTCCCAGGAGAGTGTCACAGAGCAGGACAGCAAGGAC
AGCACCTACAGCCTCAGCAGCACCCTGACGCTGAGCAAAGCAGACTACGAGAAACACAAAGTCTAC
GCCTGCGAAGTCACCCATCAGGGCCTGAGCTCGCCCGTCACAAAGAGCTTCAACAGGGGAGAGTGT
TAG

Protein Sequence Defining the Full Length Kappa Light Chain Sequence of 43.62.E2 (SEQ ID NO: 381)

DIVMTQSPDSLVVSLGERATIKCQSSQSVLYSSTNKNYLAWYQQKSGQPPKLLIYWASTRESGVPD
RFIGSGSRTDFTLTISDLQPEDAAVYYCQQYYSDYLTFGGGTKVEIKRTVAAPSVFIFPPSDEQLK
SGTASVVCLLNNFYPREAKVQWKVDNALQSGNSQESVTEQDSKDSTYSLSSTLTLSKADYEKHKVY
ACEVTHQGLSSPVTKSFNRGEC

### Example 2: Identification of Human S. aureus Antibodies

Healthy human subjects were recruited by posters located throughout the Roger Williams Medical Center campus. Blood from qualified subjects was processed through a Ficoll gradient to isolate PBMCs. Antibody-secreting plasma cells, identified by cell surface markers, were single-cell sorted into 96-well plates and subjected to RT-PCR to amplify antibody genes (Fig. 2A). As shown in Fig. 2A, the upper two panels show plasma cells with a clear CD38high/CD19+population (R3 gate). The lower two panels show the lambda positive and kappa positive plasma cell populations (R4). Cells from the lambda and kappa positive populations were sorted into individual wells of 96-well PCR plates and heavy and light chains were recovered from single cells by RT-PCR with a cognate pair recovery rate of 73% (Fig. 2B). The variation in size between individual amplicons in the heavy chain gel suggests that a single heavy chain is being amplified in each well. Sequence data from cloned antibodies supports this conclusion. Amplified products (Fig. 2B) were cloned and expressed in mammalian cells (CHO-K1 cells) in 96-well format for screening.

Expressed antibodies were screened by ELISA to identify *S. aureus* -specific binders (Fig. 2C-E). Wells in which an antibody has been detected (values greater than two times background) are shaded, with color indicating relative expression levels with respect to background. Background is calculated as the average value of negative control wells (purple), left empty during sorting. Fig. 2D is a table showing data from plate 5.6 from a screening ELISA against *S. aureus* peptide antigens. Wells displaying 2x background (average value of purple wells) were scored as positive (*e.g.,* well H9 was scored as positive). Fig. 2E is a table showing data from plate 5.6 from a screening ELISA against *S. aureus* surface proteins. Wells displaying 2x background (average value of purple wells) were scored as positive (*e.g.,* wells H2 and A12 were scored as positive). Positives were reconfirmed through further ELISA testing (Fig. 2F), sequenced and expressed in a larger scale batch culture for *in vitro* testing and animal studies. Wells displaying 2x background (average value of purple wells) were scored as positive. Wells B12 and C12 were positive controls. Clones 5.6.H2 (wells B1, B2, B7 and B8) and 5.6.H9 (wells C7, C8) were confirmed as positives in this experiment, as well as several clones from other screening plates. Clone 5.6.A12 did not confirm and was discarded.

### Example 3: Human Recombinant Polyclonal Anti-S. aureus Antibodies Protect Mice from Infection with Live S. aureus

A human recombinant polyclonal *S. aureus* antibody was generated by mixing individual antibodies (1.62.B9, 5.11.H10, 5.27.A11, 5.6.H2, and 5.6.H9; referred to herein as five antibody cocktail #1) equally to a total concentration of 1 mg/ml.

Mice were infected with a community associated methicillin-resistant (MRSA) strain, USA300, at a dose of 3.5 x 10⁸ colony forming units (CFU) or 5 x 10⁸ CFU, by intra-peritoneal injection. Immediately following infection, groups of 10 mice each were treated by intra-peritoneal injection with either PBS (control population), 110 mg/kg vancomycin, or 1.0 mg/kg of five antibody cocktail #1. Animals were observed at 2, 6, 18, 24, 48 and 72 hours post-treatment and live/dead status was noted.

At 3.5x10⁸ CFU, all of the mice treated with five antibody cocktail #1 survived the full 72 hour study. At 5x10⁸ CFU, 50% of the mice treated with five antibody cocktail #1 survived the full 72 hour study (Fig. 3). None of the PBS control mice survived to 72 hours at either dose of *S*. *aureus.* Vancomycin, the positive control, rescued all of the mice.

As shown in Fig. 4, the results of this experiment demonstrated that animals treated with PBS alone succumbed rapidly to infection with an LD₅₀ of 2.75 x 10⁸ CFU. Mice treated with five antibody cocktail #1 at a 1.0 mg/kg dose were fully protected from a dose of 3.5 x 10⁸ CFU and were more resistant to infection with an LD₅₀ of 5.0 x 10⁸ CFU, which was approximately twice the LD₅₀ dose for PBS treated animals. This represents a shift in the LD₅₀ curve from 2.75 x 10⁸ CFU to 5 x 10⁸ CFU with the five antibody cocktail, which is a significantly stronger effect than has been previously demonstrated with passive immunotherapy approaches. Therefore, the results of this experiment suggest that mice treated with five antibody cocktail #1 are more resistant to *S. aureus* infection and that fully human recombinant polyclonal antibody therapy is capable of protecting mice from lethal infections of MRSA.

A second human recombinant polyclonal *S. aureus* antibody was generated by mixing individual antibodies (1.62.B9, 5.11.H10, 5.27.A11, 5.6.H2, 5.7.D3, 5.27.G2, and 8.11.G6; referred to herein as the seven antibody cocktail) equally to a total concentration of 1 mg/ml.

Mice were infected with a methicillin-sensitive (MSSA) strain, Wood46, at a dose of 2 x 10⁸ colony forming units (CFU) or 4 x 10⁸ CFU, by intra-peritoneal injection. Immediately following infection, groups of 5 mice each were treated by intra-peritoneal injection with PBS (control population), 110 mg/kg vancomycin, or 1.0 mg/kg of the seven antibody cocktail. Animals were observed at 2, 6, 18, 24, 48 and 72 hours post-treatment and live/dead status was noted.

At 2 x 10⁸ CFU, all of the mice treated with the seven antibody cocktail survived the full 72 hour study, compared with 40% of the PBS control mice. At 4 x 10⁸ CFU, 40% of the mice treated with the seven antibody cocktail survived the full 72 hour study (Fig. 5), compared with 10% of the PBS control mice. Vancomycin, the positive control, rescued all of the mice.

As shown in Fig. 6, the results of this experiment demonstrated that compared with animals treated with PBS alone, mice treated with the seven antibody cocktail at a 1.0 mg/kg dose were fully protected from a dose of 2 x 10⁸ CFU and were more resistant to infection with a dose of 4.0 x 10⁸ CFU. This represents a shift in the LD₅₀ curve from 1.8 x 10⁸ CFU to 3.75 x 10⁸ CFU with the seven antibody cocktail, which is a significantly stronger effect than has been previously demonstrated with passive immunotherapy approaches. Therefore, the results of this experiment suggest that mice treated with the seven antibody cocktail are more resistant to *S*. *aureus* infection and that fully human recombinant polyclonal antibody therapy is capable of protecting mice from lethal infections of MSSA.

In a third study, a comparison was made between two five antibody cocktails, a ten antibody cocktail and a nineteen antibody cocktail. Five antibody cocktail #2 comprised antibodies 5.11.H10, 18.43.D8, 5.23.C9, 5.27.A11 and 5.6.H9. Five antibody cocktail #3 comprised antibodies 22.18.E9, 5.52.H10, 9.51.H9, 8.51.G10 and 5.23.C12. The ten antibody cocktail comprised antibodies 5.11.H10, 18.43.D8, 5.23.C9, 5.27.A11, 5.6.H9, 22.18.E9, 5.52.H10, 9.51.H9, 8.51.G10 and 5.23.C12. The nineteen antibody cocktail comprised antibodies 5.11.H10, 18.43.D8, 5.23.C9, 5.27.A11, 5.6.H9, 22.18.E9, 5.52.H10, 9.51.H9, 8.51.G10, 5.23.C12, 5.24.F3, 5.24.A7, 5.8.H4, 26.51.E1, 5.19.F12, 8.51.G11, 22.22 .E7, 22.21.A7, and5.17.F8. In each cocktail, component antibodies were mixed at equal ratios to a total concentration of 1 mg/ml.

Mice were infected with a community associated methicillin-resistant (MRSA) strain, USA300, at a dose of 1 x 10⁹ colony forming units (CFU) by intra-peritoneal injection. Immediately following infection, groups of 8 mice each were treated by intra-peritoneal injection with PBS (control population), 10 mg/kg vancomycin, or 1.0 mg/kg of antibody cocktail. Animals were observed at 8, 16, 24, 32, 48, 56 and 72 hours post-treatment and live/dead status was noted.

At 1x10⁹ CFU, all of the mice treated with either five antibody cocktail #2 or five antibody cocktail #3 survived the full 72 hour study, compared with 50% of the PBS control mice (Fig. 7). 75% of the mice treated with either the ten antibody cocktail or the nineteen antibody cocktail survived the full 72 hour study. Vancomycin, the positive control, rescued all of the mice.

### Example 4: Antibody Reactivity Against Various S. aureus Strains

In this example, the antibodies disclosed herein were tested for reactivity against twelve different *S*. *aureus* strains grown on Tryptic Soy Agar (TSA) medium. The skilled person would understand that TSA is one or many conditions on which *S*. *aureus* may be grown and each conditions may result in the expression of a different proteome.

Each bacterial strain was spread on TSA and a single colony from each strain was used to inoculate a 5 ml trypticase soy broth (TSB) culture. The cultures were grown at 37°C and then diluted before spread plating on TSA plates. The plates were grown overnight before being harvested. The bacteria were washed with PBS. The supernatants were filtered and stored at - 20°C before use. ELISA plates were coated with the supernatants, placed in an azide blocking buffer. The ELISA was run against the 26 different antibody clones as 1:10 diluted Fab fragments both in the presence of a protein A block. Any Fab that was reactive as determined by being sufficiently above background was marked with a (+), any Fab which was highly reactive was designated (++). Any FAb which was not reactive was designated with a (-), as is reflected in Table 4. The number of anti-S. *aureus* antibodies reacting with various *S*. *aureus* strains is shown in Fig. 8

The results suggest that not all antibodies bind each strain of *S. aureus.* A shown in Table 4, each different strain has a subset of different antibodies which bind. It is contemplated herein that these results reflect the different genomes and proteomes expressed by each strains. Under TSA conditions, antibodies 5.6.H9, 5.55.D2, 22.18A.E9, 9.51.H9, 5.11.H10, 5.23.C12, 5.52.H10, 18.43.D8, 8.51.G10 and 8.51.G11 each reacted with 8 or more *S*. *aureus* strains tested.

### Example 5: In vitro opsonization of S. aureus in human blood is augmented by specific antibody cocktails

In this example, the capacity of specific antibody cocktails to enhance the opsonization of *S*. *aureus* bacteria by human blood cells was tested.

The following antibody cocktails were tested: antibody cocktail #1 was a five antibody cocktail including antibodies 5.11.H10, 5.23.C9, 5.52.H10, 26.51.E1 and 22.21.A7 at a total concentration of 1.0 mg/ml; antibody cocktail #2 was a five antibody cocktail including antibodies 5.11.H10, 5.27.A11, 5.6.H9, 18.43.D8 and 5.23.C9 at a total concentration of 1.0 mg/ml; and antibody cocktail #3 was a three antibody cocktail including antibodies 5.11.H10, 5.23.C9 and 5.52.H10 at a total concentration of 1.0 mg/ml.

100µl of antibody (100µg total) of each of antibody cocktails 1, 2, and 100µl of *S. aureus* strain Wood-46 bacteria (1x10⁶ cells) were mixed and incubated for 30 minutes at 37°C. 100 µl of heparinized human blood was added to the antibody/bacteria suspension. The bacteria were counted at time = 0 and after a one hour incubation by taking samples from the 300µl volume and plating serial dilutions on TSA. The percent killing is calculated as [(Bacteria, Time = 0h) - Bacteria, Time = 1h)]/(Bacteria, Time=0) x 100%.

Antibody cocktails #1 and #3 each enhanced the ability of white blood cells to opsonize the *S*. *aureus* strain (Fig. 9). Antibody cocktail #2, which had previously been shown to provide protection in mice infected with a dose of 1 x 10⁹ CFUs of USA300 strain (see Example 3) and contains an antibody against the cell surface (e.g., 5.11.H10), did not enhance opsonization suggesting that opsonization is one part of the criteria needed to provide protection in vivo. The data also suggests that opsonization of *S. aureus* in human blood is enhanced by polyclonal antibodies, e.g., polyclonal antibodies that contain a mixture of cell surface binding antibodies with other antibodies targeting other cellular mechanisms.

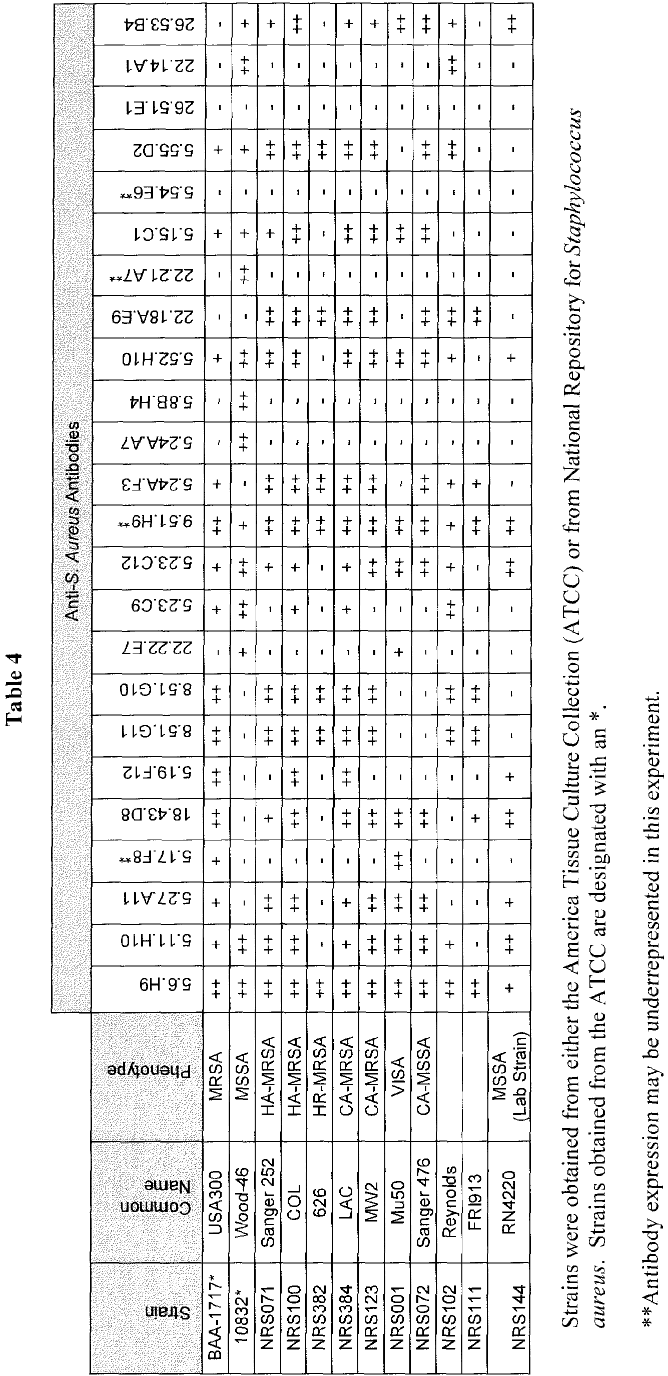

### INCORPORATION BY REFERENCE

The entire disclosure of each of the patent documents and scientific articles referred to herein is incorporated by reference for all purposes.

### EQUIVALENTS

The invention may be embodied in other specific forms without departing from the spirit or essential characteristics thereof. The foregoing embodiments are therefore to be considered in all respects illustrative rather than limiting on the invention described herein. Scope of the invention is thus indicated by the claims rather than by the foregoing description, and all changes that come within the meaning and the range of equivalency of the claims are intended to be embraced therein.

Certain embodiments of the invention are set out in the following paragraphs:
1. A human recombinant polyclonal antibody composition that binds to one or more *Staphylococcus aureus* strains comprising at least three different human antibodies that individually bind one or more *S*. *aureus* strains.
2. The polyclonal antibody composition of paragraph 1, comprising at least 5 different human antibodies that individually bind one or more *S*. *aureus* strains.
3. The polyclonal antibody composition of paragraph 1, comprising at least 10 different human antibodies that individually bind one or more *S*. *aureus* strains.
4. The polyclonal antibody composition of paragraph 1, comprising at least one of the antibodies identified in Table 3.
5. The polyclonal antibody composition of paragraph 1 or 4, comprising at least 3, at least 5, at least 8, at least 10, at least 12 or at least 15 of the antibodies identified in Table 3.
6. The polyclonal antibody composition of paragraph 1 or 4, wherein at least one of the antibodies binds to at least one of the *S*. *aureus* strains identified in Table 4.
7. The polyclonal antibody composition of paragraph 1 or 4, wherein at least one of the antibodies binds at least 5 strains, at least 6 strains, at least 7 strains, at least 8 strains, at least 9 strains, at least 10 strains, at least 11 strains, or at least 12 strains identified in Table 4.
8. The polyclonal antibody composition of paragraph 1, wherein one of the antibodies binds a cell surface antigen on one or more *S*. *aureus* strains and one of the antibodies binds a toxin produced by one or more *S*. *aureus* strains.
9. The polyclonal antibody composition of paragraph 1, wherein one of the antibodies binds a cell surface antigen on one or more *S*. *aureus* strains and one of the antibodies binds an immunomodulator produced by one or more *S*. *aureus* strains.
10. The polyclonal antibody composition of paragraph 1, wherein one of the antibodies binds a toxin produced by one or more *S*. *aureus* strains and one of the antibodies binds an immunomodulator produced by one or more *S*. *aureus* strains.
11. The polyclonal antibody composition of paragraph 1, wherein one of the antibodies binds a cell surface antigen on one or more *S*. *aureus* strains, one of the antibodies binds a toxin produced by one or more *S*. *aureus* strains, and one of the antibodies binds an immunomodulator produced by one or more *S*. *aureus* strains.
12. The polyclonal antibody composition of paragraph 1, wherein one of the antibodies binds delta-toxin.
13. The polyclonal antibody composition of paragraph 1, wherein one of the antibodies binds the toxin phenol soluble modulin beta-1.
14. The polyclonal antibody composition of paragraph 1, wherein at least one of the antibodies is selected from the group consisting of:
   (a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9;
   (b) an antibody comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9;
   (c) an antibody comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10;
   (d) an antibody comprising CDR1, CDR2 and CDR3 of heavy chain amino acid sequence SEQ ID NO: 20 and CDR1, CDR2, CDR3 of light chain amino acid sequence SEQ ID NO: 22 of 5.27.A11;
   (e) an antibody comprising CDR1, CDR2 and CDR3 of heavy chain amino acid sequence SEQ ID NO: 47 and CDR1, CDR2, CDR3 of light chain amino acid sequence SEQ ID NO: 49 of 5.17.F8;
   (f) an antibody comprising CDR1, CDR2 and CDR3 of heavy chain amino acid sequence SEQ ID NO: 56 and CDR1, CDR2, CDR3 of light chain amino acid sequence SEQ ID NO: 58 of 5.19.F12;
   (g) an antibody comprising CDR1, CDR2 and CDR3 of heavy chain amino acid sequence SEQ ID NO: 65 and CDR1, CDR2, CDR3 of light chain amino acid sequence SEQ ID NO: 67 of 5.23. C9;
   (h) an antibody comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12;
   (i) an antibody comprising CDR1, CDR2 and CDR3 of heavy chain amino acid sequence SEQ ID NO: 83 and CDR1, CDR2, CDR3 of light chain amino acid sequence SEQ ID NO: 85 of 8.51.G11;
   (j) an antibody comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9;
   (k) an antibody comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8;
   (l) an antibody comprising CDR1, CDR2 and CDR3 of heavy chain amino acid sequence SEQ ID NO: 110 and CDR1, CDR2, CDR3 of light chain amino acid sequence SEQ ID NO: 112 of 22.22.E7;
   (m) an antibody comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10;
   (n) an antibody comprising CDR1, CDR2 and CDR3 of heavy chain amino acid sequence SEQ ID NO: 153 and CDR1, CDR2, CDR3 of light chain amino acid sequence SEQ ID NO: 155 of 5.24A.A7;
   (o) an antibody comprising CDR1, CDR2 and CDR3 of heavy chain amino acid sequence SEQ ID NO: 157 and CDR1, CDR2, CDR3 of light chain amino acid sequence SEQ ID NO: 159 of 5.24A.F3;
   (p) an antibody comprising CDR1, CDR2 and CDR3 of heavy chain amino acid sequence SEQ ID NO: 161 and CDR1, CDR2, CDR3 of light chain amino acid sequence SEQ ID NO: 163 of 5.8B.H4;
   (q) an antibody comprising CDR1, CDR2 and CDR3 of heavy chain amino acid sequence SEQ ID NO: 165 and CDR1, CDR2, CDR3 of light chain amino acid sequence SEQ ID NO: 167 of 26.51.E1;
   (r) an antibody comprising CDR1, CDR2 and CDR3 of heavy chain amino acid sequence SEQ ID NO: 169 and CDR1, CDR2, CDR3 of light chain amino acid sequence SEQ ID NO: 171 of 22.21.A7;
   (s) an antibody comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10;
   (t) an antibody comprising CDR1, CDR2 and CDR3 of heavy chain amino acid sequence SEQ ID NO: 181 and CDR1, CDR2, CDR3 of light chain amino acid sequence SEQ ID NO: 183 of 5.15.C1;
   (u) an antibody comprising CDR1, CDR2 and CDR3 of heavy chain amino acid sequence SEQ ID NO: 185 and CDR1, CDR2, CDR3 of light chain amino acid sequence SEQ ID NO: 187 of 5.54.E6;
   (v) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2;
   (w) an antibody comprising CDR1, CDR2 and CDR3 of heavy chain amino acid sequence SEQ ID NO: 193 and CDR1, CDR2, CDR3 of light chain amino acid sequence SEQ ID NO: 195 of 22.14.A1;
   (x) an antibody comprising CDR1, CDR2 and CDR3 of heavy chain amino acid sequence SEQ ID NO: 197 and CDR1, CDR2, CDR3 of light chain amino acid sequence SEQ ID NO: 199 of 26.53.B4;
   (y) an antibody comprising CDR1, CDR2 and CDR3 of heavy chain amino acid sequence SEQ ID NO: 201 and CDR1, CDR2, CDR3 of light chain amino acid sequence SEQ ID NO: 203 of 5.63.E2;
   (z) an antibody comprising CDR1, CDR2 and CDR3 of heavy chain amino acid sequence SEQ ID NO: 205 and CDR1, CDR2, CDR3 of light chain amino acid sequence SEQ ID NO: 207 of 5.64.G4;
   (aa) an antibody comprising CDR1, CDR2 and CDR3 of heavy chain amino acid sequence SEQ ID NO: 209 and CDR1, CDR2, CDR3 of light chain amino acid sequence SEQ ID NO: 211 of 43.52.A11;
   (ab) an antibody comprising CDR1, CDR2 and CDR3 of heavy chain amino acid sequence SEQ ID NO: 213 and CDR1, CDR2, CDR3 of light chain amino acid sequence SEQ ID NO: 215 of 43.52.E12;
   (ac) an antibody comprising CDR1, CDR2 and CDR3 of heavy chain amino acid sequence SEQ ID NO: 217 and CDR1, CDR2, CDR3 of light chain amino acid sequence SEQ ID NO: 219 of 43.62.E2; and
   (ad) mixtures of two or more of said antibodies in (a)-(ac).
15. The polyclonal antibody composition of paragraph 1, wherein at least one of the antibodies is selected from the group consisting of:
   (a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9;
   (b) an antibody comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9; and
   (c) a mixture of (a) and (b).
16. The polyclonal antibody composition of paragraph 15, further comprising an antibody selected from the group consisting of:
   (a) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2;
   (b) an antibody comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9;
   (c) an antibody comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10;
   (d) an antibody comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12;
   (e) an antibody comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10;
   (f) an antibody comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8;
   (g) an antibody comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10; and
   (h) an antibody comprising CDR1, CDR2 and CDR3 of heavy chain amino acid sequence SEQ ID NO: 83 and CDR1, CDR2, CDR3 of light chain amino acid sequence SEQ ID NO: 85 of 8.51.G11.
17. The polyclonal antibody composition of paragraph 1, wherein at least one of the antibodies is selected from the group consisting of:
   (a) an immunoglobulin heavy chain variable region comprising the amino acid sequence of SEQ ID NO: 11 **(5.11.H10),** and an immunoglobulin light chain variable region comprising the amino acid sequence of SEQ ID NO: 13 **(5.11.H10);**
   (b) an immunoglobulin heavy chain variable region comprising the amino acid sequence of SEQ ID NO: 20 **(5.27.A11),** and an immunoglobulin light chain variable region comprising the amino acid sequence of SEQ ID NO: 22 **(5.27.A11);**
   (c) an immunoglobulin heavy chain variable region comprising the amino acid sequence of SEQ ID NO: 38 **(5.6.H9),** and an immunoglobulin light chain variable region comprising the amino acid sequence of SEQ ID NO: 40 **(5.6.H9);**
   (d) an immunoglobulin heavy chain variable region comprising the amino acid sequence of SEQ ID NO: 47 **(5.17.F8),** and an immunoglobulin light chain variable region comprising the amino acid sequence of SEQ ID NO: 49 **(5.17.F8);**
   (e) an immunoglobulin heavy chain variable region comprising the amino acid sequence of SEQ ID NO: 56 **(5.19.F12),** and an immunoglobulin light chain variable region comprising the amino acid sequence of SEQ ID NO: 58 **(5.19.F12);**
   (f) an immunoglobulin heavy chain variable region comprising the amino acid sequence of SEQ ID NO: 65 **(5.23.C9),** and an immunoglobulin light chain variable region comprising the amino acid sequence of SEQ ID NO: 67 **(5.23.C9);**
   (g) an immunoglobulin heavy chain variable region comprising the amino acid sequence of SEQ ID NO: 74 **(5.23.C12),** and an immunoglobulin light chain variable region comprising the amino acid sequence of SEQ ID NO: 76 **(5.23.C12);**
   (h) an immunoglobulin heavy chain variable region comprising the amino acid sequence of SEQ ID NO: 83 **(8.51.G11),** and an immunoglobulin light chain variable region comprising the amino acid sequence of SEQ ID NO: 85 **(8.51.G11);**
   (i) an immunoglobulin heavy chain variable region comprising the amino acid sequence of SEQ ID NO: 92 **(9.51.H9),** and an immunoglobulin light chain variable region comprising the amino acid sequence of SEQ ID NO: 94 **(9.51.H9);**
   (j) an immunoglobulin heavy chain variable region comprising the amino acid sequence of SEQ ID NO: 101 **(18.43.D8),** and an immunoglobulin light chain variable region comprising the amino acid sequence of SEQ ID NO: 103 **(18.43.D8);**
   (k) an immunoglobulin heavy chain variable region comprising the amino acid sequence of SEQ ID NO: 110 **(22.22.E7),** and an immunoglobulin light chain variable region comprising the amino acid sequence of SEQ ID NO: 112 **(22.22.E7);**
   (l) an immunoglobulin heavy chain variable region comprising the amino acid sequence of SEQ ID NO: **149(8.51.G10),** and an immunoglobulin light chain variable region comprising the amino acid sequence of SEQ ID NO: 151 **(8.51.G10);** (m) an immunoglobulin heavy chain variable region comprising the amino acid sequence of SEQ ID NO: 153 **(5.24A.A7),** and an immunoglobulin light chain variable region comprising the amino acid sequence of SEQ ID NO: 155 **(5.24A.A7);**
   (n) an immunoglobulin heavy chain variable region comprising the amino acid sequence of SEQ ID NO: 157 **(5.24A.F3),** and an immunoglobulin light chain variable region comprising the amino acid sequence of SEQ ID NO: 159 **(5.24A.F3);**
   (o) an immunoglobulin heavy chain variable region comprising the amino acid sequence of SEQ ID NO: 161 **(5.8B.H4),** and an immunoglobulin light chain variable region comprising the amino acid sequence of SEQ ID NO: 163 **(5.8B.H4);**
   (p) an immunoglobulin heavy chain variable region comprising the amino acid sequence of SEQ ID NO: 165 **(26.51.E1),** and an immunoglobulin light chain variable region comprising the amino acid sequence of SEQ ID NO: 167 **(26.51.E1);**
   (q) an immunoglobulin heavy chain variable region comprising the amino acid sequence of SEQ ID NO: 169 **(22.21.A7),** and an immunoglobulin light chain variable region comprising the amino acid sequence of SEQ ID NO: 171 **(22.21.A7);**
   (r) an immunoglobulin heavy chain variable region comprising the amino acid sequence of SEQ ID NO: 173 **(22.18A.E9),** and an immunoglobulin light chain variable region comprising the amino acid sequence of SEQ ID NO: 175 **(22.18A.E9);**
   (s) an immunoglobulin heavy chain variable region comprising the amino acid sequence of SEQ ID NO: 177 **(5.52.H10),** and an immunoglobulin light chain variable region comprising the amino acid sequence of SEQ ID NO: 179 **(5.52.H10);**
   (t) an immunoglobulin heavy chain variable region comprising the amino acid sequence of SEQ ID NO: 181 **(5.15.C1),** and an immunoglobulin light chain variable region comprising the amino acid sequence of SEQ ID NO: 191 **(5.15.C1);**
   (u) an immunoglobulin heavy chain variable region comprising the amino acid sequence of SEQ ID NO: 185 **(5.54.E6),** and an immunoglobulin light chain variable region comprising the amino acid sequence of SEQ ID NO: 187 **(5.54.E6);**
   (v) an immunoglobulin heavy chain variable region comprising the amino acid sequence of SEQ ID NO: 189 **(5.55.D2),** and an immunoglobulin light chain variable region comprising the amino acid sequence of SEQ ID NO: 191 **(5.55.D2);**
   (w) an immunoglobulin heavy chain variable region comprising the amino acid sequence of SEQ ID NO: 193 **(22.14.A1),** and an immunoglobulin light chain variable region comprising the amino acid sequence of SEQ ID NO: 195 **(22.14.A1);** and
   (x) an immunoglobulin heavy chain variable region comprising the amino acid sequence of SEQ ID NO: 197 **(26.53.B4),** and an immunoglobulin light chain variable region comprising the amino acid sequence of SEQ ID NO: 199 **(26.53.B4);**
   (y) an immunoglobulin heavy chain variable region comprising the amino acid sequence of SEQ ID NO: 201 **(5.63.E2),** and an immunoglobulin light chain variable region comprising the amino acid sequence of SEQ ID NO: 203 **(5.63.E2);**
   (z) an immunoglobulin heavy chain variable region comprising the amino acid sequence of SEQ ID NO: 205 **(5.64.G4),** and an immunoglobulin light chain variable region comprising the amino acid sequence of SEQ ID NO: 207 **(5.64.G4);**
   (aa) an immunoglobulin heavy chain variable region comprising the amino acid sequence of SEQ ID NO: 209 **(43.52.A11),** and an immunoglobulin light chain variable region comprising the amino acid sequence of SEQ ID NO: 211 **(43.52.A11);**
   (ab) an immunoglobulin heavy chain variable region comprising the amino acid sequence of SEQ ID NO: 213 **(43.52.E12),** and an immunoglobulin light chain variable region comprising the amino acid sequence of SEQ ID NO: 215 **(43.52.E12);** and
   (ac) an immunoglobulin heavy chain variable region comprising the amino acid sequence of SEQ ID NO: 217 **(43.62.E2),** and an immunoglobulin light chain variable region comprising the amino acid sequence of SEQ ID NO: 219 **(43.62.E2);** and
   (ad) mixtures of two or more of said antibodies in (a)-(ac).
18. The polyclonal antibody composition of paragraph 1, wherein at least one of the antibodies is selected from the group consisting of:
   (a) an antibody that competes with 5.6.H9 comprising the heavy chain and light chain CDR1, CDR2, and CDR3 sequences set forth in Table 3 for binding to one or more *S. aureus* strains;
   (b) an antibody that competes with 22.18A.E9 comprising the heavy chain and light chain CDR1, CDR2, and CDR3 sequences set forth in Table 3 for binding to one or more *S. aureus* strains;
   (c) an antibody that competes with 5.11.H10 comprising the heavy chain and light chain CDR1, CDR2, and CDR3 sequences set forth in Table 3 for binding to one or more *S. aureus* strains;
   (d) an antibody that competes with 5.27.A11 comprising the heavy chain and light chain CDR1, CDR2, and CDR3 sequences set forth in Table 3 for binding to one or more *S. aureus* strains;
   (e) an antibody that competes with 5.17.F8 comprising the heavy chain and light chain CDR1, CDR2, and CDR3 sequences set forth in Table 3 for binding to one or more *S. aureus* strains;
   (f) an antibody that competes with 5.19.F12 comprising the heavy chain and light chain CDR1, CDR2, and CDR3 sequences set forth in Table 3 for binding to one or more *S. aureus* strains;
   (g) an antibody that competes with 5.23.C9 comprising the heavy chain and light chain CDR1, CDR2, and CDR3 sequences set forth in Table 3 for binding to one or more *S. aureus* strains;
   (h) an antibody that competes with 5.23.C12 comprising the heavy chain and light chain CDR1, CDR2, and CDR3 sequences set forth in Table 3 for binding to one or more *S. aureus* strains;
   (i) an antibody that competes with 8.51.G11 comprising the heavy chain and light chain CDR1, CDR2, and CDR3 sequences set forth in Table 3 for binding to one or more *S. aureus* strains;
   (j) an antibody that competes with 9.51.H9 comprising the heavy chain and light chain CDR1, CDR2, and CDR3 sequences set forth in Table 3 for binding to one or more *S. aureus* strains;
   (k) an antibody that competes with 18.43.D8 comprising the heavy chain and light chain CDR1, CDR2, and CDR3 sequences set forth in Table 3 for binding to one or more *S. aureus* strains;
   (l) an antibody that competes with 22.22.E7 comprising the heavy chain and light chain CDR1, CDR2, and CDR3 sequences set forth in Table 3 for binding to one or more *S. aureus* strains;
   (m) an antibody that competes with 8.51.G10 comprising the heavy chain and light chain CDR1, CDR2, and CDR3 sequences set forth in Table 3 for binding to one or more *S. aureus* strains;
   (n) an antibody that competes with 5.24A.A7 comprising the heavy chain and light chain CDR1, CDR2, and CDR3 sequences set forth in Table 3 for binding to one or more *S. aureus* strains;
   (o) an antibody that competes with 5.24A.F3 comprising the heavy chain and light chain CDR1, CDR2, and CDR3 sequences set forth in Table 3 for binding to one or more *S. aureus* strains;
   (p) an antibody that competes with 5.8B.H4 comprising the heavy chain and light chain CDR1, CDR2, and CDR3 sequences set forth in Table 3 for binding to one or more *S. aureus* strains;
   (q) an antibody that competes with 26.51.E1 comprising the heavy chain and light chain CDR1, CDR2, and CDR3 sequences set forth in Table 3 for binding to one or more *S. aureus* strains;
   (r) an antibody that competes with 22.21.A7 comprising the heavy chain and light chain CDR1, CDR2, and CDR3 sequences set forth in Table 3 for binding to one or more *S. aureus* strains;
   (s) an antibody that competes with 5.52.H1 comprising the heavy chain and light chain CDR1, CDR2, and CDR3 sequences set forth in Table 3 for binding to one or more *S. aureus* strains;
   (t) an antibody that competes with 5.15.C1 comprising the heavy chain and light chain CDR1, CDR2, and CDR3 sequences set forth in Table 3 for binding to one or more *S. aureus* strains;
   (u) an antibody that competes with 5.54.E6 comprising the heavy chain and light chain CDR1, CDR2, and CDR3 sequences set forth in Table 3 for binding to one or more *S. aureus* strains under the conditions described in Example 4;
   (v) an antibody that competes with 5.55.D2 comprising the heavy chain and light chain CDR1, CDR2, and CDR3 sequences set forth in Table 3 for binding to one or more S. *aureus* strains;
   (w) an antibody that competes with 22.14.A1 comprising the heavy chain and light chain CDR1, CDR2, and CDR3 sequences set forth in Table 3 for binding to one or more *S*. *aureus* strains;
   (x) an antibody that competes with 26.53.B4 comprising the heavy chain and light chain CDR1, CDR2, and CDR3 sequences set forth in Table 3 for binding to one or more *S*. *aureus* strains;
   (y) an antibody that competes with 5.63.E2 comprising the heavy chain and light chain CDR1, CDR2, and CDR3 sequences set forth in Table 3 for binding to one or more *S*. *aureus* strains;
   (z) an antibody that competes with 5.64.G4 comprising the heavy chain and light chain CDR1, CDR2, and CDR3 sequences set forth in Table 3 for binding to one or more *S*. *aureus* strains;
   (aa) an antibody that competes with 43.52.A11 comprising the heavy chain and light chain CDR1, CDR2, and CDR3 sequences set forth in Table 3 for binding to one or more *S*. *aureus* strains;
   (ab) an antibody that competes with 43.52.E12 comprising the heavy chain and light chain CDR1, CDR2, and CDR3 sequences set forth in Table 3 for binding to one or more *S*. *aureus* strains; and
   (ac) an antibody that competes with 43.62.E2 comprising the heavy chain and light chain CDR1, CDR2, and CDR3 sequences set forth in Table 3 for binding to one or more *S*. *aureus* strains;
      wherein the one or more *S*. *aureus* strains are selected from the group consisting of ATCC Strain BAA-1717, ATCC Strain 10832, NRS071, NRS100, NRS382, NRS384, NRS123, NRS001, NRS072, NRS 102, NRS111, NRS 144 and USA300.
19. The polyclonal antibody composition of paragraph 1, wherein at least one of the antibodies competes with one of the antibodies set forth in Table 3 for binding to at least one of the *S. aureus* strains set forth in Table 4.
20. An isolated human antibody that binds *S. aureus* delta-toxin comprising a human antibody selected from the group consisting of:
   (a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9; and
   (b) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2.
21. A human recombinant polyclonal antibody composition that binds one or more *S. aureus* strains comprising a human antibody that binds delta-toxin comprising at least one antibody selected from the group consisting of:
   (a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9; and
   (b) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2.
22. An isolated human antibody that binds *S. aureus* phenol soluble modulin beta-1 comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9.
23. A human recombinant polyclonal antibody composition that binds one or more *S. aureus* strains comprising a human antibody that binds phenol soluble modulin beta-1 comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9.
24. An isolated human antibody that binds one or more *S. aureus* strains comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2.
25. An isolated human antibody that binds one or more *S. aureus* strains comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9.
26. An isolated human antibody that binds one or more *S. aureus* strains comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10.
27. An isolated human antibody that binds one or more *S. aureus* strains comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12.
28. An isolated human antibody that binds one or more *S. aureus* strains comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10.
29. An isolated human antibody that binds one or more *S. aureus* strains comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8.
30. An isolated human antibody that binds one or more *S. aureus* strains comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10.
31. An isolated human antibody that binds one or more *S. aureus* strains comprising CDR1, CDR2 and CDR3 of heavy chain amino acid sequence SEQ ID NO: 83 and CDR1, CDR2, CDR3 of light chain amino acid sequence SEQ ID NO: 85 of 8.5 1.G11.
32. An isolated nucleic acid comprising a nucleotide sequence encoding an immunoglobulin heavy chain variable region of paragraph 14 or 17.
33. An isolated nucleic acid comprising a nucleotide sequence encoding an immunoglobulin light chain variable region of paragraph 14 or 17.
34. An expression vector comprising the nucleic acid of paragraph 32.
35. An expression vector comprising the nucleic acid of paragraph 33.
36. The expression vector of paragraph 35, further comprising the nucleic acid of paragraph 32.
37. A host cell comprising the expression vector of paragraph 34.
38. A host cell comprising the expression vector of paragraph 35.
39. A host cell comprising the expression vector of paragraph 36.
40. The host cell of paragraph 38, further comprising the expression vector of paragraph 34.
41. A method of producing a polypeptide comprising an immunoglobulin heavy chain variable region or an immunoglobulin light chain variable region, the method comprising:
   (a) growing the host cell of paragraph 37 or 38 under conditions so that the host cell expresses the polypeptide comprising the immunoglobulin heavy chain variable region or the immunoglobulin light chain variable region; and
   (b) purifying the polypeptide comprising the immunoglobulin heavy chain variable region or the immunoglobulin light chain variable region.
42. A method of producing an antibody that binds to one or more *Staphylococcus aureus* strains, or an antigen binding fragment of the antibody, the method comprising:
   (a) growing the host cell of paragraph 39 or 40 under conditions so that the host cell expresses a polypeptide comprising the immunoglobulin heavy chain variable region and/or the immunoglobulin light chain variable region, thereby producing the antibody or the antigen-binding fragment of the antibody; and
   (b) purifying the antibody or the antigen-binding fragment of the antibody.
43. A method of inhibiting growth and/or colonization of *S*. *aureus* in a host cell, the method comprising administering an effective amount of the polyclonal antibody composition of any one of paragraphs 1-31 to inhibit growth and/or colonization of *S*. *aureus* in the host cell.
44. A method of reducing or killing *S*. *aureus,* the method comprising administering an effective amount of the polyclonal antibody composition of any one of paragraphs 1-31 to reduce or kill *S*. *aureus.*
45. A method for treating or preventing *Staphylococcus aureus* infection in a mammal, the method comprising administering an effective amount of the polyclonal antibody composition of any one of paragraphs 1-31 to a mammal in need thereof.
46. The method of paragraph 45, wherein the mammal is a human.
47. The method of paragraph 45, wherein the *Staphylococcus aureus* infection is a methicillin-resistant *Staphylococcus aureus* (MRSA) infection.
48. The method of paragraph 45, wherein the *Staphylococcus aureus* infection is a methicillin-sensitive *Staphylococcus aureus* (MSSA) infection.
49. The method of paragraph 45, wherein the *Staphylococcus aureus* infection is a vancomycin-resistant *Staphylococcus aureus* (VRSA) infection.
50. The method of paragraph 45, wherein the *Staphylococcus aureus* infection is a vancomycin-intermediate *Staphylococcus aureus* (VISA) infection.
51. A method for treating or preventing a polymicrobial infection including a *Staphylococcus aureus* infection in a mammal, the method comprising administering an effective amount of the polyclonal antibody composition of any one of paragraphs 1-31 to a mammal in need thereof.

## Claims

1. A recombinant polyclonal antibody composition for use in treating or preventing *Staphylococcus aureus* (*S. aureus)* infection in a mammal, wherein the polyclonal antibody composition comprises at least two human antibodies, each of which binds to a conserved cell surface antigen or toxin produced by more than one strain of *S. aureus;*
and wherein each antibody in the polyclonal antibody composition binds an epitope that is not bound by another member of the polyclonal antibody composition.

2. The use of claim 1, wherein the mammal is a human.

3. The use of claim 1, wherein the toxin is a secreted protein.

4. The use of claim 1, wherein the recombinant polyclonal antibody composition binds to at least three different strains of *S. aureus.*

5. The use of claim 1, wherein the *Staphylococcus aureus* infection is selected from the group consisting of:
an infection resulting from one or more strains of antibiotic-resistant *Staphylococcus aureus;*
a methicillin-resistant *Staphylococcus aureus* (MRSA) infection a methicillin-sensitive *Staphylococcus aureus* (MSSA) infection;
a vancomycin-resistant *Staphylococcus aureus* (VRSA) infection; or
a vancomycin-intermediate *Staphylococcus aureus* (VISA) infection.

6. The use of claim 1, wherein the antibody mixture comprises at least one or at least three of the antibodies identified in Table 2.

7. The use of claim 1, wherein the at least three different strains of *S. aureus* includes at least four or at least five of the strains identified in Table 4.

8. The use of claim 1, wherein the polyclonal antibody composition comprises a combination of any two antibodies as set forth in Table 1.

9. The use of claim 1, wherein at least one of the antibodies in the polyclonal antibody composition binds a toxin selected from the group consisting of alpha-toxin or panton-valentine leukocidin (PVL).

10. The use of claim 1, wherein at least one of the antibodies in the polyclonal antibody composition is selected from the group consisting of:
(a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9;
(b) an antibody comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9;
(c) an antibody comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10;
(d) an antibody comprising CDR1, CDR2 and CDR3 of heavy chain amino acid sequence SEQ ID NO: 20 and CDR1, CDR2, CDR3 of light chain amino acid sequence SEQ ID NO: 22 of 5.27.A11;
(e) an antibody comprising CDR1, CDR2 and CDR3 of heavy chain amino acid sequence SEQ ID NO: 47 and CDR1, CDR2, CDR3 of light chain amino acid sequence SEQ ID NO: 49 of 5.17.F8;
(f) an antibody comprising CDR1, CDR2 and CDR3 of heavy chain amino acid sequence SEQ ID NO: 56 and CDR1, CDR2, CDR3 of light chain amino acid sequence SEQ ID NO: 58 of 5.19.F12;
(g) an antibody comprising CDR1, CDR2 and CDR3 of heavy chain amino acid sequence SEQ ID NO: 65 and CDR1, CDR2, CDR3 of light chain amino acid sequence SEQ ID NO: 67 of 5.23.C9;
(h) an antibody comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12;
(i) an antibody comprising CDR1, CDR2 and CDR3 of heavy chain amino acid sequence SEQ ID NO: 83 and CDR1, CDR2, CDR3 of light chain amino acid sequence SEQ ID NO: 85 of 8.51.G11;
(j) an antibody comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9;
(k) an antibody comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8;
(l) an antibody comprising CDR1, CDR2 and CDR3 of heavy chain amino acid sequence SEQ ID NO: 110 and CDR1, CDR2, CDR3 of light chain amino acid sequence SEQ ID NO: 112 of 22.22.E7;
(m) an antibody comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10;
(n) an antibody comprising CDR1, CDR2 and CDR3 of heavy chain amino acid sequence SEQ ID NO: 153 and CDR1, CDR2, CDR3 of light chain amino acid sequence SEQ ID NO: 155 of 5.24A.A7;
(o) an antibody comprising CDR1, CDR2 and CDR3 of heavy chain amino acid sequence SEQ ID NO: 157 and CDR1, CDR2, CDR3 of light chain amino acid sequence SEQ ID NO: 159 of 5.24A.F3;
(p) an antibody comprising CDR1, CDR2 and CDR3 of heavy chain amino acid sequence SEQ ID NO: 161 and CDR1, CDR2, CDR3 of light chain amino acid sequence SEQ ID NO: 163 of 5.8B.H4;
(q) an antibody comprising CDR1, CDR2 and CDR3 of heavy chain amino acid sequence SEQ ID NO: 165 and CDR1, CDR2, CDR3 of light chain amino acid sequence SEQ ID NO: 167 of 26.51.E1;
(r) an antibody comprising CDR1, CDR2 and CDR3 of heavy chain amino acid sequence SEQ ID NO: 169 and CDR1, CDR2, CDR3 of light chain amino acid sequence SEQ ID NO: 171 of 22.21.A7;
(s) an antibody comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10;
(t) an antibody comprising CDR1, CDR2 and CDR3 of heavy chain amino acid sequence SEQ ID NO: 181 and CDR1, CDR2, CDR3 of light chain amino acid sequence SEQ ID NO: 183 of 5.15.C1;
(u) an antibody comprising CDR1, CDR2 and CDR3 of heavy chain amino acid sequence SEQ ID NO: 185 and CDR1, CDR2, CDR3 of light chain amino acid sequence SEQ ID NO: 187 of 5.54.E6;
(v) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2;
(w) an antibody comprising CDR1, CDR2 and CDR3 of heavy chain amino acid sequence SEQ ID NO: 193 and CDR1, CDR2, CDR3 of light chain amino acid sequence SEQ ID NO: 195 of 22.14.A1;
(x) an antibody comprising CDR1, CDR2 and CDR3 of heavy chain amino acid sequence SEQ ID NO: 197 and CDR1, CDR2, CDR3 of light chain amino acid sequence SEQ ID NO: 199 of 26.53.B4;
(y) an antibody comprising CDR1, CDR2 and CDR3 of heavy chain amino acid sequence SEQ ID NO: 201 and CDR1, CDR2, CDR3 of light chain amino acid sequence SEQ ID NO: 203 of 5.63.E2;
(z) an antibody comprising CDR1, CDR2 and CDR3 of heavy chain amino acid sequence SEQ ID NO: 205 and CDR1, CDR2, CDR3 of light chain amino acid sequence SEQ ID NO: 207 of 5.64.G4;
(aa) an antibody comprising CDR1, CDR2 and CDR3 of heavy chain amino acid sequence SEQ ID NO: 209 and CDR1, CDR2, CDR3 of light chain amino acid sequence SEQ ID NO: 211 of 43.52.A11;
(ab) an antibody comprising CDR1, CDR2 and CDR3 of heavy chain amino acid sequence SEQ ID NO: 213 and CDR1, CDR2, CDR3 of light chain amino acid sequence SEQ ID NO: 215 of 43.52.E12; and
(ac) an antibody comprising CDR1, CDR2 and CDR3 of heavy chain amino acid sequence SEQ ID NO: 217 and CDR1, CDR2, CDR3 of light chain amino acid sequence SEQ ID NO: 219 of 43.62.E2.

11. The use of claim 1, wherein at least one of the antibodies in the polyclonal antibody composition is selected from the group consisting of:
(a) an immunoglobulin heavy chain variable region comprising the amino acid sequence of SEQ ID NO: 11 (5.11.H10), and an immunoglobulin light chain variable region comprising the amino acid sequence of SEQ ID NO: 13 (5.11.H10);
(b) an immunoglobulin heavy chain variable region comprising the amino acid sequence of SEQ ID NO: 20 (5.27.A11), and an immunoglobulin light chain variable region comprising the amino acid sequence of SEQ ID NO: 22 (5.27.A11);
(c) an immunoglobulin heavy chain variable region comprising the amino acid sequence of SEQ ID NO: 38 (5.6.H9), and an immunoglobulin light chain variable region comprising the amino acid sequence of SEQ ID NO: 40 (5.6.H9);
(d) an immunoglobulin heavy chain variable region comprising the amino acid sequence of SEQ ID NO: 47 (5.17.F8), and an immunoglobulin light chain variable region comprising the amino acid sequence of SEQ ID NO: 49 (5.17.F8);
(e) an immunoglobulin heavy chain variable region comprising the amino acid sequence of SEQ ID NO: 56 (5.19.F12), and an immunoglobulin light chain variable region comprising the amino acid sequence of SEQ ID NO: 58 (5.19.F12);
(f) an immunoglobulin heavy chain variable region comprising the amino acid sequence of SEQ ID NO: 65 (5.23.C9), and an immunoglobulin light chain variable region comprising the amino acid sequence of SEQ ID NO: 67 (5.23.C9);
(g) an immunoglobulin heavy chain variable region comprising the amino acid sequence of SEQ ID NO: 74 (5.23.C12), and an immunoglobulin light chain variable region comprising the amino acid sequence of SEQ ID NO: 76 (5.23.C12);
(h) an immunoglobulin heavy chain variable region comprising the amino acid sequence of SEQ ID NO: 83 (8.51.G11), and an immunoglobulin light chain variable region comprising the amino acid sequence of SEQ ID NO: 85 (8.51.G11);
(i) an immunoglobulin heavy chain variable region comprising the amino acid sequence of SEQ ID NO: 92 (9.51.H9), and an immunoglobulin light chain variable region comprising the amino acid sequence of SEQ ID NO: 94 (9.51.H9);
(j) an immunoglobulin heavy chain variable region comprising the amino acid sequence of SEQ ID NO: 101 (18.43.D8), and an immunoglobulin light chain variable region comprising the amino acid sequence of SEQ ID NO: 103 (18.43.D8);
(k) an immunoglobulin heavy chain variable region comprising the amino acid sequence of SEQ ID NO: 110 (22.22.E7), and an immunoglobulin light chain variable region comprising the amino acid sequence of SEQ ID NO: 112 (22.22.E7);
(l) an immunoglobulin heavy chain variable region comprising the amino acid sequence of SEQ ID NO: 149(8.51.G10), and an immunoglobulin light chain variable region comprising the amino acid sequence of SEQ ID NO: 151 (8.51.G10);
(m) an immunoglobulin heavy chain variable region comprising the amino acid sequence of SEQ ID NO: 153 (5.24A.A7), and an immunoglobulin light chain variable region comprising the amino acid sequence of SEQ ID NO: 155 (5.24A.A7);
(n) an immunoglobulin heavy chain variable region comprising the amino acid sequence of SEQ ID NO: 157 (5.24A.F3), and an immunoglobulin light chain variable region comprising the amino acid sequence of SEQ ID NO: 159 (5.24A.F3);
(o) an immunoglobulin heavy chain variable region comprising the amino acid sequence of SEQ ID NO: 161 (5.8B.H4), and an immunoglobulin light chain variable region comprising the amino acid sequence of SEQ ID NO: 163 (5.8B.H4);
(p) an immunoglobulin heavy chain variable region comprising the amino acid sequence of SEQ ID NO: 165 (26.51.E1), and an immunoglobulin light chain variable region comprising the amino acid sequence of SEQ ID NO: 167 (26.51.E1);
(q) an immunoglobulin heavy chain variable region comprising the amino acid sequence of SEQ ID NO: 169 (22.21.A7), and an immunoglobulin light chain variable region comprising the amino acid sequence of SEQ ID NO: 171(22.21.A7);
(r) an immunoglobulin heavy chain variable region comprising the amino acid sequence of SEQ ID NO: 173 (22.18A.E9), and an immunoglobulin light chain variable region comprising the amino acid sequence of SEQ ID NO: 175 (22.18A.E9);
(s) an immunoglobulin heavy chain variable region comprising the amino acid sequence of SEQ ID NO: 177 (5.52.H10), and an immunoglobulin light chain variable region comprising the amino acid sequence of SEQ ID NO: 179 (5.52.H10);
(t) an immunoglobulin heavy chain variable region comprising the amino acid sequence of SEQ ID NO: 181 (5.15.C1), and an immunoglobulin light chain variable region comprising the amino acid sequence of SEQ ID NO: 191 (5.15.C1);
(u) an immunoglobulin heavy chain variable region comprising the amino acid sequence of SEQ ID NO: 185 (5.54.E6), and an immunoglobulin light chain variable region comprising the amino acid sequence of SEQ ID NO: 187 (5.54.E6);
(v) an immunoglobulin heavy chain variable region comprising the amino acid sequence of SEQ ID NO: 189 (5.55.D2), and an immunoglobulin light chain variable region comprising the amino acid sequence of SEQ ID NO: 191 (5.55.D2);
(w) an immunoglobulin heavy chain variable region comprising the amino acid sequence of SEQ ID NO: 193 (22.14.A1), and an immunoglobulin light chain variable region comprising the amino acid sequence of SEQ ID NO: 195 (22.14.A1); and
(x) an immunoglobulin heavy chain variable region comprising the amino acid sequence of SEQ ID NO: 197 (26.53.B4), and an immunoglobulin light chain variable region comprising the amino acid sequence of SEQ ID NO: 199 (26.53.B4);
(y) an immunoglobulin heavy chain variable region comprising the amino acid sequence of SEQ ID NO: 201 (5.63.E2), and an immunoglobulin light chain variable region comprising the amino acid sequence of SEQ ID NO: 203 (5.63.E2);
(z) an immunoglobulin heavy chain variable region comprising the amino acid sequence of SEQ ID NO: 205 (5.64.G4), and an immunoglobulin light chain variable region comprising the amino acid sequence of SEQ ID NO: 207 (5.64.G4);
(aa) an immunoglobulin heavy chain variable region comprising the amino acid sequence of SEQ ID NO: 209 (43.52.A11), and an immunoglobulin light chain variable region comprising the amino acid sequence of SEQ ID NO: 211 (43.52.A11);
(ab) an immunoglobulin heavy chain variable region comprising the amino acid sequence of SEQ ID NO: 213 (43.52.E12), and an immunoglobulin light chain variable region comprising the amino acid sequence of SEQ ID NO: 215 (43.52.E12); and
(ac) an immunoglobulin heavy chain variable region comprising the amino acid sequence of SEQ ID NO: 217 (43.62.E2), and an immunoglobulin light chain variable region comprising the amino acid sequence of SEQ ID NO: 219 (43.62.E2).

12. The use of claim 1, wherein the polyclonal antibody composition comprises an anti-*Staphylococcus aureus* (*S. aureus)* antibody or antigen-binding fragment thereof selected from the group consisting of:
(a) an antibody comprising CDR1, CDR2 and CDR3 of heavy chain amino acid sequence SEQ ID NO: 38 and CDR1, CDR2, CDR3 of light chain amino acid sequence SEQ ID NO: 40 of 5.6.H9;
(b) an antibody comprising CDR1, CDR2 and CDR3 of heavy chain amino acid sequence SEQ ID NO: 173 and CDR1, CDR2, CDR3 of light chain amino acid sequence SEQ ID NO: 175 of 22.18A.E9;
(c) an antibody comprising CDR1, CDR2 and CDR3 of heavy chain amino acid sequence SEQ ID NO: 11 and CDR1, CDR2, CDR3 of light chain amino acid sequence SEQ ID NO: 13 of 5.11.H10;
(d) an antibody comprising CDR1, CDR2 and CDR3 of heavy chain amino acid sequence SEQ ID NO: 20 and CDR1, CDR2, CDR3 of light chain amino acid sequence SEQ ID NO: 22 of 5.27.A11;
(e) an antibody comprising CDR1, CDR2 and CDR3 of heavy chain amino acid sequence SEQ ID NO: 47 and CDR1, CDR2, CDR3 of light chain amino acid sequence SEQ ID NO: 49 of 5.17.F8;
(f) an antibody comprising CDR1, CDR2 and CDR3 of heavy chain amino acid sequence SEQ ID NO: 56 and CDR1, CDR2, CDR3 of light chain amino acid sequence SEQ ID NO: 58 of 5.19.F12;
(g) an antibody comprising CDR1, CDR2 and CDR3 of heavy chain amino acid sequence SEQ ID NO: 65 and CDR1, CDR2, CDR3 of light chain amino acid sequence SEQ ID NO: 67 of 5.23.C9;
(h) an antibody comprising CDR1, CDR2 and CDR3 of heavy chain amino acid sequence SEQ ID NO: 74 and CDR1, CDR2, CDR3 of light chain amino acid sequence SEQ ID NO: 76 of 5.23.C12;
(i) an antibody comprising CDR1, CDR2 and CDR3 of heavy chain amino acid sequence SEQ ID NO: 83 and CDR1, CDR2, CDR3 of light chain amino acid sequence SEQ ID NO: 85 of 8.51.G11;
(j) an antibody comprising CDR1, CDR2 and CDR3 of heavy chain amino acid sequence SEQ ID NO: 92 and CDR1, CDR2, CDR3 of light chain amino acid sequence SEQ ID NO: 94 of 9.51.H9;
(k) an antibody comprising CDR1, CDR2 and CDR3 of heavy chain amino acid sequence SEQ ID NO: 101 and CDR1, CDR2, CDR3 of light chain amino acid sequence SEQ ID NO: 103 of 18.43.D8;
(l) an antibody comprising CDR1, CDR2 and CDR3 of heavy chain amino acid sequence SEQ ID NO: 110 and CDR1, CDR2, CDR3 of light chain amino acid sequence SEQ ID NO: 112 of 22.22.E7;
(m) an antibody comprising CDR1, CDR2 and CDR3 of heavy chain amino acid sequence SEQ ID NO: 149 and CDR1, CDR2, CDR3 of light chain amino acid sequence SEQ ID NO: 151 of 8.51.G10;
(n) an antibody comprising CDR1, CDR2 and CDR3 of heavy chain amino acid sequence SEQ ID NO: 153 and CDR1, CDR2, CDR3 of light chain amino acid sequence SEQ ID NO: 155 of 5.24A.A7;
(o) an antibody comprising CDR1, CDR2 and CDR3 of heavy chain amino acid sequence SEQ ID NO: 157 and CDR1, CDR2, CDR3 of light chain amino acid sequence SEQ ID NO: 159 of 5.24A.F3;
(p) an antibody comprising CDR1, CDR2 and CDR3 of heavy chain amino acid sequence SEQ ID NO: 161 and CDR1, CDR2, CDR3 of light chain amino acid sequence SEQ ID NO: 163 of 5.8B.H4;
(q) an antibody comprising CDR1, CDR2 and CDR3 of heavy chain amino acid sequence SEQ ID NO: 165 and CDR1, CDR2, CDR3 of light chain amino acid sequence SEQ ID NO: 167 of 26.51.E1;
(r) an antibody comprising CDR1, CDR2 and CDR3 of heavy chain amino acid sequence SEQ ID NO: 169 and CDR1, CDR2, CDR3 of light chain amino acid sequence SEQ ID NO: 171 of 22.21.A7;
(s) an antibody comprising CDR1, CDR2 and CDR3 of heavy chain amino acid sequence SEQ ID NO: 177 and CDR1, CDR2, CDR3 of light chain amino acid sequence SEQ ID NO: 179 of 5.52.H10;
(t) an antibody comprising CDR1, CDR2 and CDR3 of heavy chain amino acid sequence SEQ ID NO: 181 and CDR1, CDR2, CDR3 of light chain amino acid sequence SEQ ID NO: 183 of 5.15.C1;
(u) an antibody comprising CDR1, CDR2 and CDR3 of heavy chain amino acid sequence SEQ ID NO: 185 and CDR1, CDR2, CDR3 of light chain amino acid sequence SEQ ID NO: 187 of 5.54.E6;
(v) an antibody comprising CDR1, CDR2 and CDR3 of heavy chain amino acid sequence SEQ ID NO: 189 and CDR1, CDR2, CDR3 of light chain amino acid sequence SEQ ID NO: 191 of 5.55.D2;
(w) an antibody comprising CDR1, CDR2 and CDR3 of heavy chain amino acid sequence SEQ ID NO: 193 and CDR1, CDR2, CDR3 of light chain amino acid sequence SEQ ID NO: 195 of 22.14.A1;
(x) an antibody comprising CDR1, CDR2 and CDR3 of heavy chain amino acid sequence SEQ ID NO: 197 and CDR1, CDR2, CDR3 of light chain amino acid sequence SEQ ID NO: 199 of 26.53.B4;
(y) an antibody comprising CDR1, CDR2 and CDR3 of heavy chain amino acid sequence SEQ ID NO: 201 and CDR1, CDR2, CDR3 of light chain amino acid sequence SEQ ID NO: 203 of 5.63.E2;
(z) an antibody comprising CDR1, CDR2 and CDR3 of heavy chain amino acid sequence SEQ ID NO: 205 and CDR1, CDR2, CDR3 of light chain amino acid sequence SEQ ID NO: 207 of 5.64.G4;
(aa) an antibody comprising CDR1, CDR2 and CDR3 of heavy chain amino acid sequence SEQ ID NO: 209 and CDR1, CDR2, CDR3 of light chain amino acid sequence SEQ ID NO: 211 of 43.52.A11;
(ab) an antibody comprising CDR1, CDR2 and CDR3 of heavy chain amino acid sequence SEQ ID NO: 213 and CDR1, CDR2, CDR3 of light chain amino acid sequence SEQ ID NO: 215 of 43.52.E12; and
(ac) an antibody comprising CDR1, CDR2 and CDR3 of heavy chain amino acid sequence SEQ ID NO: 217 and CDR1, CDR2, CDR3 of light chain amino acid sequence SEQ ID NO: 219 of 43.62.E2.

13. The use of claim 1, wherein the polyclonal antibody composition comprises at least three, at least 4, at least 5, at least 7, or at least 10 antibodies.
